# EUROPEAN PATENT APPLICATION

(11) **EP 4 667 461 A2**
(43) Date of publication of application: **24.12.2025**
(21) Application number: 25183628.4
(22) Date of filing: 18.06.2025
(51) Int. Cl.: C07D 295/108, C07D 305/06, C07D 307/14, C07D 307/85, C07D 405/12, C07D 407/12, C07D 413/12, C07D 413/14, A61P 25/28, A61K 31/341

(54) **COMPOSITIONS AND METHODS FOR THE TREATMENT OF PARKINSON S AND GAUCHER DISEASE**

(30) Priority: 18.06.2024 US 202463661456 P
(71) Applicant: Children's Hospital Medical Center, Cincinnati, OH 45229-3039 (US)
(72) Inventor: Sun, Ying, Mason, 45040 (US); Wang, Zhaolin, Wellesley, 02481 (US); Liou, Benjamin, Cincinnati, 45249 (US)
(74) Representative: Forresters IP LLP

(57) **Abstract**

Compounds and pharmaceutical compositions are useful for the treatment of Gaucher disease and Parkinson's disease. Methods of treating Gaucher disease or Parkinson's disease include administration of one or more compounds or pharmaceutically acceptable salts or prodrugs thereof to a subject diagnosed with, or at risk of developing Gaucher disease or Parkinson's disease.

## Description

### CROSS-REFERENCE TO RELATED APPLICATIONS

This application claims priority to and the benefit of U.S. Provisional Patent Application Serial No. 63/661,456, filed June 18, 2024, the entire content of which is incorporated herein by reference.

### BACKGROUND

Neuronopathic Gaucher disease patients have shortened life expectancy. Accumulation of lipids substrates and inflammation causes neurodegeneration in neuronopathic Gaucher disease. Mutations in *GBA1,* the gene responsible for Gaucher disease, are the most commonly known genetic risk factors for Parkinson's disease.

Conventional enzyme replacement therapy with pharmacologic β-glucocerebrosidase (GCase) has been a modestly successful treatment in visceral type Gaucher disease. Because GCase is a very unstable enzyme, the enzyme must be administrated via intravenous infusion very frequently to maintain therapeutic efficacy. However, to date, there are no Food and Drug Agency (FDA) or European Medicines Agency (EMA)-approved therapeutics for use to treat neurological manifestations in neuronopathic Gaucher disease and Parkinson's disease, representing an unmet medical need.

### SUMMARY

According to embodiments of the present disclosure, compounds useful for the treatment of Gaucher disease and Parkinson's disease are represented by Formula 1, below.

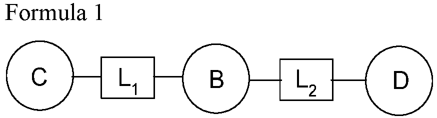

In Formula 1, ring C is selected from moieties represented by Formulae 2A and 2B, below.

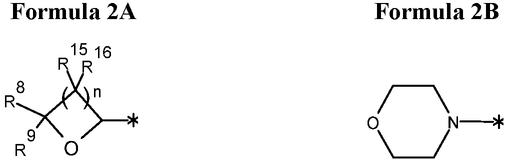

In Formulae 2A and 2B: n is 1 or 2; each of R⁸, R⁹, R¹⁵ and R¹⁶ is independently selected from hydrogen, a halide, a C1-C4 alkyl group, or a C1-C4 haloalkyl group; and when n is 2, the multiple R¹⁵s or R¹⁶s may be the same or different from each other.

Referring back to Formula 1, L₁ is selected from moieties represented by Formulae 3A, 3B, and 3C, below.

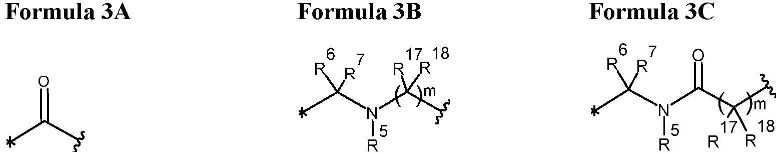

In Formulae 3A, 3B, and 3C: m is 0 or 1; and each of R⁵, R⁶, R⁷, R¹⁷ and R¹⁸ is independently selected from hydrogen, a halide, a C1-C4 alkyl group, or a C1-C4 haloalkyl group.

Referring back to Formula 1 again, ring B is selected from moieties represented by Formulae 4A, 4B, 4C, 4D, and 4E, below.

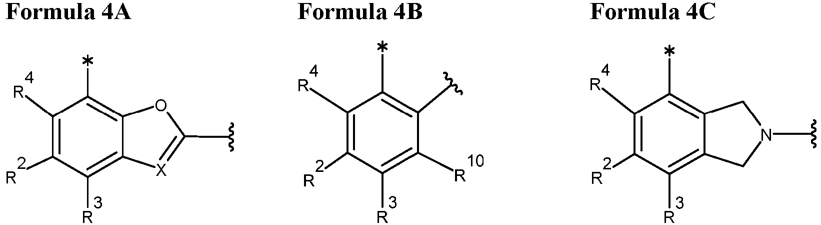

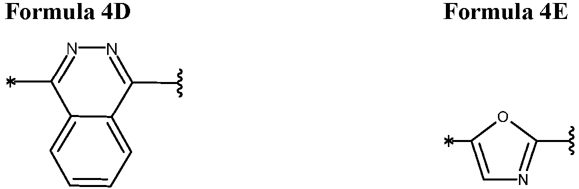

In Formulae 4A, 4B, 4C, 4D, and 4E: X is C, N or C-Y, where Y is hydrogen, a halide, or a C1-C4 alkoxy group; and each of R², R³, R⁴, and R¹⁰ is independently selected from hydrogen, a halide, a C1-C4 alkyl group, or a C1-C4 haloalkyl group.

Referring back to Formula 1, L₂ is selected from moieties represented by Formulae 5A, 5B, and 5C, below.

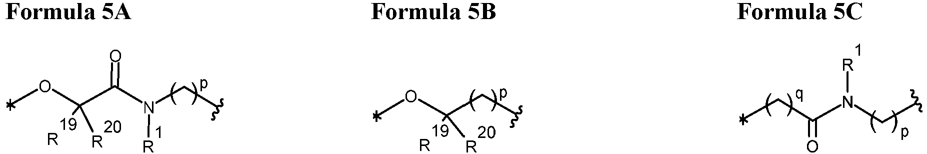

In Formulae 5A, 5B, and 5C: both p and q are independently 0 or 1; and each of R¹, R¹⁹, and R²⁰ is independently selected from hydrogen, a halide, a C1-C4 alkyl group, or a C1-C4 haloalkyl group.

Referring again to Formula 1, ring D is a substituted or unsubstituted aryl, heteroaryl, cyclic, or heterocylic ring or fused ring system.

In some embodiments, each of R⁸, R⁹, R¹⁵ and R¹⁶ in the above formulae may be independently selected from hydrogen, a halide, a methyl group, a perfluorinated methyl group, or a partially fluorinated methyl group. And in some embodiments, each of R⁵, R⁶, R⁷, R¹⁷ and R¹⁸ may be independently selected from hydrogen, a halide, a methyl group, a perfluorinated methyl group, or a partially fluorinated methyl group. In some embodiments, each of R², R³, R⁴, and R¹⁰ may be independently selected from hydrogen, a halide, a methyl group, a perfluorinated methyl group, or a partially fluorinated methyl group. And in some embodiments, each of R¹, R¹⁹, and R²⁰ may be independently selected from hydrogen, a halide, a methyl group, a perfluorinated methyl group, or a partially fluorinated methyl group.

According to some embodiments, Y may be hydrogen, a halide, or methoxy.

In some embodiments, ring D may be selected from the following moieties:

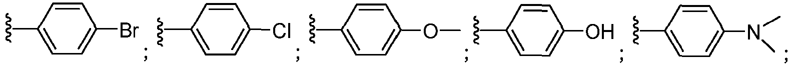

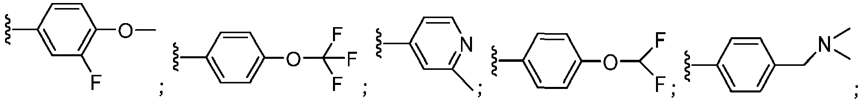

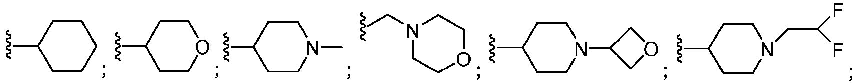

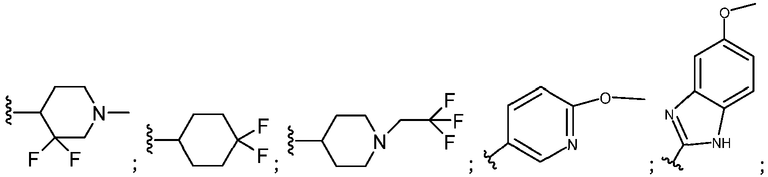

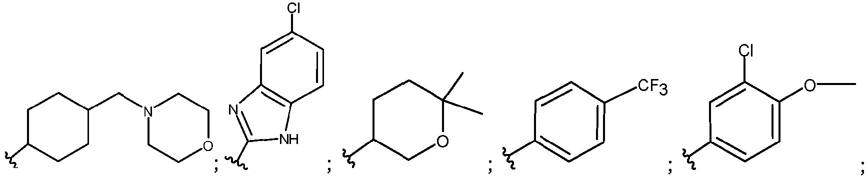

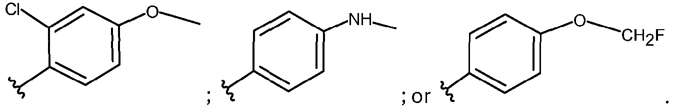

According to some embodiments, the compound may be selected from the following Compounds 1-50:

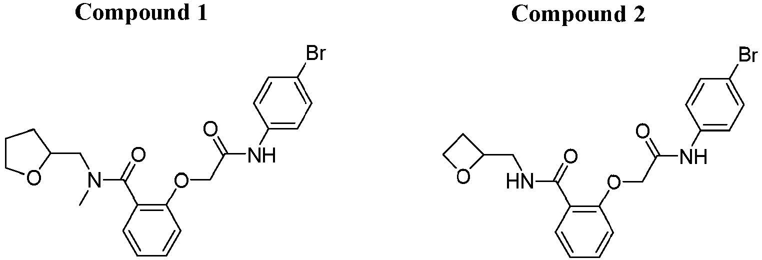

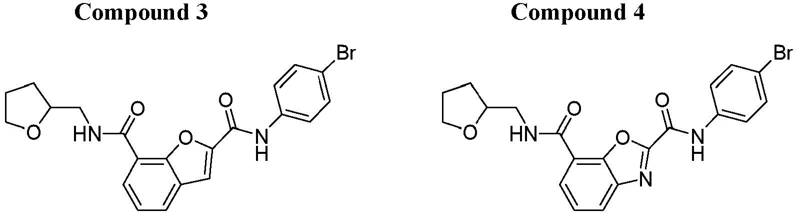

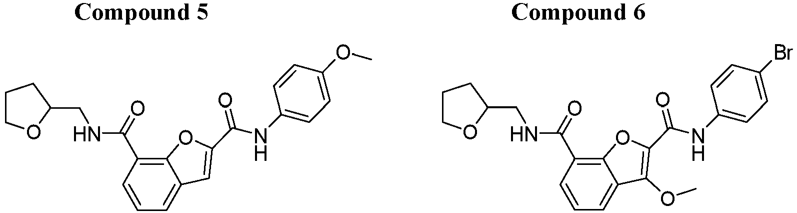

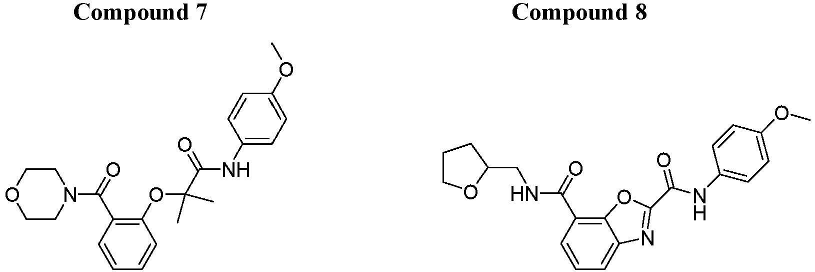

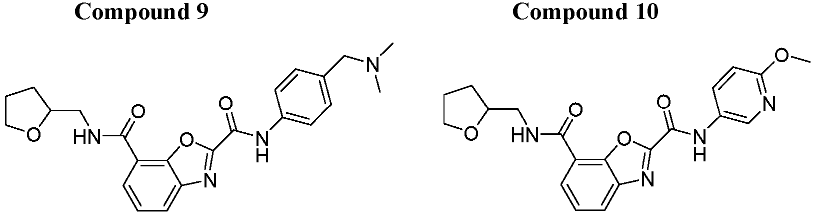

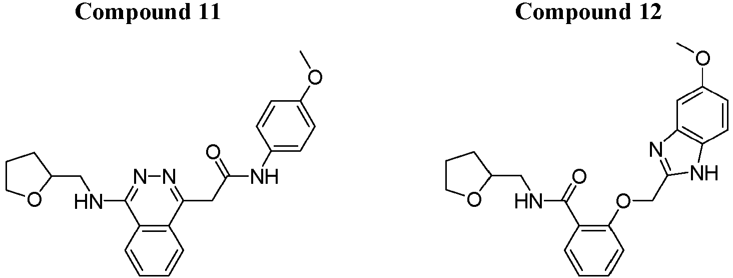

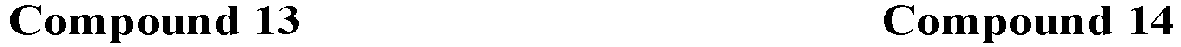

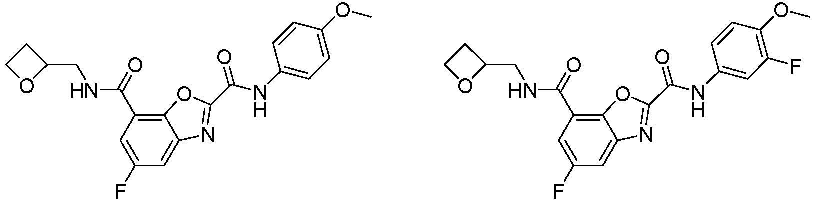

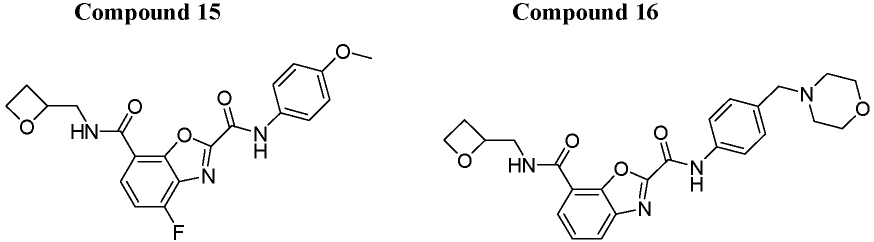

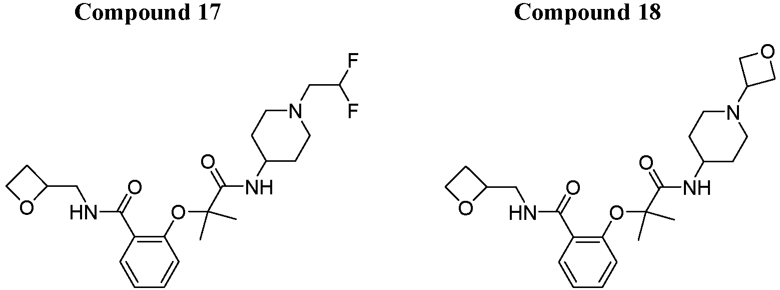

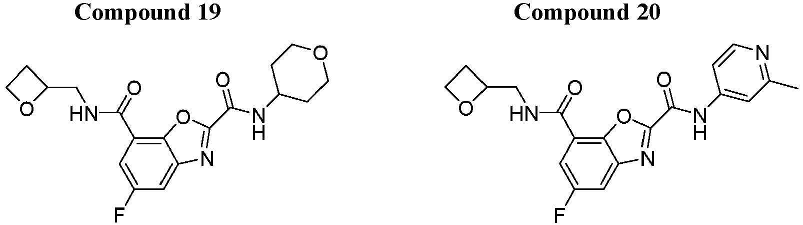

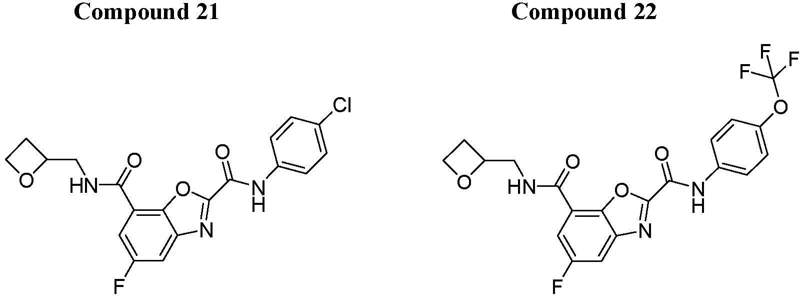

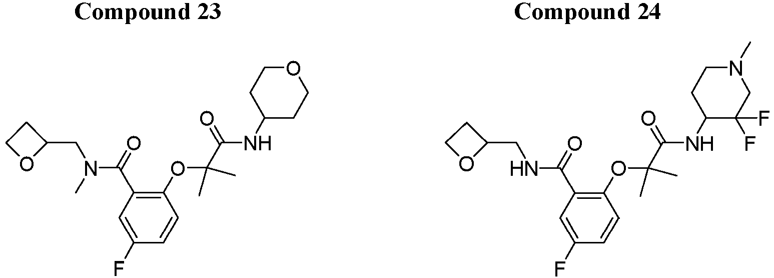

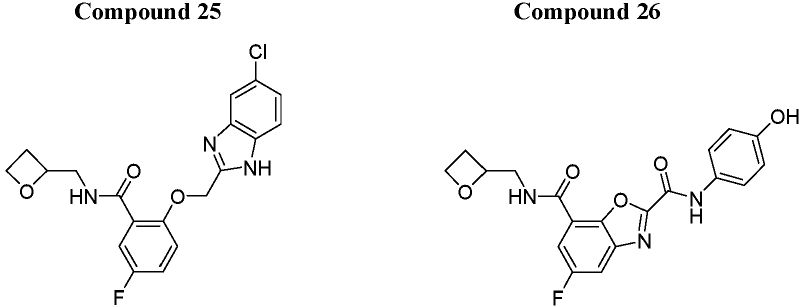

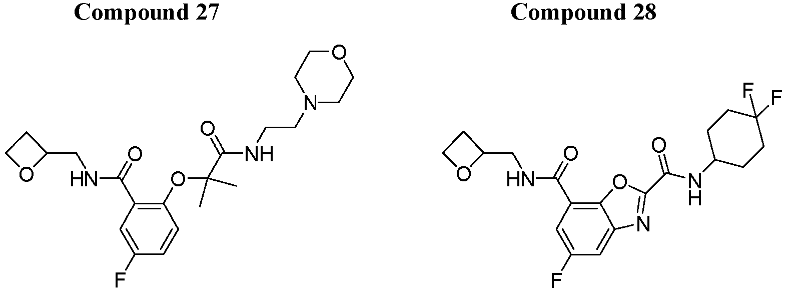

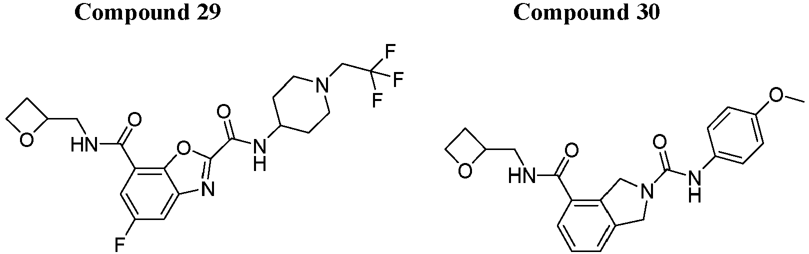

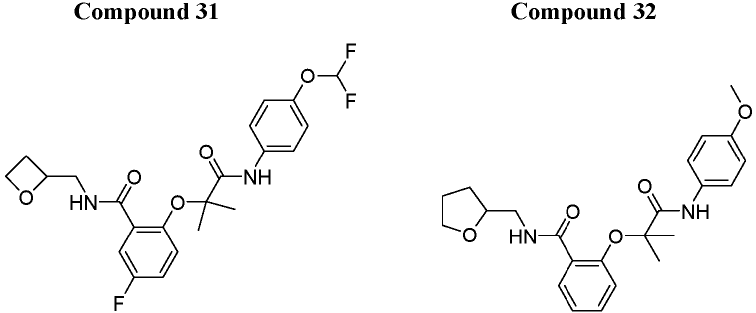

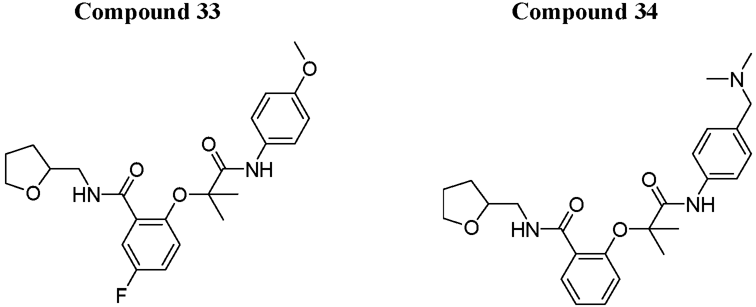

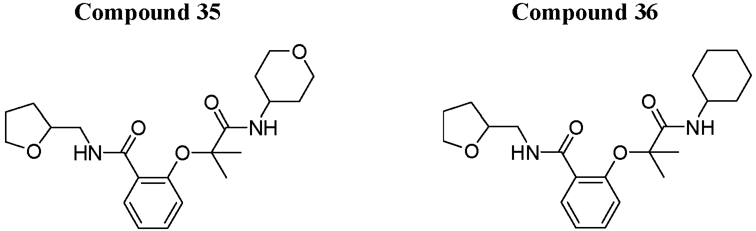

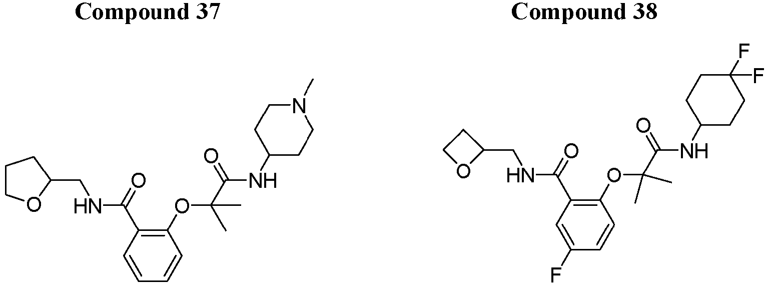

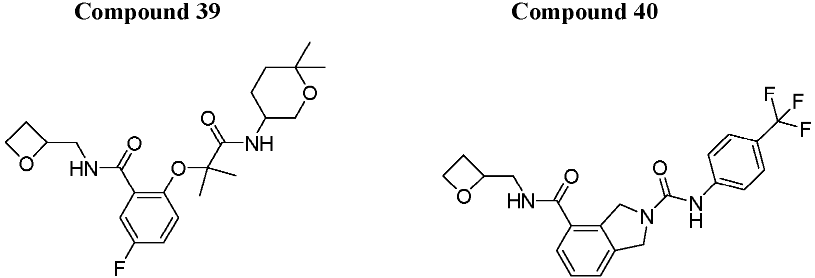

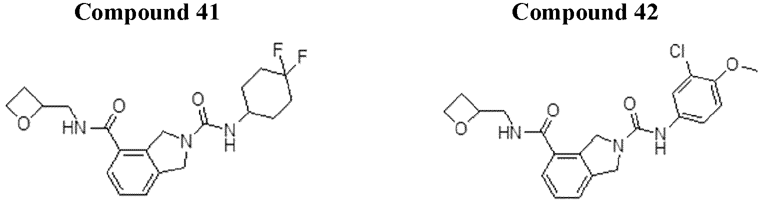

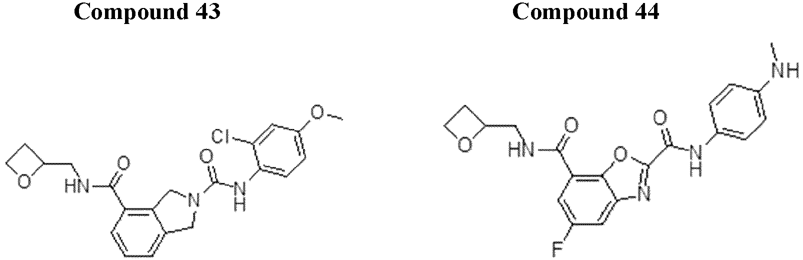

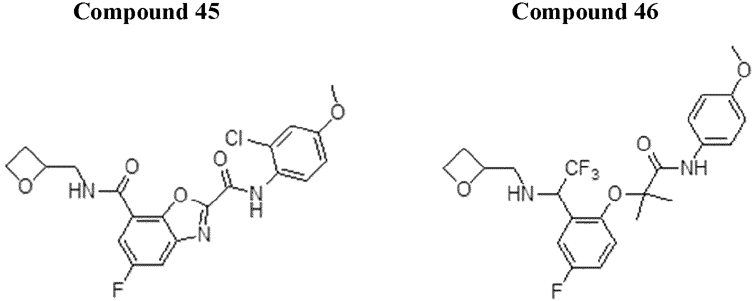

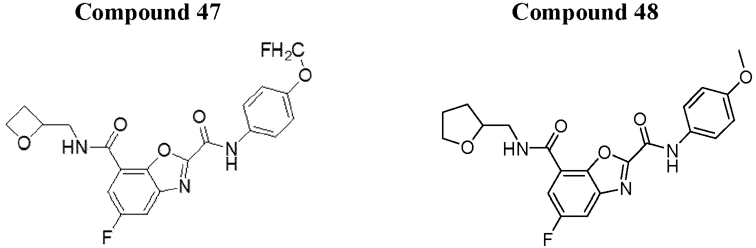

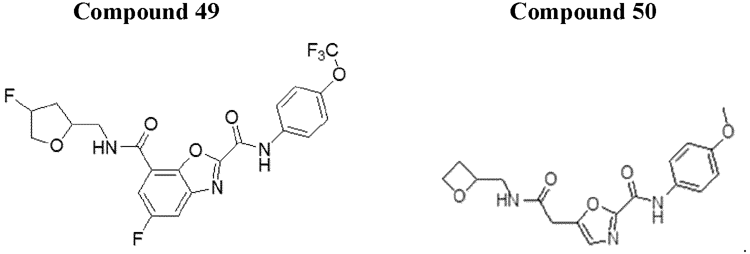

In some embodiments, the compound represented by Formula 1 may be a compound represented by one of Formula 6-9, below.

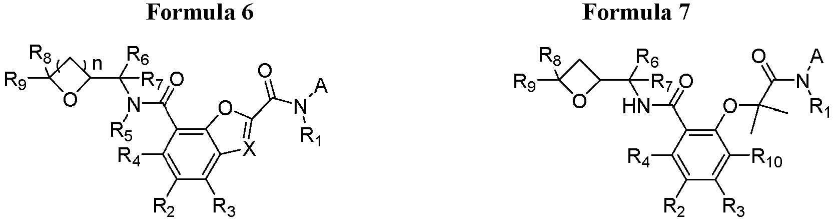

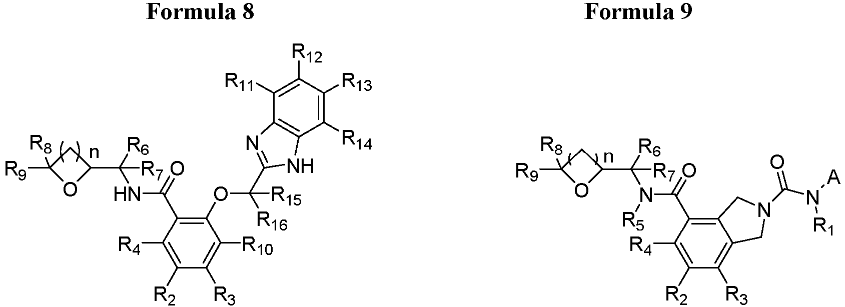

In Formulae 6 through 9: n, X, and R¹ through R¹⁶ are as defined above with respect to Formula 1; and moiety A in Formulae 6, 7 and 9 is a substituted or unsubstituted aryl, heteroaryl, cyclic, or heterocylic ring or fused ring system that is either bonded directly to Formulae 6, 7 or 9, or is bonded to Formula 6, 7, or 9 via a linking -CH₂- group.

According to some embodiments, moiety A in Formulae 6, 7, and may be selected from the following moieties: 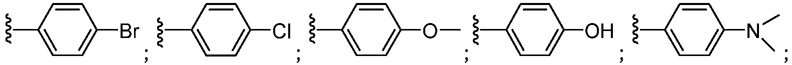 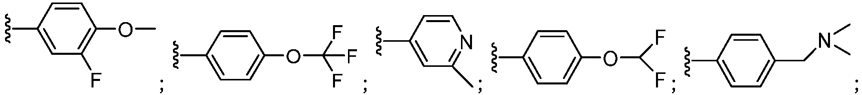 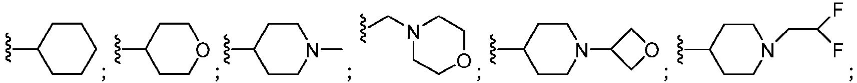 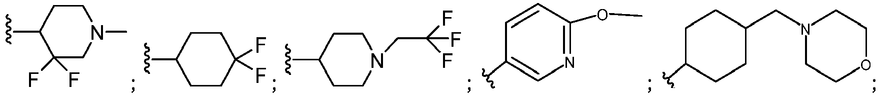 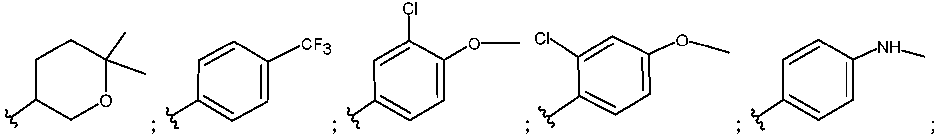 or

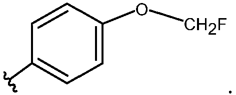

In some embodiments, the compound represented by Formula 6 may be selected from Compounds 3-6, 8-10, 13-16, 19-22, 26, 28, 29, 44, 45, and 47-49, depicted below.

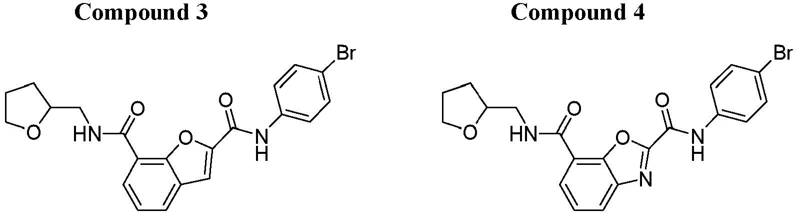

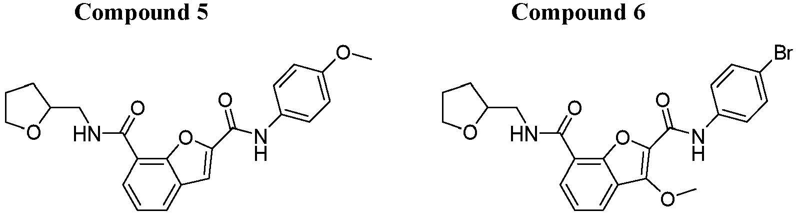

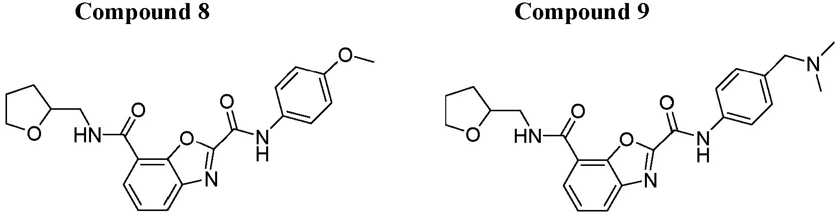

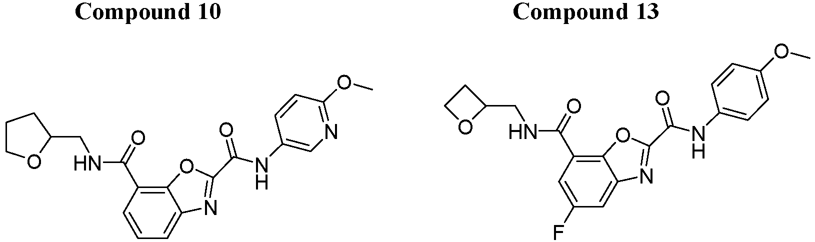

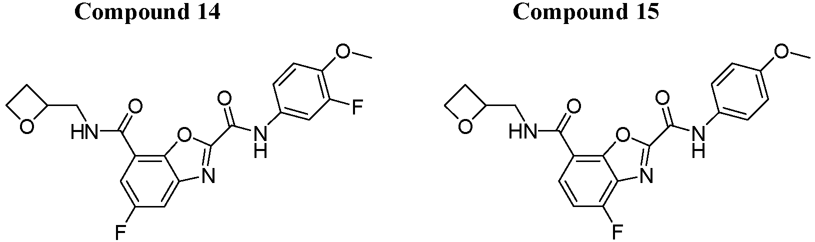

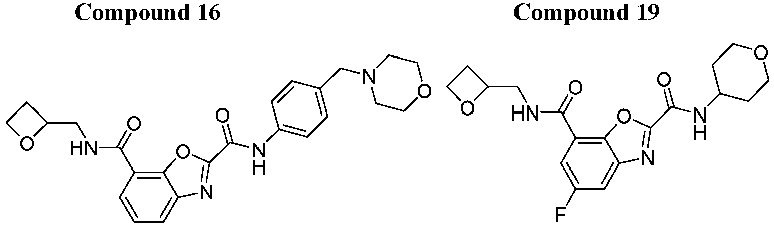

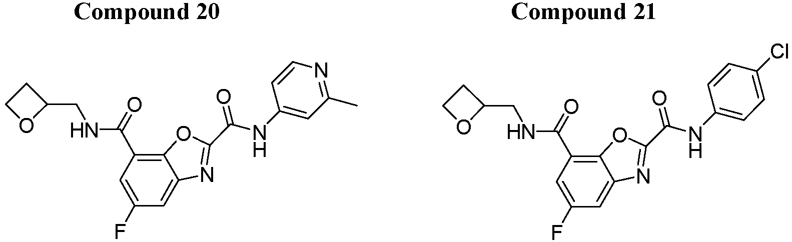

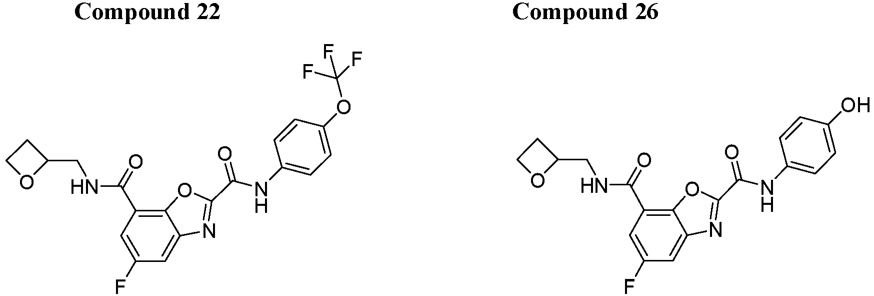

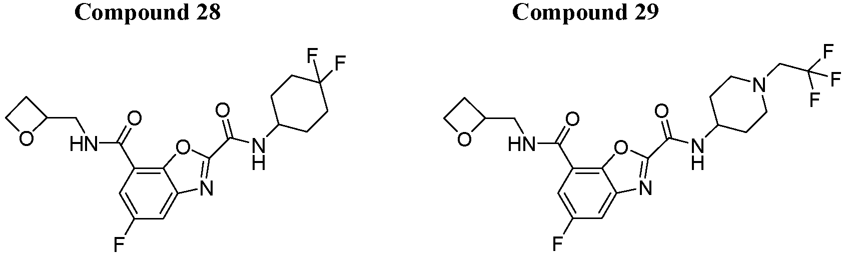

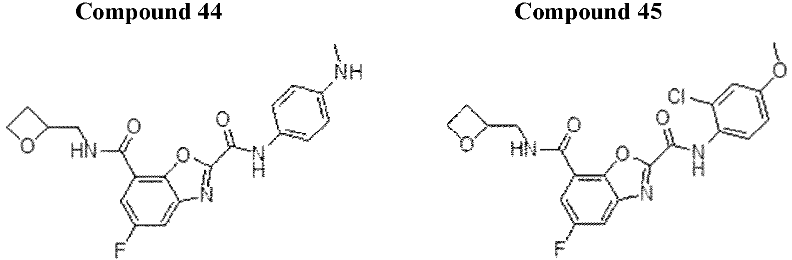

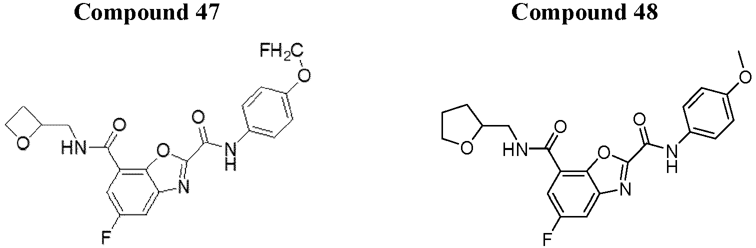

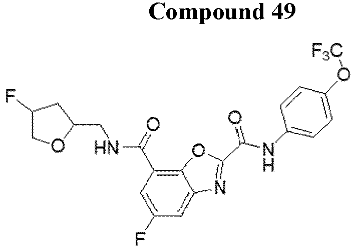

According to some embodiments, the compound represented by Formula 7 may be selected from Compounds 1, 2, 7, 12, 17, 18, 23, 24, 27, 31-39, and 46, depicted below.

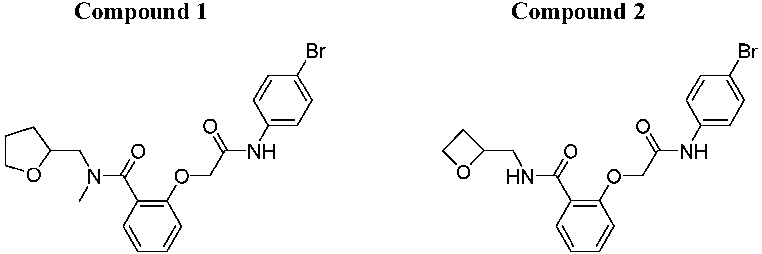

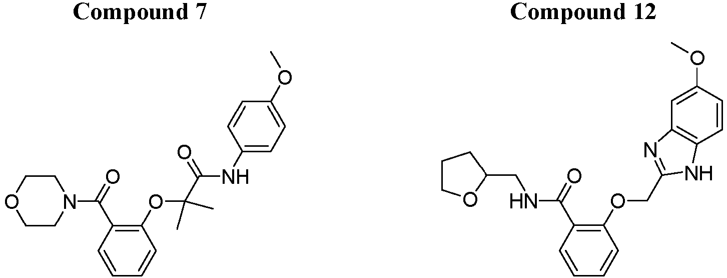

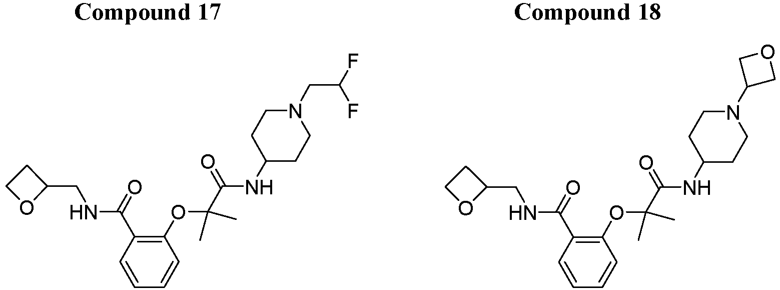

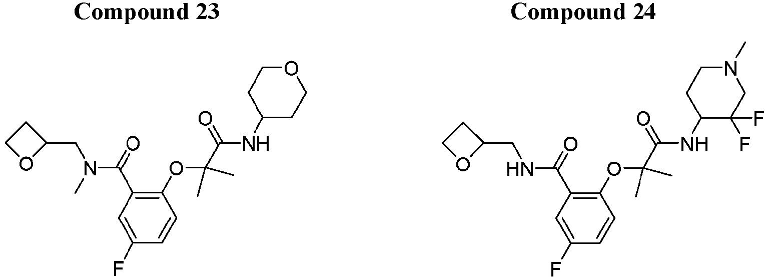

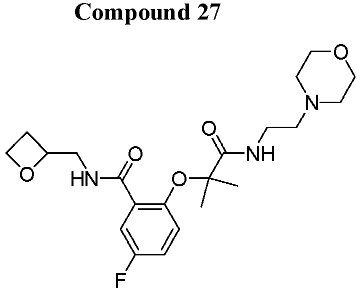

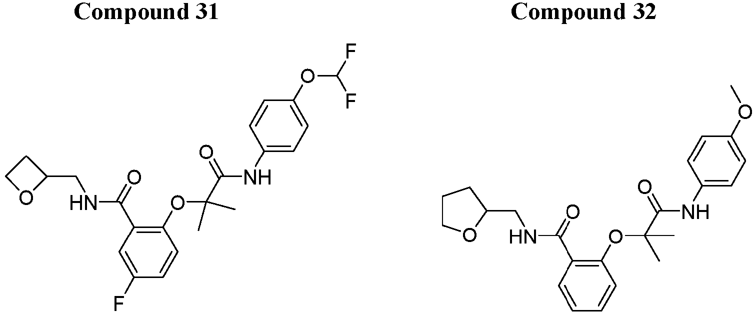

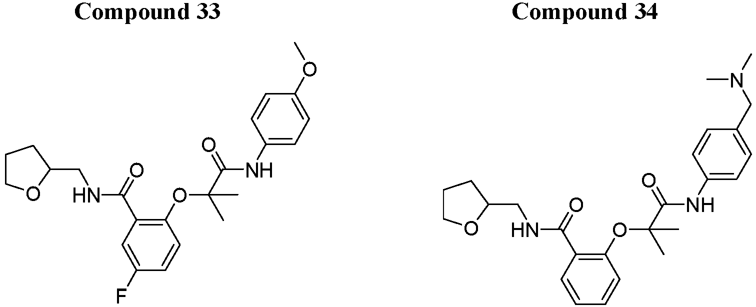

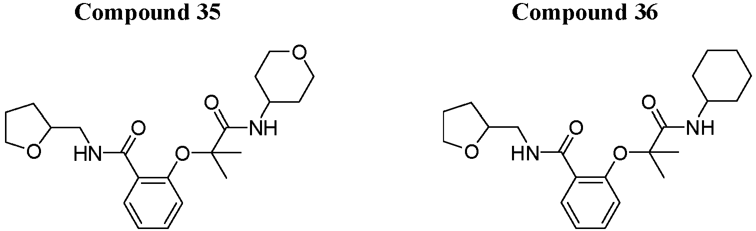

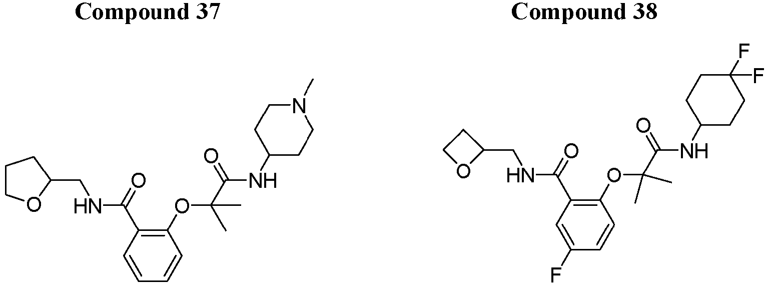

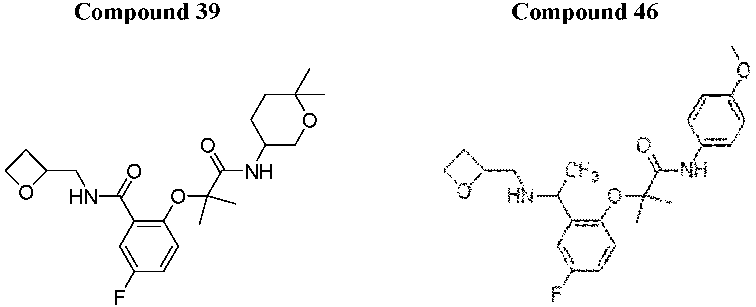

In some embodiments, the compound represented by Formula 8 may be Compound 25, depicted below.

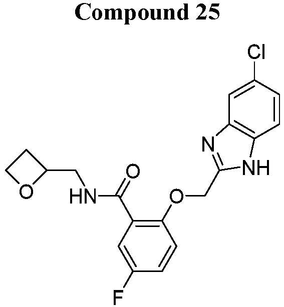

In some embodiments, the compound represented by Formula 9 may be selected from Compounds 30, and 40-43, depicted below.

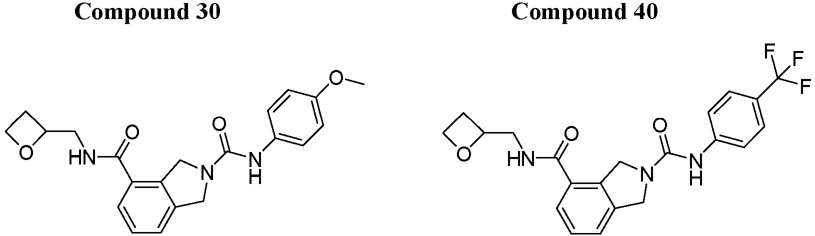

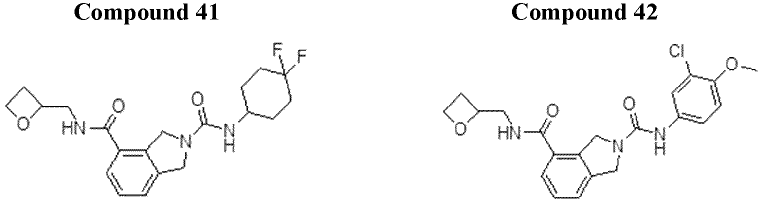

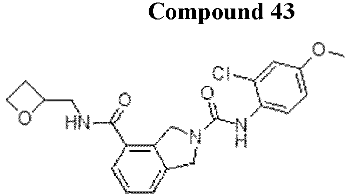

According to some embodiments, the compound(s) disclosed herein may be used for the manufacture of a medicament for the treatment of Gaucher disease or Parkinson's disease.

In some embodiments, a pharmaceutical composition includes one or more of the compounds disclosed herein or pharmaceutically acceptable salts or prodrugs thereof, and a pharmaceutically acceptable carrier and/or excipient. According to some embodiments, the pharmaceutical composition may further include one or more additional therapeutic agents for the treatment of Gaucher disease or Parkinson's disease. And in some embodiments, the pharmaceutical composition may be formulated for oral, transdermal, inhalation, infusion, or parenteral administration.

According to some embodiments, a method of treating Gaucher disease or Parkinson's disease includes administering to a subject a therapeutically effective amount of one or more of the compounds disclosed herein or pharmaceutically acceptable salts or prodrugs thereof. In some embodiments, the one or more compounds or pharmaceutically acceptable salts or prodrugs thereof may be administered in combination with one or more additional therapeutic agents for the treatment of Gaucher disease or Parkinson's disease. In some embodiments, the one or more compounds or pharmaceutically acceptable salts or prodrugs thereof may be administered orally in the form of a tablet, a capsule, or a liquid formulation. And in some embodiments, the one or more compounds or pharmaceutically acceptable salts or prodrugs thereof may be administered parenterally via intravenous, intramuscular, or subcutaneous injection. In some embodiments, the one or more compounds or pharmaceutically acceptable salts or prodrugs thereof may be administered topically as a transdermal patch or cream. And in some embodiments, the one or more compounds or pharmaceutically acceptable salts or prodrugs thereof may be administered via inhalation as an aerosolized formulation.

In some embodiments, a method of treating Gaucher disease or Parkinson's disease includes administering to a subject a pharmaceutical composition disclosed herein. According to some embodiments, the pharmaceutical composition may be administered orally in the form of a tablet, a capsule, or a liquid formulation. In some embodiments, the pharmaceutical composition may be administered parenterally via intravenous, intramuscular, or subcutaneous injection. And in some embodiments, the pharmaceutical composition may be administered topically as a transdermal patch or cream. In some embodiments, the pharmaceutical composition may be administered via inhalation as an aerosolized formulation. And in some embodiments, the pharmaceutical composition may be administered in combination with one or more additional therapeutic agents for the treatment of Gaucher disease or Parkinson's disease.

### DETAILED DESCRIPTION

### DEFINITIONS

Unless otherwise noted, terms are to be understood according to conventional usage by those of ordinary skill in the relevant art. In case of conflict, the present document, including definitions, will control. Exemplary compounds, compositions, methods, and materials are described herein, although methods and materials similar or equivalent to those described herein may be used in practice or testing of the present disclosure. All publications, patent applications, patents and other references mentioned herein are incorporated by reference in their entirety. The compositions, compounds, materials, methods, and examples disclosed herein are illustrative only and not intended to be limiting. The compounds, compositions, and methods may comprise, consist of, or consist essentially of the elements of the compositions and/or methods as described herein, as well as any additional or optional element described herein or otherwise useful in the treatment of patients or subjects having or at risk of developing Gaucher disease or Parkinson's disease.

As used herein and in the appended claims, the singular forms "a," "an," and "the" include plural referents unless the context clearly dictates otherwise. Thus, for example, reference to "a compound" or "a method" includes a plurality of such compounds or methods, and reference to "a dose" includes reference to one or more doses and equivalents thereof known to those skilled in the art, and so forth.

The term "about" or "approximately" means within an acceptable error range for the particular value as determined by one of ordinary skill in the art, which will depend in part on how the value is measured or determined, e.g., the limitations of the measurement system. For example, "about" may mean within 1 or more than 1 standard deviation, per the practice in the art. Alternatively, "about" or "approximately" may mean a range of up to 20%, or up to 10%, or up to 5%, or up to 1% of a given value. Alternatively, particularly with respect to biological systems or processes, the term may mean within an order of magnitude, preferably within 5-fold, and more preferably within 2-fold, of a value. Where particular values are described in the application and claims, unless otherwise stated, it should be assumed that the term "about" or "approximately" means within an acceptable error range for the particular value.

As used herein, the term "effective amount" means the amount of one or more active components that is sufficient to show a desired effect. This includes both therapeutic and prophylactic effects. When applied to an individual active ingredient, administered alone, the term refers to that ingredient alone. When applied to a combination, the term refers to combined amounts of the active ingredients that result in the therapeutic effect, whether administered in combination, serially or simultaneously.

The terms "individual," "host," "subject," and "patient" are used interchangeably to refer to an animal that is the object of treatment, observation and/or experiment. Generally, the term refers to a human patient, but the methods and compositions may be equally applicable to non-human subjects such as other mammals. In some aspects, the terms refer to humans. In further aspects, the terms may refer to adults or children, depending on the context.

Additional definitions are provided throughout the present disclosure, and it is understood that those definitions, as well as those provided here, apply with equal force to all aspects and parts of this disclosure.

Pharmacological chaperones are small molecules that can selectively bind to and stabilize specific proteins, typically enzymes, to promote proper folding, trafficking, and function within cells. Pharmacological chaperones offer a promising therapeutic strategy for a range of genetic diseases, including lysosomal storage disorders, cystic fibrosis, and certain forms of metabolic disorders. By restoring the activity of mutant proteins, pharmacological chaperones have the potential to alleviate disease symptoms and improve patient outcomes. Furthermore, pharmacological chaperones may also exhibit additional pharmacological properties beyond protein stabilization, such as modulating protein-protein interactions, enhancing protein solubility, or influencing intracellular trafficking pathways. These effects can further contribute to their therapeutic efficacy in treating various diseases characterized by protein misfolding or dysfunction.

These molecules are particularly relevant in the context of certain genetic disorders where mutations in genes encoding for specific proteins lead to misfolding or instability of the corresponding proteins, resulting in loss of function or reduced activity. In genetic diseases caused by protein misfolding, pharmacological chaperones can act as molecular scaffolds, assisting in the correct folding of the mutant proteins, thereby restoring their stability and function. By stabilizing the folded conformation, pharmacological chaperones can prevent premature degradation of the mutant proteins by cellular quality control mechanisms, such as the ubiquitin-proteasome system or autophagy. The mechanism of action of pharmacological chaperones may involve reversible binding to specific sites on the target protein, often within its active site or structural domains critical for stability. This binding interaction stabilizes the protein in its native conformation, facilitating proper folding and assembly into functional complexes, if applicable. According to embodiments of the present disclosure, β-glucocerebrosidase (GCase) is a druggable target for chaperone therapy, and GCase chaperones can be used to stabilize GCase, allowing prolonged drug efficacy. The improved compositions and methods for stabilizing GCase according to embodiments of the present disclosure provide improvements to current enzyme replacement therapy for visceral forms of Gaucher disease, for example, and can reduce the burden on patients.

In some embodiments, small molecule pharmaceutical chaperones can be made into an oral drug which can penetrate to the brain and restore mutant GCase. Improved GCase activity by chaperones in brain cells can reduce substrate accumulation, inflammation, mitigate alphasynuclein pathology and can improve sensorimotor and cognitive function. The non-inhibitory pharmaceutical chaperones according to embodiments of the present disclosure meet a pressing need for neuronopathic Gaucher disease, and the benefits of these chaperone therapies are expected to apply to Parkinson's disease.

In Gaucher disease and Parkinson's disease, dysfunction of GCase increases the risk factors for these diseases. According to embodiments of the present disclosure, pharmacological chaperones that address misfolded proteins may be used in methods and compositions for the treatment of Gaucher disease and/or Parkinson's disease.

According to embodiments of the present disclosure, a pharmacological chaperone is useful for the treatment of Gaucher disease and/or Parkinson's disease. In some embodiments, the compound includes an allosteric site activator compound. As used herein, the term "allosteric site activator" refers to a molecule that binds to an enzyme's allosteric site, thus increasing the enzyme's activity. Allosteric sites are separate locations on an enzyme that allow regulatory molecules to bind, typically away from the enzyme's active site. Upon binding to an enzyme's allosteric site, the binding causes a conformational change that increases the enzyme's active site affinity for its substrate. In some embodiments, the allosteric site activator compound binds to β-glucocerebrosidase (GCase).

In some embodiments, a compound useful for the treatment of Gaucher disease or Parkinson's disease includes one or more compounds represented by Formula 1. 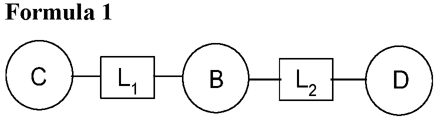 In Formula 1, C, B, and D are substituted or unsubstituted aryl, heteroaryl, cyclic, or heterocylic rings or fused ring systems, and L₁ and L₂ are linking moieties.

Ring C is selected from moieties represented by Formulae 2A and 2B.

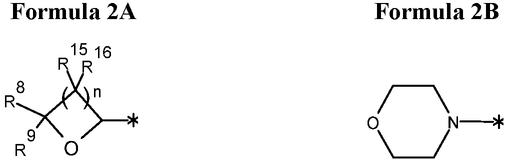

In Formulae 2A and 2B, * represents the binding site to the L₁ moiety, and n is 1 or 2. Each of R⁸, R⁹, R¹⁵ and R¹⁶ is independently selected from hydrogen, a halide, a C1-C4 alkyl group, or a C1-C4 haloalkyl group. In some embodiments, for example, each of R⁸, R⁹, R¹⁵ and R¹⁶ may be independently selected from hydrogen, a halide, a methyl group (-CH₃), or a perfluorinated or partially fluorinated methyl group. Also, when n is 2, and there are multiple R¹⁵s or R¹⁶s, each R¹⁵ or R¹⁶ may be the same or different from each other.

Referring back to Formula 1, the L₁ linking moiety is selected from moieties represented by Formulae 3A, 3B, and 3C.

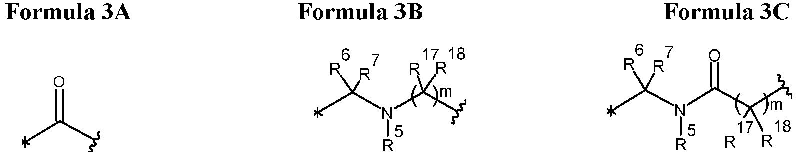

In Formulae 3A, 3B, and 3C, * represents the binding site to the Ring C moiety, 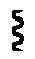 represents the binding site to the Ring B moiety, and m is 0 or 1. Each of R⁵, R⁶, R⁷, R¹⁷ and R¹⁸ is independently selected from hydrogen, a halide, a C1-C4 alkyl group, or a C1-C4 haloalkyl group. In some embodiments for example, each of R⁵*,* R⁶, R⁷, R¹⁷ and R¹⁸ may be independently selected from hydrogen, a halide, a methyl group, or a perfluorinated or partially fluorinated methyl group.

Referring again to Formula 1, Ring B is selected from moieties represented by Formulae 4A, 4B, 4C, 4D, and 4E.

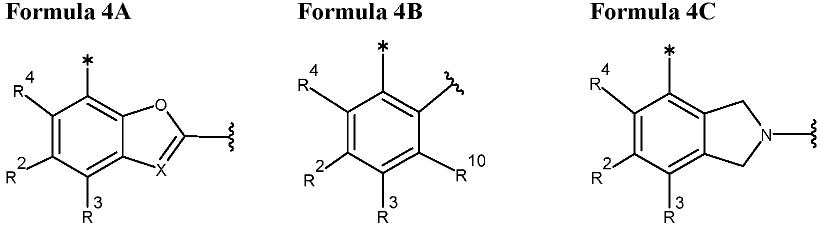

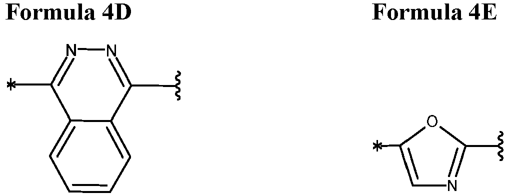

In Formulae 4A, 4B, 4C, 4D, and 4E, * represents the binding site to the L₁ linking moiety, and 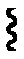 represents the binding site to the L₂ linking moiety. X is C, N or C-Y, where Y is hydrogen, a halide, or a C1-C4 alkoxy group (i.e., -O-alkyl). In some embodiments, for example, Y may be hydrogen, a halide, or methoxy (-OMe). Each of R², R³, R⁴, and R¹⁰ is independently selected from hydrogen, a halide, a C1-C4 alkyl group, or a C1-C4 haloalkyl group. In some embodiments, for example, each of R², R³, R⁴, and R¹⁰ is independently selected from hydrogen, a halide, a methyl group, or perfluorinated or partially fluorinated methyl group.

Again referring back to Formula 1, the L₂ linking moiety is selected from moieties represented by Formulae 5A, 5B, and 5C.

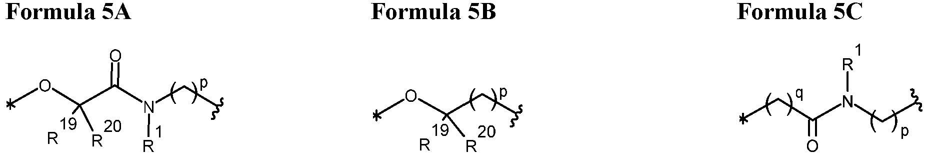

In Formulae 5A, 5B, and 5C, * represents the binding site to the Ring B moiety, and 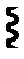 represents the binding site to the Ring D moiety, and both p and q are independently 0 or 1. Each of R¹, R¹⁹, and R²⁰ is independently selected from hydrogen, a halide, a C1-C4 alkyl group, or a C1-C4 haloalkyl group. In some embodiments, for example, each of R¹, R¹⁹, and R²⁰ is independently selected from hydrogen, a halide, a methyl group, or a perfluorinated or partially fluorinated methyl group.

Finally, in Formula 1, Ring D is a substituted or unsubstituted aryl, heteroaryl, cyclic, or heterocylic ring or fused ring system, as those terms are defined herein. Nonlimiting examples of suitable such moieties for Ring D include the following:

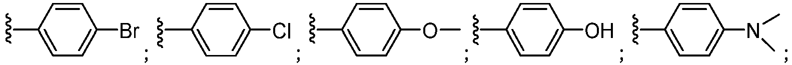

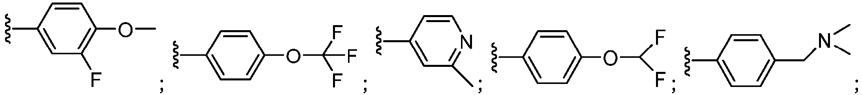

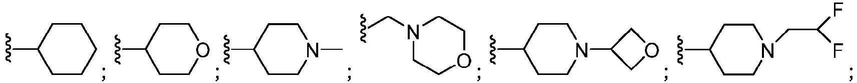

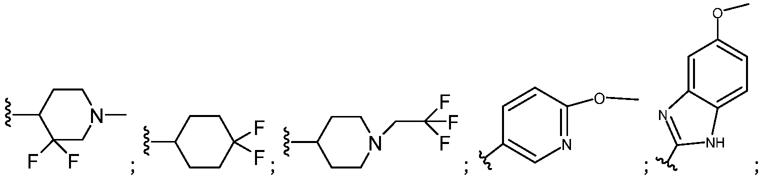

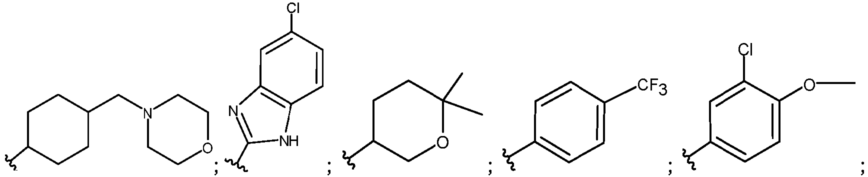

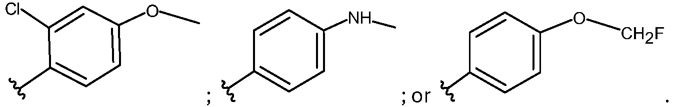

In the above moieties, 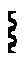 represents the binding site the L₂ moiety.

Nonlimiting examples of suitable compounds satisfying Formula 1 include Compounds 1 through 50 below.

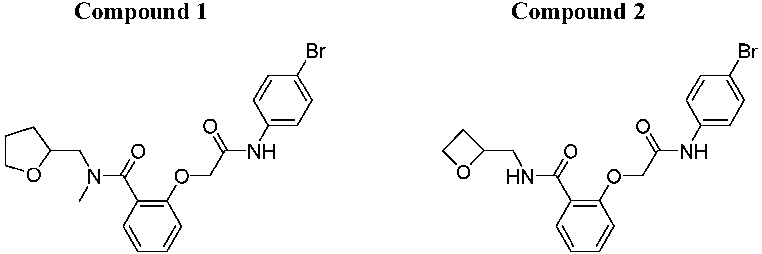

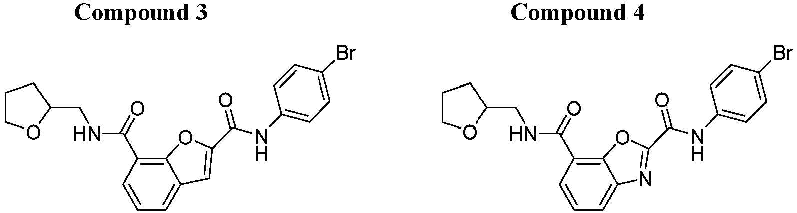

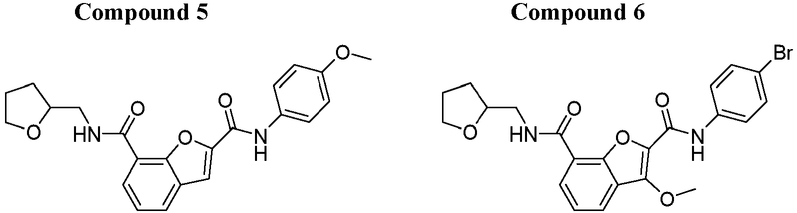

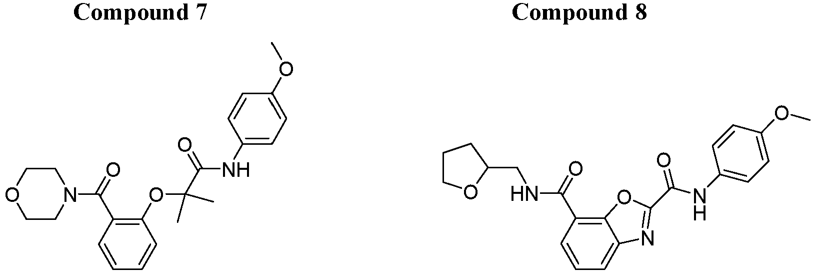

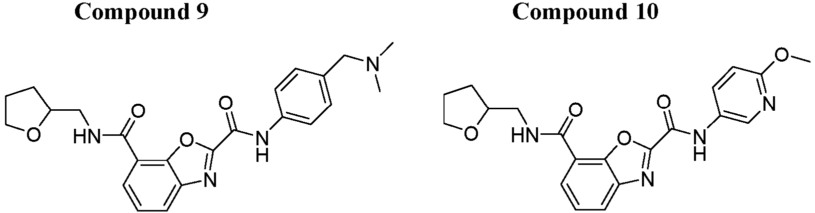

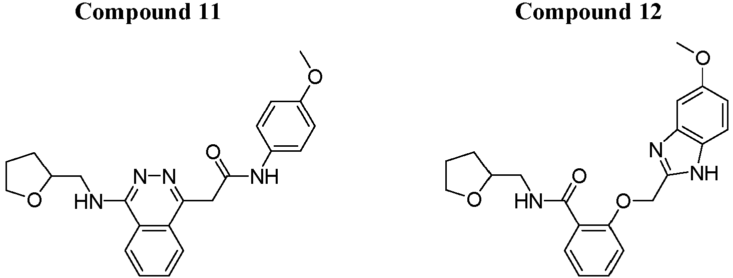

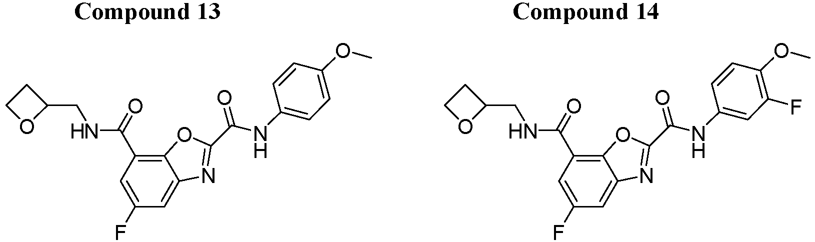

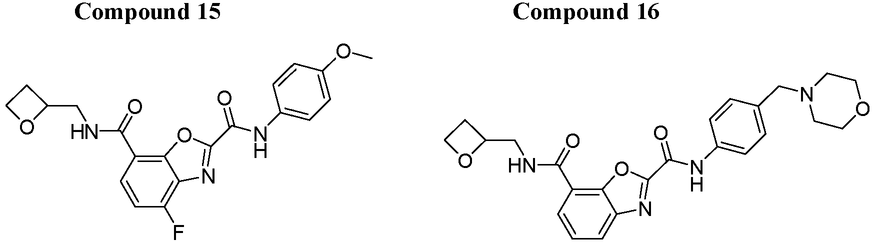

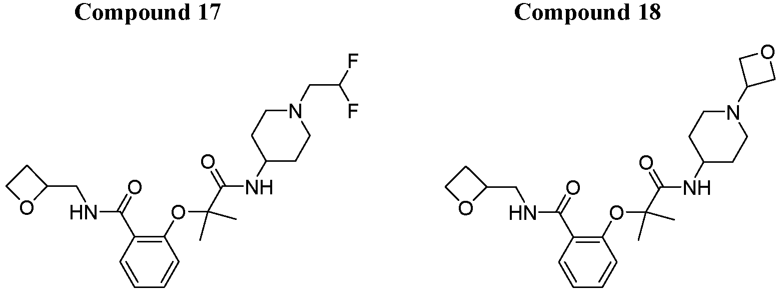

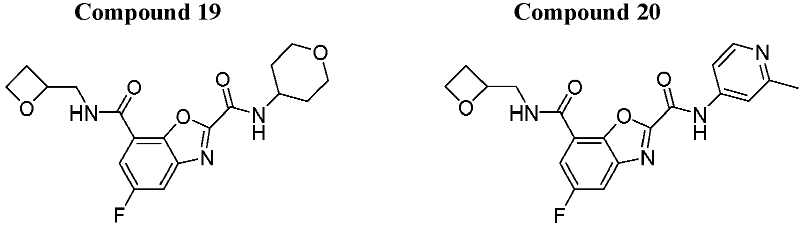

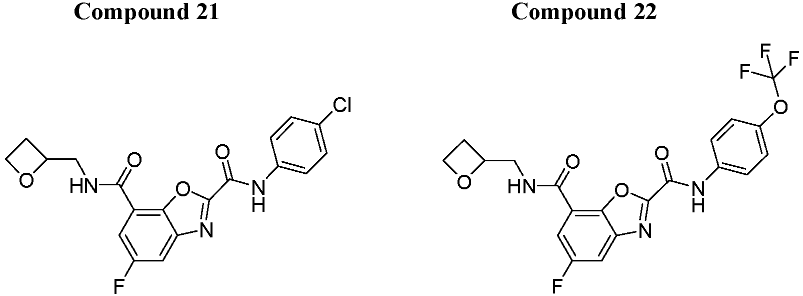

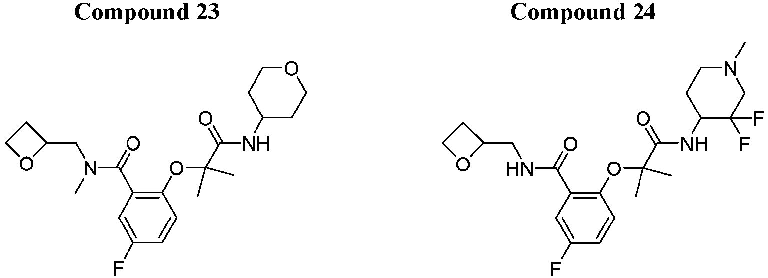

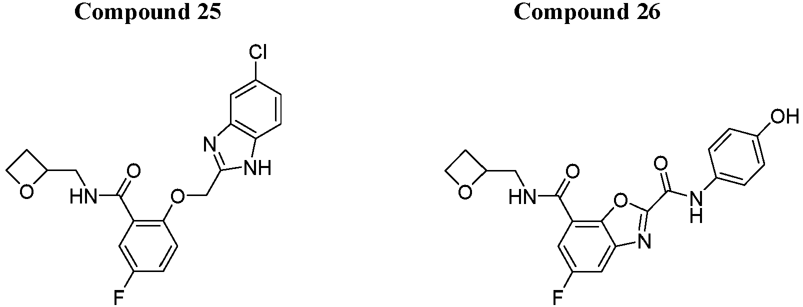

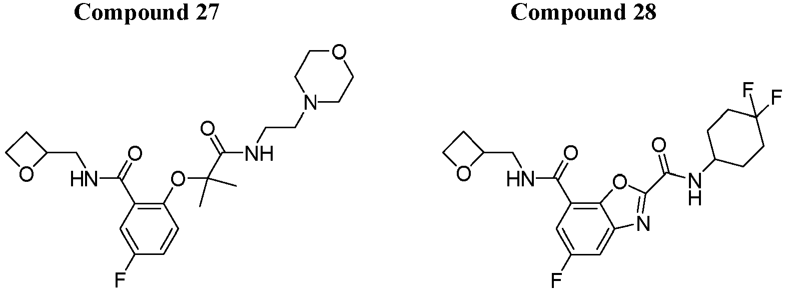

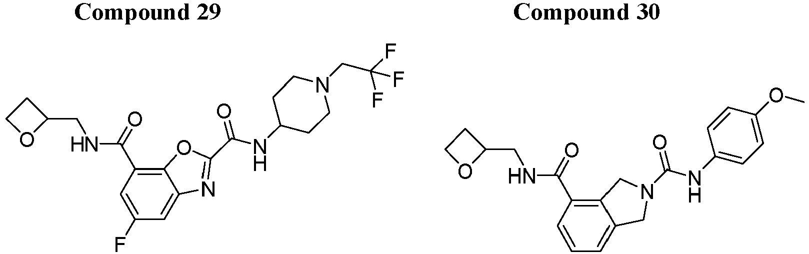

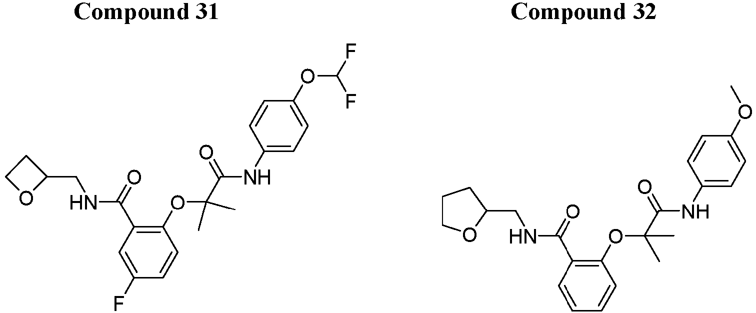

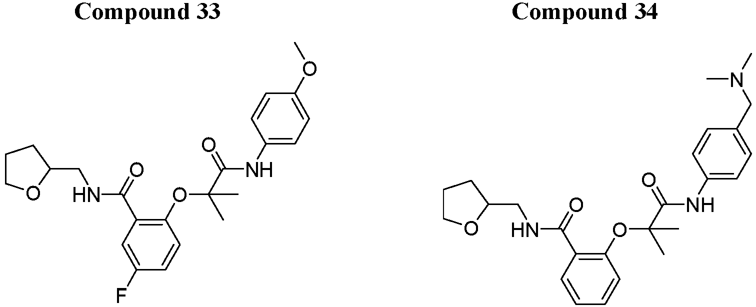

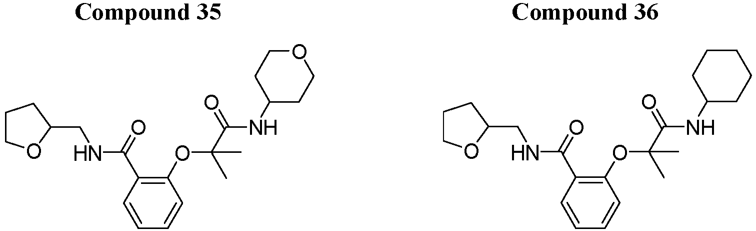

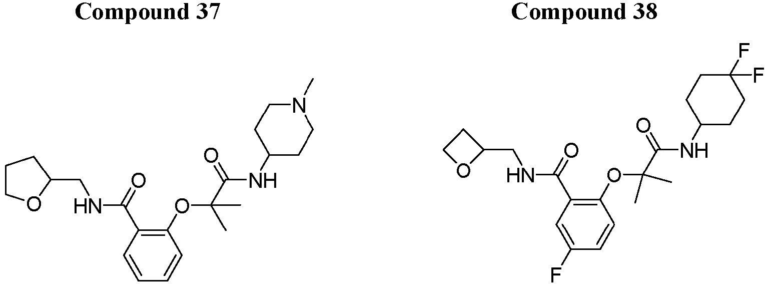

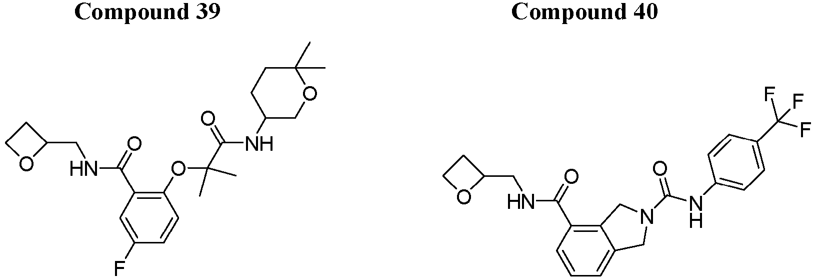

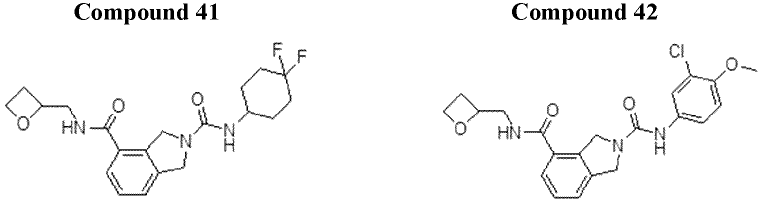

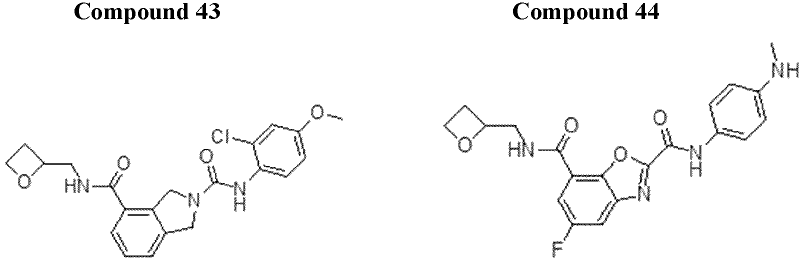

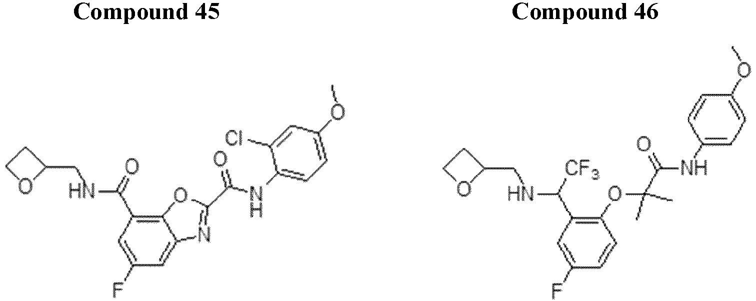

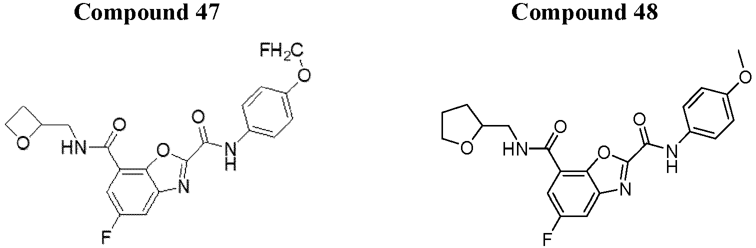

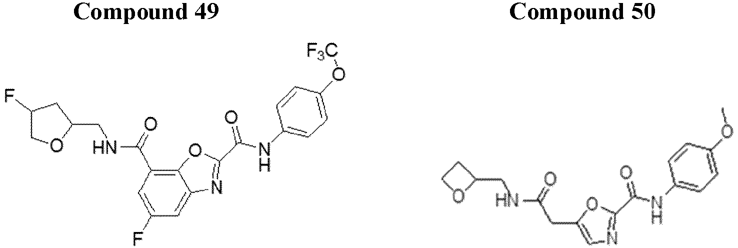

According to some embodiments, the compound satisfying Formula 1 is represented by one of the following Formulae 6 through 9.

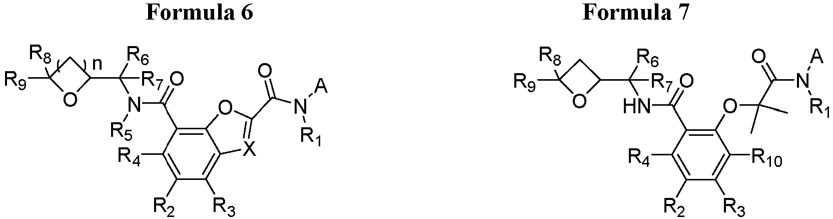

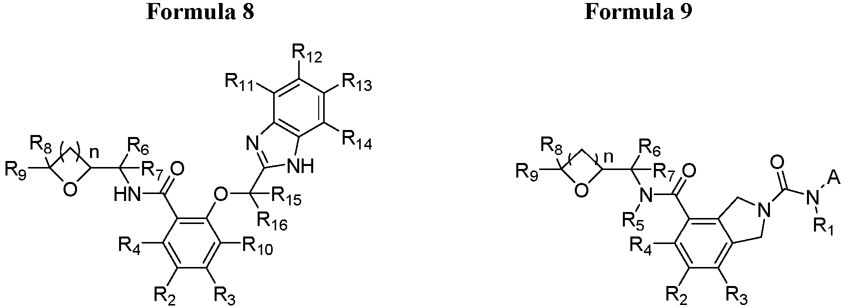

In Formulae 6 through 9, n, X, and R¹ through R¹⁶ are as defined above in connection with Formulae 1, 2A, 2B, 3A, 3B, 3C, 4A, 4B, 4C, 4D, 4E, 5A, 5B, and 5C. Moiety A in Formulae 6, 7 and 9 is a substituted or unsubstituted aryl, heteroaryl, cyclic, or heterocylic ring or fused ring system, as those terms are defined herein, and may be bonded directly to Formulae 6, 7 or 9, or may be bonded to those formulae via a linking -CH₂- group. Nonlimiting examples of suitable moieties for Moiety A include the following: 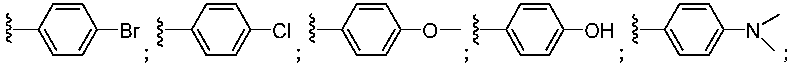 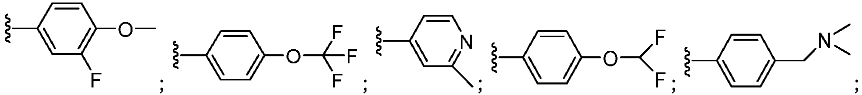 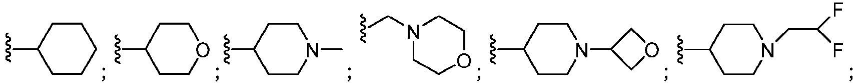 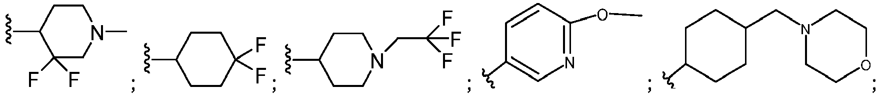 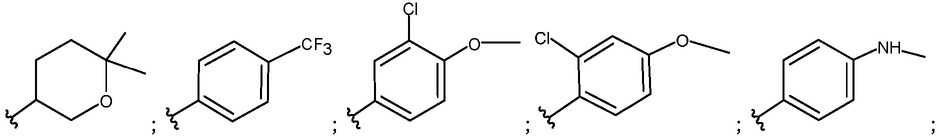 or

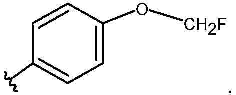

In the above moieties, 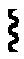 represents the binding site Formula 6, 7, or 9.

Nonlimiting examples of compounds satisfying Formula 6 include Compounds 3-6, 8-10, 13-16, 19-22, 26, 28, 29, 44, 45, and 47-49, below.

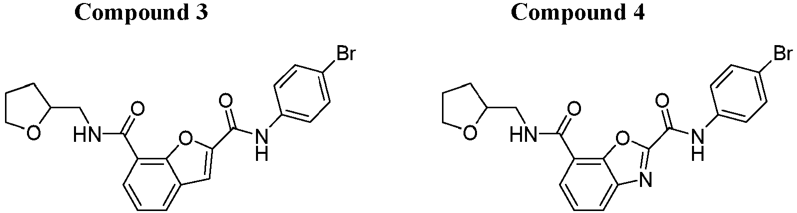

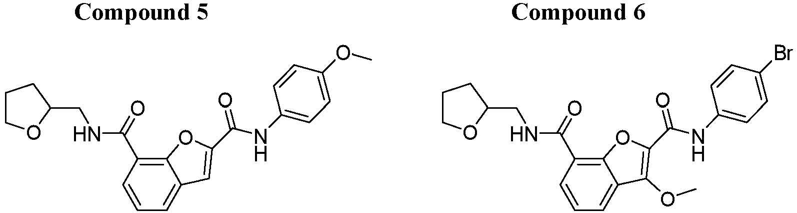

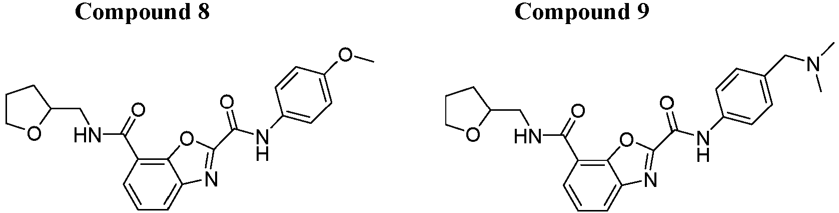

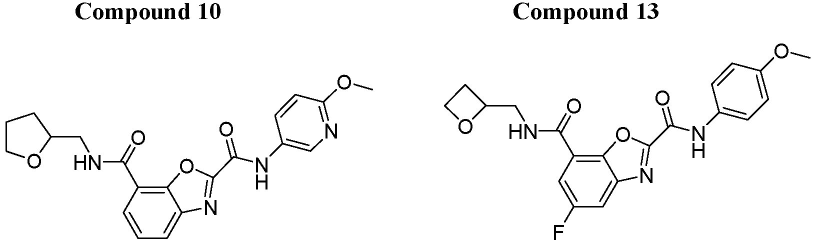

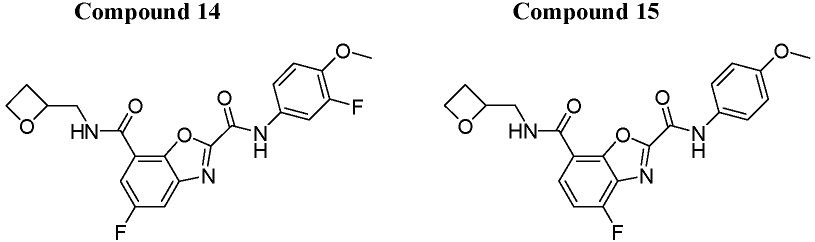

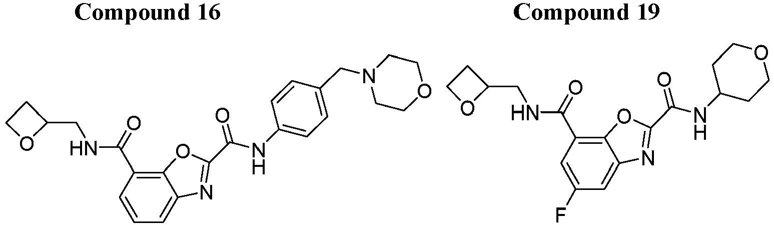

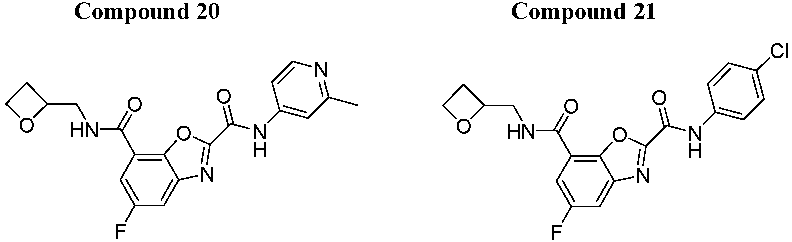

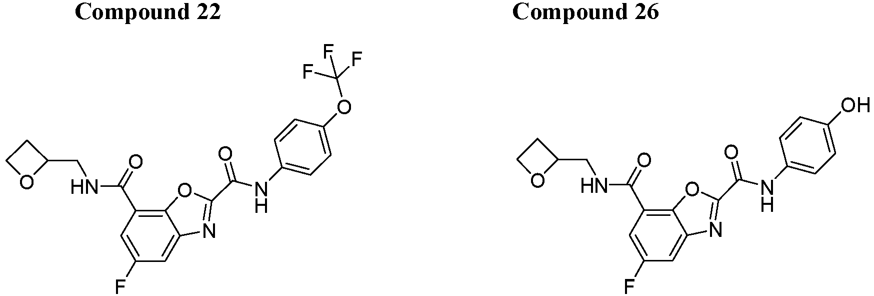

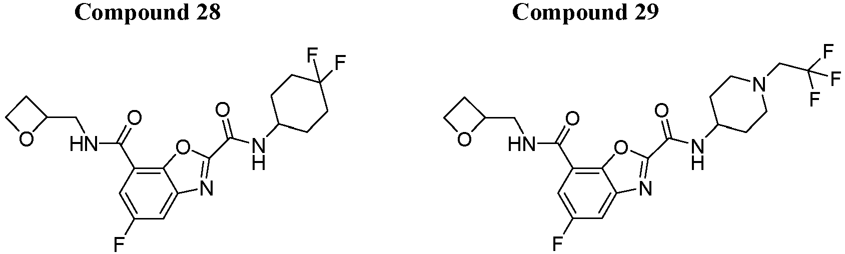

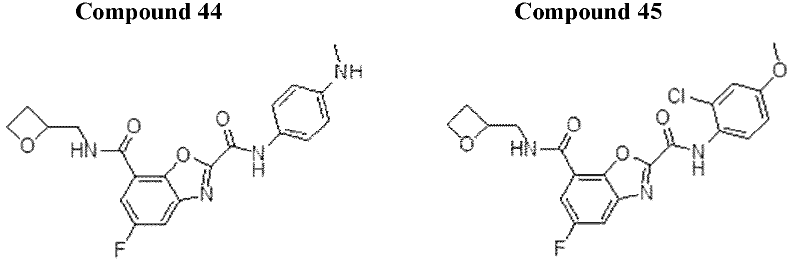

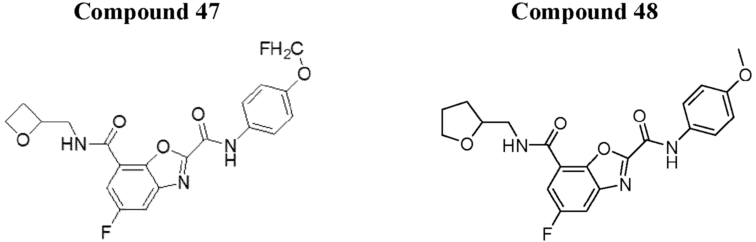

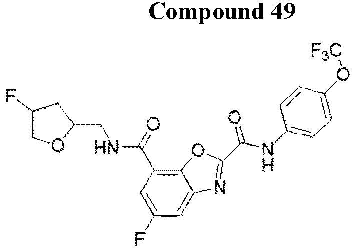

Nonlimiting examples of compounds satisfying Formula 7 include Compounds 1, 2, 7, 12, 17, 18, 23, 24, 27, 31-39, and 46, below.

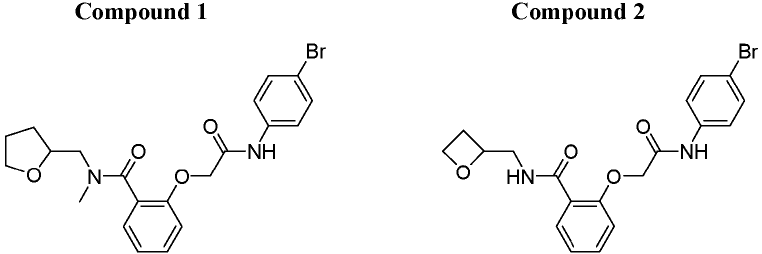

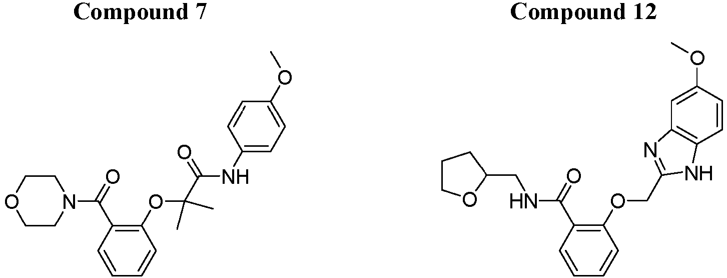

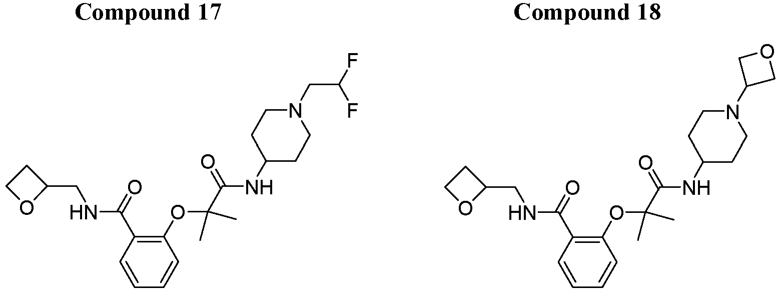

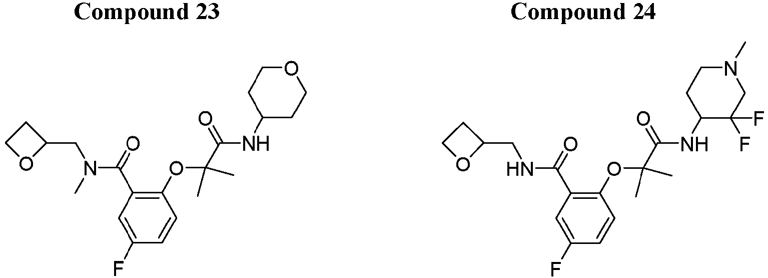

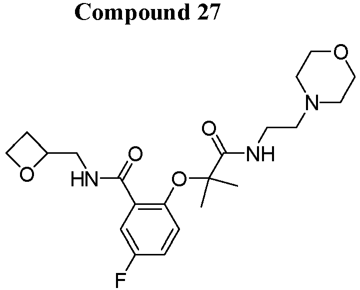

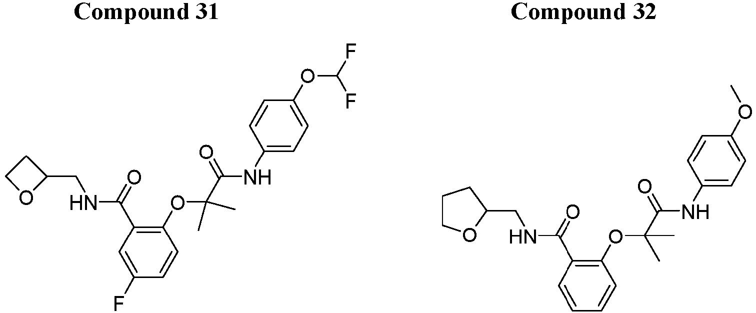

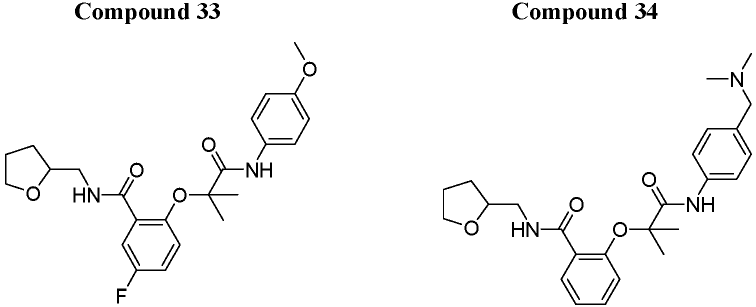

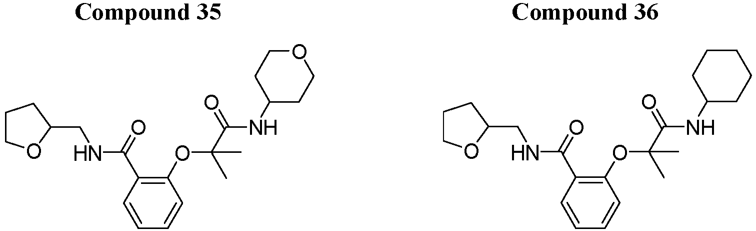

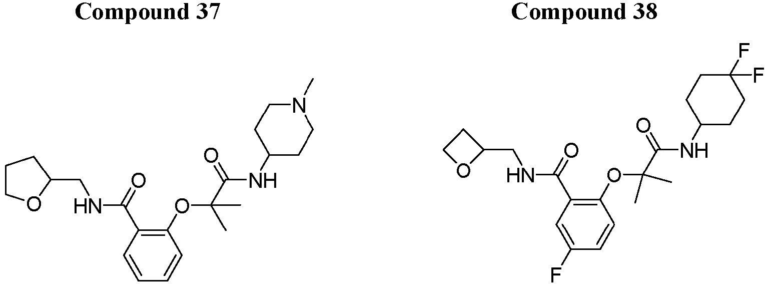

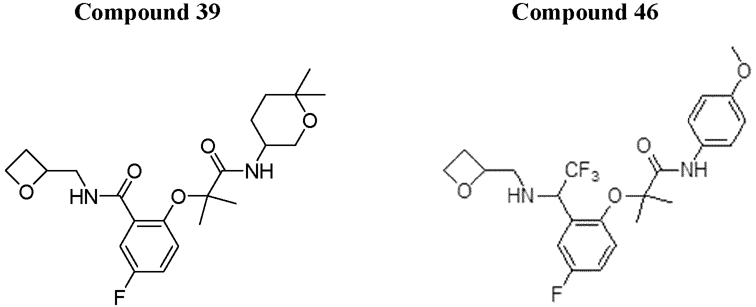

A nonlimiting example of a compound satisfying Formula 8 included Compound 25, below.

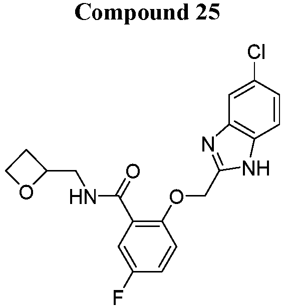

Nonlimiting examples of compounds satisfying Formula 9 include Compounds 30, and 40-43, below.

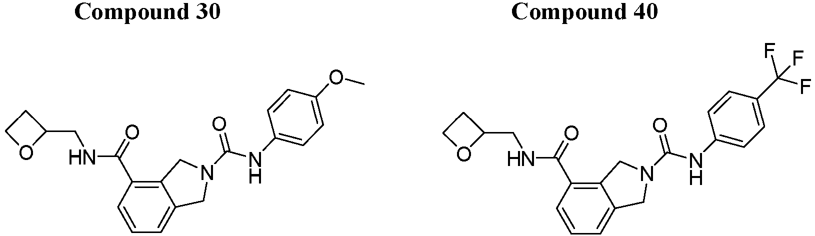

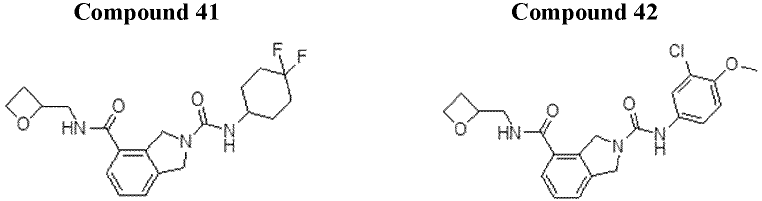

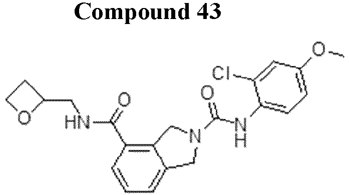

As used herein, the term "C1-C4 alkyl group" refers to any linear or branched alkyl group having from 1 to 4 carbon atoms in the alkyl chain. In some embodiments, for example, the C1-C4 alkyl group is a methyl group.

Also, as used herein, the term "haloalkyl" refers to alkyl groups in which one or more of the hydrogen atoms have been replaced by a halide. For example, in some embodiments, the haloalkyl group may include one or more hydrogen atoms substituted with F, Cl, I, or Br. And in some embodiments, the haloalkyl group may include one or more hydrogen atoms substituted with F, Cl, or Br. In some embodiments, for example, the haloalkyl may be perfluorinated (i.e., all hydrogen atoms are replaced by F) or partially fluorinated (i.e., one or more hydrogen atoms, but not all of them, are replaced by F).

As used herein, the term "C1-C4 alkoxy group" refers to a moiety having the formula, - O-alkyl, wherein the alkyl is any linear or branched alkyl group having from 1 to 4 carbon atoms in the alkyl chain. In some embodiments, for example, the C1-C4 alkoxy group is a methoxy group (i.e., -OMe).

As used herein, the term "aryl ring" refers to a monovalent carbocyclic aromatic system, which may be monocyclic or polycyclic. When the aryl ring is polycyclic, the at least two rings of the system may be fused to each other. In some embodiments, the aryl ring may be a C5 to C20 ring system, and in some embodiments, the aryl ring may be a C5 to C10 ring system.

Analogously, the term "heteroaryl ring" refers to an aryl ring as defined herein in which at least one C atom of the ring system is replaced with a heteroatom. The heteroatom may be N, O, P, or S, and in some embodiments, the heteroatom is N or O. In some embodiments, the heteroaryl ring may be 5 to 20 membered ring system, and in some embodiments, the heteroaryl ring may be a 5 to 10 membered ring system.

As used herein, the term "cyclic ring" refers to cyclic hydrocarbon groups, for example cycloalkyl groups, which may be monocyclic or polycyclic. In some embodiments, the cyclic ring may be a C3 to C20 cyclic group, and in some embodiments, the cyclic ring may be C3 to C10 cyclic group.

Analogously, the term "heterocyclic ring" refers to a cyclic ring as defined herein in which at least one C atom of the ring system is replaced with a heteroatom. The heteroatom may be N, O, P, or S, and in some embodiments, the heteroatom is N or O. In some embodiments, the heterocyclic ring may be 3 to 20 membered ring system, and in some embodiments, the heterocyclic ring may be a 3 to 10 membered ring system.

As used herein, when a moiety is "substituted," one or more hydrogen atoms of the group may be replaced with a non-hydrogen substituent. Non-limiting examples of suitable non-hydrogen substituents include halides, C1-C4 alkyl groups, C1-C4 alkoxy groups, C1-C4 haloalkyl groups having any number of substituted halide atoms, oxohaloalkyl groups (e.g., -O-haloalkyl), amino groups (e.g., -NHR, and -NRR groups), alkylamino groups (e.g., -alkyl-NHR and -alkyl-NRR groups), aryl groups, heteroaryl groups, cyclic groups, heterocylic groups, alkylaryl groups (e.g., -alkyl-aryl), arylalkyl groups (e.g., -aryl-alkyl), alkylcyclic groups (e.g., -alkyl-cyclic), alkylheterocyclic groups (e.g., -alkyl-heterocylic), and hydroxy groups.

According to embodiments of the present disclosure, a pharmaceutical composition includes a compound as disclosed herein or a mixture of such compounds in combination with a pharmaceutically acceptable carrier and/or excipient. The compound or mixture of compounds may also or alternatively be present in the pharmaceutical composition in the form of a pharmaceutically acceptable salt of the compound(s) or as a prodrug thereof. The pharmaceutical compositions may be adapted for oral, topical or transdermal, inhalation, infusion, or parenteral administration, and including an amount of the compound(s). The dose administered to a patient, particularly a human, should deliver a therapeutically effective amount of the compound(s), i.e., an amount sufficient to achieve a therapeutic response in the patient over a reasonable time frame, without lethal toxicity, and causing no more than an acceptable level of side effects or morbidity. The therapeutic response may include reduction in one or more symptoms of the treated disorder. For example, the therapeutic response may include reduction in one or more symptoms of Gaucher disease or Parkinson's disease. Those skilled in the art will recognize that such a therapeutically effective amount will depend upon a variety of factors including the condition (health) of the subject, the body weight of the subject, kind of concurrent treatment, if any, frequency of treatment, route of administration, and therapeutic ratio, as well as the severity and stage of the pathological condition. Such skilled artisans can readily determine the therapeutically effective amount using known methods.

As used herein, the term "pharmaceutically acceptable" means a non-toxic material that is compatible with a biological system such as a cell, cell culture, tissue, or organism, and that does not interfere with the effectiveness of the biological activity of the active ingredient(s). Thus, pharmaceutical compositions according to embodiments of the present disclosure may contain, in addition to the active ingredient complex, one or more diluents, fillers, salts, buffers, stabilizers, solubilizers, and/or other materials well known in the art. Nonlimiting examples of carriers or diluents for use with the compounds include ethanol, dimethyl sulfoxide, glycerol, alumina, starch, saline, and equivalent carriers and diluents.

The preparation of pharmaceutically acceptable formulations is described in detail in a number of sources which are well known and readily available to those skilled in the art. For example, Remington's Pharmaceutical Science by E.W. Martin (1995) describes formulations that can be used in connection with the disclosed compounds. In general, the pharmaceutical compositions according to embodiments of the present disclosure can be formulated such that an effective amount of the compound(s) is combined with a suitable carrier in order to facilitate effective administration of the compound. The compound(s) used can also be in a variety of forms. These include, for example, solid, semi-solid, and liquid dosage forms, such as tablets, pills, powders, liquid solutions or suspension, suppositories, injectable and infusible solutions, and sprays. The form depends on the intended mode of administration and therapeutic application. To provide for the administration of such dosages for the desired therapeutic treatment, the pharmaceutical composition according to embodiments can include from about 0.1% to about 100% by weight of the total of one or more of the subject compounds based on the weight of the total composition including carrier or diluent.

Formulations suitable for administration of the compound(s) (or the pharmaceutically acceptable salts or prodrugs thereof) include, for example, aqueous sterile injection solutions, which can contain antioxidants, buffers, bacteriostats, and solutes that render the formulation isotonic with the blood of the intended recipient; and aqueous and nonaqueous sterile suspensions, which can include suspending agents and thickening agents. The formulations can be presented in unit-dose or multi-dose containers, for example sealed ampoules and vials, and can be stored in a freeze dried (lyophilized) condition requiring only the condition of the sterile liquid carrier, for example, water for injections, prior to use. Extemporaneous injection solutions and suspensions can be prepared from sterile powder, granules, tablets, *etc.* It should be understood that in addition to the ingredients particularly mentioned above, the compositions can include other agents conventional in the art taking into account the intended type of formulation.

According to some embodiments, a kit may include the compound or mixture of such compounds disclosed herein (or the pharmaceutically acceptable salts or prodrugs thereof) in one or more containers. Such kits can optionally include pharmaceutically acceptable carriers and/or diluents. In some embodiments, a kit may include one or more other components, adjuncts, or adjuvants. In some embodiments, for example, a kit may include one or more of the compounds described herein, or their pharmaceutically acceptable salts or prodrugs thereof. In some embodiments, a kit may include instructions or packaging materials that describe how to administer a compound or composition of the kit. Containers of the kit can be of any suitable material, *e.g.,* glass, plastic, metal, *etc.,* and of any suitable size, shape, or configuration. In some embodiments, the compound(s) disclosed herein (or the pharmaceutically acceptable salts or prodrugs thereof) is provided in the kit as a solid, such as a tablet, pill, or powder form. In some embodiments, the compound(s) disclosed herein (or the pharmaceutically acceptable salts or prodrugs thereof) is provided in the kit as a liquid or solution. In some embodiments, the kit includes an ampoule or syringe containing the compound(s) disclosed herein (or the pharmaceutically acceptable salts or prodrugs thereof) in liquid or solution form.

According to embodiments of the present disclosure, the compounds (and the pharmaceutically acceptable salts and prodrugs thereof) and pharmaceutical compositions disclosed herein exhibit improved stability, improved efficacy in the treatment of Gaucher disease, and improved efficacy in the treatment of Parkinson's disease. Therefore, some embodiments of the present disclosure are directed to use of one or more of the compounds disclosed herein (or the pharmaceutically acceptable salts or prodrugs thereof) in the manufacture of a medicament for the treatment of Gaucher disease and/or Parkinson's disease. And some embodiments of the present disclosure are directed to use of the pharmaceutical compositions disclosed herein in the manufacture of a medicament for the treatment of Gaucher disease and/or Parkinson's disease. In some embodiments, the compounds (or the pharmaceutically acceptable salts or prodrugs thereof) or pharmaceutical compositions disclosed herein may be used either alone or in combination with another therapeutic agent to prepare the medicament.

According to embodiments of the present disclosure, a method of treating Gaucher disease or Parkinson's disease includes administering a therapeutically effective amount of one or more of the compounds disclosed herein (or the pharmaceutically acceptable salts or prodrugs thereof) to a patient or subject in need thereof. In some embodiments, the method includes administering the pharmaceutical composition disclosed herein to the patient or subject. For example, in some embodiments, a method of treating Gaucher disease includes administering a therapeutically effective amount of one or more of the compounds disclosed herein (or the pharmaceutically acceptable salts or prodrugs thereof) to a patient or subject diagnosed with or at risk of developing Gaucher disease. And in some embodiments, a method of treating Parkinson's disease includes administering a therapeutically effective amount of one or more of the compounds disclosed herein (or the pharmaceutically acceptable salts or prodrugs thereof) to a patient or subject diagnosed with or at risk of developing Parkinson's disease. In some embodiments, the compounds (or the pharmaceutically acceptable salts or prodrugs thereof) or pharmaceutical compositions disclosed herein may be administered to the patient or subject either alone or in combination with another therapeutic agent.

The mode of administration of the one or more compounds (or pharmaceutically acceptable salts or prodrugs thereof) or the pharmaceutical composition to the subject is not particularly limited, and may be any suitable mode of administration known to those skilled in the art. Nonlimiting examples of suitable such administration routes include parenteral administration, oral administration, buccal administration, sublingual administration, or inhalation. In some embodiments, for example, the administration route may be oral administration, and the compound(s) or pharmaceutical composition is formulated for oral administration.

In some embodiments, the administration route may be parenteral administration, for example, intravenous, intra-arterial, intra-muscular, subcutaneous, or intraperitoneal administration. Formulations of the compound(s) or pharmaceutical composition suitable for parenteral administration may include aqueous or non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. Aqueous solutions may be suitably buffered (preferably to a pH of from 3 to 9). The preparation of suitable parenteral formulations under sterile conditions is readily accomplished by standard pharmaceutical techniques well-known to those skilled in the art.

In some embodiments, the route of administration may be oral, buccal, or sublingual administration. Such pharmaceutical compositions may be in the form of tablets, capsules, ovules, elixirs, solutions, or suspensions, which may contain flavoring or coloring agents, for immediate-, delayed- or controlled-release applications. Suitable tablets may contain excipients such as microcrystalline cellulose, lactose, sodium citrate, calcium carbonate, dibasic calcium phosphate and glycine, disintegrants such as starch (for example, corn, potato or tapioca starch), sodium starch glycolate, croscarmellose sodium and certain complex silicates, and granulation binders such as polyvinylpyrrolidone, hydroxypropylmethylcellulose (HPMC), hydroxy-propylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Nonlimiting examples of excipients in this regard include lactose, starch, a cellulose, milk sugar or high molecular weight polyethylene glycols. For aqueous suspensions and/or elixirs, the compound(s) (or the pharmaceutically acceptable salts or prodrugs thereof) disclosed herein may be combined with various sweetening or flavoring agents, coloring matter or dyes, with emulsifying and/or suspending agents and with diluents such as water, ethanol, propylene glycol and glycerin, and combinations thereof.

In some embodiments, the route of administration may be intranasal administration or inhalation, such as delivered in the form of a dry powder inhaler or an aerosol spray presentation from a pressurized container, pump, spray or nebulizer with the use of a suitable propellant, e.g., dichlorodifluoromethane, trichlorofluoromethane, dichlorotetrafluoroethane, a hydrofluoroalkane, carbon dioxide or other suitable gas. In the case of a pressurized aerosol, the dosage unit may be determined by providing a valve to deliver a metered amount. The pressurized container, pump, spray, or nebulizer may contain a solution or suspension of the active compound, e.g., using a 15 mixture of ethanol and the propellant as the solvent, which may additionally contain a lubricant. Capsules and cartridges (made, for example, from gelatin) for use in an inhaler or insufflator may be formulated to contain a powder mix of the compound(s) (or pharmaceutically acceptable salts or prodrugs thereof) and a suitable powder base such as lactose or starch.

The formulations may be presented in unit-dose or multi-dose containers, for example sealed ampoules or vials, and may be stored in a freeze-dried (lyophilized) condition requiring only the addition of the sterile liquid carrier immediately prior to use. In some embodiments, the formulations can be pre-loaded in a unit-dose injection device, *e.g.,* a syringe for intravenous injection.

### EXAMPLES

These Examples are merely for illustrative purposes and are not intended to limit the scope of the appended claims. Notwithstanding that the numerical ranges and parameters setting forth the broad scope of the present disclosure are approximations, the numerical values set forth in these specific examples are reported as precisely as possible. Any numerical value, however, inherently contains certain errors necessarily resulting from the standard deviation found in their respective testing measurements. At the very least, and not as an attempt to limit the application of the doctrine of equivalents to the scope of the claims, each numerical parameter should at least be construed in light of the number of reported significant digits and by applying ordinary rounding techniques.

### SYNTHESIS EXAMPLES

The compounds and compositions disclosed herein may be manufactured by any suitable synthesis methods without limitation. The following Examples describe the synthesis of Compounds 1 through 50, though it is understood that the present disclosure is not limited to these syntheses, and any suitable synthesis may be used. Additionally, in the below Synthesis Examples, repetitive syntheses of intermediates already described in prior Synthesis Examples may be omitted for brevity and clarity.

### Synthesis Example 1: Synthesis of Compound 1

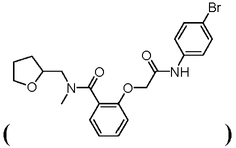

Compound 1 was synthesized according to the following Reaction Scheme 1.

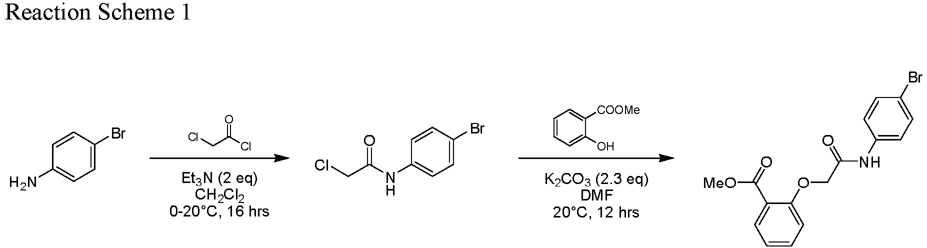

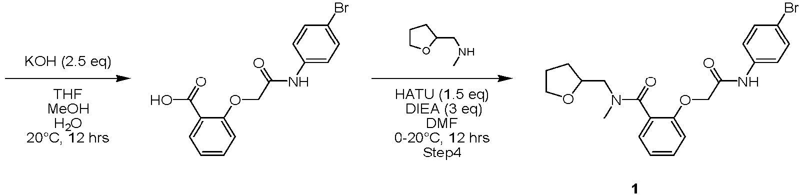

As shown in Reaction Scheme 1, N-(4-bromophenyl)-2-chloroacetamide was first prepared. To a mixture of 4-bromobenzeneamine (4.0 g, 23.25 mmol) and triethylamine (TEA; Et₃N) (2.82 g, 27.90 mmol) in dichloromethane (DCM; Ch₂Cl₂) (50 mL) was added 2-chloroacetyl chloride (2.63 g, 23.25 mmol) dropwise at 0°C under N₂. The mixture was stirred at 0°C for 30 min, then warmed to 20°C and stirred for 16 hours. Thin Layer Chromatography (TLC) showed the reaction was completed. The reaction mixture was diluted with 100 mL of dichloromethane and then washed with brine (100 mL). The combined organic phase was dried with anhydrous Na₂SO₄, filtered and concentrated in vacuum to give the crude product. The crude product was triturated with methyl tert-butyl ether (MTBE) (30 mL) at 20°C for 30 min. The filter cake was collected and dried in vacuum to give N-(4-bromophenyl)-2-chloroacetamide (5.0 g, 86.53% yield) as a pale solid. Structure of N-(4-bromophenyl)-2-chloroacetamide was confirmed by ¹H NMR: (400MHz, DMSO-*d6*)δ 10.42 (s, 1 H) 7.47 - 7.62 (m, 4 H) 4.25 (s, 2 H).

Next, methyl 2-((4-bromophenylcarbamoyl)methoxy)benzoate was prepared. To a mixture of methyl 2-hydroxybenzoate (1.22 g, 8.05 mmol) and K₂CO₃ (2.78 g, 20.12 mmol) in dimethylformamide (DMF) (30 mL) was added N-(4-bromophenyl)-2-chloroacetamide (2.0 g, 8.05 mmol) at 0°C under N₂. The mixture was stirred at 20°C and stirred for 16 hours. Liquid chromatography-mass spectrometry (LC-MS) showed the reaction was completed. The mixture was poured into ice-water (50 mL) and extracted 3 times with 60 mL ethyl acetate (3 x 60 mL). The combined organic phase was washed with brine (100 mL), dried with anhydrous Na₂SO₄, filtered and concentrated in vacuum. The residue was purified by column chromatography (SiO₂, Petroleum ether/Ethyl acetate=100/1 to 30/1) to give methyl 2-((4-bromophenylcarbamoyl)methoxy)benzoate (2.5 g, 85.29% yield) as a white solid. Structure of methyl 2-((4-bromophenylcarbamoyl)methoxy)benzoate was confirmed by ¹H NMR: (400MHz, DMSO-*d6*)6 10.26 (s, 1 H) 7.82 (dd, *J*=7.8, 1.6 Hz, 1 H) 7.64 - 7.69 (m, 2 H) 7.60 (ddd, *J*=8.6, 7.3, 1.8 Hz, 1 H) 7.52 - 7.57 (m, 2 H) 7.20 (d, *J*=8.4 Hz, 1 H) 7.07 - 7.15 (m, 1 H) 4.82 (s, 2 H) 3.88 (s, 3 H).

Next 2-((4-bromophenylcarbamoyl)methoxy)benzoic acid was prepared. To a solution of methyl 2-((4-bromophenylcarbamoyl)methoxy)benzoate (2.5 g, 6.86 mmol) in tetrahydrofuran (THF) (60 mL) and methanol (MeOH) (30 mL) was added KOH (962.92 mg, 17.16 mmol) in H₂O (15 mL) dropwise at 0°C. The reaction mixture was stirred at 20°C for 12 hours. LC-MS showed the reaction was completed. The reaction mixture was concentrated under reduced pressure to give a residue. The residue was dissolved in water (100 mL) and washed with ethyl acetate (50 mL). The aqueous phase was adjusted to pH=3 by 1 N HCl at 0°C and filtered. The filter cake was dried in vacuum to give 2-((4-bromophenylcarbamoyl)methoxy)benzoic acid (2.0 g, 83.33% yield) as a white solid. Structure of 2-((4-bromophenylcarbamoyl)methoxy)benzoic acid was confirmed by ¹H NMR: (400MHz, DMSO-*d6*)δ 12.70 - 13.96 (m, 1 H) 10.45 (s, 1 H) 7.86 (dd, *J*=7.8, 1.8 Hz, 1 H) 7.65 - 7.72 (m, 2 H) 7.53 - 7.63 (m, 3 H) 7.21 (d, *J*=8.3 Hz, 1 H) 7.11 (t, *J*=7.5 Hz, 1 H) 4.82 (s, 2 H).

Finally, 2-((4-bromophenylcarbamoyl)methoxy)-N-((tetrahydrofuran-2-yl)methyl)-N-methylbenzamide (**Compound 1**) was prepared. To a mixture of 2-((4-bromophenylcarbamoyl)methoxy)benzoic acid (150 mg, 428.37 umol), N,N-diisopropylethylamine (DIEA) (276.82 mg, 2.14 mmol) and N-methyl-1-tetrahydrofuran-2-ylmethanamine (77.95 mg, 514.05 umol) in DMF (2 mL) was added 50% of propanephosphonic acid anhydride (T₃P) in ethyl acetate (EtOAc) (408.90 mg, 642.56 umol) drop-wise at 0°C under N₂. The mixture was stirred at 20°C for 2 hours. LC-MS showed the reaction was completed. The reaction mixture was filtered, and the filtrate was collected. The filtrate was purified by preparative High-Performance Liquid Chromatography (prep-HPLC) (NH₄HCO₃) and lyophilized to give - ((4-bromophenylcarbamoyl)methoxy)-N-((tetrahydrofuran-2-yl)methyl)-N-methylbenzamide (57.2 mg, 29.85% yield)(**Compound 1**) as a white solid. Compound 1 structure was confirmed by ¹H NMR: (400MHz, DMSO-*d6*)δ 10.24 (d, *J*=4.9 Hz, 1 H) 7.58 - 7.67 (m, 2 H) 7.46 - 7.55 (m, 2 H) 7.34 - 7.43 (m, 1 H) 7.20 - 7.29 (m, 1 H) 7.15 (d, *J*=8.3 Hz, 1 H) 7.04 (q, *J*=6.8 Hz, 1 H) 4.82 (s, 2 H) 3.91 - 4.15 (m, 1 H) 3.42 - 3.80 (m, 3 H) 3.24 (br s, 1 H) 2.88 - 3.10 (m, 3 H) 1.22 - 1.99 (m, 4 H).

### Synthesis Example 2: Synthesis of Compound 2

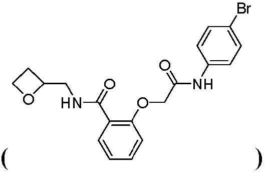

Compound 2 was synthesized according to the following Reaction Scheme 2.

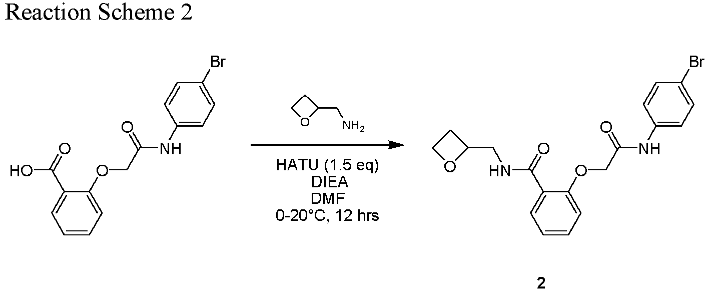

As shown in Reaction Scheme 2, 2-((4-bromophenylcarbamoyl)methoxy)-N-((oxetan-2-yl)methyl)benzamide **(Compound 2**) was prepared. To a mixture of 2-((4-bromophenylcarbamoyl)methoxy)benzoic acid (150 mg, 428.37 umol), DIEA (276.82 mg, 2.14 mmol) and oxetan-2-ylmethanamine (44.78 mg, 514.05 umol) in DMF (2 mL) was added Hexafluorophosphate Azabenzotriazole Tetramethyl Uronium (HATU) (195.46 mg, 514.05 umol) in one portion at 0°C under N₂. The mixture was stirred at 20°C for 12 hours. LC-MS showed the reaction was completed. The reaction mixture was filtered, and the filtrate was collected. The crude product was purified by prep-HPLC (NH4HCO3) to give 2-((4-bromophenylcarbamoyl)methoxy)-N-((oxetan-2-yl)methyl)benzamide(88.8 mg, 49.44% yield) (Compound 2) as a white solid. The Compound 2 structure was confirmed by 1H NMR: (400MHz, DMSO-*d6*)6 10.61 (s, 1 H) 8.86 (br t, J=5.6 Hz, 1 H) 7.70 (dd, J=7.7, 1.7 Hz, 1 H) 7.65 (d, J=8.9 Hz, 2 H) 7.46 - 7.55 (m, 3 H) 7.19 (d, J=8.4 Hz, 1 H) 7.10 (t, J=7.5 Hz, 1 H) 4.90 (s, 2 H) 4.83 - 4.89 (m, 1 H) 4.37 - 4.54 (m, 2 H) 3.59 - 3.70 (m, 1 H) 3.52 (dt, *J=*13.9*,* 5.3 Hz, 1 H) 2.60 - 2.69 (m, 1 H) 2.43 - 2.46 (m, 1 H).

### Synthesis Example 3: Synthesis of Compound 3

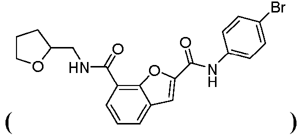

Compound 3 was synthesized according to the following Reaction Scheme 3.

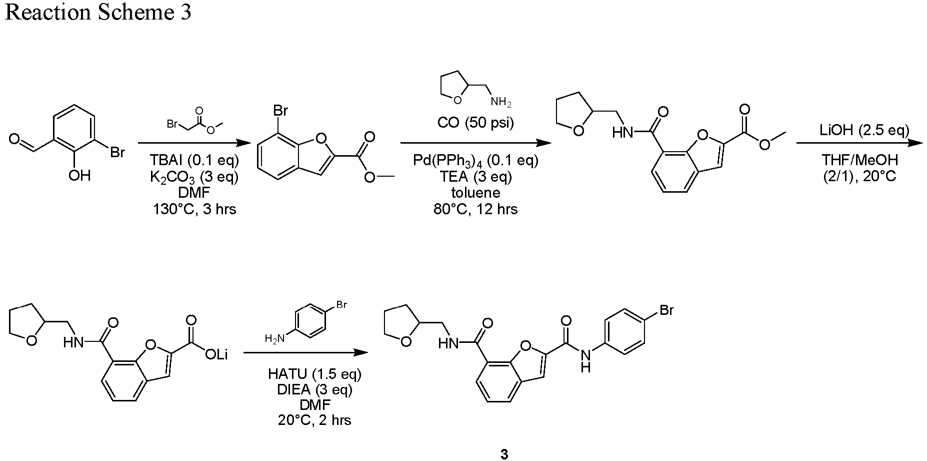

As shown in Reaction Scheme 3, first, methyl 7-bromobenzofuran-2-carboxylate was prepared. To a mixture of 3-bromo-2-hydroxybenzaldehyde (4 g, 19.90 mmol) and methyl 2-bromoacetate (6.09 g, 39.80 mmol) in DMF (80 mL) was added Tetrabutylammonium Iodide (TBAI) (735.00 mg, 1.99 mmol) and K₂CO₃ (11.00 g, 79.60 mmol) in one portion at 20°C under N₂. The mixture was heated to 130°C and stirred for 3 hours. LC-MS showed the reaction was completed. The mixture was cooled to 20°C and poured into ice-water (200 mL) and then extracted 3 times with 300 mL ethyl acetate (3 x 300 mL). The combined organic phase was washed with brine (500 mL), dried with anhydrous Na₂SO₄, filtered and concentrated under reduced pressure to give a residue. The residue was purified by column chromatography (SiO₂, Petroleum ether/Ethyl acetate=100/1 to 10/1) to give methyl 7-bromobenzofuran-2-carboxylate (2.0 g, 39.41% yield) as a white solid. The structure of methyl 7-bromobenzofuran-2-carboxylate was confirmed by ¹H NMR: (400MHz, DMSO-*d6*)δ 7.89 (s, 1 H) 7.82 (dd, *J*=7.9, 1.0 Hz, 1 H) 7.77 (dd, *J*=7.8, 1.0 Hz, 1 H) 7.32 (t, *J*=7.8 Hz, 1 H) 3.92 (s, 3 H).

Next, methyl 7-((tetrahydrofuran-2-yl)methylcarbamoyl)benzofuran-2-carboxylate was prepared. To a solution of methyl 7-bromobenzofuran-2-carboxylate (1 g, 3.92 mmol), TEA (1.19 g, 11.76 mmol) and tetrahydrofuran- 2-ylmethanamine (1.19 g, 11.76 mmol) in toluene (30 mL) was added tetrakis(triphenylphosphine)palladium(0) (Pd(PPh₃)₄) (453.05 mg, 392.06 umol) under N₂ atmosphere. The suspension was degassed and purged with CO three times. The mixture was stirred under CO (50 Psi) at 80°C for 12 hrs. LC-MS showed the reaction was completed. The reaction mixture was filtered, and the filter was concentrated under reduced pressure to give a residue. The residue was purified by column chromatography (SiO₂, Petroleum ether/Ethyl acetate=100/1to 2/1) to give methyl 7-((tetrahydrofuran-2-yl)methylcarbamoyl)benzofuran-2-carboxylate (1.0 g, 84.09% yield) as a yellow solid. The structure of methyl 7-((tetrahydrofuran-2-yl)methylcarbamoyl)benzofuran-2-carboxylate was confirmed by ¹H NMR: (400MHz, DMSO-*d*₆)δ 8.28 (br t, *J*=5.4 Hz, 1 H) 7.95 (dd, *J*=7.9, 1.0 Hz, 1 H) 7.82 - 7.89 (m, 2 H) 7.46 (t, *J*=7.7 Hz, 1 H) 4.03 (quin, *J*=6.1 Hz, 1 H) 3.82 - 3.94 (m, 4 H) 3.61 - 3.72 (m, 1 H) 3.36 - 3.55 (m, 2 H) 1.76 - 2.06 (m, 3 H) 1.60 - 1.73 (m, 1 H).

Next, lithium 7-((tetrahydrofuran-2-yl)methylcarbamoyl)benzofuran-2-carboxylate was prepared. To a solution of methyl 7-((tetrahydrofuran-2-yl)methylcarbamoyl)benzofuran-2-carboxylate (0.2 g, 659.39 umol) in THF (4 mL) and MeOH (2 mL) was added LiOH.H₂O (69.18 mg, 1.65 mmol) in H₂O (1 mL) at 20°C. The mixture was stirred at 20°C for 2 hrs. LC-MS showed the reaction was completed. The reaction mixture was lyophilized to give lithium 7-((tetrahydrofuran-2-yl)methylcarbamoyl)benzofuran-2-carboxylate (180 mg, 92.47% yield) as a white solid. The structure of lithium 7-((tetrahydrofuran-2-yl)methylcarbamoyl)benzofuran-2-carboxylate was confirmed by ¹H NMR: (400MHz, DMSO-*d*₆) δ 8.14 (br s, 1 H) 7.75 (br d, *J*=7.5 Hz, 2 H) 7.49 - 7.67 (m, 1 H) 7.29 (br t, *J*=7.5 Hz, 1 H) 7.03 (br s, 1 H) 3.97 - 4.09 (m, 1 H) 3.87 - 3.95 (m, 1 H) 3.64 (q, *J*=7.3 Hz, 1 H) 3.48 (br s, 2 H) 1.74 - 2.03 (m, 3 H) 1.59 - 1.70 (m, 1 H).

Finally, N2-(4-bromophenyl)-N7-((tetrahydrofuran-2-yl)methyl)benzofuran-2,7-dicarboxamide (**Compound 3**) was prepared. To a mixture of lithium 7-((tetrahydrofuran-2-yl)methylcarbamoyl)benzofuran-2-carboxylate (50 mg, 172.84 umol), DIEA (67.01 mg, 518.52 umol) and 4-bromoaniline (35.68 mg, 207.41 umol) in DMF (1 mL) was added HATU (98.58 mg, 259.26 umol) in one portion at 0°C under N₂. The mixture was stirred at 20°C for 2 hours. LC-MS showed the reaction was completed. The reaction mixture was filtered, and the filtrate was collected. The filtrate was purified by prep-HPLC (NH₄HCO₃) to give N2-(4-bromophenyl)-N7-((tetrahydrofuran-2-yl)methyl)benzofuran-2,7-dicarboxamide (**Compound 3**) (22.6 mg, 28.40% yield) as a white solid. The Compound 3 structure was confirmed by ¹H NMR: (400MHz, DMSO-*d*₆) δ 10.52 (s, 1 H) 10.35 (t, *J*=5.7 Hz, 1 H) 8.52 (d, *J*=2.3 Hz, 1 H) 7.79 (dd, *J*=7.5, 2.4 Hz, 1 H) 7.44 - 7.62 (m, 4 H) 6.42 (d, *J*=7.5 Hz, 1 H) 5.02 (s, 2 H) 3.87 - 3.99 (m, 1 H) 3.73 - 3.84 (m, 1 H) 3.63 (q, *J*=7.4 Hz, 1 H) 3.38 - 3.50 (m, 1 H) 3.32 - 3.37 (m, 1 H) 1.72 - 1.97 (m, 3 H) 1.43 - 1.59 (m, 1 H).

### Synthesis Example 4: Synthesis of Compound 4

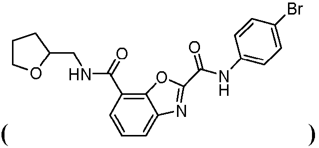

Compound 4 was synthesized according to the following Reaction Scheme 4.

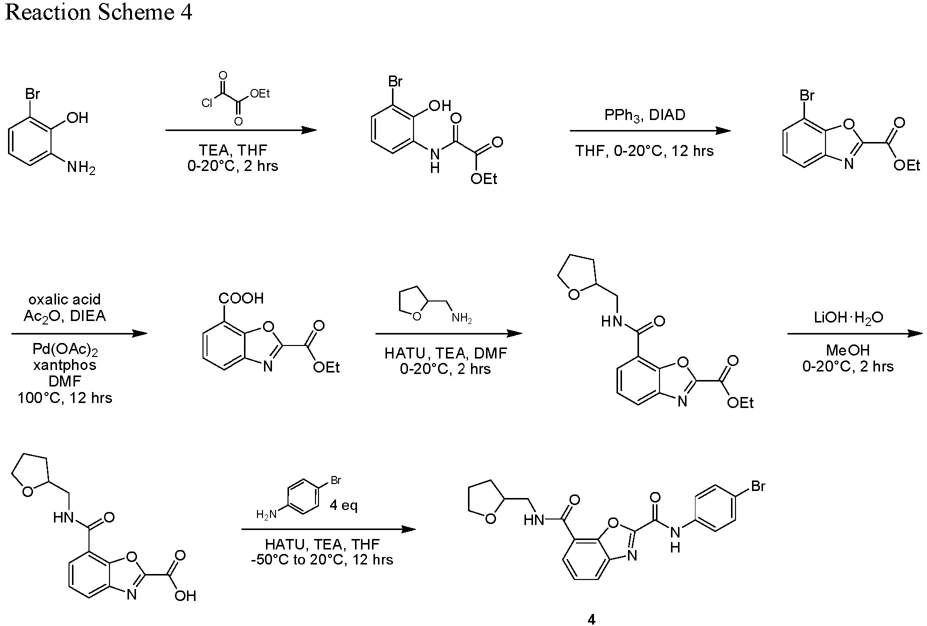

As shown in Reaction Scheme 4, first, ethyl (3-bromo-2-hydroxyphenylcarbamoyl)formate was prepared. To a solution of 2-amino-6-bromophenol (3 g, 15.96 mmol) and TEA (3.23 g, 31.91 mmol) in THF (50 mL) was added ethyl 2-chloro-2-oxo-acetate (2.18 g, 15.96 mmol) drop-wised at 0°C. Then the mixture was stirred at 20°C for 12 hrs under N₂. LC-MS showed the reaction worked well. The reaction mixture was quenched by addition of water (100 mL) at 20°C, and then diluted with ethyl acetate (20 mL). The mixture was extracted with ethyl acetate (3 x 60 mL). The combined organic layer was washed with brine (80 mL), dried over Na₂SO₄, filtered and concentrated under reduced pressure to give ethyl (3-bromo-2-hydroxyphenylcarbamoyl)formate (4.5 g, 88.11% yield) as brown oil which was used for the next step without purification.

Next, ethyl 7-bromobenzo[d]oxazole-2-carboxylate was prepared. To a solution of crude ethyl (3-bromo-2-hydroxyphenylcarbamoyl)formate (4.5 g, 14.06 mmol) and triphenylphosphine (PPh₃) (5.53 g, 21.09 mmol) in THF (50 mL) was added diisopropyl azodicarboxylate (DIAD) (4.26 g, 21.09 mmol, 4.10 mL) drop-wised at 0°C. Then the mixture was stirred at 20°C for 12 hrs under N₂. LC-MS showed the reaction worked well. The reaction mixture was quenched by addition HCl (aqueous, 100 mL, 1 N) at 20°C, and then diluted with ethyl acetate (50 mL) and extracted with ethyl acetate (3 x 50 mL). The combined organic layer was washed with brine (80 mL), dried over Na₂SO₄, filtered and concentrated under reduced pressure to give a residue. The residue was purified by column chromatography (SiO₂, Petroleum ether/Ethyl acetate=20/1 to 10/1) and the eluent was concentrated to give ethyl 7-bromobenzo[d]oxazole-2-carboxylate (1.35 g, 35.56% yield) as light brown solid. The structure of ethyl 7-bromobenzo[d]oxazole-2-carboxylate was confirmed by ¹H NMR (400 MHz, CDCl₃-*d*) δ 7.84 (dd, *J* = 0.8, 8.0 Hz, 1H), 7.69 (dd, *J* = 0.8, 7.9 Hz, 1H), 7.36 (t, *J* = 8.0 Hz, 1H), 4.58 (q, *J* = 7.1 Hz, 2H), 1.54 - 1.47 (m, 3H).

Next, 2-(ethoxycarbonyl)benzo[d]oxazole-7-carboxylic acid was prepared. To a solution of ethyl 7-bromobenzo[d]oxazole-2-carboxylate (1.2 g, 4.00 mmol) and oxalyl dichloride (1.52 g, 12.00 mmol) in DMF (8 mL) was added palladium acetate (Pd(OAc)₂) (89.78 mg, 399.88 umol), 4,5-Bis(diphenylphosphino)-9,9-dimethylxanthene (Xantphos) (462.76 mg, 799.77 umol) DIEA (1.03 g, 8.00 mmol) and acetic anhydride (Ac₂O) (612.35 mg, 6.00 mmol) in order at 20°C. The suspension was degassed and purged with N₂ three times. The mixture was stirred at 100°C for 12 hrs under N₂. LC-MS showed the reaction worked well. The reaction mixture was cooled to 20°C and diluted with water (100 mL). The aqueous layer was adjusted to pH=3 with HCl (1 N). Then the mixture was extracted three times with 50 mL ethyl acetate (3 x 50 mL). The combined organic layers were washed three times with 30 mL brine (3 x 30 mL), dried over Na₂SO₄, filtered and concentrated under reduced pressure to give a residue. The residue was purified by column chromatography (SiO₂, Petroleum ether/Ethyl acetate=10/1 to 5/1) to give 2-(ethoxycarbonyl)benzo[d]oxazole-7-carboxylic acid (850 mg, 64.02% yield) as light brown solid. The structure of 2-(ethoxycarbonyl)benzo[d]oxazole-7-carboxylic acid was confirmed by ¹H NMR (400 MHz, DMSO-*d6*) δ 13.62 (br s, 1H), 8.21 (dd, *J= 0.8,* 7.9 Hz, 1H), 8.11 (dd, *J =* 0.8, 7.7 Hz, 1H), 7.64 (t, *J =* 7.9 Hz, 1H), 4.47 (q, *J =* 7.1 Hz, 2H), 1.39 (t, *J =* 7.1 Hz, 3H).

Next, ethyl 7-((tetrahydrofuran-2-yl)methylcarbamoyl)benzo[d]oxazole-2-carboxylate was prepared. To a solution of 2-(ethoxycarbonyl)benzo[d]oxazole-7-carboxylic acid (300 mg, 1.28 mmol and tetrahydrofuran-2-ylmethanamine (193.53 mg, 1.91 mmol, 197.48 uL) in DMF (5 mL) was added TEA (258.14 mg, 2.55 mmol) and HATU (727.50 mg, 1.91 mmol) in order at 0°C. The mixture was stirred at 20°C for 2 hrs under N₂. LC-MS showed the reaction worked well. The reaction mixture was diluted with water (50 mL), and then the mixture was extracted three times with 30 mL ethyl acetate (3 x 30 mL). The combined organic layers were washed three times with 30 mL brine (3 x 30 mL), dried over Na₂SO₄, filtered and concentrated under reduced pressure to give ethyl 7-((tetrahydrofuran-2-yl)methylcarbamoyl)benzo[d]oxazole-2-carboxylate (450 mg, crude) as brown gum which was used for the next step directly without purification.

Next, 7-((tetrahydrofuran-2-yl)methylcarbamoyl)benzo[d]oxazole-2-carboxylic acid was prepared. To a solution of ethyl 7-((tetrahydrofuran-2-yl)methylcarbamoyl)benzo[d]oxazole-2-carboxylate (200 mg, 565.46 umol) in MeOH (5 mL) was added a solution of LiOH.H₂O (35.59 mg, 848.19 umol) in MeOH (0.5 mL) drop-wise at 0°C. Then, the mixture was stirred at 0°C for 2 hrs. LC-MS showed the reaction worked well, starting material remained and one peak with desired Mass was detected. To the mixture was added trifluoroacetic acid (TFA) (0.15 mL), the mixture was concentrated under reduced pressure to give the crude product 7-((tetrahydrofuran-2-yl)methylcarbamoyl)benzo[d]oxazole-2-carboxylic acid (200 mg, crude, unstable under alkaline condition) as light brown gum which was used in the next step directly.

Finally, N2-(4-bromophenyl)-N7-((tetrahydrofuran-2-yl)methyl)benzo[d]oxazole-2,7-dicarboxamide (**Compound 4**) was prepared. To a solution of 7-((tetrahydrofuran-2-yl)methylcarbamoyl)benzo[d]oxazole-2-carboxylic acid (200 mg, 689.01 umol) and 4-bromoaniline (474.10 mg, 2.76 mmol) in THF (5 mL) was added TEA (348.60 mg, 3.45 mmol) and HATU (785.95 mg, 2.07 mmol) in order at -50°C. Then, the mixture was stirred at 20°C for 12 hrs under N₂. LC-MS showed the reaction worked well, starting material remained and one peak with desired Mass was detected. The reaction mixture was dried by N₂ and diluted with DMF (3 mL). The mixture was purified by prep-HPLC under TFA system and lyophilized directly to give N2-(4-bromophenyl)-N7-((tetrahydrofuran-2-yl)methyl)benzo[d]oxazole-2,7-dicarboxamide (**Compound 4**) (26.7 mg, yield 8.55%) as off white solid. The Compound 4 structure was confirmed by ¹H NMR (400 MHz, CDCl₃-*d*) δ 9.07 (s, 1H), 8.28 (dd, *J =* 0.7, 7.7 Hz, 1H), 7.99 (dd, *J=* 0.8, 8.0 Hz, 1H), 7.70 - 7.48 (m, 6H), 4.33 - 4.19 (m, 1H), 4.15 - 3.99 (m, 1H), 3.93 - 3.76 (m, 2H), 3.68 - 3.47 (m, 1H), 2.19 - 1.86 (m, 3H), 1.80 - 1.65 (m, 1H).

### Synthesis Example 5: Synthesis of Compound 5

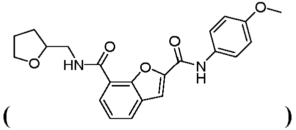

Compound 5 was synthesized according to the following Reaction Scheme 5.

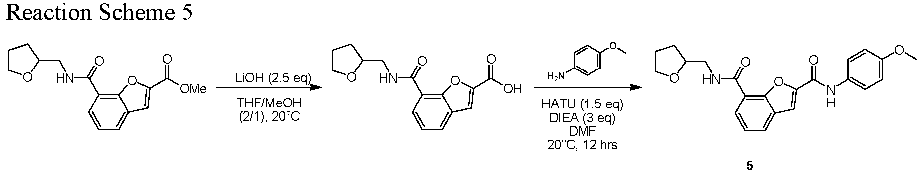

As shown in Reaction Scheme 5, first, 7-((tetrahydrofuran-2-yl)methylcarbamoyl)benzofuran-2-carboxylic acid was prepared. To a mixture of methyl 7-((tetrahydrofuran-2-yl)methylcarbamoyl)benzofuran-2-carboxylate (200 mg, 659.39 umol) in tetrahydrofuran (4 mL) and methyl alcohol (2 mL) was added a solution of LiOH.H₂O (69.18 mg, 1.65 mmol) in water (1 mL) drop-wise at 20°C under nitrogen. The mixture was stirred at 20°C for 2 hours. LC-MS showed the reaction was completed. The mixture was lyophilized to afford 7-((tetrahydrofuran-2-yl)methylcarbamoyl)benzofuran-2-carboxylic acid (190.75 mg, 100% yield) as brown solid. The structure of 7-((tetrahydrofuran-2-yl)methylcarbamoyl)benzofuran-2-carboxylic acid was confirmed by ¹H NMR: (400MHz, DMSO-*d6*) δ ppm 8.17 (br t, *J*=5.69 Hz, 1 H) 7.77 (ddd, *J*=9.94, 7.69, 1.25 Hz, 2 H) 7.50 - 7.66 (m, 1 H) 7.30 (t, *J*=7.63 Hz, 1 H) 7.09 (s, 1 H) 3.99 - 4.10 (m, 1 H) 3.84 - 3.95 (m, 1 H) 3.59 - 3.69 (m, 1 H) 3.45 - 3.55 (m, 2 H) 1.75 - 2.01 (m, 3 H) 1.60 - 1.69 (m, 1 H).

Next, N7-((tetrahydrofuran-2-yl)methyl)-N2-(4-methoxyphenyl)benzofuran-2,7-dicarboxamide(**Compound 5**) was prepared. To a mixture of 7-((tetrahydrofuran-2-yl)methylcarbamoyl)benzofuran-2-carboxylic acid (190 mg, 656.80 umol), 4-methoxyaniline (97.06 mg, 788.16 umol,) and N,N-diisopropylethylamine (254.65 mg, 1.97 mmol,) in dimethylformamide (4 mL) was added 2-(7-azabenzotriazol-1-yl)-N, N, N', N'-tetramethyluronium hexafluorophosphate (374.60 mg, 985.20 umol,) in portion at 0°C under nitrogen. The mixture was stirred at 20°C for 12 hours. LC-MS showed the reaction was completed. The reaction mixture was filtered, and the filtrate was collected. The filtrate was purified by prep-HPLC (NH₄HCO₃) and lyophilized to give N7-((tetrahydrofuran-2-yl)methyl)-N2-(4-methoxyphenyl)benzofuran-2,7-dicarboxamide (**Compound 5**) (90.1 mg, 34.29% yield) as a yellow solid. The Compound 5 structure was confirmed by ¹H NMR: (400MHz, DMSO-d6) δ 10.35 (s, 1 H) 8.47 (t, *J*=5.81 Hz, 1 H) 7.98 (dd, *J*=7.78, 0.99 Hz, 1 H) 7.92 (dd, *J*=7.67, 0.88 Hz, 1 H) 7.81 (s, 1 H) 7.65 - 7.74 (m, 2 H)7.47 (t, *J*=7.78 Hz, 1 H) 6.94 - 7.03 (m, 2 H) 4.10 (quin, *J*=6.19 Hz, 1 H) 3.79 - 3.85 (m, 1 H) 3.76 (s, 3 H) 3.63 (q, *J*=7.38 Hz, 1 H) 3.43 - 3.56 (m, 2 H) 1.92 - 2.03 (m, 1 H) 1.76 - 1.92 (m, 2 H) 1.64 - 1.76 (m, 1 H).

### Synthesis Example 6: Synthesis of Compound 6

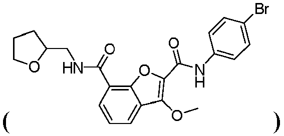

Compound 6 was synthesized according to the following Reaction Scheme 6.

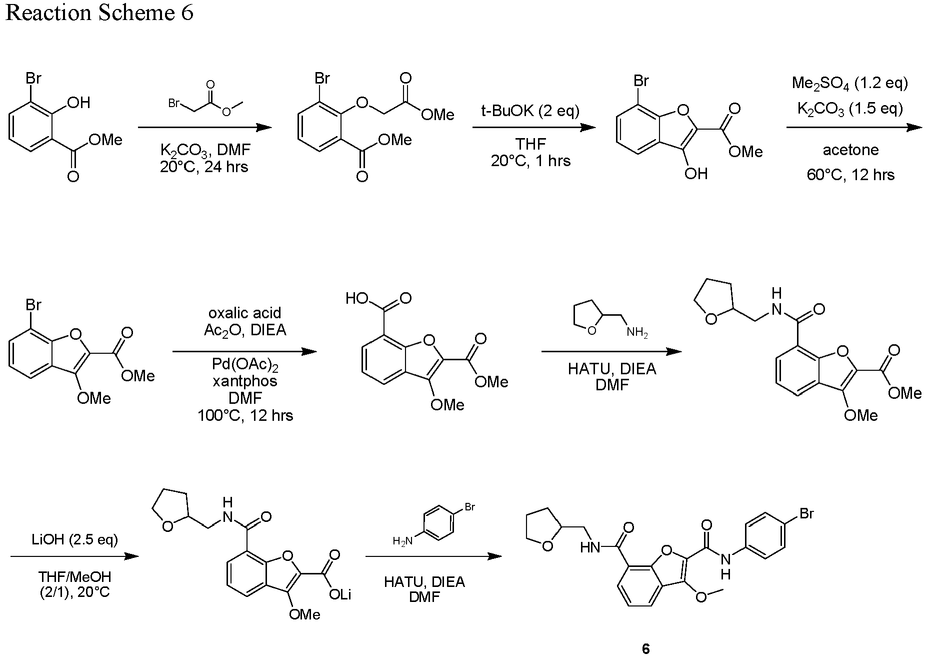

As shown in Reaction Scheme 6, first, methyl 2-((methoxycarbonyl)methoxy)-3-bromobenzoate was prepared. To a mixture of methyl 3-bromo-2-hydroxybenzoate (2 g, 8.66 mmol) and K₂CO₃ (1.79 g, 12.98 mmol) in DMF (20 mL) was drop-wise added BrCH₂CO₂Me (1.59 g, 10.39 mmol) at 20°C. The resulting reaction mixture was stirred at 20°C for 12 hours. LC-MS showed some starting material had remained. Then BrCH₂CO₂Me (264.84 mg, 1.73 mmol, 163.48 uL) was drop-wise added. And the reaction mixture was stirred at 20°C for another 12 hours. LC-MS showed 7% of starting material had remained and 43% of desired product was detected. The reaction mixture was diluted with water (100 ml) and ethyl acetate (100 ml), the biphasic mixture was cut. The organic phase was washed three times with 40 mL brine (3 x 40 ml). The combined organic phase was dried over Na₂SO₄, then concentrated to afford crude product. The crude product was purified by silica column eluted with ethyl acetate in petroleum ether from 20% to 25% to afford methyl 2-((methoxycarbonyl)methoxy)-3-bromobenzoate (2.5 g, 90.52% yield) as colorless oil. The structure of methyl 2-((methoxycarbonyl)methoxy)-3-bromobenzoate was confirmed by ¹H NMR (400 MHz, CDCl₃-*d*) δ 7.77 (dd, *J* = 1.7, 7.8 Hz, 1H), 7.73 (dd, *J =* 1.7, 7.9 Hz, 1H), 7.10 (t, *J =* 7.9 Hz, 1H), 4.73 (s, 2H), 3.90 (s, 3H), 3.85 (s, 3H).

Next, methyl 7-bromo-3-hydroxybenzofuran-2-carboxylate was prepared. To a solution of methyl 2-((methoxycarbonyl)methoxy)-3-bromobenzoate (1 g, 3.13 mmol) in THF (20 mL) was added potassium tert-butoxide (t-BuOK) (703.38 mg, 6.27 mmol) at 0°C. The resulting reaction solution was stirred at 20°C for 0.5 hr. The reaction mixture turned to a suspension. LC-MS showed the desired product was detected. The reaction was quenched with NH₄Cl (25 mL). The reaction was adjusted to pH=5-6 with 1 N HCl, then extracted three times with 100 mL ethyl acetate (3 x 100 ml), the combined organic phase was washed with brine (40 ml), dried over Na₂SO₄ and concentrated to afford crude product. The crude product was purified by silica column eluted with ethyl acetate in petroleum ether from 15% to 20% to afford methyl 7-bromo-3-hydroxybenzofuran-2-carboxylate (0.86 g, 75.92% yield) as light-yellow solid. The structure of the methyl 7-bromo-3-hydroxybenzofuran-2-carboxylate was confirmed by ¹H NMR: (400 MHz, CDCl₃-*d*) δ 8.12 (br s, 1H), 7.69 (dd, *J =* 7.8, 12.1 Hz, 2H), 7.20 (t, *J =* 7.8 Hz, 1H), 4.04 (s, 3H).

Next, methyl 7-bromo-3-methoxybenzofuran-2-carboxylate as prepared. To a solution of methyl 7-bromo-3-hydroxybenzofuran-2-carboxylate (0.65 g, 2.28 mmol), K₂CO₃ (472.28 mg, 3.42 mmol) in acetone (15 mL) was added dimethyl sulfate (344.80 mg, 2.73 mmol) at 20°C. The resulting reaction solution was stirred at 60°C for 12 hours. LC-MS showed the desired product was detected. The reaction was quenched with NH₄Cl (25 mL, and the reaction mixture was extracted twice with 30 mL ethyl acetate (2 x 30 mL), the organic phase was washed with brine (10 mL), dried over Na₂SO₄ and concentrated to afford crude product. The crude product was purified by silica column eluted with ethyl acetate in petroleum ether from 15% to 20% to afford methyl 7-bromo-3-methoxybenzofuran-2-carboxylate (0.7 g, 97.00% yield) as light-yellow solid. The structure of methyl 7-bromo-3-methoxybenzofuran-2-carboxylate was confirmed by ¹H NMR: (400 MHz, CDCl₃-*d*) δ 7.76 - 7.71 (m, 1H), 7.64 (dd, *J =* 0.9, 7.7 Hz, 1H), 7.17 (t, *J =* 7.8 Hz, 1H), 4.27 (s, 3H), 3.99 (s, 3H).

Next, 2-(methoxycarbonyl)-3-methoxybenzofuran-7-carboxylic acid was prepared. To a mixture of methyl 7-bromo-3-methoxybenzofuran-2-carboxylate (200 mg, 666.46 umol), Ac₂O (102.06 mg, 999.68 umol), oxalic acid (HOOCCOOH) (94.04 mg, 999.68 umol), DIEA (172.27 mg, 1.33 mmol), Xantphos (38.56 mg, 66.65 umol) in DMF (2 mL) was added Pd(OAc)₂ (14.96 mg, 66.65 umol) at 20°Cunder N₂. The resulting reaction solution was stirred at 100°Cfor 12 hours under N₂. LC-MS showed the desired product was detected. The reaction mixture was diluted with water (20 mL) and ethyl acetate (20 mL), the resulting biphasic mixture was cut, and the organic phase was washed three times with brine (3 x 10 mL), dried over Na₂SO₄ and concentrated to afford crude 2-(methoxycarbonyl)-3-methoxybenzofuran-7-carboxylic acid (150 mg, 89.96% yield) as yellow gum. The structure of 2-(methoxycarbonyl)-3-methoxybenzofuran-7-carboxylic acid was confirmed by ¹H NMR: (400 MHz, CDCl₃-*d*) δ 8.22 - 8.16 (m, 1H), 7.99 (br d, *J* = 7.9 Hz, 1H), 7.40 - 7.35 (m, 1H), 4.28 (s, 3H), 3.98 (s, 3H).

Next, methyl 7-((tetrahydrofuran-2-yl)methylcarbamoyl)-3-methoxybenzofuran-2-carboxylate was prepared. To a mixture of 2-(methoxycarbonyl)-3-methoxybenzofuran-7-carboxylic acid (135.00 mg, 539.56 umol), TEA (163.79 mg, 1.62 mmol), HATU (307.74 mg, 809.34 umol) in DMF (4 mL) was added tetrahydrofuran-2-ylmethanamine (81.86 mg, 809.34 umol) at 0°C under N2. The resulting reaction solution was stirred at 20°C for 12 hours under N₂. LC-MS showed the desired product was detected. The reaction mixture was diluted with water (20 mL) and ethyl acetate (20 mL), the resulting biphasic mixture was cut, and the organic phase was washed three times with brine (3 x 10 mL), dried over Na₂SO₄ and concentrated to afford methyl 7-((tetrahydrofuran-2-yl)methylcarbamoyl)-3-methoxybenzofuran-2-carboxylate (0.1 g, 55.60% yield) as yellow gum. The structure of methyl 7-((tetrahydrofuran-2-yl)methylcarbamoyl)-3-methoxybenzofuran-2-carboxylate was confirmed by ¹H NMR: (400 MHz, CDCl₃-*d*) δ 8.22 (dd, *J =* 1.3, 7.6 Hz, 1H), 7.83 (dd, *J* = 1.3*,* 7.9 Hz, 1H), 7.36 - 7.31 (m, 1H), 4.22 (s, 3H), 4.11 (dd, *J* = 4.1, 6.7 Hz, 1H), 3.92 (s, 3H), 3.79 (d, *J =* 7.5 Hz, 1H), 3.76 - 3.69 (m, 2H), 3.56 (td, *J =* 5.9, 13.8 Hz, 1H), 2.01 - 1.86 (m, 4H).

Next, lithium 7-((tetrahydrofuran-2-yl)methylcarbamoyl)-3-methoxybenzofuran-2-carboxylate was prepared. To a mixture of methyl 7-((tetrahydrofuran-2-yl)methylcarbamoyl)-3-methoxybenzofuran-2-carboxylate (0.1 g, 300.00 umol) in MeOH (1 mL), H₂O (0.5 mL), THF (1 mL) was added LiOH.H₂O (62.95 mg, 1.50 mmol) at 0°C. The resulting reaction solution was stirred at 20°C for 12 hours. LC-MS showed desired product was detected. The reaction mixture was concentrated to afford lithium 7-((tetrahydrofuran-2-yl)methylcarbamoyl)-3-methoxybenzofuran-2-carboxylate (80 mg, 81.99% yield) as yellow gum. The structure of lithium 7-((tetrahydrofuran-2-yl)methylcarbamoyl)-3-methoxybenzofuran-2-carboxylate was confirmed by ¹H NMR: (400 MHz, MeOH-d4) δ 8.05 (dd, *J =* 1.1, 7.6 Hz, 1H), 7.90 (dd, *J =* 1.3, 7.8 Hz, 1H), 7.38 (t, *J =* 7.8 Hz, 1H), 4.36 (t, *J =* 7.1 Hz, 1H), 4.17 (s, 3H), 3.68 - 3.52 (m, 4H), 2.08 - 1.93 (m, 4H).

Finally, N2-(4-bromophenyl)-N7-((tetrahydrofuran-2-yl)methyl)-3-methoxybenzofuran-2,7-dicarboxamide (**Compound 6**):To a mixture of lithium 7-((tetrahydrofuran-2-yl)methylcarbamoyl)-3-methoxybenzofuran-2-carboxylate (80 mg, 172.18 umol), TEA (26.13 mg, 258.27 umol and HATU (98.20 mg, 258.27 umol) in DMF (2 mL) was added 4-bromoaniline (35.54 mg, 206.62 umol) at 0°C. The resulting reaction solution was stirred at 20°C for 12 hours. LC-MS showed desired product was detected. The reaction mixture was concentrated to afford crude product. The crude product was purified by prep-HPLC and lyophilized to afford N2-(4-bromophenyl)-N7-((tetrahydrofuran-2-yl)methyl)-3-methoxybenzofuran-2,7-dicarboxamide (**Compound 6**) (23.1 mg, 27.27% yield) as off white solid. The structure of Compound 6 was confirmed by ¹H NMR: (400 MHz, DMSO-d6) δ 10.19 (s, 1H), 8.46 (t, *J* = 5.8 Hz, 1H), 8.04 (dd, *J* = 1.1, 7.9 Hz, 1H), 7.96 (dd, *J* = 1.1, 7.6 Hz, 1H), 7.78 - 7.73 (m, 2H), 7.62 - 7.56 (m, 2H), 7.47 (t, *J =* 7.7 Hz, 1H), 4.25 (s, 3H), 4.08 (t, *J =* 6.1 Hz, 1H), 3.81 - 3.73 (m, 1H), 3.62 - 3.55 (m, 1H), 3.48 (q, *J =* 5.5 Hz, 2H), 2.01 - 1.89 (m, 1H), 1.88 - 1.72 (m, 2H), 1.71 - 1.60 (m, 1H).

### Synthesis Example 7: Synthesis of Compound 7

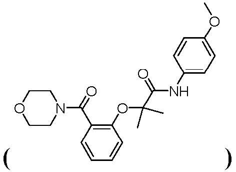

Compound 7 was synthesized according to the following Reaction Scheme 7.

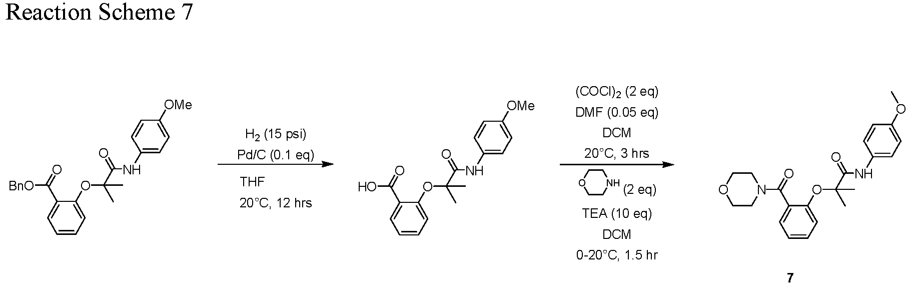

As shown in Reaction Scheme 7, to a solution of 2-(2-(4-methoxyphenylcarbamoyl)propan-2-yloxy)benzoic acid (100 mg, 303.63 umol) in DCM (2 mL) was added DMF (1.11 mg, 15.18 umol) at 20°C. Then, oxalyl chloride (77.08 mg, 607.26 umol) in DCM (1 mL) was added to the mixture at 0°C. The reaction solution was warmed to 20°C and stirred at 20°C for 3 hours. TLC indicated starting material was consumed completely and one new spot was formed. The reaction solution was quenched with water (20 mL) and extracted four times with 10 mL chloroform (4 x 10 mL). The combined organic was washed with saturated brine (10 mL), dried over anhydrous sodium sulfate, filtered and concentrated under reduced pressure to give the residue. To a solution of morpholine (52.60 mg, 603.81 umol) in DCM (2 mL) was added TEA (305.50 mg, 3.02 mmol) at 20°C. The residue (105 mg, 301.90 umol) in DCM (2 mL) was added to the previous mixture at 0°C. The reaction solution was warmed to 20°C and stirred at 20°C for 1.5 hours. LC-MS showed starting material was consumed and 91.2% of the desired compound was detected. Most volatile solvents were removed under reduced pressure to give crude product. The crude product was purified by prep-HPLC and lyophilized to give **Compound 7** (58.6 mg, 48.28% yield) as a white solid. The structure of Compound 7 was confirmed by ¹H NMR: (400MHz, DMSO-d6) δ 9.67 (s, 1 H) 7.52 - 7.60 (m, 2 H) 7.32 - 7.40 (m, 1 H) 7.28 (dd, *J*=7.53, 1.63 Hz, 1 H) 7.07 - 7.13 (m, 1 H) 7.04 (d, *J*=8.16 Hz, 1 H) 6.86 - 6.93 (m, 2 H) 3.72 (s, 3 H) 3.64 (br s, 3 H) 3.55 (br t, *J*=4.52 Hz, 3 H) 3.26 (br d, *J*=4.77 Hz, 2 H) 1.44 - 1.63 (m, 6 H).

### Synthesis Example 8: Synthesis of Compound 8

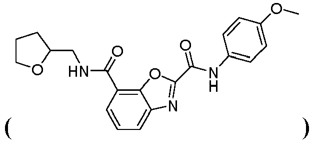

Compound 8 was synthesized according to the following Reaction Scheme 8.

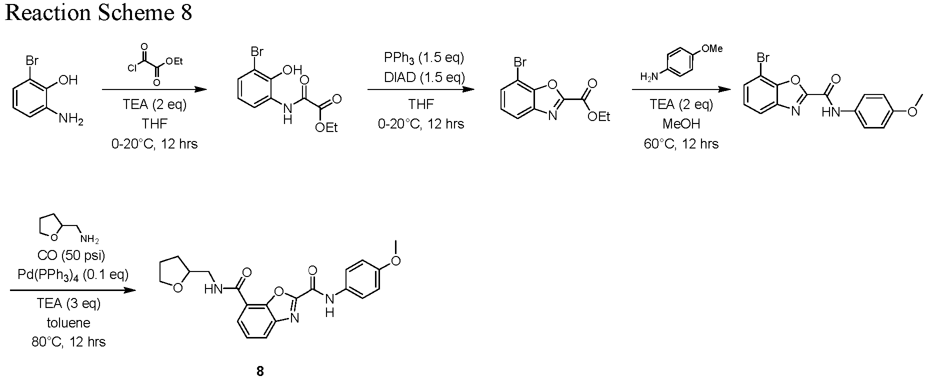

As shown in Reaction Scheme 8, to a solution of 7-bromo-N-(4-methoxyphenyl)benzo[d]oxazole-2-carboxamide (0.11 g, 301.01 umol) in toluene (10 mL) was added tetrahydrofuran-2 -ylmethanamine (36.54 mg, 361.21 umol), TEA (91.38 mg, 903.03 umol) and Pd (PPh₃)₄ (34.78 mg, 30.10 umol) at 20°C. The suspension was degassed and purged with CO three times. The reaction was stirred for 12 hours at 80°C under CO (50 psi). LC-MS showed the reaction was completed. The reaction was concentrated under reduced pressure at 40°C. The residue was purified by prep-HPLC and lyophilized to give N7-((tetrahydrofuran-2-yl)methyl)-N2-(4-methoxyphenyl)benzo[d]oxazole-2,7-dicarboxamide (**Compound 8**) (32.7 mg, 26.46% yield) as a green solid. The structure of Compound 8 was confirmed by ¹H NMR: (400 MHz, DMSO-d6) δ ppm 11.11 (s, 1 H) 8.46 (t, *J*=5.65 Hz, 1 H) 8.08 (dd, *J*=8.03, 1.00 Hz, 1 H) 7.90 (dd, *J*=7.65, 0.88 Hz, 1 H) 7.74 - 7.83 (m, 2 H) 7.58 - 7.63 (m, 1 H) 6.93 - 7.03 (m, 2 H) 4.05 (quin, *J*=6.09 Hz, 1 H) 3.83 - 3.90 (m, 1 H) 3.76 (s, 3 H) 3.62 - 3.71 (m, 1 H) 3.39 - 3.53 (m, 2 H) 1.80 - 2.01 (m, 3 H) 1.61 - 1.72 (m, 1 H).

### Synthesis Example 9: Synthesis of Compound 9

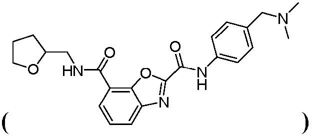

Compound 9 was synthesized according to the following Reaction Scheme 9.

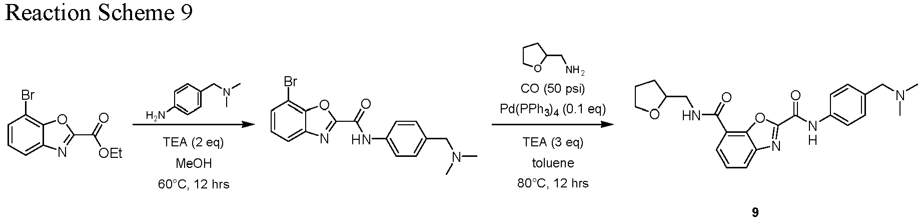

As shown in Reaction Scheme 9, first, 7-bromo-N-(4-((dimethylamino)methyl)phenyl)benzo[d]oxazole-2-carboxamide was prepared. To a solution of ethyl 7-bromobenzo[d]oxazole-2-carboxylate (0.2 g, 666.47 umol) in EtOH (0.5 mL) was added 4-[(dimethylamino) methyl]aniline (500.59 mg, 3.33 mmol) and TEA (269.76 mg, 2.67 mmol) at 20°C. The reaction was stirred for 36 hours at 60°C. LC-MS showed the reaction was completed. The reaction was concentrated under reduced pressure at 40°C. The residue was purified by pre-HPLC and lyophilized to give 7-bromo-N-(4-((dimethylamino)methyl)phenyl)benzo[d]oxazole-2-carboxamide (0.17 g, 61.34% yield) as a yellow solid.

Next, N2-(4-((dimethylamino)methyl)phenyl)-N7-((tetrahydrofuran-2-yl)methyl)benzo[d]oxazole-2,7-dicarboxamide (**Compound 9**) was prepared. To a solution of 7-bromo-N-(4-((dimethylamino)methyl)phenyl)benzo[d]oxazole-2-carboxamide (0.17 g, 408.84 umol) in toluene (15 mL) was added tetrahydrofuran-2 -ylmethanamine (49.62 mg, 490.61 umol) and TEA (124.11 mg, 1.23 mmol), Pd (PPh₃)₄ (47.24 mg, 40.88 umol) at 20°C. The suspension was degassed and purged with CO three times. The reaction mixture was stirred for 12 hours at 80°C under CO (50 psi). LC-MS showed the reaction was completed. The reaction was concentrated under reduced pressure at 30°C. The residue was purified by prep-HPLC and lyophilized to give N2-(4-((dimethylamino)methyl)phenyl)-N7-((tetrahydrofuran-2-yl)methyl)benzo[d]oxazole-2,7-dicarboxamide (**Compound 9**) (68.4 mg, 39.32% yield) as a brown solid. The Compound 9 structure was confirmed by ¹H NMR: (400 MHz, DMSO-d6) δ ppm 10.93 - 11.37 (m, 1 H) 8.46 (br t, J=5.75 Hz, 1 H) 8.09 (dd, J=8.00, 0.88 Hz, 1 H) 7.87 - 7.94 (m, 1 H) 7.81 (d, *J*=8.50 Hz, 2 H) 7.61 (t, *J*=7.82 Hz, 1 H) 7.31 (d, *J*=8.38 Hz, 2 H) 4.01 - 4.10 (m, 1 H) 3.82 - 3.90 (m, 1 H) 3.66 (q, *J*=7.46 Hz, 1 H) 3.34 - 3.53 (m, 4 H) 2.14 (br s, 6 H) 1.89 - 2.03 (m, 2 H) 1.78 - 1.87 (m, 1 H) 1.61 - 1.76 (m, 1 H).

### Synthesis Example 10: Synthesis of Compound 10

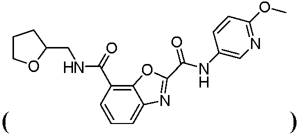

Compound 10 was synthesized according to the following Reaction Scheme 10.

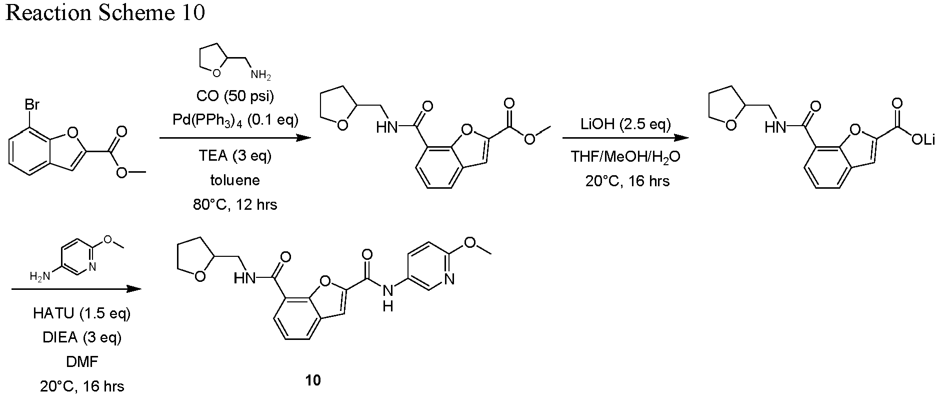

As shown in Reaction Scheme 10, first, methyl 7-((tetrahydrofuran-2-yl)methylcarbamoyl)benzofuran-2-carboxylate was prepared. To a solution of compound methyl 7-bromobenzofuran-2-carboxylate (900 mg, 3.53 mmol), tetrahydrfuran-2-ylmethanamine (1.07 g, 10.59 mmol, 1.09 mL) and triethylamine (1.07 g, 10.59 mmol, 1.47 mL) in toluene (18 mL) was added Pd(PPh₃)₄ (407.74 mg, 352.85 umol) under nitrogen atmosphere. The suspension was degassed and purged with CO three times. The mixture was stirred under CO (50 Psi) at 80°C for 16 hours. LC-MS showed the reaction was completed. The reaction mixture was filtered, and the filtrate was concentrated under reduced pressure to give a residue. The residue was purified by flash silica gel chromatography (ISCO^{®}; 25 g SEPAFLASH^{®} Silica Flash Column, Eluent of 0~50% ethyl acetate/petroleum ether gradient @ 75 mL/min) to give compound methyl 7-((tetrahydrofuran-2-yl)methylcarbamoyl)benzofuran-2-carboxylate (220 mg, 20.56% yield) as a yellow solid. The structure of methyl 7-((tetrahydrofuran-2-yl)methylcarbamoyl)benzofuran-2-carboxylate was confirmed by ¹H NMR: (400 MHz, *DMSO-d6*) δ 8.29 (br t, *J*=5.38 Hz, 1 H) 7.96 (dd, *J*=7.88, 1.00 Hz, 1 H) 7.87 (s, 1 H) 7.85 (dd, *J*=7.50, 1.00 Hz, 1 H) 7.21 - 7.30 (m, 1 H) 4.00 - 4.07 (m, 1 H) 3.92 (s, 3 H) 3.83 - 3.90 (m, 1 H) 3.64 - 3.71 (m, 1 H) 3.38 - 3.53 (m, 2 H) 1.77 - 1.99 (m, 3 H) 1.63 - 1.73 (m, 1 H).

Next, lithium 7-((tetrahydrofuran-2-yl)methylcarbamoyl)benzofuran-2-carboxylate was prepared. To a solution of compound methyl 7-((tetrahydrofuran-2-yl)methylcarbamoyl)benzofuran-2-carboxylate (200 mg, 659.39 umol) in methane (2 mL) and tetrahydrofuran (4 mL) was added a solution of LiOH.H₂O (69.17 mg, 1.65 mmol) in water (1 mL) at 20°C. The resulting mixture was stirred at 20°C for 16 hours. LC-MS showed the reaction was completed. The reaction mixture was lyophilized to give lithium 7-((tetrahydrofuran-2-yl)methylcarbamoyl)benzofuran-2-carboxylate (190 mg, 99.61% yield) as a yellow solid. Thes structure of lithium 7-((tetrahydrofuran-2-yl)methylcarbamoyl)benzofuran-2-carboxylate was confirmed by ¹H NMR: (400 MHz, DMSO-d6) δ 1.61 - 1.69 (m, 1 H) 1.74 - 1.84 (m, 1 H) 1.86 - 2.01 (m, 2 H) 3.48 (br s, 2 H) 3.63 (br d, *J*=6.80 Hz, 1 H) 3.91 (br d, *J*=7.02 Hz, 1 H) 4.03 (br t, *J*=6.03 Hz, 1 H) 7.04 (s, 1 H) 7.25 - 7.32 (m, 1 H) 7.71 - 7.80 (m, 2 H) 8.14 (br s, 1 H).

Finally, N7-((tetrahydrofuran-2-yl)methyl)-N2-(6-methoxypyridin-3-yl)benzofuran-2,7-dicarboxamide (**Compound 10**) was prepared. To a solution of lithium 7-((tetrahydrofuran-2-yl)methylcarbamoyl)benzofuran-2-carboxylate (100 mg, 345.68 umol) in dimethyl formamide (2.0 mL) was added diisopropylethylamine (268.06 mg, 2.07 mmol, 361.26 uL) and 2-(7-Azabenzotriazol -1-yl)-N,N,N',N'-tetramethyluronium hexafluorophosphate (197.16 mg, 518.52 umol) at 0°C and stirred for 30 mins. Then 6-methoxypyridin-3-amine (47.20 mg, 380.25 umol) was added to the solution at 0°C. The mixture was stirred at 20°C for 2 hours. LC-MS showed the reaction was completed. The reaction mixture was concentrated under reduced pressure to give a residue which was purified by prep-HPLC to give N7-((tetrahydrofuran-2-yl)methyl)-N2-(6-methoxypyridin-3-yl)benzofuran-2,7-dicarboxamide (**Compound 10**) (20.8 mg, 15.03% yield) as a white solid. The structure of Compound 10 was confirmed by ¹H NMR: (400 MHz, DMSO-d6) δ 1.61 - 1.75 (m, 1 H) 1.75 - 1.92 (m, 2 H) 1.92 - 2.03 (m, 1 H) 3.49 (br d, *J*=4.60 Hz, 2 H) 3.59 - 3.67 (m, 1 H) 3.78 - 3.83 (m, 1 H) 3.86 (s, 3 H) 4.09 (br s, 1 H) 6.91 (d, *J*=8.99 Hz, 1 H) 7.47 (t, *J*=7.67 Hz, 1 H) 7.84 (s, 1 H) 7.92 (br d, *J*=7.23 Hz, 1 H) 7.96 - 8.01 (m, 1 H) 8.04 (br s, 1 H) 8.43 (br s, 1 H) 8.54 (br s, 1 H) 10.54 (s, 1 H).

### Synthesis Example 11: Synthesis of Compound 11

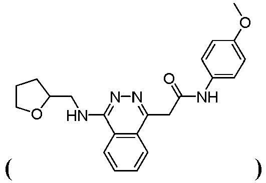

Compound 11 was synthesized according to the following Reaction Scheme 11.

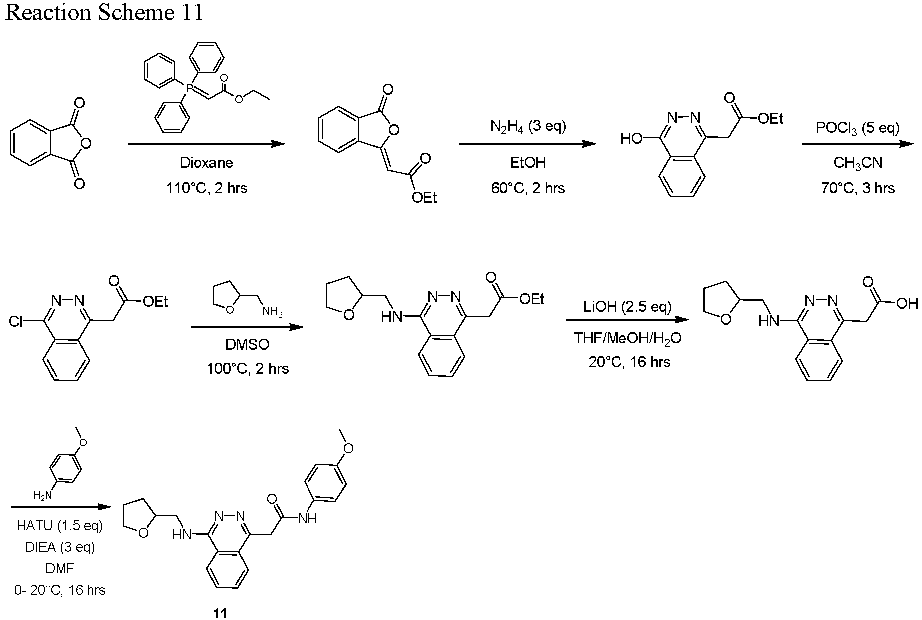

As shown in Reaction Scheme 11, first, (Z)-ethyl 2-(1-oxoisobenzofuran-3(1H)-ylidene)acetate was prepared. To a solution of isobenzofuran-1,3-dione (2 g, 13.50 mmol) in dioxane (32 mL) was added ethyl 2-(triphenyl-λ5-phosphanylidene) acetate (5.17 g, 14.85 mmol) at 20°C. The mixture was stirred at 110°C for 2 hours. TLC showed the reaction was completed. The reaction mixture was quenched by addition of water (100 mL) and extracted three times with 20 mL ethyl acetate (20 mL x 3). The combined organic layers were washed with brine (25 mL), dried over anhydrous sodium sulfate, filtered and concentrated under reduced pressure to give a residue. The residue was purified by flash silica gel chromatography (ISCO^{®}; 40 g SEPAFLASH^{®} Silica Flash Column, Eluent of 0~10% ethyl acetate/petroleum ether gradient @ 36 mL/min) to give (Z)-ethyl 2-(1-oxoisobenzofuran-3(1H)-ylidene)acetate (2.12 g, 71.95% yield) as a white solid. The structure of (Z)-ethyl 2-(1-oxoisobenzofuran-3(1H)-ylidene)acetate was confirmed by ¹H NMR: (400 MHz, CDCl₃-*d*) δ 1.37 (t, *J*=7.13 Hz, 3 H) 4.30 (q, *J*=7.23 Hz, 2 H) 6.16 (s, 1 H) 7.67 - 7.75 (m, 1 H) 7.83 (td, *J*=7.67, 1.10 Hz, 1 H) 7.97 (d, *J*=7.67 Hz, 1 H) 9.06 (d, *J*=8.11 Hz, 1 Hz, 1 H).

Next, ethyl 2-(1-hydroxyphthalazin-4-yl)acetate was prepared. To a mixture of (Z)-ethyl 2-(1-oxoisobenzofuran-3(1H)-ylidene)acetate (2.46 g, 11.27 mmol) in ethanol (50 mL) was added hydrazine hydrate (N₂H₄) (1.47 g, 28.78 mmol, 1.43 mL, 98% purity) at 20°C and stirred for 16 hours at 80°C. LC-MS showed the reaction was completed. The reaction mixture was concentrated under reduced pressure to give ethyl 2-(1-hydroxyphthalazin-4-yl)acetate (2.6 g, 99.31% yield) as a white solid. The structure of ethyl 2-(1-hydroxyphthalazin-4-yl)acetate was confirmed by ¹H NMR (400 MHz, DMSO-*d6*)6 12.63 (br s, 1 H) 8.24 - 8.28 (m, 1 H) 7.92 - 7.95 (m, 1 H) 7.84 - 7.88 (m, 2 H) 4.25 (br d, J=1.00 Hz, 1 H) 4.11 (q, *J*=7.13 Hz, 2 H) 4.05 (s, 2 H) 1.17 (t, *J*=7.07 Hz, 3 H).

Next, ethyl 2-(1-chlorophthalazin-4-yl)acetate was prepared. To a solution of ethyl 2-(1-hydroxyphthalazin-4-yl)acetate(600 mg, 2.58 mmol) in methyl cyanide (MeCN) (20 mL) was added POCl₃ (1.98 g, 12.92 mmol, 1.20 mL) at 20°C. The mixture was stirred at 70°C for 1 hour. LC-MS showed the reaction was completed. The reaction mixture was quenched by addition water (30 mL) at 20°C and extracted three times with 20 mL ethyl acetate (20 mL x 3). The combined organic layers were washed with brine (30 mL), dried over anhydrous sodium sulfate, filtered and concentrated under reduced pressure to give a residue which was purified by flash silica gel chromatography (ISCO^{®}; 25 g SEPAFLASH^{®} Silica Flash Column, Eluent of 0~8% ethyl acetate/petroleum ether gradient @ 35 mL/min) to give ethyl 2-(1-chlorophthalazin-4-yl)acetate (300 mg, 46.32% yield) as a yellow solid. The structure of ethyl 2-(1-chlorophthalazin-4-yl)acetate was confirmed by ¹H NMR: (400 MHz, MeOH-d4) δ 1.19 - 1.33 (m, 3 H) 4.18 (quin, J=7.07 Hz, 2 H) 4.86 - 4.87 (m, 2 H) 7.80 (ddd, *J=*11.19*,* 7.75, 1.31 Hz, 1 H) 7.94 - 8.09 (m, 1 H) 8.10 - 8.20 (m, 1 H) 8.23 - 8.46 (m, 1 H).

Next, ethyl 2-(1-((tetrahydrofuran-2-yl)methylamino)phthalazin-4-yl)acetate was prepared. To a solution of ethyl 2-(1-chlorophthalazin-4-yl)acetate (150 mg, 598.37 umol) in dimethylsulfide (3 mL) was added diisopropylethylamine (232.01 mg, 1.80 mmol, 312.68 uL) and tetrahydrfuran-2- ylmethanamine (605.23 mg, 5.98 mmol, 617.58 uL) at 20°C. The mixture was stirred at 100°C for 12 hours. LC-MS showed the reaction was completed. The reaction mixture was quenched by addition water (30 mL) and extracted three times with 20 mL ethyl acetate (20 mL x 3). The combined organic layers were washed with brine (30 mL), dried over anhydrous sodium sulfate, filtered and concentrated under reduced pressure to give a residue which was purified by flash silica gel chromatography (ISCO^{®}; 12 g SEPAFLASH^{®} Silica Flash Column, Eluent of 0~7% ethyl acetate/petroleum ether gradient @ 35 mL/min) to give ethyl 2-(1-((tetrahydrofuran-2-yl)methylamino)phthalazin-4-yl)acetate (130 mg, 68.89% yield) as a yellow solid. The structure of ethyl 2-(1-((tetrahydrofuran-2-yl)methylamino)phthalazin-4-yl)acetate was confirmed by ¹H NMR: (400 MHz, DMSO-*d6*) δ 1.17 (t, *J*=7.07 Hz, 3 H) 1.65 (br dd, *J=*11.69, 8.57 Hz, 1 H) 1.75 - 1.91 (m, 2 H) 1.91 - 2.01 (m, 1 H) 3.57 - 3.69 (m, 3 H) 3.77 - 3.87 (m, 1 H) 4.10 (q, *J*=7.09 Hz, 2 H) 4.18 (s, 2 H) 4.22 (t, J=6.25 Hz, 1 H) 4.20 - 4.28 (m, 1 H) 7.52 (t, *J*=5.57 Hz, 1 H) 7.83 - 7.94 (m, 3 H) 8.30 - 8.37 (m, 1 H).

Next, 2-(1-((tetrahydrofuran-2-yl)methylamino)phthalazin-4-yl)acetic acid was prepared. To a solution of ethyl 2-(1-((tetrahydrofuran-2-yl)methylamino)phthalazin-4-yl)acetate (110 mg, 348.80 umol) in tetrahydrofuran (2.2 mL) and methanol (1.1 mL) was added a solution of LiOH.H₂O (36.59 mg, 872.00 umol) in H₂O (0.55 mL) at 20°C. The mixture was stirred at 20°C for 16 hours. LC-MS showed the reaction was completed. The reaction mixture was lyophilized to give 2-(1-((tetrahydrofuran-2-yl)methylamino)phthalazin-4-yl)acetic acid (100 mg, 99.79% yield) as a white solid. The structure of 2-(1-((tetrahydrofuran-2-yl)methylamino)phthalazin-4-yl)acetic acid was confirmed by ¹H NMR: (400 MHz, DMSO-*d6*) δ 1.61 - 1.71 (m, 1 H) 1.75 - 1.99 (m, 3 H) 3.57 (br t, *J*=5.77 Hz, 2 H) 3.60 - 3.65 (m, 1 H) 3.68 (s, 2 H) 3.77 - 3.85 (m, 1 H) 4.22 (quin, *J*=6.31 Hz, 1 H) 7.19 (br t, *J*=5.46 Hz, 1 H) 7.76 (dd, *J*=6.09, 3.20 Hz, 2 H) 8.02 (dd, *J*=6.15, 3.26 Hz, 1 H) 8.23 (br dd, *J*=5.58, 3.70 Hz, 1 H).

Finally, 2-(1-((tetrahydrofuran-2-yl)methylamino)phthalazin-4-yl)-N-(4-methoxyphenyl)acetamide **(Compound 11)** was prepared. To a solution of 2-(1-((tetrahydrofuran-2-yl)methylamino)phthalazin-4-yl)acetic acid (90 mg, 313.25 umol) in dimethyl formamide (1.8 mL) was added diisopropylethylamine (121.45 mg, 939.74 umol, 163.69 uL) and 2-(7-Azabenzotriazol -1-yl)-N,N,N',N'-tetramethyluronium hexafluorophosphate (178.66 mg, 469.87 umol) at 0°C and the mixture was stirred for 30 mins. Then, 4-methoxyaniline (38.58 mg, 313.25 umol) was added to the solution at 0°C. The mixture was stirred at 20°C for 16 hours. LC-MS showed the reaction was completed. The reaction mixture was purified by prep-HPLC (neutral condition) to give 2-(1-((tetrahydrofuran-2-yl)methylamino)phthalazin-4-yl)-N-(4-methoxyphenyl)acetamide **(Compound 11)** (30.8 mg, 25.05% yield) as a light white solid. The structure of Compound 11 was confirmed by ¹H NMR: (400 MHz, DMSO-d6) δ 1.58 - 1.70 (m, 1 H) 1.72 - 2.10 (m, 4 H) 3.57 - 3.68 (m, 3 H) 3.71 (s, 3 H) 3.78 - 3.85 (m, 1 H) 4.17 (s, 2 H) 4.22 (s, 1 H) 6.83 - 6.93 (m, 2 H) 7.44 - 7.55 (m, 3 H) 7.81 - 7.91 (m, 2 H) 7.99 - 8.06 (m, 1 H) 8.32 (br d, J=9.43 Hz, 1 H) 10.18 (s, 1 H).

### Synthesis Example 12: Synthesis of Compound 12

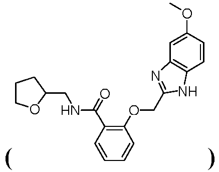

Compound 12 was synthesized according to the following Reaction Scheme 12.

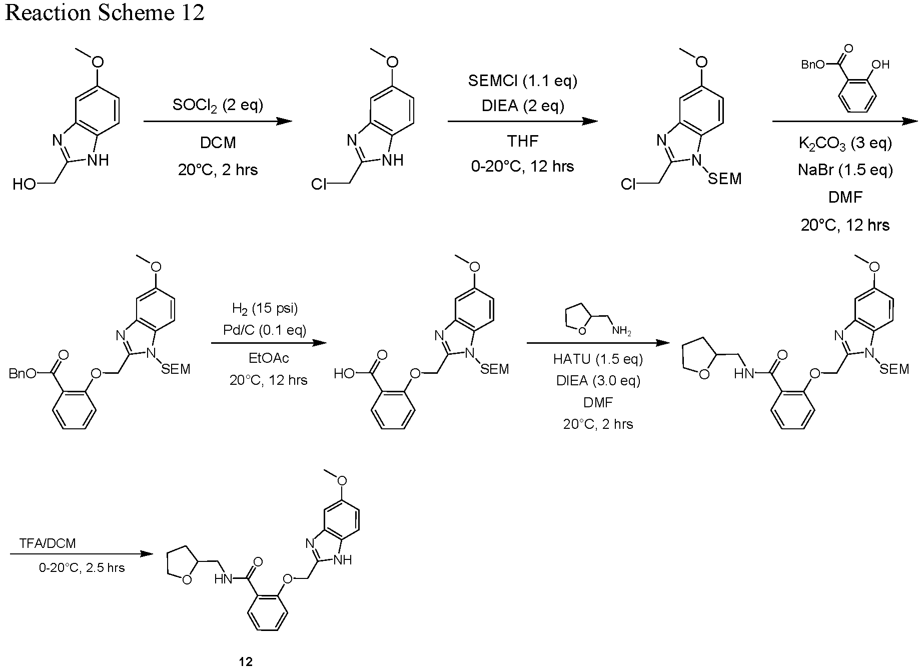

As shown in Reaction Scheme 12, first, 2-(chloromethyl)-5-methoxy-1H-benzo[d]imidazole was prepared. To a solution of (5-methoxy-1H-benzo[d]imidazol-2-yl)methanolin dichloromethane (10 mL) was added SOCl₂ (667.67 mg, 5.61 mmol, 407.11 uL) at 20°C. The mixture was stirred at 20°C for 2 hours. LC-MS showed the reaction was completed. The reaction mixture was concentrated under reduced pressure to give 2-(chloromethyl)-5-methoxy-1H-benzo[d]imidazole (551 mg, 99.86% yield) as a brown solid.

Next, 2-(chloromethyl)-5-methoxy-1H-benzo[d]imidazole was prepared. To a solution of 2-(chloromethyl)-5-methoxy-1H-benzo[d]imidazole (550 mg, 2.80 mmol,) in tetrahydrofuran (11 mL) was added N,N-Diisopropylethylamine (723.01 mg, 5.59 mmol, 974.40 uL) at 20°C. Then, 2-(chloromethoxy)ethyl-trimethyl-silane (512.97 mg, 3.08 mmol, 544.55 uL) was added to the solution at 0°C. The mixture was stirred at 20°C for 12 hours. LC-MS showed the reaction was completed. The reaction mixture was poured into water (20 mL) and extracted three times with 20 mL ethyl acetate (3 x 20 mL), the combined organic layer was washed with brine (20 mL), dried over anhydrous Na₂SO₄, filtered, concentrated under reduced pressure to give the residue which was purified by prep-TLC (SiO₂, ethyl acetate: petroleum ether = 1:2) to give 2-(chloromethyl)-5-methoxy-1H-benzo[d]imidazole (490 mg, 53.59% yield) as a yellow oil. The structure of 2-(chloromethyl)-5-methoxy-1H-benzo[d]imidazole was confirmed by ¹H NMR: (400 MHz, DMSO-*d6*) δ -0.09 (s, 9 H) 0.85 (td, *J*=8.00, 4.38 Hz, 2 H) 3.54 (dt, *J*=10.80, 8.19 Hz, 2 H) 3.80 (d, *J*=10.30 Hz, 3 H) 5.02 (d, *J*=4.38 Hz, 2 H) 5.67 (d, *J*=5.48 Hz, 2 H) 6.85 - 6.98 (m, 1 H) 7.19 (dd, *J=*17.76, 2.19 Hz, 1 H) 7.54 (t, *J*=8.44 Hz, 1 H).

Next, benzyl 2-((1-((2-(trimethylsilyl)ethoxy)methyl)-5-methoxy-1H-benzo[d]imidazol-2-yl)methoxy)benzoate was prepared. To a solution of 2-(chloromethyl)-5-methoxy-1H-benzo[d]imidazole (480 mg, 1.47 mmol) and benzyl 2-hydroxybenzoate (402.17 mg, 1.76 mmol, 19.13 uL) in DMF (9.6 mL) was added NaBr (226.63 mg, 2.20 mmol, 70.82 uL) and K₂CO₃ (608.83 mg, 4.41 mmol) at 20°C. The mixture was stirred at 20°C for 12 hours. LC-MS showed the reaction was completed. The mixture was diluted with water (30 mL) and extracted three times with 15 mL ethyl acetate (3 x 15 mL). The combined organic layer was washed with brine (15 mL), dried over sodium sulfate, filtered, and concentrated under reduced pressure to give a residue which was purified by prep-TLC (SiO₂, ethyl acetate : petroleum ether = 1:2) to give benzyl 2-((1-((2-(trimethylsilyl)ethoxy)methyl)-5-methoxy-1H-benzo[d]imidazol-2-yl)methoxy)benzoate (560 mg, 73.53% yield) as a yellow oil. The structure of benzyl 2-((1-((2-(trimethylsilyl)ethoxy)methyl)-5-methoxy-1H-benzo[d]imidazol-2-yl)methoxy)benzoate was confirmed by ¹H NMR: (400MHz, DMSO-*d6*) δ = -0.17 (d, J=2.41 Hz, 9 H) 0.70 - 0.79 (m, 2 H) 3.34 (s, 4 H) 3.42 (dt, J=12.61, 8.06 Hz, 2 H) 3.81 (d, J=7.89 Hz, 3 H) 5.25 (s, 2 H) 5.45 (d, J=8.33 Hz, 2 H) 5.62 (d, J=9.21 Hz, 2 H) 6.86 - 6.98 (m, 1 H) 7.08 (t, J=7.56 Hz, 1 H) 7.18 - 7.26 (m, 4 H) 7.32 (br d, J=7.67 Hz, 2 H) 7.42 (d, J=8.33 Hz, 1 H) 7.51 - 7.60 (m, 2 H) 7.73 (dd, J=7.67, 1.32 Hz, 1 H).

Next, 2-((1-((2-(trimethylsilyl)ethoxy)methyl)-5-methoxy-1H-benzo[d]imidazol-2-yl)methoxy)benzoic acid was prepared. To a solution of Pd/C (523.20 mg, 491.64 umol, 10% purity) in ethyl acetate (10.2 mL) was added benzyl 2-((1-((2-(trimethylsilyl)ethoxy)methyl)-5-methoxy-1H-benzo[d]imidazol-2-yl)methoxy)benzoate (510 mg, 983.28 umol) at 20°C. The mixture was degassed and purged with H₂ three times. The mixture was stirred at 20°C for 2 hours under 15 psi H₂ atmosphere. LC-MS showed the reaction was completed. The reaction mixture was filtered, and the filtrate was concentrated under reduced pressure to give 2-((1-((2-(trimethylsilyl)ethoxy)methyl)-5-methoxy-1H-benzo[d]imidazol-2-yl)methoxy)benzoic acid (424 mg, 91.47% yield) as a yellow oil. The structure of 2-((1-((2-(trimethylsilyl)ethoxy)methyl)-5-methoxy-1H-benzo[d]imidazol-2-yl)methoxy)benzoic acid was confirmed by ¹H NMR: (400MHz, DMSO-d6) δ = -0.17 - -0.06 (m, 9 H) 0.75 - 0.87 (m, 2 H) 2.53 (d, J=6.25 Hz, 1 H) 3.43 - 3.56 (m, 2 H) 3.75 - 3.83 (m, 3 H) 5.45 - 5.59 (m, 2 H) 5.69 - 5.78 (m, 1 H) 6.76 - 6.97 (m, 2 H) 7.01 - 7.09 (m, 1 H) 7.15 - 7.26 (m, 1 H) 7.35 - 7.46 (m, 1 H) 7.47 - 7.60 (m, 1 H) 7.64 - 7.79 (m, 1 H).

Next, 2-((1-((2-(trimethylsilyl)ethoxy)methyl)-5-methoxy-1H-benzo[d]imidazol-2-yl)methoxy)-N-((tetrahydrofuran-2-yl)methyl)benzamide was prepared. To a solution of 2-((1-((2-(trimethylsilyl)ethoxy)methyl)-5-methoxy-1H-benzo[d]imidazol-2-yl)methoxy)benzoic acid (200 mg, 466.69 umol) in dimethylformamide (4 mL) was added 2-(7-Azabenzotriazol-1-yl)-N,N,N',N'-tetramethyluronium hexafluorophosphate (266.17 mg, 700.03 umol) and N, N-Diisopropylethylamine (180.94 mg, 1.40 mmol, 243.86 uL) at 0°C. Then, tetrahydrofuran-2-ylmethanamine (70.81 mg, 700.03 umol, 72.25 uL) was added to the solution at 0°C. The mixture was stirred at 20°C for 2 hours. LC-MS showed the reaction was completed. The reaction mixture was diluted with water (15 mL) and extracted three times with 15 mL ethyl acetate (3 x 15 mL). The combined organic layer was washed with brine (15 mL), dried over sodium sulfate, filtered, and concentrated under reduced pressure to give the residue which was purified by flash silica gel chromatography (ISCO^{®}; 25 g SEPAFLASH^{®} Silica Flash Column, Eluent of 0~54% ethyl acetate/petroleum ether gradient @ 72mL/min) to give 2-((1-((2-(trimethylsilyl)ethoxy)methyl)-5-methoxy-1H-benzo[d]imidazol-2-yl)methoxy)-N-((tetrahydrofuran-2-yl)methyl)benzamide (97 mg, 40.62% yield) as yellow oil. The structure of 2-((1-((2-(trimethylsilyl)ethoxy)methyl)-5-methoxy-1H-benzo[d]imidazol-2-yl)methoxy)-N-((tetrahydrofuran-2-yl)methyl)benzamide was confirmed by ¹H NMR: (400MHz, DMSO-d6) δ = -0.13 (s, 9 H) 0.79 (br t, J=8.00 Hz, 2 H) 1.44 - 1.55 (m, 1 H) 1.60 - 1.80 (m, 3 H) 3.33 (s, 7 H) 3.35 - 3.41 (m, 2 H) 3.42 - 3.54 (m, 3 H) 3.57 - 3.65 (m, 1 H) 3.76 - 3.83 (m, 3 H) 3.86 - 3.95 (m, 1 H) 5.54 - 5.61 (m, 2 H) 5.68 - 5.73 (m, 2 H) 6.86 - 6.98 (m, 1 H) 7.08 (t, J=7.45 Hz, 1 H) 7.18 - 7.28 (m, 1 H) 7.39 (d, J=8.11 Hz, 1 H) 7.45 - 7.51 (m, 1 H) 7.52 - 7.62 (m, 1 H) 7.73 - 7.82 (m, 1 H) 8.77 (br t, J=5.48 Hz, 1 H).

Finally, 2-((5-methoxy-1H-benzo[d]imidazol-2-yl)methoxy)-N-((tetrahydrofuran-2-yl)methyl)benzamide **(Compound 12)** was prepared. To a solution of 2-((1-((2-(trimethylsilyl)ethoxy)methyl)-5-methoxy-1H-benzo[d]imidazol-2-yl)methoxy)-N-((tetrahydrofuran-2-yl)methyl)benzamide (87 mg, 170.03 umol) in dichloromethane (1.8 mL) and trifluoroacetic acid (693.00 mg, 6.08 mmol, 0.45 mL) at 20°C and stirred for 16 hours. The mixture was concentrated under reduced pressure to give a residue. The residue was dissolved in methanol (1.8 mL) and triethylamine (344.10 mg, 3.40 mmol, 473.31 uL) was added to the solution at 20°C. The reaction mixture was stirred at 20°C for 1 hour. LC-MS showed the reaction was completed. The reaction mixture was concentrated under reduced pressure to give a residue which was purified by prep-HPLC and lyophilized to give 2-((5-methoxy-1H-benzo[d]imidazol-2-yl)methoxy)-N-((tetrahydrofuran-2-yl)methyl)benzamide **(Compound 12)** (30.3 mg, 79.44 umol, 42.47% yield) as a white solid. The structure of Compound 12 was confirmed by ¹H NMR: (400MHz, DMSO-d6) δ = 1.43 - 1.53 (m, 1 H) 1.57 - 1.78 (m, 3 H) 3.35 - 3.47 (m, 3 H) 3.54 - 3.61 (m, 1 H) 3.78 (s, 3 H) 3.89 (t, J=6.25 Hz, 1 H) 5.36 - 5.51 (m, 1 H) 5.43 (s, 1 H) 6.82 (dd, J=8.76, 2.38 Hz, 1 H) 7.03 - 7.12 (m, 2 H) 7.31 (d, J=8.00 Hz, 1 H) 7.42 - 7.51 (m, 2 H) 7.78 (dd, J=7.75, 1.75 Hz, 1 H) 8.58 (br t, J=5.07 Hz, 1 H) 12.36 - 12.63 (m, 1 H).

### Synthesis Example 13: Synthesis of Compound 13

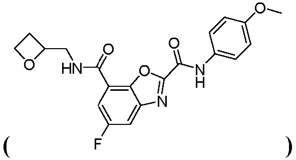

Compound 13 was synthesized according to the following Reaction Schemes 13A and 13B.

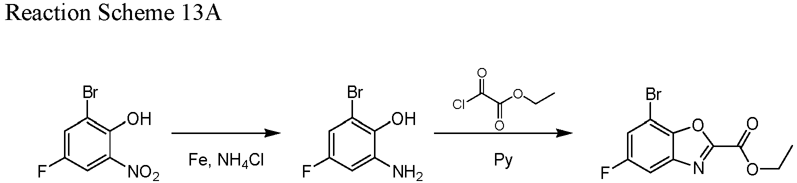

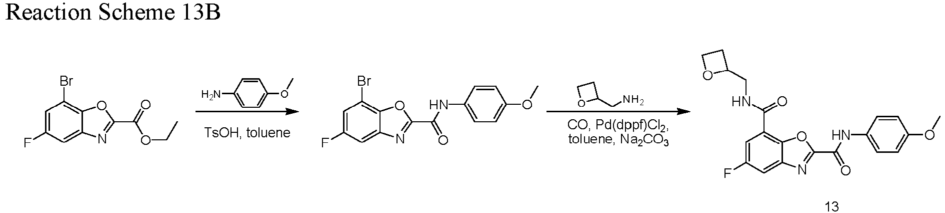

As shown in Reaction Scheme 13A, first, 2-amino-6-bromo-4-fluoro-phenol was prepared. To a solution of 2-bromo-4-fluoro-6-nitro-phenol (5 g, 21.19 mmol, 1 eq) in H₂O (25 mL) and EtOH (25 mL) was added Fe (7.10 g, 127.12 mmol, 6 eq) and NH₄Cl (9.07 g, 169.49 mmol, 8 eq). After stirring at 25°C for 2h, the reaction mixture was quenched by addition H₂O (50 mL) and extracted three times with 50 mL EtOAc (50 mL x 3). Concentrating gave 2-amino-6-bromo-4-fluoro-phenol (4 g, 19.42 mmol, 91.6% yield) as a black brown solid. Electrospray ionization (ESI) calculation for C₆H₆BrFNO [MH]⁺ 205.9; found 205.9.

Next, ethyl 7-bromo-5-fluoro-1,3-benzoxazole-2-carboxylate was prepared. To a solution of 2-amino-6-bromo-4-fluoro-phenol (4 g, 19.42 mmol, 1 eq) in pyridine (Py) (50 mL) was added ethyl 2-chloro-2-oxo-acetate (7.95 g, 58.25 mmol, 6.52 mL, 3 eq). After stirring at 100°C for 4hr, the reaction mixture was quenched by addition H₂O (50 mL) and extracted three times with 50 mL EtOAc (50 mL x 3). The combined organic phase was concentrated and purified by column chromatography (SiO₂, Petroleum ether/Ethyl acetate=0% to 5%) to give ethyl 7-bromo-5-fluoro-1,3-benzoxazole-2-carboxylate (800.0 mg, 2.78 mmol, 14.3% yield) as a light-yellow solid. ESI calculation for C₁₀H₈BrFNO₃ [MH₂]⁺ 289.9; found 289.8.

As shown in Reaction Scheme 13B, 7-bromo-5-fluoro-N-(4-methoxyphenyl)-1,3-benzoxazole-2-carboxamide was prepared. To a solution of ethyl 7-bromo-5-fluoro-1,3-benzoxazole-2-carboxylate (400.0 mg, 1.39 mmol, 1 eq) in toluene (4 mL) was added p-toluenesulfonic acid (TsOH) (23.9 mg, 138.86 µmol, 0.1 eq) and 4-methoxyaniline (171.0 mg, 1.39 mmol, 1 eq). After stirring at 100°C for 16hr, the reaction mixture was quenched by addition H₂O (5 mL) and extracted three times with 5 mL EtOAc (5 mL x 3). The combined organic phase was concentrated and purified by column chromatography (SiO2, Petroleum ether/Ethyl acetate=0% to 5%) to give 7-bromo-5-fluoro-N-(4-methoxyphenyl)-1,3-benzoxazole-2-carboxamide (500.0 mg, 1.37 mmol, 98.6% yield) as a yellow solid. The structure of 7-bromo-5-fluoro-N-(4-methoxyphenyl)-1,3-benzoxazole-2-carboxamide was confirmed by ¹H NMR (400 MHz, CDCl3) δ = 8.89 (s, 1H), 7.66 (d, J = 8.8Hz, 2H), 7.48-7.44 (m, 2H), 6.96 (d, J = 8.8Hz, 2H), 3.83 (s, 3H).¹⁹F NMR (376.5 MHz, CDCl3-*d*) δ = -113.491. ESI calculation for C₁₅H₁₀BrFN₂O₃Na [MNa]⁺ 386.9; found 386.9.

Next, 5-fluoro-N2-(4-methoxyphenyl)-N7-(oxetan-2-ylmethyl)-1,3-benzoxazole-2,7-dicarboxamide **(Compound 13)** was prepared. To a solution of 7-bromo-5-fluoro-N-(4-methoxyphenyl)-1,3-benzoxazole-2-carboxamide (150.0 mg, 410.79 µmol, 1 eq) in toluene (1 mL) was added oxetan-2-ylmethanamine (71.5 mg, 821.57 µmol, 2 eq), Pd(OAc)₂ (1.8 mg, 8.22 µmol, 0.02 eq), Xantphos (4.7 mg, 8.22 µmol, 0.02 eq) and Na₂CO₃ (130.6 mg, 1.23 mmol, 3 eq). After stirring at 80°C for 18hr under CO atmosphere (15 Psi), the reaction mixture was filtered and washed three times with 2 mL toluene (3 x 2 mL). The combined organic phase was concentrated and purified by Supercritical Fluid Chromatography (SFC) (column: DAICEL CHIRALPAK^{®} AD (250mm*30mm, 10um);mobile phase: [CO₂-EtOH/ACN];B%:55%, isocratic elution mode) to give 5-fluoro-N2-(4-methoxyphenyl)-N7-(oxetan-2-ylmethyl)-1,3-benzoxazole-2,7-dicarboxamide **(Compound 13)** (10.4 mg, 26.04 µmol, 6.3% yield) as a yellow solid. The structure of Compound 13 was confirmed by ¹H NMR (400MHz, CDCl3-*d*) δ = 8.96 (s, 1H), 8.01 (d, J = 6.0Hz, 1H), ,7.81 (s, 1H), 7.65 (d, J = 8.4Hz, 3H), 6.95 (d, J = 8.8Hz, 2H), 5.15 (s, 1H), 4.77 (t, J = 7.6Hz, 2H), 3.89-3.88 (m, 1H), 3.83 (s, 3H), 3.82-3.76 (m, 1H), 2.73-2.61 (m, 2H). ¹⁹F NMR (376.5 MHz, CDCl3-*d*) δ = -113.311. ESI calculation for C₂₀H₁₉FN₃O₅ [MH]⁺ 400.1; found 399.9.

### Synthesis Example 14: Synthesis of Compound 14

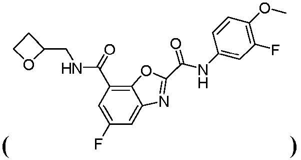

Compound 14 was synthesized according to the following Reaction Scheme 14.

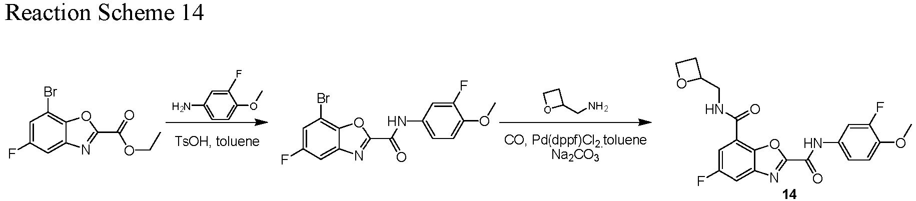

First, ethyl 7-bromo-5-fluoro-1,3-benzoxazole-2-carboxylate was prepared according to the reaction described in Synthesis Example 13 for Reaction Scheme 13A.

Next, as shown in Reaction Scheme 13, 7-bromo-5-fluoro-N-(3-fluoro-4-methoxyphenyl)-1,3-benzoxazole-2-carboxamide was prepared. To a solution of ethyl 7-bromo-5-fluoro-1,3-benzoxazole-2-carboxylate (1.6 g, 5.55 mmol, 1 eq) in toluene (20 mL) was added TsOH (95.6 mg, 555.42 µmol, 0.1 eq) and 3-fluoro-4-methoxy-aniline (783.9 mg, 5.55 mmol, 1 eq). After stirring at 100°C for 16hr, the reaction mixture was quenched by addition H₂O (16 mL) and extracted three times with 16 mL EtOAc (16 mL x 3), concentrated and purified by column chromatography (SiO₂, Petroleum ether/Ethyl acetate=0% to 20%) to give 7-bromo-5-fluoro-N-(3-fluoro-4-methoxy-phenyl)-1,3-benzoxazole-2-carboxamide (600.0 mg, 1.57 mmol, 28.2% yield) as a yellow solid. The structure of 7-bromo-5-fluoro-N-(3-fluoro-4-methoxy-phenyl)-1,3-benzoxazole-2-carboxamide was confirmed by ¹H NMR (400 MHz, CDCl3-*d*) δ = 8.88 (s, 1H), 7.66 (dd, J = 2.8Hz, 20.8Hz, 1H), 7.48-7.45 (m, 1H), 7.39-7.36 (m, 1H), 6.99 (t, J = 8.8Hz, 1H), 3.91 (s, 3H). ¹⁹F NMR (376.5 MHz, CDCl3) δ = -113.281, -132.096. ESI calculation for C₁₅H₁₀BrF₂N₂O₃ [MH]⁺ 382.9; found 382.9.

Finally, 5-fluoro-N2-(3-fluoro-4-methoxy-phenyl)-N7-(oxetan-2-ylmethyl)-1,3-benzoxazole-2,7-dicarboxamide **(Compound 14).** To a solution of 7-bromo-5-fluoro-N-(3-fluoro-4-methoxy-phenyl)-1,3-benzoxazole-2-carboxamide (400.0 mg, 1.04 mmol, 1 eq) in toluene (1 mL) was added oxetan-2-ylmethanamine (181.9 mg, 2.09 mmol, 2 eq), Pd(OAc)₂ (4.6 mg, 20.88 µmol, 0.02 eq), Xantphos (12.0 mg, 20.88 µmol, 0.02 eq) and Na₂CO₃ (331.9 mg, 3.13 mmol, 3 eq). After stirring at 80°C for 18hr under CO atmosphere (15 Psi), the reaction mixture was filtered and washed three times with 2 mL toluene (3 x 2 mL), concentrated and purified by column chromatography (SiO₂, Petroleum ether/Ethyl acetate=0% to 60%) to give 5-fluoro-N2-(3-fluoro-4-methoxy-phenyl)-N7-(oxetan-2-ylmethyl)-1,3-benzoxazole-2,7-dicarboxamide **(Compound 14)** (14.2 mg, 34.02 µmol, 3.2% yield) as a yellow solid. The structure of Compound 14 was confirmed by ¹H NMR (400MHz, CDCl3) δ = 8.93 (s, 1H), 8.04 (d, J = 2.4Hz, 1H), 7.73 (s, 1H), 7.67-7.63 (m, 2H), 7.38 (d, J = 10.8Hz, 1H), 6.99 (t, J = 8.4Hz, 1H), 5.17 (s, 1H), 4.79 (t, J = 9.6Hz, 2H), 3.91 (s, 3H), 3.89-3.86 (m, 1H), 3.81-3.79 (m, 1H), 2.78-2.61 (m, 2H). ¹⁹F NMR (376.5 MHz, CDCl3-*d*) δ = -113.085, -132.044. ESI calculation for C₂₀H₁₈F₂N₃O₅ [MH]⁺ 418.1; found 417.9.

### Synthesis Example 15: Synthesis of Compound 15

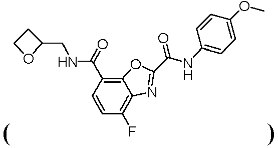

Compound 15 was synthesized according to the following Reaction Scheme 15.

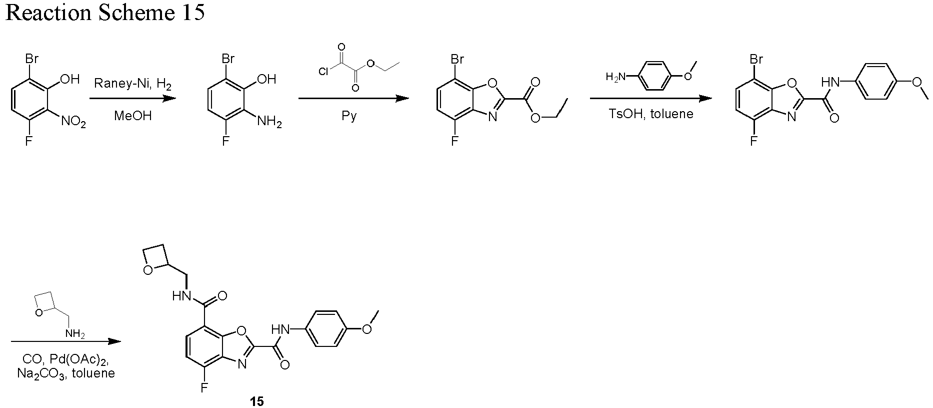

As shown in Reaction Scheme 15, first, 2-amino-6-bromo-3-fluoro-phenol was prepared. To a solution of 6-bromo-3-fluoro-2-nitro-phenol (2 g, 8.47 mmol, 1 eq) in MeOH (8 mL) was added Raney-Ni (2.18 g, 25.42 mmol, 3 eq). After stirring under H₂ (15 psi) at 25°C for 2h, the reaction mixture was filtered through a pad of CELITE^{®} and washed with MeOH (3 x 8 mL), concentrated to give 2-amino-6-bromo-3-fluoro-phenol (1.6 g, crude) as a brown solid. ESI calculation for C₆H₆BrFNO [MH]⁺ 205.9; found 206.0.

Next, ethyl 7-bromo-4-fluoro-1,3-benzoxazole-2-carboxylate was prepared. To a solution of 2-amino-6-bromo-3-fluoro-phenol (1.6 g, 7.77 mmol, 1 eq) in Py (16 mL) was added ethyl 2-chloro-2-oxo-acetate (5.30 g, 38.83 mmol, 4.35 mL, 5 eq). After stirring at 100°C for 4hr, the reaction mixture was quenched by addition H₂O (16 mL) and extracted three times with 16 mL EtOAc (16 mL x 3). The organic layers were concentrated and purified by column chromatography (SiO₂, Petroleum ether/Ethyl acetate= 0% to 5%) to give ethyl 7-bromo-4-fluoro-1,3-benzoxazole-2-carboxylate (550.0 mg, 1.91 mmol, 24.5% yield) as a yellow oil. The structure of ethyl 7-bromo-4-fluoro-1,3-benzoxazole-2-carboxylate was confirmed by ¹H NMR (400 MHz, CDCl3) δ = 7.62 (dd, J = 4.4Hz, 8.8Hz, 1H), 7.10 (t, J = 8.8Hz, 1H), 4.58-4.51 (m, 2H), 1.48 (t, J = 7.2Hz, 3H). ¹⁹F NMR (376.5 MHz, CDCl3) δ = -123.225. ESI calculation for C₁₀H₈BrFNO₃[MH₂]²⁺ 289.9; found 289.9.

Next, ethyl 7-bromo-4-fluoro-N-(4-methoxyphenyl)-1,3-benzoxazole-2-carboxamide was prepared. To a solution of 7-bromo-4-fluoro-1,3-benzoxazole-2-carboxylate (250.0 mg, 867.85 µmol, 1 eq) in toluene (1 mL) was added TsOH (14.94 mg, 86.78 µmol, 0.1 eq) and 4-methoxyaniline (106.88 mg, 867.85 µmol, 1 eq). After stirring at 100°C for 16hr, the reaction mixture was quenched by addition H₂O (5 mL) and extracted three times with 5 mL EtOAc (5 mL x 3). The combined organic phase was concentrated and purified by column chromatography (SiO₂, Petroleum ether/Ethyl acetate=0% to 5%) to give ethyl 7-bromo-4-fluoro-N-(4-methoxyphenyl)-1,3-benzoxazole-2-carboxamide (130.0 mg, 356.01 µmol, 41.0% yield) as a yellow solid. The structure of ethyl 7-bromo-4-fluoro-N-(4-methoxyphenyl)-1,3-benzoxazole-2-carboxamide was confirmed by ¹H NMR (400 MHz, CDCl3) δ = 8.84 (s, 1H), 7.67-7.59 (m, 3H), 7.12 (t, J = 9.2Hz, 1H), 6.96 (d, J = 9.2Hz, 2H), 3.83 (s, 3H). ¹⁹F NMR (376.5 MHz, CDCl3) δ = -125.041. ESI calculation for C₁₅H₁₁BrFN₂O₃ [MH₂]²⁺ 367.0; found 366.9.

Finally, 4-fluoro-N2-(4-methoxyphenyl)-N7-(oxetan-2-ylmethyl)-1,3-benzoxazole-2,7-dicarboxamide **(Compound 15)** was prepared. To a solution of 7-bromo-4-fluoro-N-(4-methoxyphenyl)-1,3-benzoxazole-2-carboxamide (130.0 mg, 356.01 µmol, 1 eq) in toluene (1 mL) was added oxetan-2-ylmethanamine (62.0 mg, 712.03 µmol, 2 eq), Pd(OAc)₂ (1.6 mg, 7.12 µmol, 0.02 eq), Xantphos (4.1 mg, 7.12 µmol, 0.02 eq) and Na₂CO₃ (113.2 mg, 1.07 mmol, 3 eq). After stirring under CO atmosphere (15 Psi) at 80°C for 18hr, the reaction mixture was filtered and washed with toluene (3 x 2 mL), concentrated and purified by prep-TLC (SiO₂, PE: EtOAc= 1: 1) to give 4-fluoro-N2-(4-methoxyphenyl)-N7-(oxetan-2-ylmethyl)-1,3-benzoxazole-2,7-dicarboxamide **(Compound 15)** (10.3 mg, 25.79 µmol, 7.2% yield) as a yellow solid. The structure of Compound 15 was confirmed by ¹H NMR (400MHz, CDCl3) δ = 9.00 (s, 1H), 8.32-8.28 (m, 1H), 7.69-7.64 (m, 3H), 7.31 (t, J = 8.8Hz, 1H), 6.95 (d, J = 8.8Hz, 2H), 5.16-5.14 (m, 1H), 4.81-4.74 (m, 2H), 3.91-3.86 (m, 1H), 3.83 (s, 3H), 3.78-3.74 (m, 1H), 2.76-2.61 (m, 2H).¹⁹F NMR (376.5 MHz, CDCl3) δ = -117.393. ESI calculation for C₂₀H₁₉FN₃O₅ [MH]⁺ 400.1; found 400.0.

### Synthesis Example 16: Synthesis of Compound 16

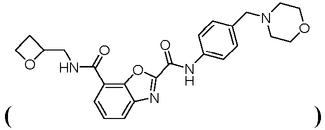

Compound 16 was synthesized according to the following Reaction Scheme 16.

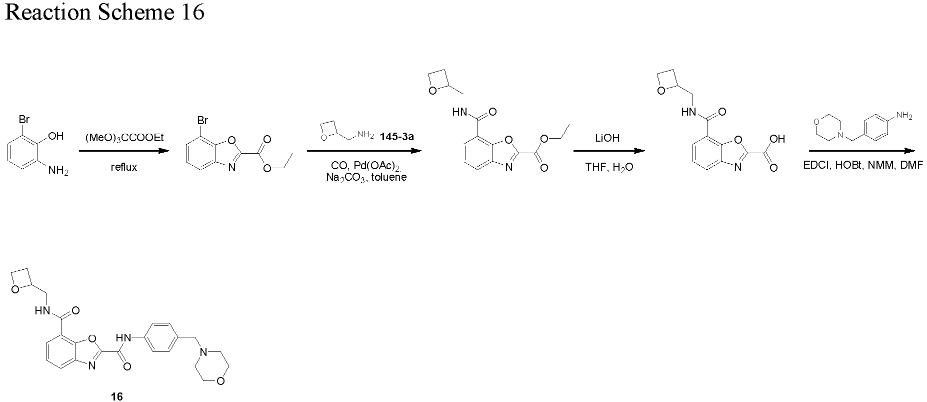

As shown in Reaction Scheme 16, first, ethyl 7-bromo-1,3-benzoxazole-2-carboxylate was prepared. To a solution of 2-amino-6-bromo-phenol (5 g, 26.59 mmol, 1 eq) in Py (50 mL) was added ethyl 2-chloro-2-oxo-acetate (18.15 g, 132.96 mmol, 14.88 mL, 5 eq). After stirring at 100°C for 4hr, the reaction mixture was quenched by addition H₂O (50 mL) and extracted three times with 50 mL EtOAc (50 mL x 3). The combined organic phase was concentrated and purified by column chromatography (SiO₂, Petroleum ether/Ethyl acetate=0% to 4%) to give ethyl 7-bromo-1,3-benzoxazole-2-carboxylate (5.5 g, 20.36 mmol, 76.5% yield) as a pink solid. The structure of ethyl 7-bromo-1,3-benzoxazole-2-carboxylate was confirmed by ¹H NMR (400 MHz, CDCl3) δ = 7.83 (dd, J = 0.8Hz, 8.0Hz, 1H), 7.67 (dd, J = 0.8Hz, 8.0Hz, 1H), 7.34 (t, J = 8.0Hz, 1H), 4.7 (dd, J = 0.8Hz, 8.0Hz, 2H), 1.49 (t, J = 7.2Hz, 3H). ESI calculation for C₁₀H₉BrNO₃ [MH]⁺ 269.9; found 270.0.

Next, ethyl 7-(oxetan-2-ylmethylcarbamoyl)-1,3-benzoxazole-2-carboxylate was prepared. To a solution of ethyl 7-bromo-1,3-benzoxazole-2-carboxylate (500.0 mg, 1.85 mmol, 1 eq) in toluene (1 mL) was added oxetan-2-ylmethanamine (129.0 mg, 1.48 mmol, 0.8 eq), Pd(OAc)₂ (8.3 mg, 37.03 µmol, 0.02 eq), TEA (588.6 mg, 5.82 mmol, 809.71 µL, 3.14 eq) and Xantphos (21.4 mg, 37.03 µmol, 0.02 eq). After stirring under CO (15 psi) at 80°C for 16hr, the reaction mixture was filtered and washed three times with 5 mL toluene (3 x 5 mL), concentrated and purified by column chromatography (SiO₂, Petroleum ether/Ethyl acetate=0% to 25%) to give ethyl 7-(oxetan-2-ylmethylcarbamoyl)-1,3-benzoxazole-2-carboxylate (120.0 mg, 394.35 µmol, 21.3% yield) as yellow oil. The structure of ethyl 7-(oxetan-2-ylmethylcarbamoyl)-1,3-benzoxazole-2-carboxylate was confirmed by ¹H NMR (400 MHz, CDCl3) δ = 8.15 (d, J = 7.6Hz, 1H), 8.01 (d, J = 8.0Hz, 1H), 7.77 (s, 1H), 7.51 (t, J = 8.0Hz, 1H), 5.07-5.05 (m, 1H), 4.74-4.69 (m, 1H), 4.61-4.48 (m, 4H), 3.90-3.84 (m, 1H), 3.73-3.67 (m, 1H), 2.75-2.71 (m, 1H), 2.55-2.51 (m, 1H), 1.46 (t, J = 7.2Hz, 3H). ESI calculation for C₁₅H₁₇N₂O₅ [MH]⁺ 305.1; found 305.1.

Next, 7-(oxetan-2-ylmethylcarbamoyl)-1,3-benzoxazole-2-carboxylic acid was prepared. To a solution of ethyl 7-(oxetan-2-ylmethylcarbamoyl)-1,3-benzoxazole-2-carboxylate (400.0 mg, 1.31 mmol, 1 eq) in THF (3 mL) and MeOH (3 mL) and H₂O (0.5 mL) was added LiOH.H₂O (82.7 mg, 1.97 mmol, 1.5 eq). After stirring at 25°C for 0.5hr, the reaction mixture was acidified with 1M HCl (3 mL) and extracted three times with 5 mL EtOAc (5 mL x 3). The combined organic phase was filtered and concentrated to give 7-(oxetan-2-ylmethylcarbamoyl)-1,3-benzoxazole-2-carboxylic acid (300.0 mg, crude) as a yellow solid. ESI calculation for C13H13N2O5 [MH]⁺ 277.0; found 277.1.

Finally, N2-[4-(morpholinomethyl)phenyl]-N7-(oxetan-2-ylmethyl)-1,3-benzoxazole-2,7-dicarboxamide **(Compound 16)** was prepared. To a solution of 7-(oxetan-2-ylmethylcarbamoyl)-1,3-benzoxazole-2-carboxylic acid (300.0 mg, 1.09 mmol, 1 eq) in DMF (3 mL) was added 4-methylmorpholine (549.2 mg, 5.43 mmol, 596.98 µL, 5 eq), 1-Ethyl-3-(3-dimethylaminopropyl)carbodiimide (EDCI) (416.3 mg, 2.17 mmol, 2 eq), 1-Hydroxybenzotriazole (HOBt) (293.4 mg, 2.17 mmol, 2 eq), and 4-(morpholinomethyl)aniline (313.1 mg, 1.63 mmol, 1.5 eq). After stirring at 25°C for 2hr, the reaction mixture was quenched by addition H₂O (3 mL) and extracted three times with 3 mL EtOAc (3 mL x 3). The combined organic phase was concentrated and purified by prep-HPLC (column: Waters XBRIDGE^{®} 150x25mm 10um; mobile phase: [water( NH₄HCO₃)-ACN]; gradient: 14%-44% B over 18 min) to give N2-[4-(morpholinomethyl)phenyl]-N7-(oxetan-2-ylmethyl)-1,3-benzoxazole-2,7-dicarboxamide **(Compound 16)** (83.2 mg, 184.69 µmol, 17.0% yield) as a yellow solid. The structure of Compound 16 was confirmed by ¹H NMR (400 MHz, CDCl3) δ = 8.98 (s, 1H), 8.36 (d, J = 6.8Hz, 1H), 8.00 (d, J = 6.8Hz ,1H), 7.82-7.72 (m, 3H), 7.61 (t, J = 9.2Hz, 1H), 7.36 (d, J = 8.4Hz, 2H), 5.10-5.06 (m, 1H), 4.76-4.70 (m, 1H), 4.62-4.58 (m, 1H), 3.90-3.84 (m, 1H), 3.74-3.69 (m, 5H), 3.51 (s, 2H), 2.77-2.73 (m, 1H), 2.56-2.46 (m, 5H). ESI calculation for C₂₄H₂₇N₄O₅ [MH]⁺ 451.1; found 451.1.

### Synthesis Example 17: Synthesis of Compound 17

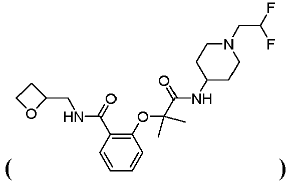

Compound 17 was synthesized according to the following Reaction Schemes 17A and 17B.

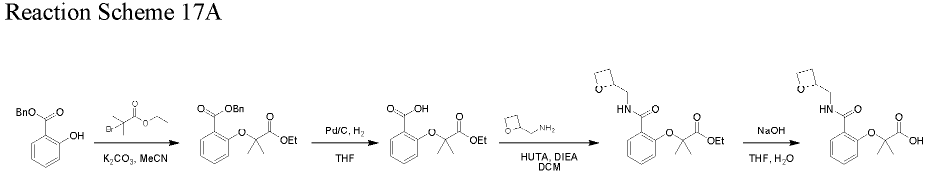

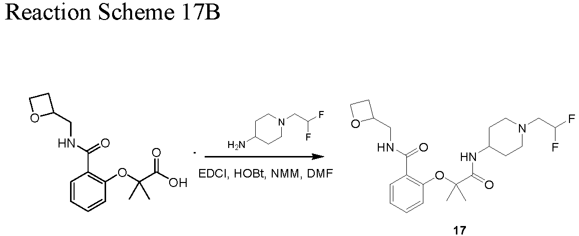

As shown in Reaction Scheme 17A, first, benzyl 2-(2-ethoxy-1,1-dimethyl-2-oxo-ethoxy)benzoate was prepared. To a solution of benzyl 2-hydroxybenzoate (10 g, 43.81 mmol, 1 eq) in MeCN (100 mL) was added ethyl 2-bromo-2-methyl-propanoate (17.09 g, 87.63 mmol, 12.85 mL, 2 eq) and K₂CO₃ (12.11 g, 87.63 mmol, 2 eq). After stirring at 85°C for 16h, the reaction mixture was quenched by addition of NH₄Cl (10 mL) and extracted three times with 60 mL EtOAc (60 mL x 3). The combined organic phase was concentrated and purified by column chromatography (SiO₂, Petroleum ether/Ethyl acetate=0 to 5%) to give benzyl 2-(2-ethoxy-1,1-dimethyl-2-oxo-ethoxy)benzoate (4 g, 11.68 mmol, 26.7% yield) as a white oil. The structure of benzyl 2-(2-ethoxy-1,1-dimethyl-2-oxo-ethoxy)benzoate was confirmed by ¹H NMR (400 MHz, CDCl3) δ = 7.80-7.76 (m, 1H), 7.47-7.43 (m, 2H), 7.39-7.35 (m, 2H), 7.35-7.31 (m, 2H), 7.04-6.99 (m, 1H), 6.85-6.82 (m, 1H), 5.35-5.33 (m, 2H), 4.26-4.19 (m, 2H), 1.54 (s, 6H), 1.25-1.21 (m, 3H). ESI calculation for C₂₀H₂₃O₅ [MH]⁺ 343.4; found 343.2.

Next, 2-(2-ethoxy-1,1-dimethyl-2-oxo-ethoxy)benzoic acid was prepared. To a solution of benzyl 2-(2-ethoxy-1,1-dimethyl-2-oxo-ethoxy)benzoate (4 g, 11.68 mmol, 1 eq) in THF (140 mL) was added Pd/C (0.5 g, 10% purity) under N₂. The suspension was degassed under vacuum and purged with H₂ several times. After stirring at 20°C for 20h under H₂ (15 psi), the reaction mixture was filtered and concentrated to give 2-(2-ethoxy-1,1-dimethyl-2-oxo-ethoxy)benzoic acid (2.7 g, 10.70 mmol, 91.6% yield) as a white oil. The structure of 2-(2-ethoxy-1,1-dimethyl-2-oxo-ethoxy)benzoic acid was confirmed by ¹H NMR (400 MHz, CDCl3) δ = 8.20-8.14 (m, 1H), 7.48-7.42 (m, 1H), 7.14 (t, J = 7.6Hz, 1H), 6.91 (d, J = 8.4Hz, 1H), 4.28 - 4.21 (m, 2H), 1.74 (s, 6H), 1.24 (t, J = 7.2Hz, 3H). ESI calculation for C13H17O5 [MH]⁺ 253.3; found 253.1.

Next, ethyl 2-methyl-2-[2-(oxetan-2-ylmethylcarbamoyl)phenoxy]propanoate was prepared. To a solution of 2-(2-ethoxy-1,1-dimethyl-2-oxo-ethoxy)benzoic acid (600.00 mg, 2.38mmol, 1 eq) in DCM (6 mL) was added HATU (1.36 g, 3.57 mmol, 1.5 eq) and DIEA (1.54 g, 11.89 mmol, 2.07 mL, 5 eq). After stirring at 25°C for 0.5h, oxetan-2-ylmethanamine (207.21 mg, 2.38 mmol, 1 eq) was added. After stirring at 25°C for another 0.5h, the reaction mixture was quenched by addition of water 10 mL at 25°C and extracted three times with 5 mL EtOAc (5 mL x 3). The combined organic phase was concentrated and purified by column chromatography (SiO₂, Petroleum ether/Ethyl acetate=0 to 30%) to give ethyl 2-methyl-2-[2-(oxetan-2-ylmethylcarbamoyl)phenoxy]propanoate (400 mg, 1.24 mmol, 52.3% yield) as a white oil. The structure of ethyl 2-methyl-2-[2-(oxetan-2-ylmethylcarbamoyl)phenoxy]propanoate was confirmed by ¹H NMR (400 MHz, CDCl3) δ = 8.47-8.38 (m, 1H), 8.24-8.20 (m, 1H), 7.37-7.31 (m, 1H), 7.14-7.09 (m, 1H), 6.83-6.77 (m, 1H), 4.30-4.22 (m, 2H), 1.71 (d, J = 6.8Hz, 6H), 1.24 (t, J = 6.8Hz, 3H). ESI calculation for C₁₇H₂₄NO₅ [MH]⁺ 322.4; found 322.2.

Next, 2-methyl-2-[2-(oxetan-2-ylmethylcarbamoyl)phenoxy]propanoic acid was prepared. To a solution of ethyl 2-methyl-2-[2-(oxetan-2-ylmethylcarbamoyl)phenoxy]propanoate (150 mg, 466.76 µmol, 1 eq) in MeOH (2 mL), THF (1 mL), H₂O (0.5 mL) was added LiOH (16.77 mg, 700.13 µmol, 1.5 eq). After stirring at 25°C for 1hr, the reaction mixture was quenched by addition of 2M HCl (3 mL), and then diluted with H₂O (10 mL), extracted three times with 10 mL EtOAc (10 mL x 3). The combined organic phase was concentrated to give 2-methyl-2-[2-(oxetan-2-ylmethylcarbamoyl)phenoxy]propanoic acid (120 mg, 409.12 µmol, 87.6% yield) as white oil. ESI calculation for C₁₅H₂₀NO₅ [MH]⁺ 294.3; found 294.3.

As shown in Reaction Scheme 17B, 2-[2-[[1-(2,2-difluoroethyl)-4-piperidyl]amino]-1,1-dimethyl-2-oxo-ethoxy]-N-(oxetan-2-ylmethyl)benzamide **(Compound 17)** was prepared. To a solution of 2-methyl-2-[2-(oxetan-2-ylmethylcarbamoyl)phenoxy]propanoic acid (150 mg, 511.40 µmol, 1 eq) in DMF (1 mL) was added EDCI (196.07 mg, 1.02 mmol, 2 eq), HOBt (207.31 mg, 1.53 mmol, 3 eq), and 4-methylmorpholine (258.63 mg, 2.56 mmol, 281.12 µL, 5 eq). After stirring at 50°C for 0.5hr, 1-(2,2-difluoroethyl)piperidin-4-amine (167.94 mg, 1.02 mmol, 2 eq) was added. After stirring at 50°C for 1hr, the reaction mixture was concentrated and purified by prep-HPLC (neutral condition: column: Waters XBRIDGE^{®} 150x25mm 10um; mobile phase: [water(NH₄HCO₃)-ACN]; B%: 17%-47%, 15min) to give 2-[2-[[1-(2,2-difluoroethyl)-4-piperidyl]amino]-1,1-dimethyl-2-oxo-ethoxy]-N-(oxetan-2-ylmethyl)benzamide **(Compound 17)** (90 mg, 204.78 µmol, 40.0% yield) as a white oil. The structure of Compound 17 was confirmed by ¹H NMR (400 MHz, CDCl3) δ = 8.03 (dd, J = 1.6Hz, 7.6Hz, 1H), 7.78 (s, 1H), 7.39-7.32 (m, 1H), 7.13 (t, J = 7.6 Hz, 1H), 6.90 (d, J = 8.4 Hz, 1H), 6.81-6.72 (m, 1H), 6.01-5.68 (m, 1H), 5.07-4.98 (m, 1H), 4.75-4.66 (m, 1H), 4.57-4.49 (m, 1H), 3.84-3.67 (m, 3H), 2.87 (br d, J = 11.8 Hz, 2H), 2.77-2.67 (m, 3H), 2.56-2.47 (m, 1H), 2.34 (br t, J = 11.2 Hz, 2H), 1.88 (br d, J = 12.0 Hz, 2H), 1.64 (d, J = 2.0 Hz, 6H), 1.54-1.43 (m, 2H). ESI calculation for C₂₂H₃₂F₂N₃O₄ [MH]⁺ 440.5; found 440.1.

### Synthesis Example 18: Synthesis of Compound 18

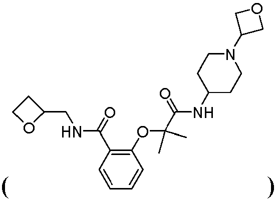

Compound 18 was synthesized according to the following Reaction Scheme 18.

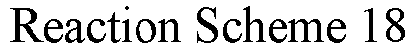

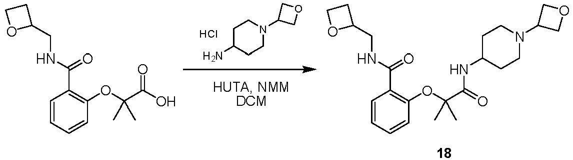

First, 2-methyl-2-[2-(oxetan-2-ylmethylcarbamoyl)phenoxy]propanoic acid was prepared according to the reaction described in Synthesis Example 17 for Reaction Scheme 17A.

Next, 2-[1,1-dimethyl-2-[[1-(oxetan-3-yl)-4-piperidyl]amino]-2-oxo-ethoxy]-N-(oxetan-2-ylmethyl)benzamide **(Compound 18)** was prepared. To a solution of 2-methyl-2-[2-(oxetan-2-ylmethylcarbamoyl)phenoxy]propanoic acid (110 mg, 375.02 µmol, 1eq) and 1-(oxetan-3-yl)piperidin-4-amine (58.59 mg, 375.02 µmol, 1 eq) in DMF (1 mL) was added EDCI (107.84 mg, 562.54 µmol, 1.5 eq), HOBt (76.01 mg, 562.54 µmol, 1.5 eq), and 4-methylmorpholine (189.66 mg, 1.88 mmol, 206.16 µL, 5 eq) .The mixture was stirred at 50°C for 1 hr .The reaction mixture was quenched by addition of NH₄Cl 2mL at 25°C, and then diluted with H₂O (3 mL) and extracted three times with 3 mL EtOAc (3mL x 3), concentrated under reduced pressure to give a residue, and purified by prep-HPLC (neutral condition: column: Waters XBRIDGE^{®} 150x25mm 10um; mobile phase: [water(NH₄HCO₃)-ACN]; gradient:8%-38% B over 14 min) to give 2-[1,1-dimethyl-2-[[1-(oxetan-3-yl)-4-piperidyl]amino]-2-oxo-ethoxy]-N-(oxetan-2-ylmethyl)benzamide **(Compound 18)** (45 mg, 104.28 µmol, 27.8% yield) as white oil. The structure of Compound 18 was confirmed by ¹H NMR (400 MHz, CDCl3) δ = 8.05-8.01 (m, 1H), 7.81-7.75 (m, 1H), 7.38-7.32 (m, 1H), 7.13 (t, J = 7.6 Hz, 1H), 6.91 (d, J = 8.4 Hz, 1H), 6.78 (br d, J = 7.6 Hz, 1H), 5.07-5.00 (m, 1H), 4.75-4.68 (m, 1H), 4.66-4.61 (m, 2H), 4.59-4.55 (m, 2H), 4.54-4.49 (m, 1H), 3.86-3.77 (m, 2H), 3.74-3.67 (m, 1H), 3.49-3.42 (m, 1H), 2.74-2.63 (m, 3H), 2.56-2.49 (m, 1H), 1.99-1.90 (m, 4H), 1.64 (d, J = 2.0 Hz, 6H), 1.53-1.44 (m, 2H). ESI calculation for C₂₃H₃₃N₃O₅ [MH]⁺ 432.5; found 432.1.

### Synthesis Example 19: Synthesis of Compound 19

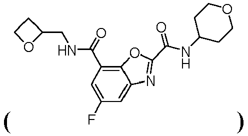

Compound 19 was synthesized according to the following Reaction Scheme 19.

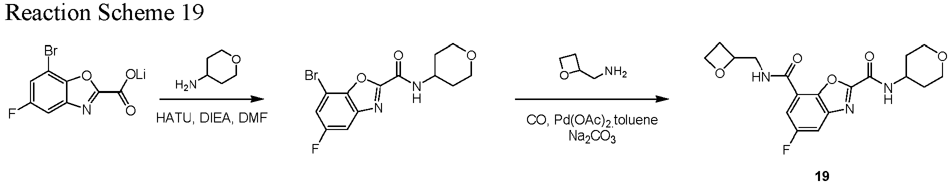

Following Reaction Scheme 19, 5-fluoro-N2-(tetrahydro-2H-pyran-4-yl)-N7-((oxetan-2-yl)methyl)benzo[d]oxazole-2,7-dicarboxamide **(Compound 19)** (22.2 mg) was obtained as a white solid. The structure of Compound 19 was confirmed by ¹H NMR (400 MHz, CDCl3) δ 7.98 (d, *J=* 10.8 Hz, 1H), 7.87 (s, 1H), 7.62-7.59 (m, 1H), 7.36-7.34 (m, 1H), 5.15-5.14 (m, 1H), 4.78-4.73 (m, 2H), 4.04-4.01 (m, 1H), 3.85-3.79 (m, 2H), 3.56-3.50 (m, 4H), 2.74-2.71 (m, 1H), 2.03-2.00 (m, 2H), 1.69-1.66 (m, 2H). ¹⁹F NMR (376.5 MHz, CDCl₃) δ -113.466. MS *m*/*z:* 378 [MH⁺].

### Synthesis Example 20: Synthesis of Compound 20

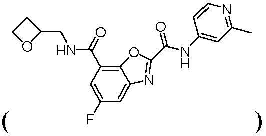

Compound 20 was synthesized according to the following Reaction Scheme 20.

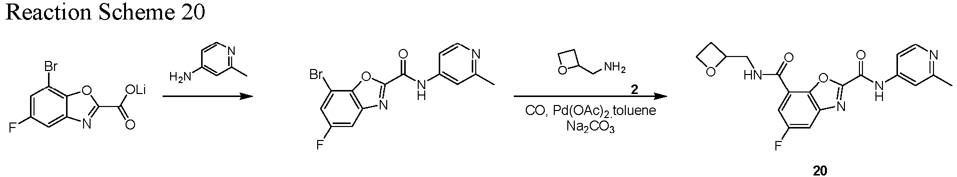

Following Reaction Scheme 20, 5-fluoro-N2-(2-methylpyridin-4-yl)-N7-((oxetan-2-yl)methyl)benzo[d]oxazole-2,7-dicarboxamide **(Compound 20)** (15 mg) was obtained as a white solid. The structure of Compound 20 was confirmed by ¹H NMR (400 MHz, CDCl₃) δ 9.21 (br, 1H), 9.21 (br, 1H), 8.50 (d, J = 5.6 Hz, 1H), 8.06-8.03 (m, 1H), 7.68-7.66 (m, 1H), 7.57-7.27 (m, 3H), 5.18-5.15 (m, 1H), 4.82-4.77 (m, 2H), 3.92-3.87 (m, 1H), 3.82-3.78 (m, 1H), 2.77-2.74 (m, 1H), 2.66-2.63 (m, 1H), 2.60 (s, 3H). ¹⁹F NMR (376.5 MHz, CDCl₃) δ -112.656. MS *m*/*z*: 385 [MH⁺].

### Synthesis Example 21: Synthesis of Compound 21

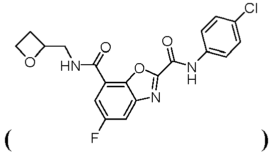

Compound 21 was synthesized according to the following Reaction Scheme 21.

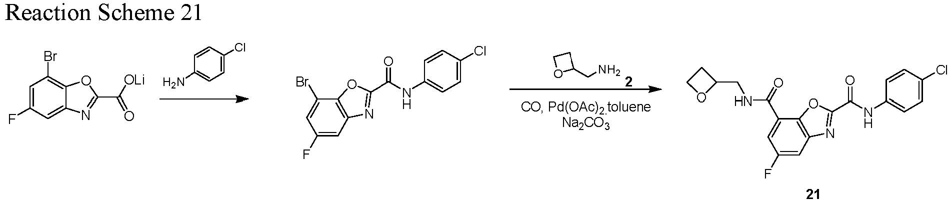

Following Reaction Scheme 21, N2-(4-chlorophenyl)-5-fluoro-N7-((oxetan-2-yl)methyl)benzo[d]oxazole-2,7-dicarboxamide **(Compound 21)** (20.5 mg) was obtained as a white solid. The structure of Compound 21 was confirmed by ¹H NMR (400MHz, DMSO-d6) δ 11.42 (s, 1H), 8.79 (d, *J* = 5.6 Hz, 1H), 8.04-7.47 (m, 6H), 4.95-4.93 (m, 1H), 4.60-4.53 (m, 2H), 3.68-3.67 (m, 1H), 3.57-3.54 (m, 1H), 2.67-2.66 (m, 1H). ¹⁹F NMR (376.5 MHz, DMSO-d6) δ - 115.413. MS *m*/*z:* 404 [MH⁺].

### Synthesis Example 22: Synthesis of Compound 22

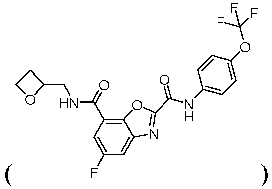

Compound 22 was synthesized according to the following Reaction Scheme 22.

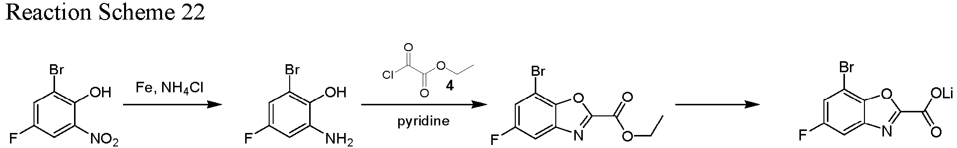

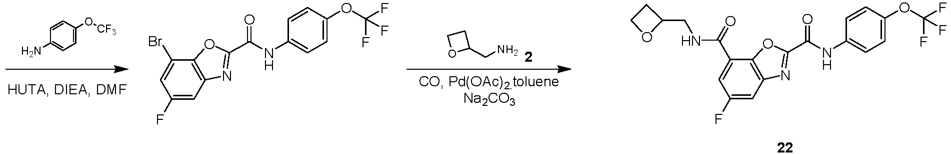

Following Reaction Scheme 22, 5-fluoro-N7-((oxetan-2-yl)methyl)-N2-(4-(trifluoromethoxy)phenyl)benzo[d]oxazole-2,7-dicarboxamide **(Compound 22)** (62.9 mg) was obtained as a white solid. The structure of Compound 22 was confirmed by ¹H NMR (400MHz, DMSO-d6) δ 10.65 (s, 1H), 7.97-7.94 (m, 1H), 7.20-7.14 (m, 3H), 6.94-6.91 (m, 1H), 6.60-6.58 (m, 2H), 4.11-4.09 (m, 1H), 3.76-3.69 (m, 2H), 2.84-2.81 (m, 1H), 2.71-2.67 (m, 1H), 1.85-1.83 (m, 1H). ¹⁹F NMR (376.5 MHz, DMSO-d6) δ -56.973, -115.437. MS *m*/*z:* 454 [MH⁺].

### Synthesis Example 23: Synthesis of Compound 23

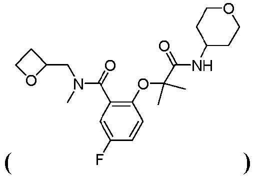

Compound 23 was synthesized according to the following Reaction Scheme 23.

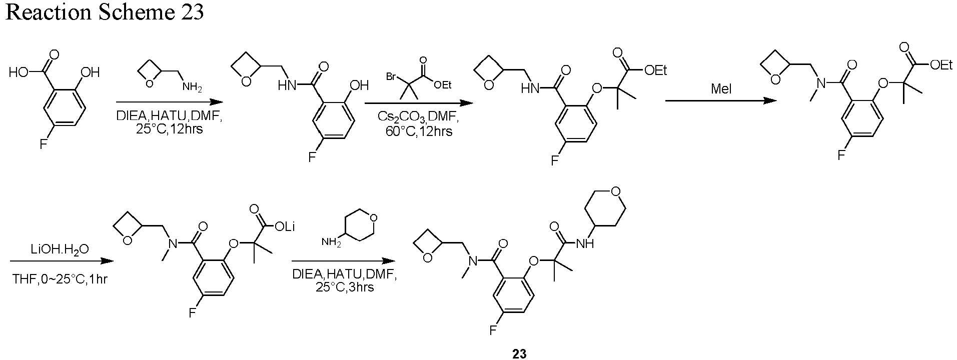

Following Reaction Scheme 23, , 2-(2-(tetrahydro-2H-pyran-4-ylcarbamoyl)propan-2-yloxy)-5-fluoro-N-methyl-N-((oxetan-2-yl)methyl)benzamide **(Compound 23)** (5.65mg) was obtained as a yellow solid. The structure of Compound 23 was confirmed by ¹H NMR (400 MHz, MeOD) δ 7.03-6.83 (m, 3H), 5.08-5.04 (m, 1H), 4.90-4.88 (m, 3H), 3.83-3.80 (m, 4H), 3.39-3.35 (m, 2H), 3.33-2.63 (m, 5H), 1.66-1.40 (m, 10 H).¹⁹F NMR (376.5 MHz, MeOD-d) δ = -121.90.MS *m*/*z:* 409 [MH⁺].

### Synthesis Example 24: Synthesis of Compound 24

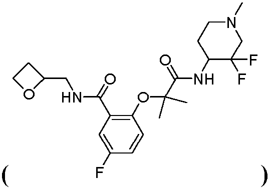

Compound 24 was synthesized according to the following Reaction Scheme 24.

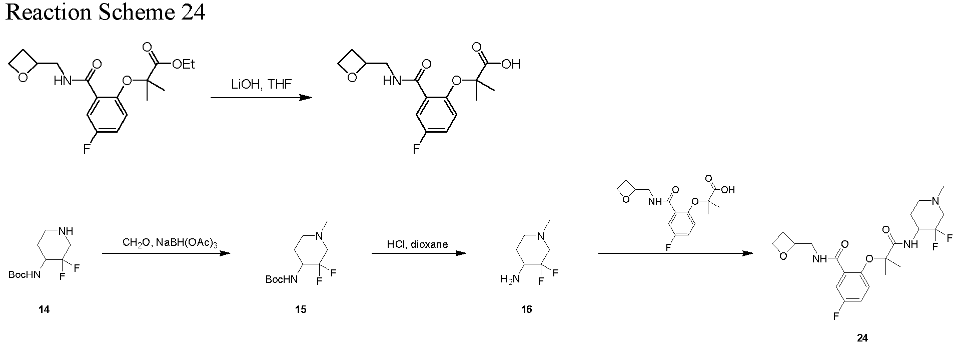

Following Reaction Scheme 24, 2-(2-(3,3-difluoro-1-methylpiperidin-4-ylcarbamoyl)propan-2-yloxy)-5-fluoro-N-((oxetan-2-yl)methyl)benzamide (Compound 24) (31.1 mg) was obtained as a white solid. The structure of Compound 24 was confirmed by ¹H NMR (400MHz, DMSO-d6) δ 8.58-8.56 (1H), 8.32 (d, *J =* 9.2 Hz, 1H), 7.41-7.38 (m, 1H), 7.25-7.23 (m, 1H), 6.99-6.96 (m, 1H), 4.86-4.82 (m, 1H), 4.53-4.51 (m, 1H), 4.44-4.42 (m, 1H), 3.57-3.46 (m, 2H), 2.98-2.61 (m, 3H), 2.43-2.22 (m, 6H), 1.76-1.64 (m, 2H), 1.45-1.44 (m, 6H). ¹⁹F NMR (376.5 MHz, DMSO*-d*₆) δ -106.12, -114.32, -120.58. MS *m*/*z:* 444 [MH⁺].

### Synthesis Example 25: Synthesis of Compound 25

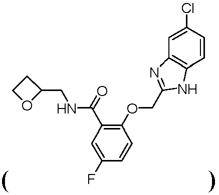

Compound 25 was synthesized according to the following Reaction Scheme 25.

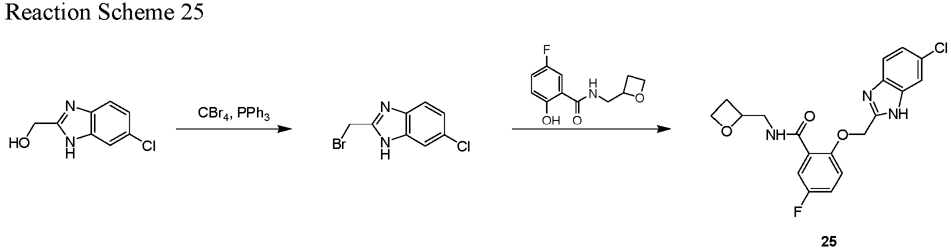

Following Reaction Scheme 25, 2-((6-chloro-1H-benzo[d]imidazol-2-yl)methoxy)-5-fluoro-N-((oxetan-2-yl)methyl)benzamide **(Compound 25)** (52.8 mg) was obtained as a white solid. The structure of Compound 25 was confirmed by ¹H NMR (400MHz, CDCl3) δ 11.25 (m, 1H), 7.77-6.91 (m, 7H), 5.27-4.96 (m, 5H), 3.63-3.61 (m, 1H), 3.52-3.49 (m, 1H), 2.61-2.59 (m, 1H), 2.37-2.35 (m, 1H). ¹⁹F NMR (376.5 MHz, CDCl₃) δ -120.0. MS *m*/*z:* 390 [MH⁺].

### Synthesis Example 26: Synthesis of Compound 26

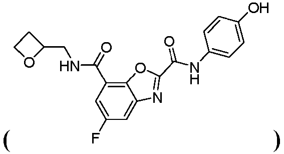

Compound 26 was synthesized according to the following Reaction Scheme 26.

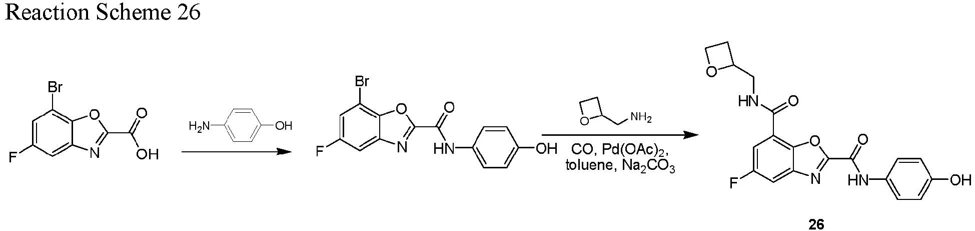

As shown in Reaction Scheme 26, first, 7-bromo-5-fluoro-N-(4-hydroxyphenyl)benzo[d]oxazole-2-carboxamide was prepared. To a solution of 7-bromo-5-fluoro-1,3-benzoxazole-2-carboxylic acid (1 g, 3.85 mmol, 1 eq) in DMF (5 mL) was added N-methylmorpholine (NMM) (1.95 g, 19.23 mmol, 2.11 mL, 5 eq), HOBt (1.04 g, 7.69 mmol, 2 eq), and 4-aminophenol (629.53 mg, 5.77 mmol, 1.5 eq). After stirring at 25°C for 0.5 h, EDCI (1.47 g, 7.69 mmol, 2 eq) was added. The mixture was stirred at 25°C for 16 hr and quenched with H₂O (10 mL), and extracted three times with 20 mL EtOAc (20 mL x 3). The combined organic layers were washed three times with 30 mL brine (30 mL x 3), dried over Na₂SO₄, filtered and concentrated to give 7-bromo-5-fluoro-N-(4-hydroxyphenyl)-1,3-benzoxazole-2-carboxamide (280 mg, 20.73% yield, 100% purity) as a yellow solid. ESI calculation for C₁₄H₉BrFN₂O₃ [MH]⁺ 351.0; found 351.0.

Next 5-fluoro-N2-(4-hydroxyphenyl)-N7-(oxetan-2-ylmethyl)benzo[d]oxazole-2,7-dicarboxamide **(Compound 26)** was prepared. To a solution of 7-bromo-5-fluoro-N-(4-hydroxyphenyl)-1,3-benzoxazole-2-carboxamide (280 mg, 797.43 µmol, 1 eq) in toluene (3 mL) was added oxetan-2-ylmethanamine (104.21 mg, 1.20 mmol, 1.5 eq), Pd(OAc)₂ (3.58 mg, 15.95 µmol, 0.02 eq), Xantphos (9.23 mg, 15.95 µmol, 0.02 eq) and Na₂CO₃ (253.56 mg, 2.39 mmol, 3 eq). After stirring at 80°C for 16hr under CO atmosphere (15 Psi), the reaction mixture was filtered and washed three times with 2 mL toluene (3 x 2 mL). The combined organic phase was concentrated and triturated with isopropyl ether (3 mL) at 25°C for 0.5 h to give 5-fluoro-N2-(4-hydroxyphenyl)-N7-(oxetan-2-ylmethyl)benzo[d]oxazole-2,7-dicarboxamide **(Compound 26)** (17.7 mg, 3.3% yield, 100% purity) as a yellow solid. The structure of Compound 26 was confirmed by ¹H NMR (400MHz, DMSO-d6) δ = 11.01 (s, 1H), 8.76 (s, 1H), 8.00 (dd, J = 8.0, 2.4 Hz, 1H), 7.73 (dd, J = 10, 2.4 Hz, 1H), 7.63 (d, J = 8.8 Hz, 2H), 6.78 (d, J = 8.8 Hz, 2H), 4.93 (d, J = 6.8 Hz, 1H), 4.63-4.50 (m, 2H), 3.73-3.63 (m, 1H), 3.57-3.51 (m, 1H), 2.71-2.61 (m, 2H).¹⁹F NMR (376.5 MHz, DMSO-d6) δ = -115.68.ESI calculation for C₁₉H₁₇FN₃O₅ [MH]⁺ 386.1; found 386.0.

### Synthesis Example 27: Synthesis of Compound 27

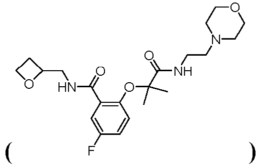

Compound 27 was synthesized according to the following Reaction Scheme 27.

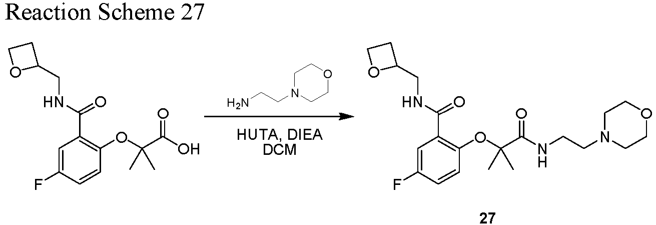

As shown in Reaction Scheme 27, 2-[1, 1-dimethyl-2-(2-morpholinoethylamino)-2-oxo-ethoxy]-5-fluoro-N-(oxetan-2-ylmethyl)benzamide **(Compound 27)** was prepared. To a solution of 2-[4-fluoro-2-(oxetan-2-ylmethylcarbamoyl)phenoxy]-2-methyl-propanoic acid (0.3 g, 963.69 µmol, 1 eq) in DMF (3 mL) was added HATU (549.63 mg, 1.45 mmol, 1.5 eq), and DIEA (498.20 mg, 3.85 mmol, 671.43 µL, 4 eq). After stirring at 25°C for 15min, 2-morpholinoethanamine (150.55 mg, 1.16 mmol, 151.77 µL, 1.2 eq) was added. After stirring at 25°C for 16hr, the reaction mixture was quenched by addition of H₂O (10 mL) at 25°C and extracted three times with 5 mL EtOAc (5 mL x 3). The combined organic layers were dried over Na₂SO₄, filtered, concentrated, and purified by pre-HPLC (column: CD02-Waters XBRIDGE^{®} BEH C18 150*25*10um; mobile phase: [water( NH₄HCO₃)-ACN]; gradient:16%-46% B over 11 min) to give 2-[1, 1-dimethyl-2-(2-morpholinoethylamino)-2-oxo-ethoxy]-5-fluoro-N-(oxetan-2-ylmethyl)benzamide **(Compound 27)** (134.3 mg, 32.15% yield) as a yellow solid. The structure of Compound 27 was confirmed by ¹H NMR (400 MHz, CDCl₃) δ = 8.19 (s, 1H), 7.86 (dd, *J* = 9.2, 3.2Hz, 1H), 7.07-7.01 (m, 2H), 6.88-6.84 (m, 1H), 5.04-4.99 (m, 1H), 4.73-4.67(m, 1H), 4.55-4.49(m, 1H), 3.79-3.71 (m, 2H), 3.57-3.52 (m, 4H), 3.43-3.37 (m, 2H), 2.75-2.66(m, 1H), 2.52-2.43(m, 3H), 2.38-2.33 (m, 4H), 1.62(d, *J* = 2.4Hz, 6H). ¹⁹F NMR(376.5 MHz, CDCl₃) δ = - 119.345. ESI calculation for C₂₁H₃₁FN₃O₅ [MH]⁺ 424.2; found 424.2.

### Synthesis Example 28: Synthesis of Compound 28

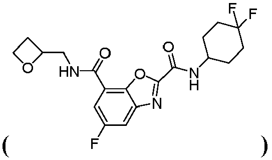

Compound 28 was synthesized according to the following Reaction Scheme 28.

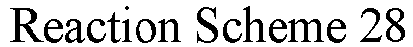

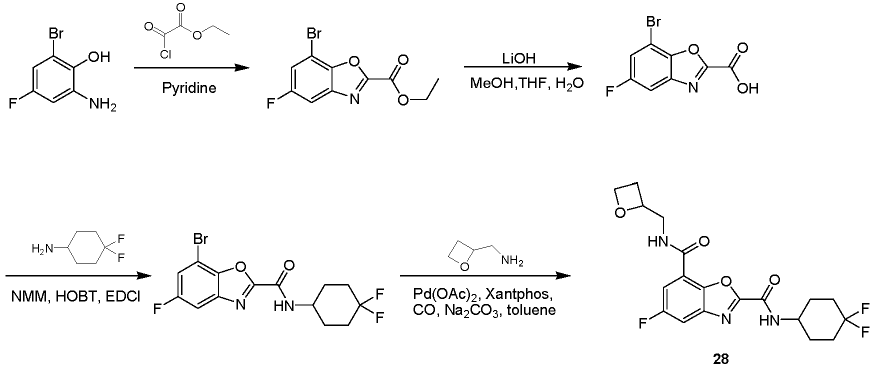

As shown in Reaction Scheme 28, 7-bromo-N-(4,4-difluorocyclohexyl)-5-fluorobenzo[d]oxazole-2-carboxamide was prepared. To a solution of 7-bromo-5-fluorobenzo[d]oxazole-2-carboxylic acid (200 mg, 769.18 µmol, 1 eq) in DMF (5 mL) was added NMM (389.00 mg, 3.85 mmol, 422.83 µL, 5 eq), HOBT (207.87 mg, 1.54 mmol, 2 eq), and 4,4-difluorocyclohexan-1-amine (155.94 mg, 1.15 mmol, 1.5 eq). After stirring at 25°C for 20 mins, EDCI (294.91 mg, 1.54 mmol, 2 eq) was added. The mixture was stirred at 25°C for 16 hr to give a dark brown solution. The reaction was quenched with H₂O (10 mL) and extracted three times with 20 mL EtOAc (20 mL x 3). The combined organic layers were washed with brine (30 mL), dried over Na₂SO₄, filtered, and concentrated under vacuum. The residue was purified by column chromatography (SiO₂, Petroleum ether/Ethyl acetate=20/1 to 8/1) to give 7-bromo-N-(4,4-difluorocyclohexyl)-5-fluorobenzo[d]oxazole-2-carboxamide (160 mg, 424.23 µmol, 55.2% yield) as a white solid. The structure of 7-bromo-N-(4,4-difluorocyclohexyl)-5-fluorobenzo[d]oxazole-2-carboxamide was confirmed by ¹H NMR (400MHz, CDCl3) δ = 7.45 - 7.42 (m, 2H), 7.10 (d, J = 7.8 Hz, 1H), 4.17 - 4.09 (m, 1H), 2.22 - 2.13 (m, 4H), 2.02 - 1.85 (m, 2H), 1.78 - 1.68 (m, 2H). 19F NMR (376.5 MHz, CDCl3) δ = -95.18 (d, J = 243.2 Hz), -101.33 (d, J = 243.2 Hz), -113.52. ESI calculation for C₁₄H₁₂BrF₃N₂O₂Na [MNa]⁺ 401.0; found 400.8.

Then, N2-(4,4-difluorocyclohexyl)-5-fluoro-N7-(oxetan-2-ylmethyl)benzo[d]oxazole-2,7-dicarboxamide **(Compound 28)** was prepared. A mixture of 7-bromo-N-(4,4-difluorocyclohexyl)-5-fluorobenzo[d]oxazole-2-carboxamide (180 mg, 477.26 µmol, 1 eq), Xantphos (5.52 mg, 9.55 µmol, 0.02 eq), Pd(OAc)₂ (2.14 mg, 9.55 µmol, 0.02 eq), oxetan-2-ylmethanamine (62.37 mg, 715.88 µmol, 1.5 eq), and Na₂CO₃ (151.75 mg, 1.43 mmol, 3 eq) in Toluene (5 mL) was degassed and purged with Ar and then CO three times, and then the mixture was stirred at 80°C for 16 hr under CO (15 psi) in a 30 mL of sealed tube to give a yellow solution. The reaction mixture was filtered, and the solvent was removed under vacuum. The residue was purified by Prep-TLC (SiO₂, Petroleum ether/Ethyl acetate = 1:2) to give N2-(4,4-difluorocyclohexyl)-5-fluoro-N7-(oxetan-2-ylmethyl)benzo[d]oxazole-2,7-dicarboxamide **(Compound 28)** (38 mg, 92.37 µmol, 19.4% yield) as a white solid. The structure of Compound 28 was confirmed by ¹H NMR (400MHz, CDCl3) δ = 8.00 (dd, J = 9.6, 2.4 Hz, 1H), 7.69 (brs, 1 H), 7.62 (dd, J = 7.2, 2.4 Hz, 1H), 7.18 (d, J = 8.0 Hz, 1H), 5.17 - 5.12 (m, 1H), 4.79 (t, J = 8.0 Hz, 2H), 4.17 - 4.08 (m, 1H), 3.90 - 3.74 (m, 2H), 2.79 - 2.58 (m, 2H), 2.24 - 2.12 (m, 4H), 2.02 - 1.84 (m, 2H), 1.77 - 1.66 (m, 2H). ¹⁹F NMR (376.5 MHz, CDCl3) δ = -95.18 (d, J = 240.2 Hz), -101.38 (d, J = 240.2 Hz), -113.34. ESI calculation for C₁₉H₂₁F₃N₃O₄ [MH]⁺ 412.1; found 412.0.

### Synthesis Example 29: Synthesis of Compound 29

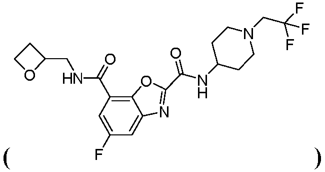

Compound 29 was synthesized according to the following Reaction Scheme 29.

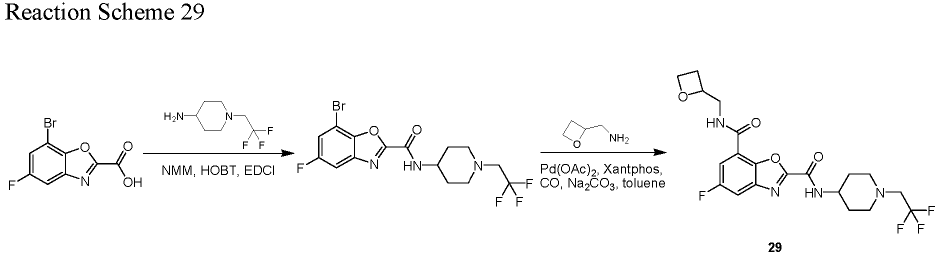

As shown in Reaction Scheme 29, first, 7-bromo-5-fluoro-N-(1-(2,2,2-trifluoroethyl)piperidin-4-yl)benzo[d]oxazole-2-carboxamide was prepared. To a solution of 7-bromo-5-fluorobenzo[d]oxazole-2-carboxylic acid (200.00 mg, 769.18 µmol, 1 eq) in DMF (5 mL) was added NMM (389.00 mg, 3.85 mmol, 422.83 µL, 5 eq), HOBT (207.87 mg, 1.54 mmol, 2 eq), and 1-(2,2,2-trifluoroethyl)piperidin-4-amine (210.20 mg, 1.15 mmol, 1.5 eq). After stirring at 25°C for 20 mins, EDCI (294.91 mg, 1.54 mmol, 2 eq) was added. The mixture was stirred at 25°C for 16 hr to give a dark brown solution. The reaction was quenched with H₂O (10 mL) and extracted three times with 20 mL EtOAc (20 mL x 3). The combined organic layers were washed with brine (30 mL), dried over Na₂SO₄, filtered and concentrated under vacuum. The residue was purified by column chromatography (SiO₂, Petroleum ether/Ethyl acetate=20/1 to 5/1) to give 7-bromo-5-fluoro-N-(1-(2,2,2-trifluoroethyl)piperidin-4-yl)benzo[d]oxazole-2-carboxamide (160 mg, 377.19 µmol, 49.0% yield) as a white solid. The structure of 7-bromo-5-fluoro-N-(1-(2,2,2-trifluoroethyl)piperidin-4-yl)benzo[d]oxazole-2-carboxamide was confirmed by ¹H NMR (400MHz, CDCl3) δ = 7.43 (d, J = 8.0 Hz, 2H), 7.10 (d, J = 8.0 Hz, 1H), 4.08 - 3.99 (m, 1H), 3.05 - 2.98 (m, 4H), 2.58 (t, J = 10.8 Hz, 2H), 2.07 - 2.04 (m, 2H), 1.75 - 1.65 (m, 2H). ¹⁹F NMR (376.5 MHz, CDCl3) δ = -69.157, -113.64. ESI calculation for C₁₅H₁₅BrF₄N₃O₂ [MH]⁺ 424.0; found 423.9.

Next, 5-fluoro-N7-(oxetan-2-ylmethyl)-N2-(1-(2,2,2-trifluoroethyl)piperidin-4-yl)benzo[d]oxazole-2,7-dicarboxamide **(Compound 29)** was prepared. To a solution of 7-bromo-5-fluoro-N-(1-(2,2,2-trifluoroethyl)piperidin-4-yl)benzo[d]oxazole-2-carboxamide (160 mg, 377.19 µmol, 1 eq) in toluene (5 mL) was added oxetan-2-ylmethanamine (49.29 mg, 565.79 µmol, 1.5 eq), Xantphos (4.36 mg, 7.54 µmol, 0.02 eq), Pd(OAc)₂ (1.69 mg, 7.54 µmol, 0.02 eq) and Na₂CO₃ (119.93 mg, 1.13 mmol, 1.5 eq). The reaction mixture was stirred under CO (15 Psi) at 80°C for 16hr in a 30 mL of sealed tube to give a dark green solution. The reaction mixture was filtered, and the solvent was removed under vacuum. The residue was purified by Prep-TLC (SiO₂, Petroleum ether/Ethyl acetate = 1:1) to give 5-fluoro-N7-(oxetan-2-ylmethyl)-N2-(1-(2,2,2-trifluoroethyl)piperidin-4-yl)benzo[d]oxazole-2,7-dicarboxamide **(Compound 29)** (33 mg, 71.99 µmol, 19.1% yield) as a white solid. The structure of Compound 29 was confirmed by ¹H NMR (400MHz, CDCl3) δ = 7.99 (dd, J = 9.6, 2.4 Hz, 1H), 7.80 (brs, 1 H), 7.61 (dd, J = 7.2, 2.4 Hz, 1H), 7.22 (d, J = 8.0 Hz, 1H), 5.16 - 5.10 (m, 1H), 4.77 (t, J = 7.2 Hz, 2H), 4.05 - 3.99 (m, 1H), 3.90 - 3.74 (m, 2H), 3.04 - 2.97 (m, 4H), 2.78 - 2.69 (m, 1H), 2.65 - 2.52 (m, 3H), 2.05 - 1.98 (m, 2H), 1.74 - 1.63 (m, 2H).¹⁹F NMR (376.5 MHz, CDCl3) δ = -69.28, -113.49. ESI calculation for C₂₀H₂₃F₄N₄O₄ [MH]⁺ 459.2; found 458.9.

### Synthesis Example 30: Synthesis of Compound 30

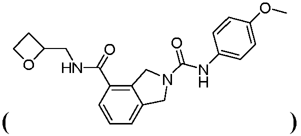

Compound 30 was synthesized according to the following Reaction Scheme 30.

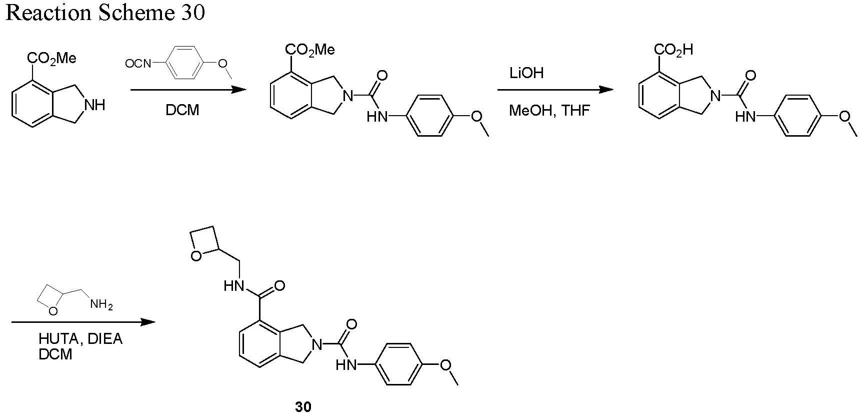

As shown in Reaction Scheme 30, first, methyl 2-((4-methoxyphenyl)carbamoyl)isoindoline-4-carboxylate was prepared. To a solution of methyl 2,3-dihydro-1H-isoindole-4-carboxylate (300 mg, 1.40 mmol, 1 eq, HCl salt) in DCM (5 mL) was added TEA (142.08 mg, 1.40 mmol, 195.43 µL, 1 eq) and 1-isocyanato-4-methoxy-benzene (209.42 mg, 1.40 mmol, 180.85 µL, 1 eq) at 25°C. The mixture was stirred at 25°C for 3hr to give a white suspension. The solvent was removed under reduced pressure. The residue was purified by column chromatography (SiO₂, Petroleum ether/Ethyl acetate=20/1 to 5/1) to give methyl 2-((4-methoxyphenyl)carbamoyl)isoindoline-4-carboxylate (450 mg, 1.38 mmol, 98.2% yield) as a white solid. The structure of methyl 2-((4-methoxyphenyl)carbamoyl)isoindoline-4-carboxylate was confirmed by ¹H NMR (400MHz, CDCl3) δ = 7.97 (d, J = 7.6 Hz, 1H), 7.49 (d, J = 7.6 Hz, 1H), 7.42 - 7.35 (m, 3H), 6.86 - 6.82 (m, 2H), 6.39 (s, 1H), 5.10 (s, 2H), 4.86 (s, 2H), 3.96 (s, 3H), 3.77 (s, 3H). ESI calculation for C₁₈H₁₉N₂O₄ [MH]⁺ 327.1; found 327.2.

Next, 2-((4-methoxyphenyl)carbamoyl)isoindoline-4-carboxylic acid was prepared. To a solution of methyl 2-((4-methoxyphenyl)carbamoyl)isoindoline-4-carboxylate (450 mg, 1.38 mmol, 1 eq) in MeOH (10 mL), THF (5 mL) and H₂O (1 mL) was added LiOH (49.53 mg, 2.07 mmol, 1.5 eq). The mixture was stirred at 25°C for 0.5 hr to give a pale pink solution. The reaction mixture was diluted with toluene (20 mL) and concentrated under vacuum. The crude product 2-((4-methoxyphenyl)carbamoyl)isoindoline-4-carboxylic acid (400 mg, 1.28 mmol, 92.9% yield) was obtained as a white solid and was used in the next step without further purification. ESI calculation for C₁₇H₁₇N₂O₄ [MH]⁺ 313.3; found 313.0.

Finally, N2-(4-methoxyphenyl)-N4-((oxetan-2-yl)methyl)isoindoline-2,4-dicarboxamide **(Compound 30)** was prepared. To a solution of 2-((4-methoxyphenyl)carbamoyl)isoindoline-4-carboxylic acid (400 mg, 1.28 mmol, 1 eq) in DMF (5 mL) was added HATU (973.95 mg, 2.56 mmol, 2 eq), DIEA (1.66 g, 12.81 mmol, 2.23 mL, 10 eq), and oxetan-2-ylmethanamine (111.58 mg, 1.28 mmol, 1 eq). The mixture was stirred at 50°C for 3 hr to give a brown solution. The mixture was quenched with H₂O (30 mL), diluted with EtOAc (30 mL), extracted three times with 30 mL EtOAc (30 mL x 3). The combined organic layers were washed with brine (30 mL), dried over Na₂SO₄, filtered and concentrated under vacuum. The residue was purified by column chromatography (SiO₂, Petroleum ether/Ethyl acetate=5/1 to 2/1) to give N2-(4-methoxyphenyl)-N4-((oxetan-2-yl)methyl)isoindoline-2,4-dicarboxamide **(Compound 30)** (38 mg, 99.63 µmol, 7.8% yield) as a white foam. The Compound 30 structure was confirmed by ¹H NMR (400MHz, CDCl3) δ = 7.54 (d, J = 7.6 Hz, 1H), 7.47 (d, J = 7.2 Hz, 1H), 7.42 (d, J = 7.2 Hz, 1H), 7.39 - 7.36 (m, 2H), 6.88 - 6.85 (m, 2H), 6.70 (brs, 1H), 6.31 (s, 1H), 6.13 (s, 2H), 5.08 - 5.02 (m, 1H), 4.87 (s, 2H), 4.75 - 4.70 (m, 1H), 4.58 - 4.52 (m, 1H), 3.83 - 3.76 (m, 1H), 3.79 (s, 3H), 3.68 - 3.63 (m, 1H), 2.76 - 2.68 (m, 1H), 2.56 - 2.47 (m, 1H). ESI calculation for C₂₁H₂₄N₃O₄ [MH]⁺ 382.2; found 382.1.

### Synthesis Example 31: Synthesis of Compound 31

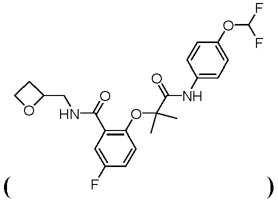

Compound 31 was synthesized according to the following Reaction Scheme 31.

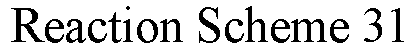

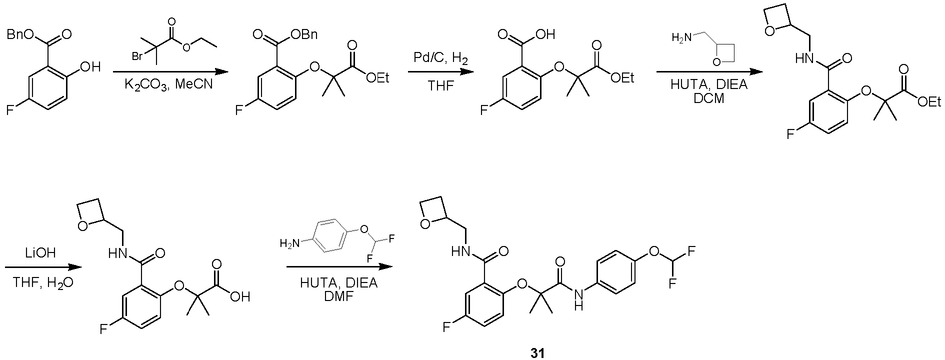

As shown in Reaction Scheme 31, first, benzyl 2-((1-ethoxy-2-methyl-1-oxopropan-2-yl)oxy)-5-fluorobenzoate was prepared. To a solution of benzyl 5-fluoro-2-hydroxybenzoate (3 g, 12.18 mmol, 1 eq) in MeCN (30 mL) was added ethyl 2-bromo-2-methyl-propanoate (8.32 g, 42.64 mmol, 6.26 mL, 3.5 eq) and K₂CO₃ (5.89 g, 42.64 mmol, 3.5 eq). The mixture was stirred at 80°C for 20 hr to give a white suspension. The suspension was filtered through a pad of CELITE^{®} and the filter cake was washed with EtOAc (50 mL). Then, the filtrate was washed with water (100 mL) and extracted three times with 50 mL EtOAc (50 mL x 3). The combined organic layers were dried over Na₂SO₄, filtered and concentrated under vacuum. The residue was purified by column chromatography (SiO₂, Petroleum ether/Ethyl acetate=99/1 to 95/5) to give: benzyl 2-((1-ethoxy-2-methyl-1-oxopropan-2-yl)oxy)-5-fluorobenzoate (3.1 g, 8.60 mmol, 70.6% yield) as a light-yellow liquid. The structure of benzyl 2-((1-ethoxy-2-methyl-1-oxopropan-2-yl)oxy)-5-fluorobenzoate was confirmed by ¹H NMR (400MHz, CDCl₃) δ = 7.48 - 7.30 (m, 6H), 7.09 - 7.04 (m, 1H), 6.87 (dd, J = 8.8, 4.4 Hz, 1H), 5.35 (s, 2H), 4.23 (q, J = 6.8 Hz, 2H), 1.49 (s, 6H), 1.26 (t, J = 7.2 Hz, 3H). ¹⁹F NMR (376.SMHz, CDCl3) δ = -120.37. ESI calculation for C₂₀H₂₂FO₅ [MH]⁺ 361.1; found 361.0.

Next, 2-((1-ethoxy-2-methyl-1-oxopropan-2-yl)oxy)-5-fluorobenzoic acid was prepared. To a 100 mL sealed tube was carefully added wet Pd/C (310 mg, 291.30 µmol, 10% purity, 2 eq) under Ar. A solution of benzyl 2-((1-ethoxy-2-methyl-1-oxopropan-2-yl)oxy)-5-fluorobenzoate (3.1 g, 8.60 mmol, 1 eq) in THF (30 mL) was added into the tube. And the tube was purged with Ar, then evacuated under vacuum three times, and then purged with H₂ three times. The reaction mixture was stirred under H₂ (15 Psi) at 25°C for 16 hr to give a black suspension. The catalyst was carefully removed by filtration under Ar to give a colorless solution, and the filtrate was concentrated under vacuum. The residue was purified by column chromatography (SiO₂, Petroleum ether/Ethyl acetate=20/1 to 5/1) to give 2-((1-ethoxy-2-methyl-1-oxopropan-2-yl)oxy)-5-fluorobenzoic acid (2.2 g, 8.14 mmol, 94.6% yield) as a white solid. The structure of 2-((1-ethoxy-2-methyl-1-oxopropan-2-yl)oxy)-5-fluorobenzoic acid was confirmed by ¹H NMR (400MHz, CDCl3) δ = 11.19 (brs, 1H), 7.89 (dd, J = 8.4, 3.2 Hz, 1H), 7.21 - 7.16 (m, 1H), 6.95 (dd, J = 8.8, 4.0 Hz, 1H), 4.28 (q, J = 6.8 Hz, 2H), 1.73 (s, 6 H), 1.28 (t, J = 6.8 Hz, 3H).¹⁹F NMR (376.5MHz, CDCl3) δ = -117.93. ESI calculation for C₁₃H₁₅FO₅Na [MNa]⁺ 293.1; found 292.9.

Next, ethyl 2-(4-fluoro-2-((oxetan-2-ylmethyl)carbamoyl)phenoxy)-2-methylpropanoate was prepared. To a solution of 2-((1-ethoxy-2-methyl-1-oxopropan-2-yl)oxy)-5-fluorobenzoic acid (500 mg, 1.85 mmol, 1 eq) in DMF (5 mL) was added HATU (1.41 g, 3.70 mmol, 2 eq) and DIEA (1.20 g, 9.25 mmol, 1.61 mL, 5 eq) at 25°C, and the reaction mixture was stirred for 30 mins, followed by addition of oxetan-2-ylmethanamine (193.42 mg, 2.22 mmol, 1.2 eq). Then, the reaction mixture was stirred at 25°C for 16 hr to give a dark brown solution. The reaction mixture was quenched with H₂O (10 mL) and extracted three times with 10 mL EtOAc (10 mL x 3). The combined organic layers were washed three times with 10 mL brine (10 mL x 3), dried over Na₂SO₄, filtered, concentrated and purified by column chromatography (SiO2, Petroleum ether/Ethyl acetate=20/1 to 8/1) to give ethyl 2-(4-fluoro-2-((oxetan-2-ylmethyl)carbamoyl)phenoxy)-2-methylpropanoate (420 mg, 1.24 mmol, 66.9% yield) as a light yellow liquid. The structure of ethyl 2-(4-fluoro-2-((oxetan-2-ylmethyl)carbamoyl)phenoxy)-2-methylpropanoate was confirmed by ¹H NMR (400MHz, CDCl3) δ = 8.47 (s, 1H), 7.90 (dd, J = 9.6, 3.2 Hz, 1H), 7.06 - 7.01 (m, 1H), 6.81 (dd, J = 9.2, 4.4 Hz, 1H), 5.05 - 4.99 (m, 1H), 4.69 - 4.64 (m, 1H), 4.55 - 4.49 (m, 1H), 4.25 (q, J = 7.2 Hz, 2H), 3.85 - 3.78 (m, 1H), 3.67 - 3.61 (m, 1H), 2.72 - 2.62 (m, 1H), 2.53 - 2.45 (m, 1H), 1.66 (d, J = 8.8 Hz, 6H), 1.25 (t, J = 7.2 Hz, 3H). ¹⁹F NMR (376.5MHz, CDCl3) δ = -119.83. ESI calculation for C₁₇H₂₃FNO₅ [MH]⁺ 340.2; found 340.1.

Next, 2-(4-fluoro-2-((oxetan-2-ylmethyl)carbamoyl)phenoxy)-2-methylpropanoic acid was prepared. To a solution of ethyl 2-(4-fluoro-2-((oxetan-2-ylmethyl)carbamoyl)phenoxy)-2-methylpropanoate (420 mg, 1.24 mmol, 1 eq) in THF (0.75 mL), MeOH (1.5 mL) and H₂O (0.1 mL) was added LiOH (32.60 mg, 1.36 mmol, 1.1 eq) at 25°C. The mixture was stirred at 25°C for 1hr to give a light-yellow solution. The reaction mixture was diluted with toluene (20 mL) and concentrated under vacuum. 2-(4-fluoro-2-((oxetan-2-ylmethyl)carbamoyl)phenoxy)-2-methylpropanoic acid (380 mg, 1.22 mmol, 98.6% yield) was obtained as a light-yellow solid. The structure of 2-(4-fluoro-2-((oxetan-2-ylmethyl)carbamoyl)phenoxy)-2-methylpropanoic acid was confirmed by ¹H NMR (400MHz, CDCl3) δ = 10.09 (s, 1H), 7.90 (s, 1H), 7.24 - 7.16 (m, 1H), 7.06 (brs, 2H), 5.06 (s, 1H), 4.63 - 4.50 (m, 2H), 3.95 - 3.64 (m, 2H), 2.63 - 2.47 (m, 2H), 1.56 (s, 6H).¹⁹F NMR (376.5MHz, CDCl3) δ = -119.63. ESI calculation for C₁₅H₁₉FNO₅ [MH]⁺ 312.1; found 311.9.

Finally, 2-((1-((4-(difluoromethoxy)phenyl)amino)-2-methyl-1-oxopropan-2-yl)oxy)-5-fluoro-N-(oxetan-2-ylmethyl)benzamide **(Compound 31)** was prepared. To a solution of 2-(4-fluoro-2-((oxetan-2-ylmethyl)carbamoyl)phenoxy)-2-methylpropanoic acid (380 mg, 1.22 mmol, 1 eq) in DMF (8 mL) was added HATU (1.16 g, 3.05 mmol, 2.5 eq) DIEA (788.81 mg, 6.10 mmol, 1.06 mL, 5 eq) at 25°C, and the reaction mixture was stirred for 30 mins to give an orange solution, followed by addition of 4-(difluoromethoxy)aniline (291.37 mg, 1.83 mmol, 1.5 eq). The mixture was stirred at 25°C for 16 hr to give a brown solution. The mixture was quenched with H₂O (30 mL), diluted with EtOAc (30 mL), and extracted three times with 30 mL EtOAc (30 mL x 3). The combined organic layers were washed with brine (30 mL), dried over Na₂SO₄, filtered and concentrated under vacuum. The residue was purified by column chromatography (SiO₂, Petroleum ether/Ethyl acetate=6/1 to 2/1) to give 2-((1-((4-(difluoromethoxy)phenyl)amino)-2-methyl-1-oxopropan-2-yl)oxy)-5-fluoro-N-(oxetan-2-ylmethyl)benzamide **(Compound 31)** (229 mg, 506.16 µmol, 41.5% yield) as a white solid. The structure of Compound 31 was confirmed by ¹H NMR (400MHz, CDCl3) δ = 9.15 (s, 1H), 7.71 - 7.67 (m, 2H), 7.58 (dd, J = 8.8, 3.2 Hz, 1H), 7.37 (brs, 1H), 7.12 - 7.10 (m, 2H), 7.09 - 6.99 (m, 2H), 6.47 (t, J = 74 Hz, 1H), 5.02 - 4.96 (m, 1H), 4.71 - 4.65 (m, 1H), 4.52 - 4.47 (m, 1H), 3.74 - 3.71 (m, 2H), 2.71 - 2.63 (m, 1H), 2.51 - 2.43 (m, 1H), 1.68 (d, J = 3.2 Hz, 6H). ¹⁹F NMR (376.5MHz, CDCl3) δ = -80.67, -118.11. ESI calculation for C₂₂H₂₄F₃N₂O₅ [MH]⁺ 453.2; found 452.9.

### Synthesis Example 32: Synthesis of Compound 32

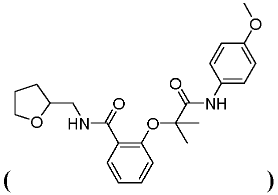

Compound 32 was synthesized according to the following Reaction Scheme 32.

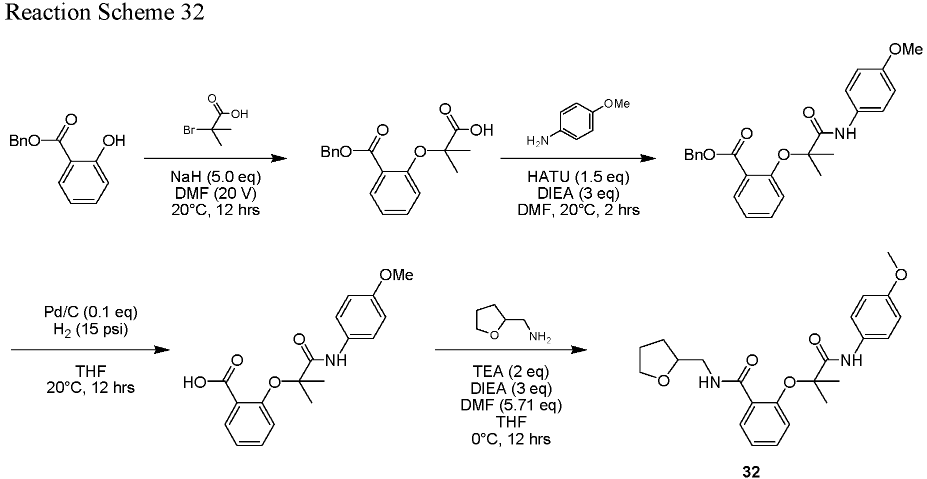

As shown in Reaction Scheme 32, 2-(2-(4-methoxyphenylcarbamoyl)propan-2-yloxy)-N-((tetrahydrofuran-2-yl)methyl)benzamide (compound 32) was prepared. To a solution of 2-(2-(4-methoxyphenylcarbamoyl)propan-2-yloxy)benzoic acid (0.15 g, 455.45 umol) in DMF (3 mL) was added DIEA (176.59 mg, 1.37 mmol) and HATU (285.74 mg, 751.49 umol) at 0°C, and the resulting solution was stirred at 20°C for 12 hrs. LC-MS showed 79% of the product with desired mass was detected. The reaction mixture was quenched by water (5 mL) at 20°C. The mixture was extracted three times with 5 mL ethyl acetate (3 x 5 mL). The combined organic layers were washed three times with 5 mL water (3 x 5 mL), dried over Na2SO4, filtered and concentrated under reduced pressure. The residue was purified by prep-HPLC (NH₄HCO₃) to give 2-(2-(4-methoxyphenylcarbamoyl)propan-2-yloxy)-N-((tetrahydrofuran-2-yl)methyl)benzamide **(Compound 32)** (109.9 mg, 58.5% yield) as a white solid. The structure of Compound 32 was confirmed by ¹H NMR: (400MHz, DMSO-d6) δ 1.57 (d, J=2.88 Hz, 6 H) 1.72 - 1.84 (m, 2 H) 1.84 - 1.95 (m, 1 H) 3.33 - 3.45 (m, 2 H) 3.59 (q, J=7.50 Hz, 1 H) 3.69 - 3.79 (m, 4 H) 3.90 - 3.99 (m, 1 H) 6.90 (d, J=9.13 Hz, 2 H) 7.01 (d, J=8.00 Hz, 1 H) 7.08 - 7.14 (m, 1 H) 7.38 - 7.45 (m, 1 H) 7.58 (d, J=9.01 Hz, 2 H) 7.65 (dd, J=7.63, 1.75 Hz, 1 H) 8.48 (br t, J=5.57 Hz, 1 H) 10.09 (s, 1 H).

### Synthesis Example 33: Synthesis of Compound 33

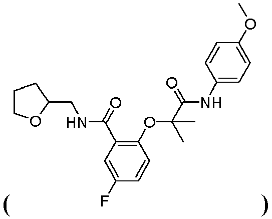

Compound 33 was synthesized according to the following Reaction Scheme 33.

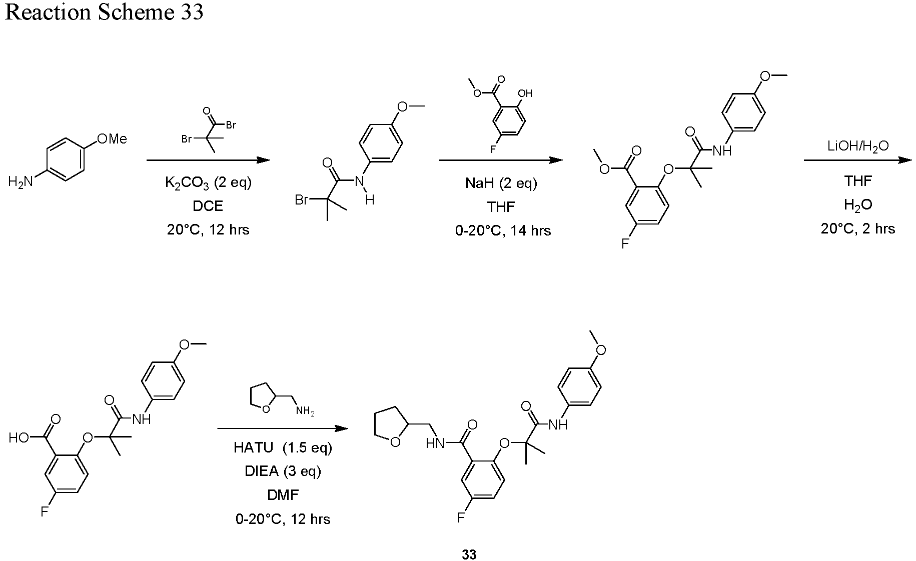

As shown in Reaction Scheme 33, first, 2-bromo-N-(4-methoxyphenyl)-2-methylpropanamide was prepared. To a solution of 4-methoxybenzenamine (1.2 g, 9.74 mmol) in 1,2-dichloroethane (24 mL) was added K₂CO₃ (2.69 g, 19.49 mmol) and 2-bromo-2-methylpropanoyl bromide (2.46 g, 10.72 mmol, 1.32 mL) at 20°C. Then, the reaction was stirred at 20°C for 12 hrs. LC-MS showed the reaction was completed. The reaction mixture was poured into ice water (20 mL) and extracted three times with 20 mL ethyl acetate (3 x 20 mL). The combined organic layers were washed with brine (30 mL), dried over Na₂SO₄, filtered and concentrated under reduced pressure to give crude product. Then, the crude product was purified by column chromatography on silica gel (eluted with ethyl acetate in petroleum ether from 0% to 6%) to give 2-bromo-N-(4-methoxyphenyl)-2-methylpropanamide (2.57 g, 87.23% yield) as a white solid. The structure of 2-bromo-N-(4-methoxyphenyl)-2-methylpropanamide was confirmed by ¹H NMR: (400 MHz, DMSO-d6) δ 9.66 (s, 1 H) 7.49 - 7.57 (m, 2 H) 6.86 - 6.93 (m, 2 H) 3.73 (s, 3 H) 1.98 (s, 6 H).

Next, methyl 2-(2-(4-methoxyphenylcarbamoyl)propan-2-yloxy)-5-fluorobenzoate was prepared. To a solution of methyl 5-fluoro-2-hydroxy-benzoate (450.14 mg, 2.65 mmol) in THF (10 mL) was added NaH (176.36 mg, 4.41 mmol) at 0°C. Then, the reaction mixture was stirred at 0°C for 2 hrs. Then 2-bromo-N-(4-methoxyphenyl)-2-methylpropanamide (600 mg, 2.20 mmol) was added to the reaction mixture at 0°C. The reaction mixture was stirred at 20°C for 12 hrs. LC-MS showed the starting material was consumed and the desired MS was detected. The reaction mixture was added drop-wise to saturated ammonium chloride aqueous solution (20 mL) at 0°C and extracted three times with 20 mL ethyl acetate (3 x 20 mL). The combined organic layers were washed with brine (30 mL), dried over Na₂SO₄, filtered and concentrated under reduced pressure to give crude product. Then crude product was purified by column chromatography on silica gel (elute ethyl acetate in petroleum ether from 0% to 6%) to afford methyl 2-(2-(4-methoxyphenylcarbamoyl)propan-2-yloxy)-5-fluorobenzoate (280 mg, 17.57% yield) as a white solid. The structure of methyl 2-(2-(4-methoxyphenylcarbamoyl)propan-2-yloxy)-5-fluorobenzoate was confirmed by ¹H NMR: (400 MHz, DMSO-d6) δ 10.16 (s, 1 H) 7.63 - 7.68 (m, 2 H) 7.30 - 7.36 (m, 3 H) 6.89 - 6.94 (m, 2 H) 3.85 (s, 3 H) 3.73 (s, 3 H) 1.54 (s, 6 H).

Next, 2-(2-(4-methoxyphenylcarbamoyl)propan-2-yloxy)-5-fluorobenzoic acid was prepared. To a solution of methyl 2-(2-(4-methoxyphenylcarbamoyl)propan-2-yloxy)-5-fluorobenzoate (341 mg, 471.82 umol) in THF (3.5 mL) was added the solution of LiOH·H₂O (39.60 mg, 943.65 umol) in H₂O (3.5 mL) at 20°C. Then, the reaction was stirred at 20°C for 2 hrs. LC-MS showed the starting material was consumed and the desired MS was detected. Then the reaction mixture was poured into water (5 mL) and washed twice with 5 mL ethyl acetate (2 x 5 mL). The aqueous phase was cooled to 0°C and adjusted to pH=2 by 1 N HCl at 0°C and then extracted three times with 5 mL ethyl acetate (3 x 5 mL). The combined organic was washed with brine (10 mL), dried over Na₂SO₄, filtered and concentrated under reduced pressure to give 2-(2-(4-methoxyphenylcarbamoyl)propan-2-yloxy)-5-fluorobenzoic acid (80 mg, 43.93% yield) as a white solid. The structure of 2-(2-(4-methoxyphenylcarbamoyl)propan-2-yloxy)-5-fluorobenzoic acid was confirmed by ¹H NMR: (400 MHz, DMSO-d6) δ 13.03 - 13.85 (m, 1 H) 10.41 (s, 1 H) 7.65 (d, J=8.88 Hz, 2 H) 7.61 (dd, J=8.88, 3.25 Hz, 1 H) 7.41 (td, J=8.50, 3.25 Hz, 1 H) 7.26 (dd, J=9.07, 4.57 Hz, 1 H) 6.92 (d, J=8.88 Hz, 2 H) 3.74 (s, 3 H) 1.56 (s, 6 H).

Finally, 2-(2-(4-methoxyphenylcarbamoyl)propan-2-yloxy)-5-fluoro-N-((tetrahydrofuran-2-yl)methyl)benzamide **(Compound 33)** was prepared. To a solution of 2-(2-(4-methoxyphenylcarbamoyl)propan-2-yloxy)-5-fluorobenzoic acid (80 mg, 230.32 umol) in DMF (1 mL) was added tetrahydrofuran -2-ylmethanamine (34.94 mg, 345.49 umol) and DIEA (89.30 mg, 690.97 umol) at 0°C. Then, HATU (87.58 mg, 230.32 umol) was added to the reaction mixture at 0°C. Then, the reaction mixture was stirred at 20°C for 12 hrs. LC-MS showed the starting material was consumed and the desired mass was detected. The reaction mixture was filtered, and the filtrate was purified by pre-HPLC and lyophilized to give 2-(2-(4-methoxyphenylcarbamoyl)propan-2-yloxy)-5-fluoro-N-((tetrahydrofuran-2-yl)methyl)benzamide **(Compound 33)** (26.9 mg, 27.13% yield) as a white solid. The structure of Compound 33 was confirmed by ¹H NMR: (400 MHz, DMSO-d6) δ 10.04 (s, 1 H) 8.57 (t, J=5.69 Hz, 1 H) 7.54 - 7.61 (m, 2 H) 7.42 (dd, J=8.94, 3.31 Hz, 1 H) 7.29 (ddd, J=8.94, 8.07, 3.38 Hz, 1 H) 7.04 (dd, J=9.07, 4.44 Hz, 1 H) 6.87 - 6.94 (m, 2 H) 3.89 - 3.98 (m, 1 H) 3.69 - 3.77 (m, 4 H) 3.53 - 3.61 (m, 1 H) 3.24 - 3.36 (m, 2 H) 1.56 - 1.93 (m, 4 H) 1.54 (d, J=3.38 Hz, 6 H).

### Synthesis Example 34: Synthesis of Compound 34

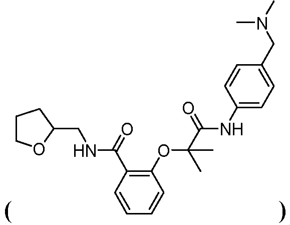

Compound 34 was synthesized according to the following Reaction Scheme 34.

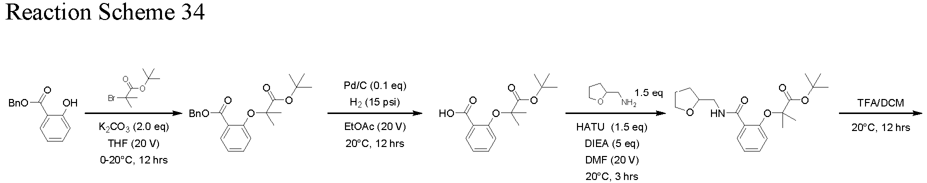

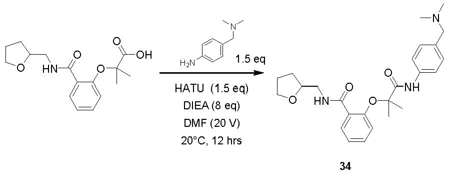

As shown in Reaction Scheme 34, 2-(2-(4-((dimethylamino)methyl)phenylcarbamoyl)propan-2-yloxy)-N-((tetrahydrofuran-2-yl)methyl)benzamide(**Compound 34)** was prepared. To a solution of 2-(2-((tetrahydrofuran-2-yl)methylcarbamoyl)phenoxy)-2-methylpropanoic acid (185 mg, 439.05 umol, TFA salt) in DMF (3.7 mL) was added DIEA (453.94 mg, 3.51 mmol) and 4-[(dimethylamino)methyl]aniline (98.93 mg, 658.58 umol) at 20°C. Then, HATU (250.41 mg, 658.58 umol) was added to the solution at 0°C. The mixture was stirred at 20°C for 12 hours. LC-MS showed the starting material was consumed and 88.1% of the desired compound was detected. The mixture solution was concentrated under reduced pressure to give crude product which was purified by prep-HPLC (neutral condition) to give 2-(2-(4-((dimethylamino)methyl)phenylcarbamoyl)propan-2-yloxy)-N-((tetrahydrofuran-2-yl)methyl)benzamide **(Compound 34)** (39.1 mg, 19.84% yield) as a light-yellow solid. The structure of Compound 34 was confirmed by ¹H NMR: (400MHz, DMSO-d6) δ 10.21 (s, 1 H) 8.51 (br t, J=5.63 Hz, 1 H) 7.61 - 7.69 (m, 3 H) 7.37 - 7.46 (m, 1 H) 7.23 (d, J=8.38 Hz, 2 H) 7.09 - 7.16 (m, 1 H) 7.04 (d, J=8.25 Hz, 1 H) 3.90 - 4.00 (m, 1 H) 3.69 - 3.78 (m, 1 H) 3.57 (q, J=7.50 Hz, 1 H) 3.37 - 3.45 (m, 1 H) 3.35 (br s, 2 H) 3.27 - 3.32 (m, 1 H) 2.12 (s, 6 H) 1.83 - 1.93 (m, 1 H) 1.69 - 1.82 (m, 2 H) 1.58 (d, J=4.00 Hz, 6 H).

### Synthesis Example 35: Synthesis of Compound 35

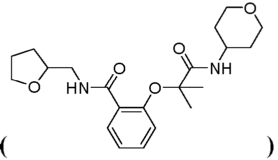

Compound 35 was synthesized according to the following Reaction Schemes 35A and 35B.

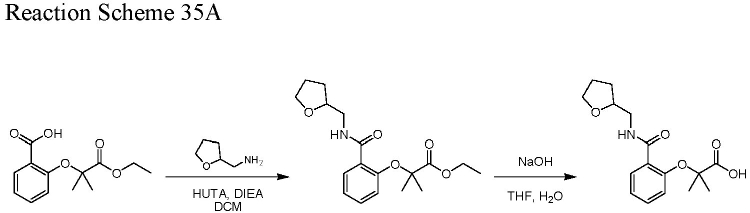

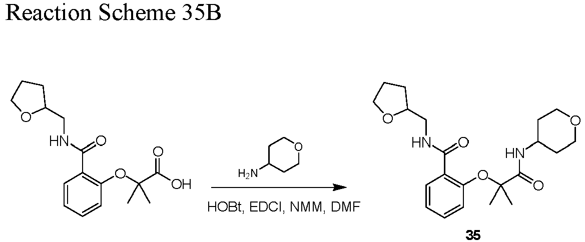

As shown in Reaction Scheme 35A, first, ethyl 2-methyl-2-[2-(tetrahydrofuran-2-ylmethylcarbamoyl)phenoxy]propanoate was prepared. To a solution of 2-(2-ethoxy-1,1-dimethyl-2-oxo-ethoxy)benzoic acid (450.0 mg, 1.78 mmol, 1 eq) in DCM (5 mL) was added DIEA (1.15 g, 8.92 mmol, 1.55 mL, 5 eq) and HATU (1.02 g, 2.68 mmol, 1.5 eq) at 25°C. (tetrahydrofuran-2-yl)methanamine (270.6 mg, 2.68 mmol, 276.17 µL, 1.5 eq) was added to the mixture at 25°C. After stirring at 25°C for 1h, the reaction mixture was quenched by addition of H₂O (5 mL), and extracted three times with 5 mL DCM (5 mL x 3). The combined organic phase was concentrated and purified by column chromatography (EtOAc in PE= 0 to 7%) to give ethyl 2-methyl-2-[2-(tetrahydrofuran-2-ylmethylcarbamoyl)phenoxy]propanoate (400.0 mg, 1.19 mmol, 66.9% yield) as a white solid. The structure of ethyl 2-methyl-2-[2-(tetrahydrofuran-2-ylmethylcarbamoyl)phenoxy]propanoate was confirmed by ¹H NMR(400 MHz, CDCl₃) δ = 8.24-8.17 (m, 2H), 7.33-7.31 (m, 1H), 7.10 (t, *J =* 0.8Hz, 1H), 6.76 (d, *J =* 8.4Hz, 1H), 4.23 (dd, *J =* 6.8Hz, 14.0Hz, 2H), 4.14-4.05 (m, 2H), 3.88-3.84 (m, 1H), 3.77-3.73 (m, 2H), 3.44-3.39 (m, 1H), 2.04-1.98 (m, 2H), 1.96-1.88 (m, 2H), 1.68 (d, *J =* 6.8Hz, 6H), 1.65-1.60 (m, 2H).

Next, 2-methyl-2-[2-(tetrahydrofuran-2-ylmethylcarbamoyl)phenoxy]propanoic acid was prepared. To a solution of ethyl 2-methyl-2-[2-(tetrahydrofuran-2-ylmethylcarbamoyl)phenoxy]propanoate (400.0 mg, 1.19 mmol, 1 eq) in MeOH (12 mL) and THF (6 mL) and H₂O (3 mL) was added LiOH (1.35 g, 56.48 mmol, 47.36 eq). After stirring at 25°C for 1hr, the reaction mixture was acidified with 1M HCl (3 mL) and extracted three times with 5 mL EtOAc (5 mL x 3). The combined organic phase was filtered and concentrated to give 2-methyl-2-[2-(tetrahydrofuran-2-ylmethylcarbamoyl)phenoxy]propanoic acid (400.0 mg, crude) as a white solid. The structure of 2-methyl-2-[2-(tetrahydrofuran-2-ylmethylcarbamoyl)phenoxy]propanoic acid was confirmed by ¹H NMR(400 MHz, CDCl₃) δ = 7.82-7.80 (m, 1H), 7.44-7.35 (m, 2H), 7.13 (t, *J* = 8.4Hz, 1H), 7.09-7.02 (m, 1H), 4.07-4.05 (m, 1H), 3.90-3.84 (m, 1H), 3.79-3.73 (m, 2H), 3.35-3.28 (m, 1H), 2.05-1.99 (m, 1H), 1.95-1.88 (m, 2H), 1.70 (d, *J* = 2.0Hz, 6H), 1.65-1.58 (m, 1H). ESI calculation for C₁₆H₂₂NO₅ [MH]⁺308.1; found 308.2.

As shown in Reaction Scheme 35 B, 2-[1,1-dimethyl-2-oxo-2-(tetrahydropyran-4-ylamino)ethoxy]-N-(tetrahydrofuran-2-ylmethyl)benzamide **(Compound 35)** was prepared. To a solution of 2-methyl-2-[2-(tetrahydrofuran-2-ylmethylcarbamoyl)phenoxy]propanoic acid (200.0 mg, 650.74 µmol, 1 eq) in DMF (2 mL) was added 4-methylmorpholine (NMM) (329.1 mg, 3.25 mmol, 357.73 µL, 5 eq), tetrahydropyran-4-amine (98.7 mg, 976.12 µmol, 1.5 eq), HOBt (131.8 mg, 976.12 µmol, 1.5 eq) and EDCI (187.1 mg, 976.12 µmol, 1.5 eq). After stirring at 25°C for 2hr, the reaction mixture was quenched by addition of H₂O (2 mL) and extracted three times with 2 mL EtOAc (2 mL x 3). The combined organic phase was concentrated and purified by prep-HPLC (column: Waters XBRIDGE^{®} 150x25mm 10um; mobile phase: [water( NH₄HCO₃)-ACN];B%: 18%-48%,10min) to give 2-[1,1-dimethyl-2-oxo-2-(tetrahydropyran-4-ylamino)ethoxy]-N-(tetrahydrofuran-2-ylmethyl)benzamide **(Compound 35)** (29.5 mg, 75.55 µmol, 11.6% yield) as a white solid. The structure of Compound 35 was confirmed by ¹H NMR(400 MHz, CDCl₃) δ = 7.98 (dd, *J* = 1.6Hz, 7.6Hz, 1H), 7.60 (brs, 1H), 7.35-7.31 (m, 1H), 7.10 (t, *J* = 7.6Hz, 1H), 6.91-6.86 (m, 2H), 4.06-3.97 (m, 2H), 3.92-3.73 (m, 4H), 3.50-3.43 (m, 2H), 3.33-3.26 (m, 1H), 2.08-2.01 (m, 1H), 1.96-1.89 (m, 2H), 1.86-1.82 (m, 2H), 1.63 (s, 6H), 1.62-1.57 (m, 2H), 1.53-1.43 (m, 2H). ESI calculation for C₂₁H₃₁N₂O₅ [MH]⁺ 391.2; found 391.2.

### Synthesis Example 36: Synthesis of Compound 36

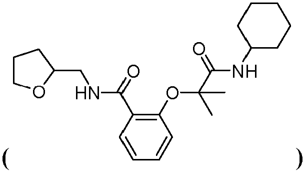

Compound 36 was synthesized according to the following Reaction Scheme 36.

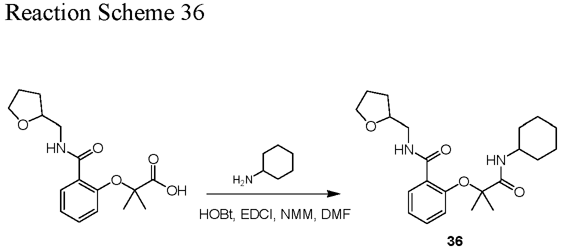

First, 2-methyl-2-[2-(tetrahydrofuran-2-ylmethylcarbamoyl)phenoxy]propanoic acid was prepared according to described Synthesis Example 35 for Reaction Scheme 35A.

Next, 2-[2-(cyclohexylamino)-1,1-dimethyl-2-oxo-ethoxy]-N-(tetrahydrofuran-2-ylmethyl)benzamide **(Compound 36)** was prepared. To a solution of 2-methyl-2-[2-(tetrahydrofuran-2-ylmethylcarbamoyl)phenoxy]propanoic acid (250.0 mg, 813.43 µmol, 1 eq) in DMF (2.5 mL) was added 4-methylmorpholine (NMM) (411.3 mg, 4.07 mmol, 447.15 µL, 5 eq), EDCI (233.9 mg, 1.22 mmol, 1.5 eq), HOBt (164.8 mg, 1.22 mmol, 1.5 eq), and cyclohexanamine (121.0 mg, 1.22 mmol, 139.65 µL, 1.5 eq). After stirring at 25°C for 2hr, the reaction mixture was quenched by addition of H₂O (2 mL) and extracted three times with 2 mL EtOAc (2 mL x 3). The combined organic phase was concentrated and purified by prep-HPLC (column: Phenomenex LUNA^{®} C18 150*25mm* 10um; mobile phase: [water(FA)-ACN]; B%: 36%-66%, 15min) to give 2-[2-(cyclohexylamino)-1,1-dimethyl-2-oxo-ethoxy]-N-(tetrahydrofuran-2-ylmethyl)benzamide **(Compound 36)** (82.4 mg, 212.10 µmol, 26.0% yield) as a white solid. The structure of Compound 36 was confirmed by ¹H NMR (400MHz, CDCl₃) δ = 8.08 (dd, *J =* 2.0, 8.0 Hz, 1H), 7.87 (s, 1H), 7.34-7.30 (m, 1H) 7.12 (t, *J* = 8.0 Hz, 1H), 6.88 (d, *J* = 8.4 Hz, 1H), 6.50 (d, *J* = 8.0 Hz, 1H), 4.06-4.02 (m, 1H), 3.89-3.72 (m, 4H), 3.35-3.28 (m, 1H), 2.05-1.99 (m, 1H), 1.95-1.88 (m, 4H), 1.71-1.65 (m, 2H), 1.62 (s, 6H), 1.60-1.57 (m, 1H), 1.40-1.29 (m, 2H), 1.17-1.07 (m, 3H). ESI calculation for C₂₂H₃₃N₂O₄ [M+H]+ 389.2; found 389.2.

### Synthesis Example 37: Synthesis of Compound 37

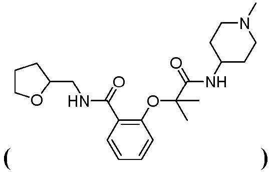

Compound 37 was synthesized according to the following Reaction Scheme 37.

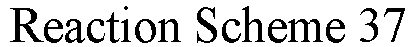

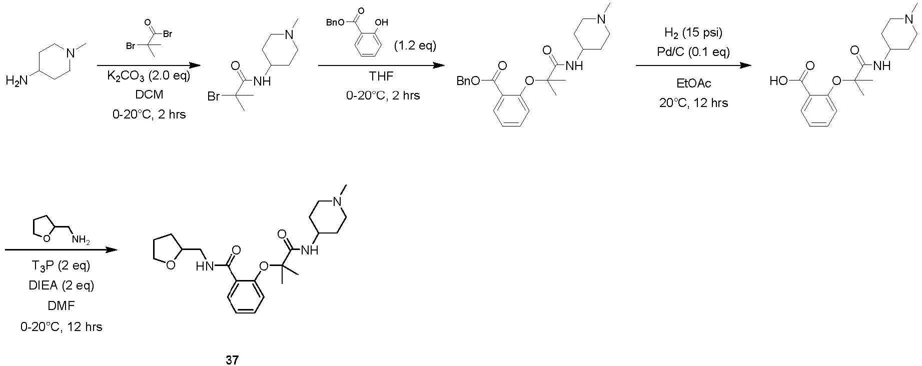

As shown in Reaction Scheme 37, first, 2-bromo-2-methyl-N-(1-methylpiperidin-4-yl)propenamide was prepared. To a solution of 1-methylpiperidin-4-amine (1 g, 8.76 mmol) in dichloromethane (10 mL) was added K₂CO₃ (2.42 g, 17.51 mmol) and 2-bromo-2-methylpropanoyl bromide (2.21 g, 9.63 mmol) at 20°C. The mixture was stirred for 2 hours at 20°C. LC-MS showed the reaction was completed. The mixture was diluted with water (20 mL), extracted three times with 20 mL dichloromethane (3 x 20 mL). The organic phase was dried over Na₂SO₄, filtered and concentrated under reduced pressure to give 2-bromo-2-methyl-N-(1-methylpiperidin-4-yl)propanamide (2 g, 78.10% yield) as a yellow solid which was used in the next step directly. The structure of 2-bromo-2-methyl-N-(1-methylpiperidin-4-yl)propenamide was confirmed by ¹H NMR: (400 MHz, CDCl3-*d*) δ 6.60 (br d, J = 2.1 Hz, 1H), 3.80 - 3.65 (m, 1H), 2.77 (br d, J = 11.1 Hz, 2H), 2.30 (s, 3H), 2.20 - 2.06 (m, 2H), 1.99 - 1.91 (m, 8H), 1.62 - 1.47 (m, 2H).

Next, benzyl 2-(2-(1-methylpiperidin-4-ylcarbamoyl)propan-2-yloxy)benzoate was prepared. To a solution of benzyl 2-hydroxybenzoate (867.27 mg, 3.80 mmol) in THF (5 mL) was added NaH (189.97 mg, 4.75 mmol) at 0°C. The mixture was stirred for 1 hour at 0°C. 2-bromo-2-methyl-N-(1-methylpiperidin-4-yl)propenamide (500 mg, 1.90 mmol) was added into the mixture at 0°C. The reaction was stirred for 12 hours at 20°C. LC-MS showed the reaction was completed. The reaction was quenched with aqueous NH₄Cl (20 mL) at 0°C. The mixture was adjusted to pH=3 with HCl (2N). The mixture was extracted twice with 10 mL ethyl acetate (2 x 10 mL). The water phase was adjusted to pH 8 with saturated NaHCO₃, and extracted three times with 15 mL ethyl acetate (3 x 15 mL). The organic phase was washed with brine (20 mL), dried over Na₂SO₄, filtered and concentrated under reduced pressure to give benzyl 2-(2-(1-methylpiperidin-4-ylcarbamoyl)propan-2-yloxy)benzoate (270 mg, 31.16% yield) as a white solid. The structure of benzyl 2-(2-(1-methylpiperidin-4-ylcarbamoyl)propan-2-yloxy)benzoate was confirmed by ¹H NMR: (400 MHz, CDCl3-d) δ 8.45 (br d, J = 6.6 Hz, 1H), 7.95 (dd, J = 1.8, 7.9 Hz, 1H), 7.49 - 7.32 (m, 7H), 7.15 (d, J = 8.3 Hz, 1H), 7.09 - 7.01 (m, 1H), 5.33 (s, 2H), 3.86 - 3.72 (m, 1H), 2.86 (br d, J = 11.5 Hz, 2H), 2.31 (s, 3H), 2.13 (br t, J = 10.9 Hz, 2H), 1.94 (br d, J = 14.4 Hz, 2H), 1.77 - 1.60 (m, 11H).

Next, 2-(2-(1-methylpiperidin-4-ylcarbamoyl)propan-2-yloxy)benzoic acid was prepared. To a solution of benzyl 2-(2-(1-methylpiperidin-4-ylcarbamoyl)propan-2-yloxy)benzoate (270 mg, 657.73 umol) in MeOH (5 mL) was added Pd/C (50 mg, 46.73 umol) under N₂ atmosphere. The suspension was degassed and purged with H₂ three times. The mixture was stirred under H₂ (15 Psi) at 20°C for 12 hours. LC-MS showed the reaction was completed. The mixture was filtered, and the filtrate was concentrated under reduced pressure to give 2-(2-(1-methylpiperidin-4-ylcarbamoyl)propan-2-yloxy)benzoic acid (200 mg, 85.42% yield) as a brown solid. The structure of 2-(2-(1-methylpiperidin-4-ylcarbamoyl)propan-2-yloxy)benzoic acid was confirmed by ¹H NMR: (400 MHz, CDCl₃-d) δ 9.67 (br d, J = 4.0 Hz, 1H), 7.82 (br d, J = 6.5 Hz, 1H), 7.34 - 7.27 (m, 1H), 7.08 - 6.93 (m, 2H), 3.90 - 3.77 (m, 1H), 3.48 (s, 2H), 3.42 - 3.30 (m, 2H), 2.68 - 2.54 (m, 4H), 2.01 (br s, 4H), 1.64 (s, 6H).

Finally, 2-(2-(1-methylpiperidin-4-ylcarbamoyl)propan-2-yloxy)-N-((tetrahydrofuran-2-yl)methyl)benzamide **(Compound 37)** was prepared. To a solution of 2-(2-(1-methylpiperidin-4-ylcarbamoyl)propan-2-yloxy)benzoic acid (100 mg, 280.91 umol), tetrahydrofuran-2-ylmethanamine (56.83 mg, 561.83 umol) and DIEA (72.61 mg, 561.83 umol) in DMF (1.5 mL) was added T₃P (440 mg, 691.43 umol) at 0°C. The reaction mixture was stirred for 12 hours at 20°C. LC-MS showed the reaction was completed. The reaction was purified by prep-HPLC and lyophilized to give 2-(2-(1-methylpiperidin-4-ylcarbamoyl)propan-2-yloxy)-N-((tetrahydrofuran-2-yl)methyl)benzamide **(Compound 37)** (33.7 mg, 29.60% yield) as a white solid. The structure of Compound 37 was confirmed by ¹H NMR: (400 MHz, DMSO-d6) δ 8.35 (br t, J = 5.6 Hz, 1H), 8.12 (br d, J = 8.0 Hz, 1H), 7.65 (dd, J = 1.8, 7.6 Hz, 1H), 7.42 - 7.34 (m, 1H), 7.07 (t, J = 7.5 Hz, 1H), 6.89 (d, J = 8.3 Hz, 1H), 4.01 - 3.91 (m, 1H), 3.83 - 3.75 (m, 1H), 3.67 - 3.60 (m, 1H), 3.56 (dt, J = 3.8, 7.5 Hz, 1H), 3.43 - 3.36 (m, 2H), 2.71 (br d, J = 11.1 Hz, 2H), 2.13 (s, 3H), 1.98 - 1.87 (m, 3H), 1.86 - 1.79 (m, 2H), 1.63 (br d, J = 2.6 Hz, 1H), 1.61 (br s, 2H), 1.49 (br d, J = 3.5 Hz, 1H), 1.47 (s, 6H), 1.44 (br s, 1H).

### Synthesis Example 38: Synthesis of Compound 38

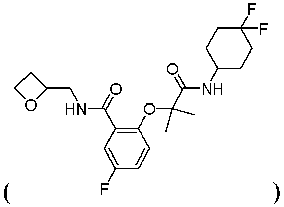

Compound 38 was synthesized according to the following Reaction Scheme 38.

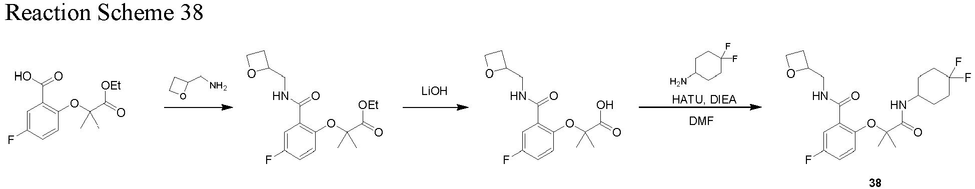

As shown in Reaction Scheme 38, first, ethyl 2-[4-fluoro-2-(oxetan-2-ylmethylcarbamoyl)phenoxy]-2-methyl-propanoate was prepared. To a solution of 2-(2-(ethoxycarbonyl)propan-2-yloxy)-5-fluorobenzoic acid (400 mg, 1.48 mmol, 1 eq) in DMF (3 mL) was added HATU (1.13 g, 2.96 mmol, 2 eq) and DIEA (956.45 mg, 7.40 mmol, 1.29 mL, 5 eq). After stirring at 25°C for 0.5 h, oxetan-2-ylmethanamine (154.74 mg, 1.78 mmol, 1.2 eq) was added. The mixture was stirred at 25°C for 3 h. The reaction mixture was quenched by addition of H₂O (10 mL) and extracted twice with 15 mL EtOAc (15 mL x 2). The combined organic layers were washed twice with 15 mL of ~3% LiOH (15 mL x 2), dried over Na₂SO₄, filtered, concentrated and purified by column chromatography (SiO₂, Ethyl acetate/Petroleum ether= 0 to 16%) to give ethyl 2-[4-fluoro-2-(oxetan-2-ylmethylcarbamoyl)phenoxy]-2-methyl-propanoate (380 mg, 1.12 mmol, 75.65% yield) as light yellow oil. The structure of 2-[4-fluoro-2-(oxetan-2-ylmethylcarbamoyl)phenoxy]-2-methyl-propanoate was confirmed by ¹H NMR (400MHz, DMSO-d6) δ = 8.38 (t, *J =* 5.6 Hz, 1H), 7.49 (dd, *J =* 3.2, 9.2 Hz, 1H), 7.29-7.24 (m, 1H), 6.88 (dd, *J* = 4.4, 9.2 Hz, 1H), 4.87-4.84 (m, 1H), 4.55-4.49 (m, 1H), 4.45-4.40 (m, 1H), 4.22-4.16 (m, 2H), 3.64-3.57 (m, 1H), 3.51-3.45 (m, 1H), 2.65-2.57 (m, 1H), 2.44-2.35 (m, 1H), 1.55 (d, *J* = 2.4 Hz, 6H), 1.18 (t, J=6.8, 3H). ¹⁹F NMR (376.5MHz, DMSO-d6) δ = -120.597. ESI calculation for C₁₇H₂₃FNO₅ [MH]⁺ 340.1; found 340.2.

Next, 2-[4-fluoro-2-(oxetan-2-ylmethylcarbamoyl)phenoxy]-2-methyl-propanoic acid was prepared. To a solution of ethyl 2-[4-fluoro-2-(oxetan-2-ylmethylcarbamoyl)phenoxy]-2-methyl-propanoate (380 mg, 1.12 mmol, 1 eq) in MeOH (2 mL), THF (1.5 mL) and H₂O (0.1 mL) was added LiOH.H₂O (70.48 mg, 1.68 mmol, 1.5 eq). The mixture was stirred at 25°C for 1h. The reaction mixture was pH adjusted to acidic and extracted twice with 20 mL EtOAc (20 mL x 2). The combined organic layers were washed twice with 10 mL brine (10 mL x 2), dried over Na₂SO₄, filtered and concentrated to give 2-[4-fluoro-2-(oxetan-2-ylmethylcarbamoyl)phenoxy]-2-methyl-propanoic acid (300 mg, 963.69 µmol, 86.06% yield) as a yellow solid. ESI calculation for C₁₅H₁₉FNO₅ [MH]⁺ 312.1; found 312.1.

Finally, 2-[2-[(4,4-difluorocyclohexyl)amino]-1,1-dimethyl-2-oxo-ethoxy]-5-fluoro-N-(oxetan-2-ylmethyl)benzamide **(Compound 38)** was prepared. To a solution of 2-[4-fluoro-2-(oxetan-2-ylmethylcarbamoyl)phenoxy]-2-methyl-propanoic acid (150 mg, 481.84 µmol, 1 eq) in DMF (2 mL) was added HATU (366.42 mg, 963.69 µmol, 2 eq) and DIEA (311.37 mg, 2.41 mmol, 419.64 µL, 5 eq). After stirring at 25°C for 0.5 h, 4,4-difluorocyclohexanamine (97.68 mg, 722.76 µmol, 1.5 eq) was added. The mixture was stirred at 25°C for 2h, quenched by addition of H₂O (3 mL) and extracted twice with 10 mL EtOAc (10 mL x 2). The combined organic layers were washed twice with 10 mL of ~3% LiOH (10 mL x 2), dried over Na₂SO₄, filtered, concentrated and purified by prep-HPLC (Neutral condition; column: CD02-Waters XBRIDGE^{®} BEH C18 150x25x10um; mobile phase: [water( NH₄HCO₃)-ACN]; gradient: 26%-56% B over 10 min) to give 2-[2-[(4,4-difluorocyclohexyl)amino]-1,1-dimethyl-2-oxo-ethoxy]-5-fluoro-N-(oxetan-2-ylmethyl)benzamide **(Compound 38)** (58.8 mg, 137.47 µmol, 28.53% yield) as a white solid. The structure of Compound 38 was confirmed by ¹H NMR (400MHz, DMSO-d6) δ = 8.55 (t, *J =* 6.0 Hz, 1H), 8.17 (d, *J* = 8.0 Hz, 1H), 7.42 (dd, *J* = 3.2, 8.4 Hz, 1H), 7.30-7.25 (m, 1H), 6.95 (dd, *J* = 4.4, 9.2 Hz, 1H), 4.87-4.81 (m, 1H), 4.55-4.40 (m, 2H), 3.88-3.75 (m, 1H), 3.62-3.45 (m, 2H), 2.67-2.57 (m, 1H), 2.45-2.37 (m, 1H), 2.05-1.93 (m, 3H), 1.91-1.83 (m, 1H), 1.79-1.72 (m, 2H), 1.58-1.49 (m, 2H), 1.44 (s, 6H). ¹⁹F NMR (376.5MHz, DMSO-d6) δ = -120.723. ESI calculation for C₂₁H₂₈F₃N₂O₄ [MH]⁺ 429.2; found 429.1

### Synthesis Example 39: Synthesis of Compound 39

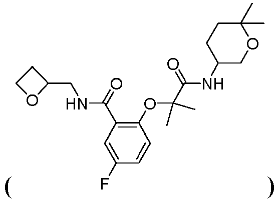

Compound 39 was synthesized according to the following Reaction Scheme 39.

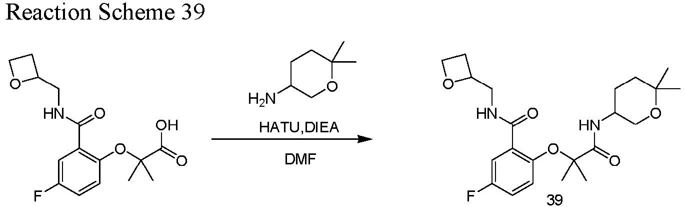

As shown in Reaction Scheme 39, 2-[2-[(6,6-dimethyltetrahydropyran-3-yl)amino]-1,1-dimethyl-2-oxo-ethoxy]-5-fluoro-N-(oxetan-2-ylmethyl)benzamide **(Compound 39)** was prepared. To a solution of 2-[4-fluoro-2-(oxetan-2-ylmethylcarbamoyl)phenoxy]-2-methyl-propanoic acid (150 mg, 481.84 µmol, 1 eq) in DMF (1 mL) was added HATU (366.42 mg, 963.69 µmol, 2 eq) and DIEA (311.37 mg, 2.41 mmol, 419.64 µL, 5 eq). After stirring at 25°C for 0.5h, 6,6-dimethyltetrahydropyran-3-amine (68.48 mg, 530.03 µmol, 1.1 eq) was added. The mixture was stirred at 25°C for 2h, quenched by addition of H₂O (3 mL), and extracted twice with 10 mL EtOAc (10 mL x 2). The combined organic layers were washed twice with 10 mL of ~3% LiOH (10 mL x 2), dried over Na₂SO₄, filtered, concentrated and purified by prep-HPLC (Neutral condition; column: CD02-Waters XBRIDGE^{®} BEH C18 150x25x10um; mobile phase: [water(NH₄HCO₃)-ACN]; gradient:2 2%-52% B over 10 min) to give 2-[2-[(6,6-dimethyltetrahydropyran-3-yl)amino]-1,1-dimethyl-2-oxo-ethoxy]-5-fluoro-N-(oxetan-2-ylmethyl)benzamide **(Compound 39)** (32.2 mg, 84.97 µmol, 17.63% yield) as a white solid. The structure of Compound 39 was confirmed by ¹H NMR (400MHz, DMSO-d6) δ = 8.49 (t, *J =* 6.0 Hz, 1H), 8.25 (t, *J* = 6.0 Hz, 1H), 7.45-7.38 (m, 1H), 7.28-7.23 (m, 1H), 6.91 (dd, *J* = 4.4, 9.2 Hz, 1H), 4.87-4.81 (m, 1H), 4.56-4.42 (m, 2H), 3.94 (quin, *J* = 6.0 Hz, 1H), 3.64-3.56 (m, 1H), 3.51-3.45 (m, 1H), 3.24-3.12 (m, 2H), 2.68-2.60 (m, 1H), 2.45-2.38 (m, 1H), 1.92-1.83 (m, 1H), 1.66-1.58 (m, 3H), 1.44 (s, 6H), 1.14 (d, *J =* 1.6 Hz, 3H), 1.11 (s, 3H).¹⁹F NMR (376.5MHz, DMSO-d6) δ = -121.054. ESI calculation for C₂₂H₃₂FN₂O₅ [MH]⁺ 423.2; found 423.0.

### Synthesis Example 40: Synthesis of Compound 40

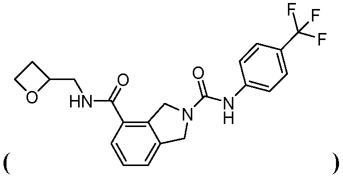

Compound 40 was synthesized according to the following Reaction Scheme 40.

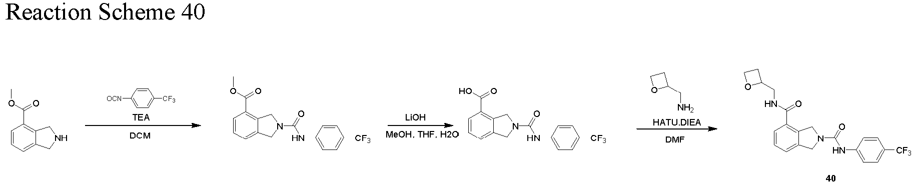

As shown in Reaction Scheme 40, first, methyl 2-[[4-(trifluoromethyl)phenyl]carbamoyl]isoindoline-4-carboxylate was prepared. To a solution of methyl isoindoline-4-carboxylate (500 mg, 2.82 mmol, 1 eq) and 1-isocyanato-4-(trifluoromethyl)benzene (527.99 mg, 2.82 mmol, 403.04 µL, 1 eq) in DCM (5 mL) was added TEA (285.53 mg, 2.82 mmol, 392.75 µL, 1 eq). After stirring at 25°C for 3 h, the reaction mixture was quenched by addition of H₂O (5 mL) and extracted twice with 5 mL DCM (5 mL x 2). The combined organic layers were washed twice with 5 mL brine (5 mL x 2), dried over Na₂SO₄, filtered and concentrated to give methyl 2-[[4-(trifluoromethyl)phenyl]carbamoyl]isoindoline-4-carboxylate (1 g, 2.74 mmol, 97.28% yield) as a yellow solid. ESI calculations for C₁₈H₁₆F₃N₂O₃ [MH]⁺ 365.1; found 365.2.

Next, 2-[[4-(trifluoromethyl)phenyl]carbamoyl]isoindoline-4-carboxylic acid was prepared. To a solution of methyl 2-[[4-(trifluoromethyl)phenyl] carbamoyl] isoindoline-4-carboxylate (500 mg, 1.37 mmol, 1 eq) in MeOH (4 mL), THF (3 mL) and H₂O (0.1 mL) was added LiOH.H₂O (86.39 mg, 2.06 mmol, 1.5 eq). The mixture was stirred at 25°C for 3 h. The reaction mixture was pH adjusted to acidic and extracted twice with 10 mL EtOAc (10 mL x 2). The combined organic layers were washed twice with 10 mL brine (10mL x 2), dried over Na₂SO₄, filtered and concentrated to give 2-[[4-(trifluoromethyl)phenyl]carbamoyl]isoindoline-4-carboxylic acid (300 mg, crude) as a yellow solid. ESI calculation for C₁₇H₁₄F₃N₂O₃ [MH]⁺ 351.1; found 351.1.

Finally, N4-(oxetan-2-ylmethyl)-N2-[4-(trifluoromethyl)phenyl]isoindoline-2,4-dicarboxamide **(Compound 40)** was prepared. To a solution of 2-[[4-(trifluoromethyl)phenyl]carbamoyl]isoindoline-4-carboxylic acid (300 mg, 856.43 µmol, 1 eq) in DMF (3 mL) was added HATU (651.28 mg, 1.71 mmol, 2 eq) and DIEA (553.44 mg, 4.28 mmol, 745.87 µL, 5 eq). After stirring at 25°C for 0.5 h, oxetan-2-ylmethanamine (89.53 mg, 1.03 mmol, 1.2 eq) was added. The mixture was stirred at 25°C for 3 h, quenched by addition of H₂O (5 mL) and extracted with EtOAc (20 mL x 2). The combined organic layers were washed twice with 10 mL of ~3% LiOH (10 mL x 2), dried over Na₂SO₄, filtered, concentrated and purified by prep-HPLC (Neutral condition; column: CD02-Waters XBRIDGE^{®} BEH C18 150x25x10um; mobile phase: [water(NH₄HCO₃)-ACN]; gradient: 25%-58% B over 10 min) to give N4-(oxetan-2-ylmethyl)-N2-[4-(trifluoromethyl)phenyl]isoindoline-2,4-dicarboxamide **(Compound 40)** (68.5 mg, 164.28 µmol, 19.18% yield) as a white solid. The structure of Compound 40 was confirmed by ¹H NMR (400MHz, DMSO-d6) δ = 8.84 (s, 1H), 8.68 (t, *J =* 5.6 Hz, 1H), 7.81 (d, J = 8.4 Hz, 2H), 7.72 (d, *J =* 7.6 Hz, 1H), 7.61 (d, *J =* 8.8 Hz, 2H), 7.52 (d, *J =* 7.2 Hz, 1H), 7.43(t, *J =* 7.6, 1H), 5.01 (s, 2H), 4.87-4.77 (m, 3H), 4.55-4.42 (m, 2H), 3.62-3.55 (m, 1H), 3.49-3.44 (m, 1H), 2.67-2.60 (m, 1H), 2.46-2.37 (m, 1H). ¹⁹F NMR (376.5MHz, DMSO-d6) δ = -59.986. ESI calculation for C₂₁H₂₁F₃N₃O₃ [MH]⁺ 420.1; found 420.0.

### Synthesis Example 41: Synthesis of Compound 41

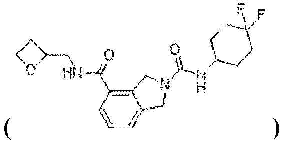

Compound 41 was synthesized according to the following Reaction Scheme 41.

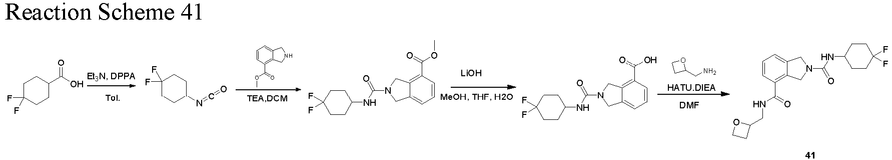

As shown in Reaction Scheme 41, first, 1,1-difluoro-4-isocyanatocyclohexane was prepared. To a solution of 4,4-difluorocyclohexanecarboxylic acid (295.0 mg, 1.80 mmol, 1 eq) in toluene (Tol.) (5 mL) was added Et₃N (TEA) (218.2 mg, 2.16 mmol, 300.17 µL, 1.2 eq) and diphenylphosphoryl azide (DPPA) (544.0 mg, 1.98 mmol, 426.69 µL, 1.1 eq). The mixture was stirred at 110°C for 4 hr. The mixture was quenched with H₂O (20 mL) and extracted three times with 25 mL EtOAc (25 mL × 3). The combined organic phase was dried over anhydrous sodium sulfate, filtered and concentrated to give 1,1-difluoro-4-isocyanato-cyclohexane (300.0 mg, crude) as a yellow solid. The structure of 1,1-difluoro-4-isocyanato-cyclohexane was confirmed by ¹H NMR (400MHz, CDCl₃) δ = 3.77-3.66 (m, 1H), 2.16-2.05 (m, 2H), 1.99-1.77 (m, 6H). ¹⁹F NMR (376.5MHz, CDCl₃) δ = -95.846, -99.456, -100.199.

Next, methyl 2-((4,4-difluorocyclohexyl)carbamoyl)isoindoline-4-carboxylate was prepared. To a solution of methyl isoindoline-4-carboxylate (300.0 mg, 1.40 mmol, 1 eq, HCl) in DCM (3 mL) was added TEA (142.1 mg, 1.40 mmol, 195.43 µL, 1 eq) and 1,1-difluoro-4-isocyanato-cyclohexane (226.3 mg, 1.40 mmol, 1 eq). The mixture was stirred at 25°C for 2hr. The mixture was quenched with H₂O (10 mL) and extracted three times with 10 mL DCM (10 mL × 3). The combined organic phase was dried over anhydrous sodium sulfate, filtered and concentrated to give methyl 2-[(4,4-difluorocyclohexyl)carbamoyl]isoindoline-4-carboxylate (600.0 mg, crude) as a yellow oil. The structure of methyl 2-[(4,4-difluorocyclohexyl)carbamoyl]isoindoline-4-carboxylate was confirmed by ¹H NMR (400MHz, CDCl₃) δ = 8.00-7.92 (m, 1H), 7.51-7.44 (m, 1H), 7.42-7.37 (m, 1H), 7.08-6.93 (m, 1H), 4.94 (s, 2H), 4.76 (s, 2H), 4.36-4.28 (m, 1H), 3.93 (s, 3H), 2.16-2.02 (m, 8H).¹⁹F NMR (376.5MHz, CDCl₃) δ = -94.367, -95.005, -101.220, -101.580. ESI calculation for C₁₇H₂₁F₂N₂O₃ [MH]⁺ 338.1; found 339.2.

Next, 2-((4,4-difluorocyclohexyl)carbamoyl)isoindoline-4-carboxylic acid was prepared. To a solution of methyl 2-[(4,4-difluorocyclohexyl)carbamoyl]isoindoline-4-carboxylate (600.0 mg, 1.77 mmol, 1 eq) in MeOH (4.5 mL), THF (2.1 mL) and H₂O (0.1 mL) was added LiOH.H₂O (96.7 mg, 2.31 mmol, 1.3 eq). The mixture was stirred at 25°C for 2hr. The mixture was concentrated and HCl (2M,2mL) was added, then the mixture was filtered to give 2-[(4,4-difluorocyclohexyl)carbamoyl]isoindoline-4-carboxylic acid (460.0 mg, crude) as a yellow solid. The structure of 2-[(4,4-difluorocyclohexyl)carbamoyl]isoindoline-4-carboxylic acid was confirmed by ¹H NMR (400MHz, CDCl₃) δ = 8.05 (t, *J =* 7.6 Hz, 1H), 7.54-7.48 (m, 1H), 7.45-7.39 (m, 1H), 5.01 (s, 2H), 4.76 (s, 2H), 3.99-3.88 (m, 1H), 2.02-1.88 (m, 8H). ¹⁹F NMR (376.5MHz, CDCl₃) δ = -94.983. ESI calculation for C₁₆H₁₉F₂N₂O₃ [MH]⁺ 325.1; found 325.2.

Finally, N2-(4,4-difluorocyclohexyl)-N4-(oxetan-2-ylmethyl)isoindoline-2,4-dicarboxamide **(Compound 41)** was prepared. To a solution of 2-[(4,4-difluorocyclohexyl)carbamoyl]isoindoline-4-carboxylic acid (200.0 mg, 616.67 µmol, 1 eq) and oxetan-2-ylmethanamine (53.7 mg, 616.67 µmol, 1 eq) in DMF (3 mL) was added HATU (468.9 mg, 1.23 mmol, 2 eq) and DIEA (398.5 mg, 3.08 mmol, 537.06 µL, 5 eq). The mixture was stirred at 25°C for 2hr. The mixture was quenched with H₂O (10 mL) and extracted three times with 10 mL EtOAc (10 mL × 3). The combined organic phase was dried over anhydrous sodium sulfate, filtered, concentrated and purified by prep-TLC (SiO₂, DCM:MeOH=10:1) to give N2-(4,4-difluorocyclohexyl)-N4-(oxetan-2-ylmethyl)isoindoline-2,4-dicarboxamide **(Compound 41)** (40.1 mg, 145.80 µmol, 23.64% yield, 98.9% purity) as a white solid. The structure of Compound 41 was confirmed by ¹H NMR (400MHz, DMSO*-d*₆) δ = 8.63 (t, *J =* 5.6 Hz, 1H), 7.67 (d, *J =* 7.6 Hz, 1H), 7.46 (d, *J* = 7.6 Hz, 1H), 7.38 (t, *J =* 7.6 Hz, 1H), 6.21 (d, *J =* 7.6 Hz, 1H), 4.86-4.81 (m, 1H), 4.79 (s, 2H), 4.59 (s, 2H), 4.54-4.48 (m, 1H), 4.47-4.40 (m, 1H), 3.74-3.63 (m, 1H), 3.59-3.52 (m, 1H), 3.49-3.43 (m, 1H), 2.69-2.59 (m, 1H), 2.46-2.39 (m, 1H), 2.05-1.80 (m, 6H), 1.65-1.55 (m, 2H). ¹⁹F NMR (376.5MHz, DMSO*-d*₆) δ = -91-794, -92.409, -98.675, -99.283. ESI calculation for C₂₀H₂₆F₂N₃O₃ [MH]⁺ 394.1; found 394.0.

### Synthesis Example 42: Synthesis of Compound 42

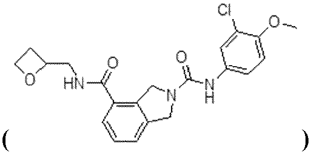

Compound 42 was synthesized according to the following Reaction Scheme 42.

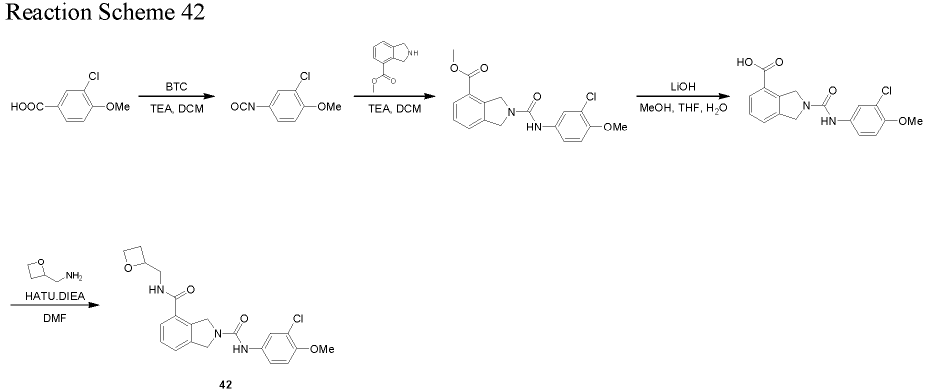

As shown in Reaction Scheme 42, first, 2-chloro-4-isocyanato-1-methoxybenzene was prepared. To a solution of bis(trichloromethyl) carbonate (282.44 mg, 951.79 µmol, 0.5 eq) in DCM (3 mL) was added 3-chloro-4-methoxybenzoic acid (300.00 mg, 1.90 mmol, 1 eq) and TEA (385.24 mg, 3.81 mmol, 529.90 µL, 2 eq) in DCM (3 mL) at 0°C under N₂. The mixture was stirred at 25°C for 0.5hr. The mixture was concentrated under reduced pressure to give 2-chloro-4-isocyanato-1-methoxybenzene (350 mg, crude) as a brown solid. The structure of 2-chloro-4-isocyanato-1-methoxybenzene was confirmed by ¹H NMR (400MHz, CDCl₃) δ = 7.12 (d, *J* = 2.4Hz,1H), 6.95 (dd, *J =* 2.4Hz, 8.8Hz, 1H), 6.85 (s, 1H), 3.87 (s, 3H).

Next, methyl 2-((3-chloro-4-methoxyphenyl) carbamoyl) isoindoline-4-carboxylate was prepared. To a solution of methyl isoindoline-4-carboxylate (200 mg, 1.13 mmol, 1 eq) in DCM (2 mL) was added TEA (114.21 mg, 1.13 mmol, 157.10 µL, 1 eq) at 25°C, and the reaction mixture was stirred for 30mins, followed by addition of 2-chloro-4-isocyanato-1-methoxybenzene(207.21 mg, 1.13 mmol, 1 eq). The mixture was stirred at 25°C for 16hr. The mixture was quenched by H₂O (2 mL), extracted three times with 2 mL EtOAc (2 mL x 3). The combined organic layers were washed with brine (3 mL), dried over Na₂SO₄, and concentrated to give methyl 2-((3-chloro-4-methoxyphenyl) carbamoyl) isoindoline-4-carboxylate(400 mg, crude) as a yellow solid. ESI calculation for C₁₈H₁₈ClN₂O₄ [MH]⁺ 360.1; found 361.1.

Next, 2-((3-chloro-4-methoxyphenyl) carbamoyl) isoindoline-4-carboxylic acid was prepared. To a solution of methyl 2-((3-chloro-4-methoxyphenyl) carbamoyl) isoindoline-4-carboxylate (300.00 mg, 831.51 µmol, 1 eq) in MeOH (3 mL) THF (1.5 mL) and H₂O (0.1 mL) was added LiOH.H₂O (45.36 mg, 1.08 mmol, 1.3 eq). The mixture was stirred at 25°C for 1hr. The mixture was diluted with toluene (2 mL) and concentrated under vacuum to give 2-((3-chloro-4-methoxyphenyl) carbamoyl) isoindoline-4-carboxylic acid (200 mg, crude) as a yellow solid. ESI calculation for C₁₇H₁₆ClN₂O₄ [MH]⁺ 347.0; found 347.2.

Finally, N2-(3-chloro-4-methoxyphenyl)-N4-(oxetan-2-ylmethyl) isoindoline-2,4-dicarboxamide **(Compound 42)** was prepared. To a solution of 2-((3-chloro-4-methoxyphenyl) carbamoyl) isoindoline-4-carboxylic acid (200.00 mg, 576.76 µmol, 1 eq) in DMF (2 mL) was added HATU (438.60 mg, 1.15 mmol, 2 eq) and DIEA (372.70 mg, 2.88 mmol, 502.29 µL, 5 eq) at 25°C, and the reaction mixture was stirred for 30 mins, followed by addition of oxetan-2-ylmethanamine (50.25 mg, 576.76 µmol, 1 eq). The mixture was stirred at 25°C for 16hr. The mixture was quenched by H₂O (2 mL), extracted three times with 2 mL EtOAc (2 mL x 3). The combined organic layers were washed with brine (3 mL), dried over Na₂SO₄, concentrated and purified by column chromatography (SiO₂, MeOH in DCM=0% to 5%) to give N2-(3-chloro-4-methoxyphenyl)-N4-(oxetan-2-ylmethyl) isoindoline-2,4-dicarboxamide **(Compound 42)** (30 mg, 72.14 µmol, 12.51% yield) as a yellow solid. The structure of Compound 42 was confirmed by ¹H NMR (400MHz, CDCl₃) δ = 7.64-7.51 (m, 2H), 7.49-7.45 (m, 1H), 7.44-7.38 (m, 1H), 7.30 (dd, *J* = 2.4Hz, 8.8Hz, 1H), 6.87 (d, *J* = 9.2Hz, 1H), 6.75 (brs, 1H), 6.38 (s, 1H), 5.12 (s, 2H), 5.09-5.01 (m, 1H), 4.86 (s, 2H), 4.77-4.68 (m, 1H), 4.59-4.49 (m, 1H), 3.87 (s, 3H), 3.82-3.73 (m, 1H), 3.70-3.61 (m, 1H), 2.77-2.65 (m, 1H), 2.58-2.43 (m, 1H). ESI calculation for C₂₁H₂₃ClN₃O₄ [MH]⁺ 416.1; found 416.1.

### Synthesis Example 43: Synthesis of Compound 43

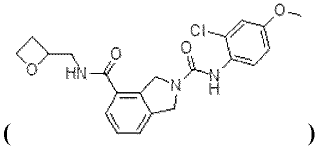

Compound 43 was synthesized according to the following Reaction Scheme 43.

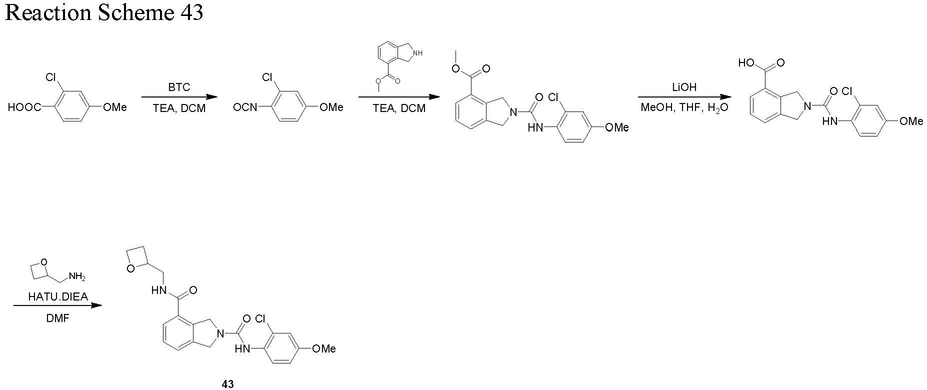

As shown in Reaction Scheme 43, first, 2-chloro-1-isocyanato-4-methoxybenzene was prepared. To a solution of bis(trichloromethyl) carbonate (282.44 mg, 951.79 µmol, 0.5 eq) in DCM (3 mL) was added 2-chloro-4-methoxybenzoic acid (300 mg, 1.90 mmol, 1 eq) and TEA (385.24 mg, 3.81 mmol, 529.91 µL, 2 eq) in DCM (3 mL) at 0°C under N₂. The mixture was stirred at 25°C for 0.5hr. The mixture was concentrated under reduced pressure to give 2-chloro-1-isocyanato-4-methoxybenzene (350 mg, crude) as a brown solid. The structure of 2-chloro-1-isocyanato-4-methoxybenzene was confirmed by ¹H NMR (400MHz, CDCl₃) δ = 7.00 (d, *J* = 8.8Hz, 1H), 6.92 (d, *J* = 2.4Hz, 1H), 6.72 (dd, *J* = 2.8Hz, 8.8Hz, 1H), 3.75 (s, 3H).

Next, methyl 2-((2-chloro-4-methoxyphenyl)carbamoyl)isoindoline-4-carboxylate was prepared. To a solution of methyl isoindoline-4-carboxylate (200 mg, 1.13 mmol, 1 eq) in DCM (2 mL) was added methyl isoindoline-4-carboxylate(207.21 mg, 1.13 mmol, 1 eq) and TEA (114.21 mg, 1.13 mmol, 157.10 µL, 1 eq). The mixture was stirred at 25°C for 16hr. The mixture was quenched by H₂O (2 mL), extracted three times with 2 mL EtOAc (2 mL x 3). The combined organic layers were washed with brine (3 mL), dried over Na₂SO₄, and concentrated to give methyl 2-((2-chloro-4-methoxyphenyl)carbamoyl)isoindoline-4-carboxylate (400 mg, crude) as a yellow oil. The structure of methyl 2-((2-chloro-4-methoxyphenyl)carbamoyl)isoindoline-4-carboxylate was confirmed by ¹H NMR (400MHz, CDCl₃) δ = 8.10 (d, *J* = 9.2Hz, 1H), 8.00 (d, *J =* 8.0Hz, 1H), 7.52 (d, *J* = 7.2Hz, 1H), 7.43 (t, *J* = 7.6Hz, 1H), 6.94 (d, *J* = 2.4Hz, 1H), 6.84 (dd, *J* = 2.8Hz, 9.2Hz, 1H), 6.73 (s, 1H), 5.16 (s, 2H), 4.86 (s, 2H), 3.95(s, 3H), 3.78 (s, 3H).

Next, 2-((2-chloro-4-methoxyphenyl)carbamoyl)isoindoline-4-carboxylic acid was prepared. To a solution of methyl 2-((2-chloro-4-methoxyphenyl)carbamoyl)isoindoline-4-carboxylate ((350.00 mg, 970.09 µmol, 1 eq) in MeOH (3 mL), THF (1 mL) and H₂O (0.1 mL) was added LiOH.H₂O (52.92 mg, 1.26 mmol, 1.3 eq). The mixture was stirred at 25°C for 1hr. The mixture was diluted with toluene (2 mL) and concentrated under vacuum to give 2-((2-chloro-4-methoxyphenyl)carbamoyl)isoindoline-4-carboxylic acid (350 mg, crude) as a yellow solid. ESI calculation for C₁₇H₁₆ClN₂O₄ [MH]⁺ 347.0; found 347.1.

Finally, N2-(2-chloro-4-methoxyphenyl)-N4-(oxetan-2-ylmethyl)isoindoline-2,4-dicarboxamide **(Compound 43):** To a solution of 2-((2-chloro-4-methoxyphenyl)carbamoyl)isoindoline-4-carboxylic acid(350 mg, 1.01 mmol, 1 eq) in DMF (4 mL) was added HATU (767.56 mg, 2.02 mmol, 2 eq) and DIEA (652.23 mg, 5.05 mmol, 879.02 µL, 5 eq) at 25°C, and the reaction mixture was stirred for 30 mins, followed by addition of oxetan-2-ylmethanamine (87.93 mg, 1.01 mmol, 1 eq). The mixture was stirred at 25°C for 16hr. The mixture was quenched by H₂O (2 mL), extracted with EtOAc (2 mL x 3). The combined organic layers were washed with brine (3 mL), dried over Na₂SO₄, concentrated and purified by column chromatography (SiO₂, MeOH in DCM=0% to 5%) to give N2-(2-chloro-4-methoxyphenyl)-N4-(oxetan-2-ylmethyl)isoindoline-2,4-dicarboxamide **(Compound 43)** (29 mg, 69.73 µmol, 14.50% yield) as a yellow solid. The structure of Compound 43 was confirmed by ¹H NMR (400MHz, CDCl₃) δ = 8.08 (d, *J =* 8.8Hz, 1H), 7.58 (d, *J* = 7.2Hz, 1H), 7.52-7.40 (m, 2H), 6.96 (d, *J* = 2.8Hz, 1H), 6.87 (dd, *J* = 2.8Hz, 8.8Hz, 1H), 6.77-6.63 (m, 2H), 5.20 (s, 2H), 5.11-5.03 (m, 1H), 4.91 (s, 2H), 4.80-4.71 (m, 1H), 4.62-4.54 (m, 1H), 3.86-3.79 (m, 4H), 3.72-3.65 (m, 1H), 2.80-2.69 (m, 1H), 2.61-2.50 (m, 1H). ESI calculation for C₂₁H₂₃ClN₃O₄ [MH]⁺ 416.1; found 416.1.

### Synthesis Example 44: Synthesis of Compound 44

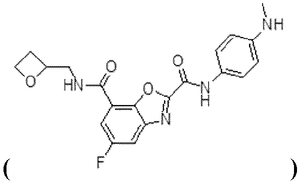

Compound 44 was synthesized according to the following Reaction Scheme 44.

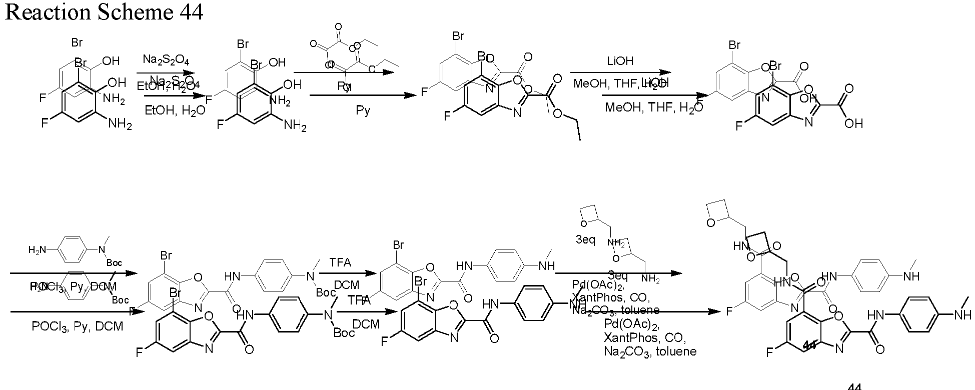

As shown in Reaction Scheme 44, tert-butyl (4-(7-bromo-5-fluorobenzo[d]oxazole-2-carboxamido)phenyl)(methyl)carbamate was prepared. To a solution of 7-bromo-5-fluoro-1,3-benzoxazole-2-carboxylic acid (400.0 mg, 1.54 mmol, 1 eq) and tert-butyl N-(4-aminophenyl)-N-methyl-carbamate (341.9 mg, 1.54 mmol, 1 eq) in DCM (4 mL) was added Py (608.4 mg, 7.69 mmol, 620.84 µL, 5 eq) and POCl₃ (4718 mg, 3.08 mmol, 286.78 µL, 2 eq). The mixture was stirred at 25°C for 2hr. The mixture was quenched with NaHCO₃ (30 mL) and extracted three times with 10 mL EtOAc (10 mL × 3), the combined organic phase was dried over anhydrous sodium sulfate, filtered and concentrated to give tert-butyl N-[4-[(7-bromo-5-fluoro-1,3-benzoxazole-2-carbonyl)amino]phenyl]-N-methyl-carbamate (400.0 mg, crude) as a yellow oil. ESI calculation for C₁₆H₁₁BrFN₃O₄ [M-56+H]⁺ 407.9; found 407.9.

Next, 7-bromo-5-fluoro-N-(4-(methylamino)phenyl)benzo[d]oxazole-2-carboxamide was prepared. To a solution of tert-butyl N-[4-[(7-bromo-5-fluoro-1,3-benzoxazole-2-carbonyl)amino]phenyl]-N-methyl-carbamate (400.0 mg, 861.54 µmol, 1 eq) in DCM (4 mL) was added TFA (98.2 mg, 861.54 µmol, 64.00 µL, 1 eq). The mixture was stirred at 25°C for 2hr. The mixture was quenched with NaHCO₃(10 mL) and extracted three times with 5 mL EtOAc (5 mL × 3). The combined organic phase was dried over anhydrous sodium sulfate, filtered, concentrated and purified by flash silica gel chromatography (Eluent of 0~2% MeOH/DCM @30 mL/min) to give 7-bromo-5-fluoro-N-[4-(methylamino)phenyl]-1,3-benzoxazole-2-carboxamide (170.0 mg, crude) as a yellow solid. ESI calculatin for C₁₅H₁₂BrFN₃O₂ [MH]⁺ 364.0; found 363.9.

Finally, 5-fluoro-N2-(4-(methylamino)phenyl)-N7-(oxetan-2-ylmethyl)benzo[d]oxazole-2,7-dicarboxamide **(Compound 44)** was prepared. A mixture of 7-bromo-5-fluoro-N-[4-(methylamino)phenyl]-1,3-benzoxazole-2-carboxamide (170 mg, 466.82 µmol, 1 eq), oxetan-2-ylmethanamine (162.68 mg, 1.87 mmol, 4 eq), Pd(OAc)₂ (2.10 mg, 9.34 µmol, 0.02 eq), (5-diphenylphosphanyl-9,9-dimethyl-xanthen-4-yl)-diphenyl-phosphane (5.40 mg, 9.34 µmol, 0.02 eq), and Na₂CO₃ (148.43 mg, 1.40 mmol, 3 eq) in Tol. (4 mL) was degassed and purged with CO three times, and then the mixture was stirred at 80°C for 16hr under CO atmosphere. The mixture was filtered and concentrated to give a residue. The residue was purified by prep-TLC (SiO₂, DCM: MeOH = 10:1) and prep-HPLC (column: CD24-WePure Biotech XPT C18 150x25x7um; mobile phase: [H₂O(10mM NH₄HCO₃)-ACN]; gradient: 18%-48% B over 15.0 min) to give 5-fluoro-N2-(4-(methylamino)phenyl)-N7-(oxetan-2-ylmethyl)benzo[d]oxazole-2,7-dicarboxamide **(Compound 44)** (10 mg, 25.10 µmol, 20.00% yield) was obtained as a yellow solid. The structure of Compound 44 was confirmed by ¹H NMR (400MHz, CDCl₃) δ = 8.91 (s, 1H), 8.01 (dd, *J* = 2.4Hz, 9.6Hz, 1H), 7.88-7.81 (m, 1H), 7.64 (dd, *J* = 2.8Hz, 7.2Hz, 1H), 7.59-7.51 (m, 2H), 7.28-7.26 (m, 1H), 6.69 (d, *J* = 8.8Hz, 2H), 5.22-5.11 (m, 1H), 4.80-4.75 (m, 2H), 3.93-3.78 (m, 2H), 2.87 (s, 3H), 2.78-2.72 (m, 1H), 2.66-2.60 (m, 1H). ¹⁹F NMR (376.5MHz, CDCl₃) δ = -113.483.ESI calculcation for C₂₀H₂₀FN₄O₄ [M+H]⁺ 399.1; found 399.0.

### Synthesis Example 45: Synthesis of Compound 45

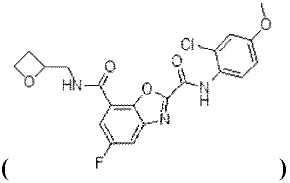

Compound 45 was synthesized according to the following Reaction Scheme 45.

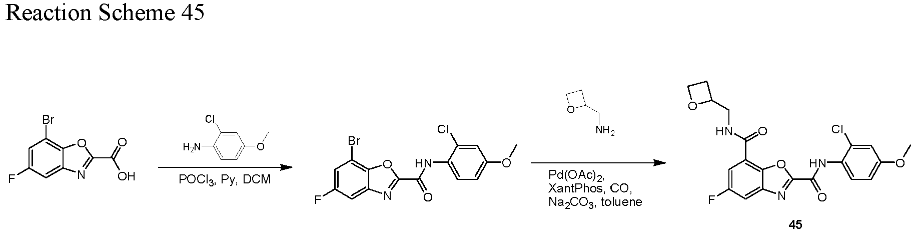

As shown in Reaction Scheme 45, first, 7-bromo-N-(2-chloro-4-methoxyphenyl)-5-fluorobenzo[d]oxazole-2-carboxamide was prepared. To a solution of 7-bromo-5-fluoro-1,3-benzoxazole-2-carboxylic acid (2 g, 7.69 mmol, 1 eq) in DCM (20 mL) was added 2-chloro-4-methoxy-aniline (1.21 g, 7.69 mmol, 1 eq), Py (3.04 g, 38.46 mmol, 3.10 mL, 5 eq) and POCl₃ (2.36 g, 15.38 mmol, 1.43 mL, 2 eq). The mixture was stirred at 25°C for 2hr. The mixture was quenched with H₂O (30 mL) and extracted three times with 10 mL DCM (10 mL x 3). The combined organic phase was dried over anhydrous sodium sulfate, concentrated and purified by column chromatography (SiO₂, Ethyl acetate in Petroleum ether = 0% to 10%) to give 7-bromo-N-(2-chloro-4-methoxy-phenyl)-5-fluoro-1,3-benzoxazole-2-carboxamide (300 mg, 750.75 µmol, 9.76% yield) as a yellow solid. The structure of 7-bromo-N-(2-chloro-4-methoxy-phenyl)-5-fluoro-1,3-benzoxazole-2-carboxamide was confirmed by ¹H NMR (400MHz, CDCl₃) δ = 9.41 (s, 1H), 8.41 (d, *J* = 8.8Hz, 1H), 7.60-7.38 (m, 2H), 7.01 (d, *J* = 2.4Hz, 1H), 6.91 (dd, *J* = 3.2Hz, 9.2Hz, 1H), 3.83 (s, 3H).¹⁹F NMR (376.5MHz, CDCl₃) δ = -113.399. ESI calculation for C₁₅H₁₀BrClFN₂O₃ [MH]⁺ 398.9; found 399.0.

Finally, N2-(2-chloro-4-methoxyphenyl)-5-fluoro-N7-(oxetan-2-ylmethyl)benzo[d]oxazole-2,7-dicarboxamide **(Compound 45):** A mixture of 7-bromo-N-(2-chloro-4-methoxy-phenyl)-5-fluoro-1,3-benzoxazole-2-carboxamide (300 mg, 750.75 µmol, 1 eq), oxetan-2-ylmethanamine (196.22 mg, 2.25 mmol, 3 eq), Pd(OAc)₂ (3.37 mg, 15.02 µmol, 0.02 eq), XantPhos (8.69 mg, 15.02 µmol, 0.02 eq), and Na₂CO₃ (238.72 mg, 2.25 mmol, 3 eq) in toluene (3 mL) was degassed and purged with CO three times, and then the mixture was stirred at 80°C for 16hr under CO atmosphere (20 psi). The mixture was filtered and concentrated to give a residue. The residue was purified by column chromatography (SiO₂, EtOAC in DCM = 0% to 30%) to give N2-(2-chloro-4-methoxyphenyl)-5-fluoro-N7-(oxetan-2-ylmethyl)benzo[d]oxazole-2,7-dicarboxamide **(Compound 45)** (50 mg, 115.26 µmol, 15.35% yield) as a yellow solid. The structure of Compound 45 was confirmed by ¹H NMR (400MHz, CDCl₃) δ = 9.44 (s, 1H), 8.36 (d, *J* = 9.2Hz, 1H), 8.03 (dd, *J* = 2.4Hz, 9.6Hz, 1H), 7.83-7.65 (m, 2H), 7.02 (d, *J* = 2.8Hz, 1H), 6.90 (dd, *J* = 2.8Hz, 9.2Hz, 1H), 5.21-5.05 (m, 1H), 4.86-4.70 (m, 2H), 3.96-3.86 (m, 1H), 3.83 (s, 3H), 3.80-3.72 (m, 1H), 2.79-2.60 (m, 2H). ¹⁹F NMR (376.5MHz, CDCl₃) δ = -113.228. ESI calculation for C₂₀H₁₈ClFN₃O₅ [MH]⁺ 434.0; found 434.1.

### Synthesis Example 46: Synthesis of Compound 46

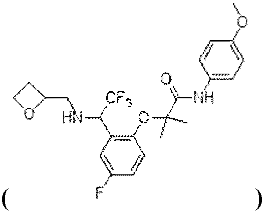

Compound 46 was synthesized according to the following Reaction Scheme 46.

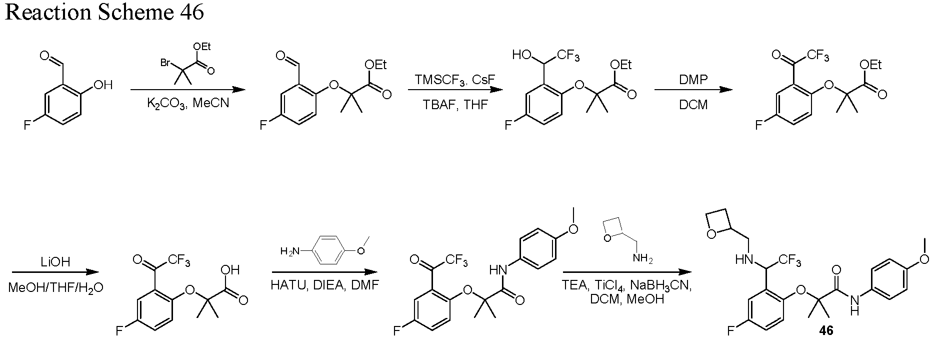

As shown in Reaction Scheme 46, first, ethyl 2-(4-fluoro-2-formylphenoxy)-2-methylpropanoate was prepared. To a solution of 5-fluoro-2-hydroxy-benzaldehyde (5 g, 35.69 mmol, 1 eq) in MeCN (50 mL) was added K₂CO₃ (17.26 g, 124.90 mmol, 3.5 eq) and ethyl 2-bromo-2-methyl-propanoate (24.36 g, 124.90 mmol, 18.33 mL, 3.5 eq). The mixture was stirred at 80°C for 20hr. The mixture was filtered and concentrated to give ethyl 2-(4-fluoro-2-formyl-phenoxy)-2-methyl-propanoate (9.5 g, crude) as a yellow oil. The structure of ethyl 2-(4-fluoro-2-formyl-phenoxy)-2-methyl-propanoate was confirmed by ¹H NMR (400MHz, CDCl₃) δ = 10.43 (d, *J =* 3.2Hz, 1H), 7.51 (dd, *J* = 3.2Hz,8.0Hz, 1H), 7.19-7.11 (m, 1H), 6.82 (dd, *J =* 4.0Hz, 9.2Hz, 1H), 4.27-4.20 (m, 2H), 1.65 (s, 6H), 1.24 (t, *J* = 7.2Hz, 3H). ¹⁹F NMR (376.5MHz, CDCl₃) δ = - 120.110. ESI calculation for C₁₃H₁₆FO₄ [MH]⁺ 255.1; found 255.1.

Next, ethyl 2-(4-fluoro-2-(2,2,2-trifluoro-1-hydroxyethyl)phenoxy)-2-methylpropanoate was prepared. To a solution of ethyl 2-(4-fluoro-2-formyl-phenoxy)-2-methylpropanoate (9 g, 35.40 mmol, 1 eq) in THF (100 mL) was added trifluoromethyltrimethylsilane (TMSCF₃) (10.07 g, 70.80 mmol, 2 eq) and CsF (107.54 mg, 707.95 µmol, 0.02 eq). The mixture was stirred at 25°C for 4hr. Tetrabutylammonium fluoride (TBAF) (1 M, 35.40 mL, 1 eq) was added to the reaction mixture. The mixture was stirred at 20°C for 0.5hr. H₂O (50 mL) was slowly added to the reaction mixture, and the mixture was extracted three times with 50 mL EtOAc (50 mL × 3). The mixture was concentrated and purified by column chromatography (SiO₂, Ethyl acetate in Petroleum ether = 0% to 10%) to give ethyl 2-[4-fluoro-2-(2,2,2-trifluoro-1-hydroxyethyl)phenoxy]-2-methyl-propanoate (6.5 g, 20.05 mmol, 56.63% yield) as a yellow oil. The structure of ethyl 2-[4-fluoro-2-(2,2,2-trifluoro-1-hydroxy-ethyl)phenoxy]-2-methyl-propanoate was confirmed by ¹H NMR (400MHz, CDCl₃) δ = 7.23-7.13 (m, 1H), 7.00-6.91 (m, 1H), 6.79-6.64 (m, 1H), 5.42-5.33 (m, 1H), 4.30-4.18 (m, 2H), 3.62 (d, *J* = 6.8Hz, 1H), 1.63 (s, 6H), 1.23 (t, *J =* 7.2Hz, 3H). ¹⁹F NMR (376.5MHz, CDCl₃) δ = -77.601, -120.958.

Next, ethyl 2-(4-fluoro-2-(2,2,2-trifluoroacetyl)phenoxy)-2-methylpropanoate was prepared. To a solution of ethyl 2-[4-fluoro-2-(2,2,2-trifluoro-1-hydroxy-ethyl)phenoxy]-2-methyl-propanoate (6.5 g, 20.05 mmol, 1 eq) in DCM (70 mL) was added Dess-Martin periodinane (DMP) (12.75 g, 30.07 mmol, 9.32 mL, 1.5 eq). The mixture was stirred at 20°C for 4hr. Na₂S₂O₃ : NaHCO₃ = 1:1 (100 mL) was slowly added to the reaction mixture, and the mixture was extracted with DCM (100 mL x 3). The organic phase was filtered and concentrated to give ethyl 2-[4-fluoro-2-(2,2,2-trifluoroacetyl)phenoxy]-2-methyl-propanoate (6.3 g, crude) as a yellow oil. The structure of ethyl 2-[4-fluoro-2-(2,2,2-trifluoroacetyl)phenoxy]-2-methyl-propanoate was confirmed by ¹H NMR (400MHz, CDCl₃) δ = 7.30 (dd, *J =* 3.2Hz, 8.0Hz, 1H), 7.23-7.10 (m, 1H), 6.74 (dd, *J =* 4.0Hz, 9.2Hz, 1H), 4.33-4.14 (m, 2H), 1.64 (s, 6H), 1.22 (t, *J* = 7.2Hz, 3H). ¹⁹F NMR (376.5MHz, CDCl₃) δ = -73.856, -121.048.

Next, 2-(4-fluoro-2-(2,2,2-trifluoroacetyl)phenoxy)-2-methylpropanoic acid was prepared. To a solution of ethyl 2-[4-fluoro-2-(2,2,2-trifluoroacetyl)phenoxy]-2-methylpropanoate (6 g, 18.62 mmol, 1 eq) in THF (60 mL), H₂O (5 mL) and MeOH (10 mL) was added LiOH (535.11 mg, 22.34 mmol, 1.2 eq). The mixture was stirred at 25°C for 2hr, quenched by addition of 1M HCl (100 mL),and then extracted three times with 50 mL EtOAc (50 mL x 3). The combined organic layers were washed with brine (30 mL), dried over Na₂SO₄, filtered and concentrated to give 2-[4-fluoro-2-(2,2,2-trifluoroacetyl)phenoxy]-2-methyl-propanoic acid (5.5 g, crude) as a yellow oil. ESI calculation for C₁₂H₉F₄O₄ [M-H]⁺ 293.0; found 293.0.

Next, 2-(4-fluoro-2-(2,2,2-trifluoroacetyl)phenoxy)-N-(4-methoxyphenyl)-2-methylpropanamide was prepared. To a solution of 2-[4-fluoro-2-(2,2,2-trifluoroacetyl)phenoxy]-2-methyl-propanoic acid (5.5 g, 18.69 mmol, 1 eq) in DMF (55 mL) was added DIEA (12.08 g, 93.47 mmol, 16.28 mL, 5 eq) and HATU (14.22 g, 37.39 mmol, 2 eq) at 25°C, and the reaction mixture was stirred for 30 mins, followed by addition of 4-methoxyaniline (2.76 g, 22.43 mmol, 1.2 eq). The mixture was stirred at 25°C for 16hr, quenched by H₂O (50 mL), and then extracted three times with 50 mL EtOAc (50 mL x 3). The combined organic layers were washed with brine (30 mL), dried over Na₂SO₄, concentrated and purified by column chromatography (SiO₂, Commercial hexanes: Ethyl acetate = 0% to 15%) to give 2-[4-fluoro-2-(2,2,2-trifluoroacetyl)phenoxy]-N-(4-methoxyphenyl)-2-methyl-propanamide (2 g, 5.01 mmol, 26.79% yield) as a yellow oil. The structure of 2-[4-fluoro-2-(2,2,2-trifluoroacetyl)phenoxy]-N-(4-methoxyphenyl)-2-methyl-propanamide was confirmed by ¹H NMR (400MHz, CDCl₃) δ = 9.03 (s, 1H), 7.66-7.56 (m, 2H), 7.53-7.49 (m, 1H), 7.35-7.28 (m, 1H), 7.17-7.12 (m, 1H), 6.93-6.88 (m, 2H), 3.80 (s, 3H), 1.71 (s, 6H). ¹⁹F NMR (376.5MHz, CDCl₃) δ = -72.159, -118.669.

Finally, 2-(4-fluoro-2-(2,2,2-trifluoro-1-((oxetan-2-ylmethyl)amino)ethyl)phenoxy)-N-(4-methoxyphenyl)-2-methylpropanamide **(Compound 46)** was prepared. To a solution of 2-[4-fluoro-2-(2,2,2-trifluoroacetyl)phenoxy]-N-(4-methoxyphenyl)-2-methyl-propanamide (700 mg, 1.75 mmol, 1 eq) in DCM (7 mL) was added oxetan-2-ylmethanamine (229.07 mg, 2.63 mmol, 1.5 eq), TEA (532.13 mg, 5.26 mmol, 731.95 µL, 3 eq) and TiCl₄ (166.25 mg, 876.46 µmol, 0.5 eq) .The mixture was stirred at 25°C for 16h. NaBH₃CN (330.47 mg, 5.26 mmol, 3 eq) in MeOH (7 mL) was added to the mixture. The mixture was stirred at 25°C for 16h. The mixture was quenched by H₂O (5 mL) and then extracted three times with 5 mL DCM (5 mL x 3). The combined organic layers were washed with brine (3 mL), dried over Na₂SO₄, concentrated and purified by column chromatography (SiO₂, Commercial hexanes: Ethyl acetate = 0% to 15%) to give 2-(4-fluoro-2-(2,2,2-trifluoro-1-((oxetan-2-ylmethyl)amino)ethyl)phenoxy)-N-(4-methoxyphenyl)-2-methylpropanamide **(Compound 46)** (30.9 mg, 65.68 µmol, 3.75% yield) as a yellow solid. The structure of Compound 46 was confirmed by ¹H NMR (400MHz, CDCl₃) δ = 8.41 (s, 1H), 7.44-7.37 (m, 2H), 7.23-7.19 (m, 1H), 6.99-6.92 (m, 1H), 6.88-6.81 (m, 3H), 5.04-4.94 (m, 1H), 4.91-4.79 (m, 1H), 4.69-4.58 (m, 1H), 4.54-4.43 (m, 1H), 3.78 (s, 3H), 2.85 (d, *J =* 4.0Hz, 2H), 2.67-2.58 (m, 2H), 1.64 (d, *J* = 6.8Hz, 6H). ESI calculation for C₂₃H₂₇F₄N₂O₄ [MH]⁺ 471.1; found 471.1.

### Synthesis Example 47: Synthesis of Compound 47

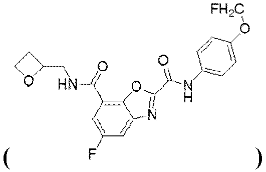

Compound 47 was synthesized according to the following Reaction Scheme 47.

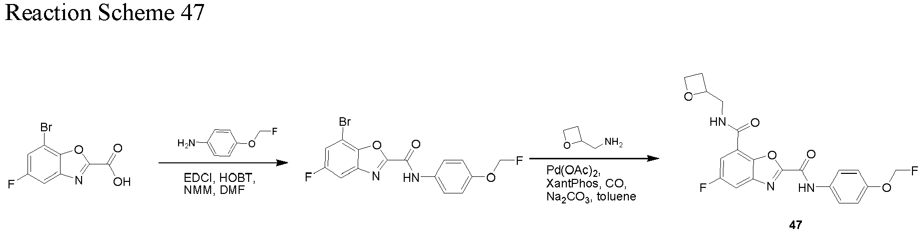

As show in Reaction Scheme 47, first, 7-bromo-5-fluoro-N-[4-(fluoromethoxy)phenyl]-1,3-benzoxazole-2-carboxamide was prepared. To a solution of 7-bromo-5-fluoro-1,3-benzoxazole-2-carboxylic acid (450 mg, 1.73 mmol, 1 eq) in DMF (8 mL) was added 4-methylmorpholine (NMM) (525.15 mg, 5.19 mmol, 570.82 µL, 3 eq), HOBt (292.32 mg, 2.16 mmol, 1.25 eq), and 4-(fluoromethoxy)aniline (293.12 mg, 2.08 mmol, 1.2 eq) at 25°C. After addition, the mixture was stirred at this temperature for 10 min, and then EDCI (364.95 mg, 1.90 mmol, 1.1 eq) was added at 25°C. The resulting mixture was stirred at 50°C for 3hr. The reaction mixture was quenched by addition of NH₄Cl (5 mL) and extracted twice with 5 mL EtOAc (EA) (5 mL x 2). The combined organic layers were washed with H₂O (10 mL), dried over Na₂SO₄, filtered, concentrated and triturated with MeCN (10 mL) at 25°C for 30 min to give 7-bromo-5-fluoro-N-[4-(fluoromethoxy)phenyl]-1,3-benzoxazole-2-carboxamide (420 mg, 1.10 mmol, 63.34% yield) as a yellow solid. ESI calculation for C₁₅H₁₀BrF₂N₂O₃ [MH]⁺ 383.0; found 383.0.

Finally, 5-fluoro-N2-[4-(fluoromethoxy)phenyl]-N7-(oxetan-2-ylmethyl)-1,3-benzoxazole-2,7-dicarboxamid **(Compound 47)** was prepared. To a solution of 7-bromo-5-fluoro-N-[4-(fluoromethoxy)phenyl]-1,3-benzoxazole-2-carboxamide (420 mg, 1.10 mmol, 1 eq) in toluene (10 mL) was added (5-diphenylphosphanyl-9,9-dimethyl-xanthen-4-yl)-diphenyl-phosphane (12.69 mg, 21.92 µmol, 0.02 eq), disodium:carbonate (348.55 mg, 3.29 mmol, 3 eq), diacetoxypalladium (4.92 mg, 21.92 µmol, 0.02 eq), and oxetan-2-ylmethanamine (143.25 mg, 1.64 mmol, 1.5 eq) under N₂ atmosphere. The suspension was degassed and purged with CO three times. The mixture was stirred under CO (15 Psi) at 80°C for 16hr. The catalyst was removed by filtration. The solvent was removed under vacuum and purified by flash silica gel chromatography (ISCO^{®}; 12 g SEPAFLASH^{®} Silica Flash Column, Eluent of 0~30% Ethyl acetate/Commercial hexanes gradient @ 30 mL/min) to give 5-fluoro-N2-[4-(fluoromethoxy)phenyl]-N7-(oxetan-2-ylmethyl)-1,3-benzoxazole-2,7-dicarboxamid **(Compound 47)** (73 mg, 174.91 µmol, 15.96% yield) as a white solid. The structure of Compound 47 was confirmed by ¹H NMR (400MHz, CDCl₃) δ = 8.98 (s, 1H), 8.10-8.01 (m, 1H), 7.74-7.65 (m, 4H), 7.15 (d, J = 8.0 Hz, 2H), 5.79 (s, 1H), 5.66 (s, 1H), 5.24-5.10 (m, 1H), 4.85-4.77 (m, 2H), 3.95-3.85 (m, 1H), 3.84-3.74 (m, 1H), 2.78-2.61 (m, 2H). ¹⁹F NMR (376.5MHz, CDCl₃) δ = -113.138, -148.675. ESI calculation for C₂₀H₁₇F₂N₃O₅ [M+H]⁺ 418.1; found 418.0.

### Synthesis Example 48: Synthesis of Compound 48

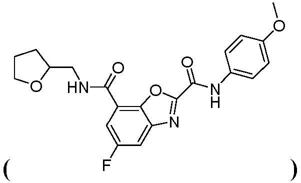

Compound 48 was synthesized according to the following Reaction Scheme 48.

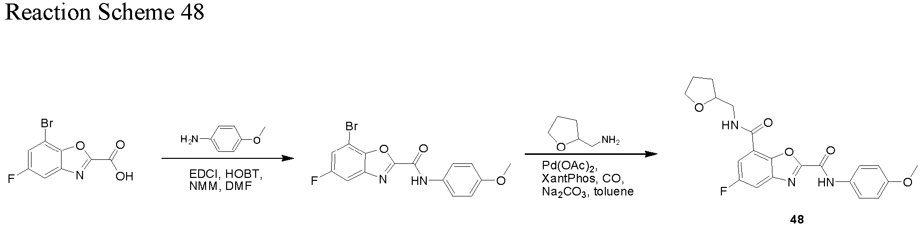

As shown in Reaction Scheme 48, first, 7-bromo-5-fluoro-N-(4-methoxyphenyl)-1,3-benzoxazole-2-carboxamide was prepared. To a solution of 7-bromo-5-fluoro-1,3-benzoxazole-2-carboxylic acid (500 mg, 1.92 mmol, 1 eq) in DMF (5 mL) was added EDCI (405.50 mg, 2.12 mmol, 1.1 eq), HOBt (324.79 mg, 2.40 mmol, 1.25 eq), 4-methylmorpholine (NMM) (583.50 mg, 5.77 mmol, 634.24 µL, 3 eq) and 4-methoxyaniline (284.18 mg, 2.31 mmol, 1.2 eq). The mixture was stirred at 30°C for 16hr, quenched by addition of NH₄Cl (10 mL) and extracted twice with 10 mL EtOAc (EA) (10 mL x 2). The combined organic layers were washed with H₂O (15 mL), dried over Na₂SO₄, filtered, concentrated and purified by flash silica gel chromatography (ISCO^{®}; 12 g SEPAFLASH^{®} Silica Flash Column, Eluent of 0~6% Ethyl acetate/Commercial hexanes gradient @ 20 mL/min) to give 7-bromo-5-fluoro-N-(4-methoxyphenyl)-1,3-benzoxazole-2-carboxamide (230 mg, 629.87 µmol, 32.76% yield) as a yellow solid. ESI calculation for C₁₅H₁₁BrFN₂O₃ [MH]⁺ 365.0; found 365.0.

Finally, 5-fluoro-N2-(4-methoxyphenyl)-N7-(tetrahydrofuran-2-ylmethyl)-1,3-benzoxazole-2,7-dicarboxamide **(Compound 48)** was prepared. To a solution of 7-bromo-5-fluoro-N-(4-methoxyphenyl)-1,3-benzoxazole-2-carboxamide (230 mg, 629.87 µmol, 1 eq) in toluene (5 mL) was added (5-diphenylphosphanyl-9, 9-dimethyl-xanthen-4-yl)-diphenyl-phosphane (7.29 mg, 12.60 µmol, 0.02 eq), tetrahydrofuran-2-ylmethanamine (95.56 mg, 944.81 µmol, 97.51 µL, 1.5 eq), Na₂CO₃ (200.28 mg, 1.89 mmol, 3 eq) and Pd(OAc)₂ (2.83 mg, 12.60 µmol, 0.02 eq) under N₂ atmosphere. The suspension was degassed and purged with CO three times. The mixture was stirred under CO (15 Psi) at 80°C for 16 hr. The catalyst was removed by filtration. The solvent was removed under vacuum and purified by flash silica gel chromatography (ISCO^{®}; 12 g SEPAFLASH^{®} Silica Flash Column, Eluent of 0~30% Ethyl acetate/Commercial hexanes gradient @ 30 mL/min) to give 5-fluoro-N2-(4-methoxyphenyl)-N7-(tetrahydrofuran-2-ylmethyl)-1,3-benzoxazole-2,7-dicarboxamide **(Compound 48)** (21.6 mg, 52.25 µmol, 8.30% yield) as a white solid. The structure of Compound 48 was confirmed by ¹H NMR (400MHz, CDCl₃) δ = 8.92 (s, 1H), 8.01 (d, J = 9.6 Hz, 1H), 7.71-7.63 (m, 3H), 7.56 (s, 1H), 6.97-6.94 (m, 2H), 4.24-4.19 (m, 1H), 4.13-4.05 (m, 1H), 3.86-3.81 (m, 5H), 3.62-3.54 (m, 1H), 2.08-1.93 (m, 3H), 1.75-1.69 (m, 1H). ¹⁹F NMR (376.5MHz, CDCl₃) δ = -113.453. ESI calculation for C₂₁H₂₁FN₃O₅ [MH]⁺ 414.1; found 414.1.

### Synthesis Example 49: Synthesis of Compound 49

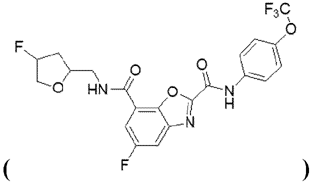

Compound 49 was synthesized according to the following Reaction Scheme 49.

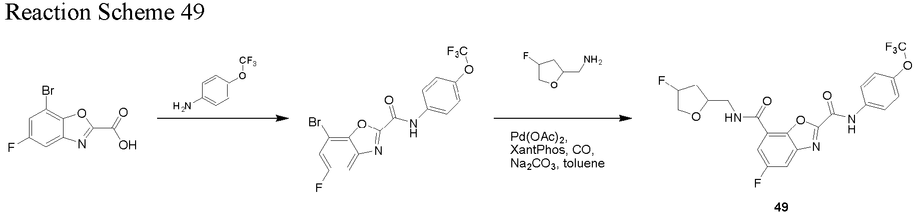

As shown in Reaction Scheme 49, first, 7-bromo-5-fluoro-N-[4-(trifluoromethoxy)phenyl]-1,3-benzoxazole-2-carboxamide was prepared. To a solution of ethyl 7-bromo-5-fluoro-1, 3-benzoxazole-2-carboxylate (400 mg, 1.39 mmol, 1 eq) in toluene (5 mL) was added TsOH (23.91 mg, 138.86 µmol, 0.1 eq) and 4-(trifluoromethoxy)aniline (319.73 mg, 1.81 mmol, 244.07 µL, 1.3 eq). The mixture was stirred at 100°C for 16 hr. The reaction mixture was quenched by addition of NH₄Cl (10 mL) and extracted twice with 10 mL EA (10mL x 2). The combined organic layers were washed with H₂O (20 mL), dried over Na₂SO₄, filtered, concentrated and purified by flash silica gel chromatography (ISCO^{®}; 12 g SEPAFLASH^{®} Silica Flash Column, Eluent of 0~5% Ethyl acetate/Commercial hexanes gradient @ 15 mL/min) to give 7-bromo-5-fluoro-N-[4-(trifluoromethoxy)phenyl]-1,3-benzoxazole-2-carboxamide (300 mg, 715.78 µmol, 51.55% yield) as a black brown solid. ESI calculation for C₁₅H₈BrF₄N₂O₃ [MH]⁺419.0; found 418.9.

Finally, 5-fluoro-N7-[(4-fluorotetrahydrofuran-2-yl)methyl]-N2-[4-(trifluoromethoxy)phenyl]-1,3-benzoxazole-2,7-dicarboxamide **(Compound 49):** To a solution of 7-bromo-5-fluoro-N-[4-(trifluoromethoxy)phenyl]-1,3-benzoxazole-2-carboxamide (170 mg, 405.61 µmol, 1 eq) in toluene (10 mL) was added (5-diphenylphosphanyl-9,9-dimethyl-xanthen-4-yl)-diphenyl-phosphane (4.69 mg, 8.11 µmol, 0.02 eq), disodium carbonate (128.97 mg, 1.22 mmol, 3 eq), diacetoxypalladium (1.82 mg, 8.11 µmol, 0.02 eq), and (4-fluorotetrahydrofuran-2-yl)methanamine (96.65 mg, 811.21 µmol, 2 eq) under N₂ atmosphere. The suspension was degassed and purged with CO three times. The mixture was stirred under CO (15 Psi) at 80°C for 16hr. The catalyst was removed by filtration. The solvent was removed under vacuum and purified by flash silica gel chromatography (ISCO^{®}; 12 g SEPAFLASH^{®} Silica Flash Column, Eluent of 0~25% Ethyl acetate/Commercial hexanes gradient @ 20 mL/min) to give 5-fluoro-N7-[(4-fluorotetrahydrofuran-2-yl)methyl]-N2-[4-(trifluoromethoxy)phenyl]-1,3-benzoxazole-2,7-dicarboxamide **(Compound 49)** (45.1 mg, 92.92 µmol, 22.91% yield) as a white solid. The structure of Compound 49 was confirmed by ¹H NMR (400MHz, CDCl₃) δ = 9.04 (s, 1H), 8.05-7.99 (m, 1H), 7.78 (d, J = 8.8 Hz, 2H), 7.68-7.65 (m, 1H), 7.50-7.45 (m, 1H), 7.30 (d, J = 8.6 Hz, 2H), 5.48-5.29 (m, 1H), 4.53-4.46 (m, 1H), 4.42-4.29 (m, 1H), 4.17-4.07 (m, 1H), 3.90-3.83 (m, 1H), 3.81-3.74 (m, 1H), 2.38-2.28 (m, 1H), 1.99-1.88 (m, 1H). ¹⁹F NMR (376.5MHz, CDCl₃) δ = -58.065, -112.838, -174.193. ESI calculation for C₂₁H₁₇F₅N₃O₅ [MH]⁺ 486.1; found 486.0.

### Synthesis Example 50: Synthesis of Compound 50

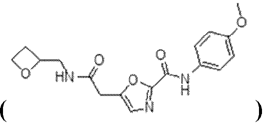

Compound 50 was synthesized according to the following Reaction Scheme 50.

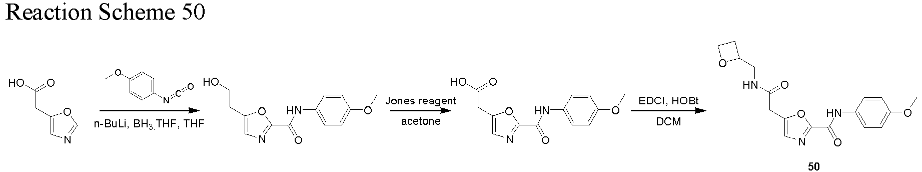

As shown in Reaction Scheme 50, first, 5-(2-hydroxyethyl)-N-(4-methoxyphenyl)oxazole-2-carboxamide was prepared. To a solution of 2-oxazol-5-ylacetic acid (2 g, 15.74 mmol, 1 eq) in THF (20 mL) was added borane-tetrahydrofuran (BH₃-THF) (1 M, 31.47 mL, 2 eq) at 25°C and stirred at 25°C for 1h. n-BuLi (2.5 M, 15.74 mL, 2.5 eq) was added to the mixture at -78°C. The mixture was stirred at -78°C for 40 min. 1-isocyanato-4-methoxybenzene (4.69 g, 31.47 mmol, 4.05 mL, 2 eq) was added to the mixture at -78°C. The mixture was stirred at -78°C for 2h. The reaction mixture was quenched by addition of NH₄Cl (20 mL) and then extracted three times with 20 mL EtOAc (20 mL x 3). The combined organic layers were washed twice with 20 mL brine (20 mL x 2), dried over Na₂SO₄, concentrated and purified by column chromatography (SiO₂, Commercial hexanes: Ethyl acetate = 0% to 70%) to give 5-(2-hydroxyethyl)-N-(4-methoxyphenyl)oxazole-2-carboxamide (420 mg, 1.60 mmol, 10.18% yield) as a white solid. The structure of 5-(2-hydroxyethyl)-N-(4-methoxyphenyl)oxazole-2-carboxamide was confirmed by ¹H NMR (400MHz, CDCl₃) δ = 8.71 (s, 1H), 7.61-7.53 (m, 2H), 7.01 (s, 1H), 6.95-6.89 (m, 2H), 3.97 (t, *J* = 6.4Hz, 2H), 3.80 (s, 3H), 3.03 (t, *J* = 6.0Hz, 2H).

Next, 2-(2-((4-methoxyphenyl)carbamoyl)oxazol-5-yl)acetic acid was prepared. To a solution of 5-(2-hydroxyethyl)-N-(4-methoxyphenyl)oxazole-2-carboxamide (420 mg, 1.60 mmol, 1 eq) in acetone (5 mL) was added Jones reagent (2.54 g, 12.81 mmol, 8 eq) at 25°C. The mixture was stirred at 20°C for 8hr. 1M HCl (10 mL) was added to the reaction mixture, and the mixture was extracted three times with 5 mL EtOAc (5 mL x 3). The mixture was concentrated to give 2-[2-[(4-methoxyphenyl)carbamoyl]oxazol-5-yl]acetic acid (230 mg, crude) as a yellow oil. The structure of 2-[2-[(4-methoxyphenyl)carbamoyl]oxazol-5-yl]acetic acid was confirmed by ¹H NMR (400MHz, DMSO) δ = 10.67 (m, 1H), 7.69 (d, *J =* 9.2Hz, 2H), 7.30 (s, 1H), 6.92 (d, *J* = 9.2Hz, 2H), 3.93 (s, 2H), 3.73 (s, 3H).ESI calculation for C₁₃H₁₃N₂O₅ [MH]⁺ 277.2; found 277.2.

Finally, N-(4-methoxyphenyl)-5-(2-((oxetan-2-ylmethyl)amino)-2-oxoethyl)oxazole-2-carboxamide **(Compound 50)** was prepared. To a solution of 2-[2-[(4-methoxyphenyl)carbamoyl]oxazol-5-yl]acetic acid (230 mg, 832.60 µmol, 1 eq) in DCM (2.5 mL) was added HOBt (112.50 mg, 832.60 µmol, 1 eq) and EDCI (239.41 mg, 1.25 mmol, 1.5 eq) at 25°C, and the reaction mixture was stirred for 30 mins, followed by addition of oxetan-2-ylmethanamine (79.79 mg, 915.86 µmol, 1.1 eq).The mixture was stirred at 25°C for 16h, quenched by H₂O (3 mL), and extracted three times with 3 mL EtOAc (3 mL x 3). The combined organic layers were washed with brine (3 mL), dried over Na₂SO₄, concentrated and purified by column chromatography (SiO₂, MeOH in DCM= 0% to 3%) to give N-(4-methoxyphenyl)-5-(2-((oxetan-2-ylmethyl)amino)-2-oxoethyl)oxazole-2-carboxamide **(Compound 50)** (22.1 mg, 63.99 µmol, 7.69% yield) as a yellow solid. The structure of Compound 50 was confirmed by ¹H NMR (400MHz, CDCl₃) δ = 8.69 (s, 1H), 7.57 (d, *J* = 8.8Hz, 2H), 7.18 (s, 1H), 6.91 (d, *J* = 9.2Hz, 2H), 6.25 (s, 1H), 4.99-4.88 (m, 1H), 4.72-4.63 (m, 1H), 4.56-4.37 (m, 1H), 3.81 (s, 3H), 3.78 (s, 2H), 3.65-3.57 (m, 1H), 3.52-3.44 (m, 1H), 2.73-2.54 (m, 1H), 2.52-2.33 (m, 1H). ESI calculation for C₁₇H₂₀N₃O₅ [MH]⁺ 346.3; found 346.0.

### EXPERIMENTAL EXAMPLES

Each of Compounds 1-50 were evaluated using a GCase AC₅₀ enzyme activity assay, as well as EC₅₀ cell-based assays for GCase according to the assay protocols noted below.

### GCase AC₅₀ Enzyme Activity Assay

An *in vitro* enzyme assay was performed through a dose response curve of Compounds (0-40 µM) with purified enzyme (Velaglucerase alfa, VPRIV^{®}, Shire Human Genetic Therapies, Cambridge, MA) in citric phosphate [0.025 mM] buffer (pH 5.6) where 4-MuGlu [4 mM] was the substrate. Compounds were pre-incubated with the enzyme for 40 minutes at room temperature prior to substrate addition, and then incubated at 37°C for 1 hour. AC₅₀ represents the concentration of a Compound that produces 50% of its maximal response in the *in vitro* GCase activity assay.

### EC₅₀ Cell-based Assay for GCase

In cell assays, GCase activity measurements were carried out on harvested cell pellets on the 5th day of Compound treatment. Healthy human fibroblasts (GCase cell), mutant N370/ΔGG (GCase N370S) or L444P/L444P (GCase L444P) were incubated with the Compound (0-20 µM) for 5 days with 15% fetal bovine serum (FBS) in Dulbecco's Modified Eagle Medium (DMEM) in 100 mm culture dishes. Fresh Medium with Compound were replaced every other day. On the 5th day of Compound treatment, harvested cell pellets were lysed with 1% Na-taurocholate (Tc) and 1% Triton X-100 (Tx), and mixed with the reaction buffer which contained 0.025 mM Citric phosphate buffer (pH 5.6), 0.25%Tc/Tx and 4 mM 4-MuGlu, then incubated at 37°C for 1 hour. EC₅₀ represents the concentration of a Compound that produces 50% of its maximal response in the cell-based GCase activity assay.

GCase activities were determined fluorometrically with 4-MuGlu. The relative fluorescence unit (RFU) of 4-MuGlu standard curve is generated with the plate reader (M5, Molecular Devise) as 1 n mole= 56500 RFU. The human mutant Gaucher fibroblasts were purchased from Coriell Institute (Camden, NJ): N370/ΔGG (Cat#: GM00852) and L444P/L444P (cat#: GM00877)

Selected active compounds were tested on N370S and L444P variants. Assay results are shown below in Table 1. In Table 1, "A" denotes less than 10 µM, and B denotes 10-100 µM.

**Table 1 - GCase AC₅₀ (enzyme assay) and EC₅₀ (cell-based assay)**

| **Compound** | **GCase enzyme AC₅₀ (µM)** | **GCase cell EC₅₀ (µM)** | **GCase N370S cell EC₅₀ (µM)** | **GCase L444P cell EC₅₀ (µM)** |
|---|---|---|---|---|
| 1 | A | A | | |
| 2 | A | B | A | |
| 3 | A | A | | |
| 4 | A | A | A | |
| 5 | A | A | | |
| 6 | A | A | | |
| 7 | A | A | | |
| 8 | A | A | A | A |
| 9 | A | A | | |
| 10 | A | A | | |
| 11 | A | A | | |
| 12 | A | A | | |
| 13 | A | A | | |
| 14 | A | | | |
| 15 | A | | | |
| 16 | A | | | |
| 17 | A | A | | |
| 18 | A | A | | |
| 19 | A | A | | |
| 20 | A | A | | |
| 21 | A | A | | |
| 22 | A | A | | |
| 23 | A | A | | |
| 24 | A | A | | |
| 25 | A | A | | |
| 26 | A | A | A | A |
| 27 | A | A | | |
| 28 | B | A | | |
| 29 | B | | | |
| 30 | A | A | | |
| 31 | A | | | |
| 32 | A | A | B | |
| 33 | A | A | | |
| 34 | A | A | | |
| 35 | A | A | | |
| 36 | A | A | | |
| 37 | A | A | A | A |
| 38 | A | A | | |
| 39 | A | A | | |
| 40 | A | A | | |
| 41 | A | A | | |
| 42 | A | A | | |
| 43 | B | A | | |
| 44 | A | A | | |
| 45 | A | A | | |
| 46 | A | A | | |
| 47 | A | A | | |
| 48 | A | A | | |
| 49 | A | A | | |
| 50 | A | A | | |

All percentages and ratios are calculated by weight unless otherwise indicated.

All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a volume disclosed as "20 mL" is intended to mean "about 20 mL."

Every document cited herein, including any cross-referenced or related patent or application, is hereby incorporated herein by reference in its entirety unless expressly excluded or otherwise limited. All accessioned information (e.g., as identified by PUBMED, PUBCHEM, NCBI, UNIPROT, or EBI accession numbers) and publications in their entireties are incorporated into this disclosure by reference in order to more fully describe the state of the art as known to those skilled therein as of the date of this disclosure. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications may be made without departing from the spirit and scope of the invention. It is therefore intended to cover in the appended claims all such changes and modifications that are within the scope of this invention.

### ILLUSTRATIVE COMBINATIONS

The following illustrative examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A compound represented by Formula 1: 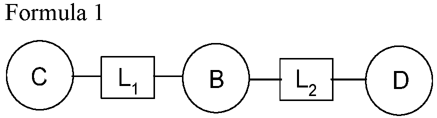
in Formula 1, ring C is selected from moieties represented by Formulae 2A and 2B: 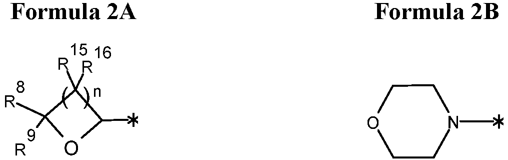 in Formulae 2A and 2B:
   n is 1 or 2,
   each of R⁸, R⁹, R¹⁵ and R¹⁶ is independently selected from hydrogen, a halide, a C1-C4 alkyl group, or a C1-C4 haloalkyl group, and
   when n is 2, the multiple R¹⁵s or R¹⁶s may be the same or different from each other;
in Formula 1, L₁ is selected from moieties represented by Formulae 3A, 3B, and 3C: 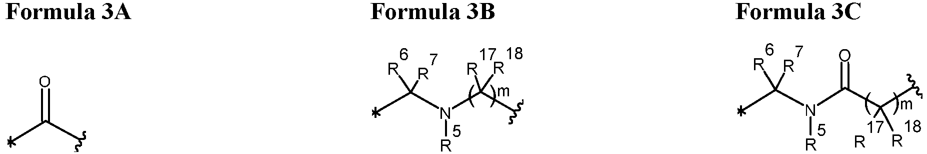 in Formulae 3A, 3B, and 3C:
   m is 0 or 1,
   each of R⁵, R⁶, R⁷, R¹⁷ and R¹⁸ is independently selected from hydrogen, a halide, a C1-C4 alkyl group, or a C1-C4 haloalkyl group;
in Formula 1, ring B is selected from moieties represented by Formulae 4A, 4B, 4C, 4D, and 4E: 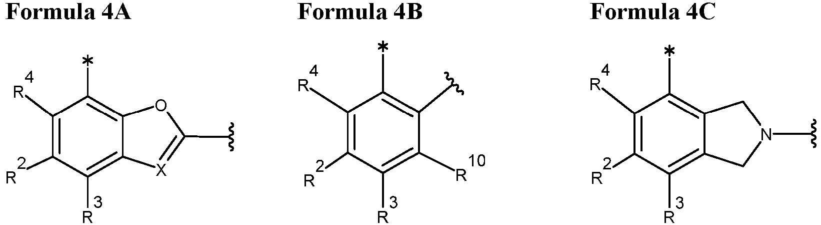 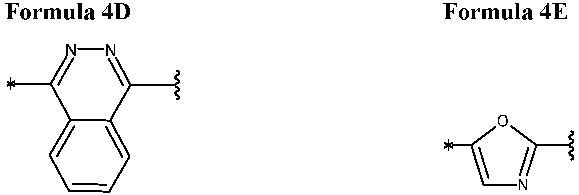 in Formulae 4A, 4B, 4C, 4D, and 4E:
   X is C, N or C-Y, where Y is hydrogen, a halide, or a C1-C4 alkoxy group;
   each of R², R³, R⁴, and R¹⁰ is independently selected from hydrogen, a halide, a C1-C4 alkyl group, or a C1-C4 haloalkyl group;
in Formula 1, L₂ is selected from moieties represented by Formulae 5A, 5B, and 5C: 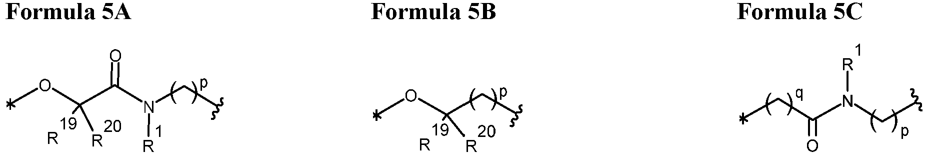 in Formulae 5A, 5B, and 5C:
   both p and q are independently 0 or 1; and
   each of R¹, R¹⁹, and R²⁰ is independently selected from hydrogen, a halide, a C1-C4 alkyl group, or a C1-C4 haloalkyl group;
in Formula 1, ring D is a substituted or unsubstituted aryl, heteroaryl, cyclic, or heterocylic ring or fused ring system.

### Example 2

The compound according to Example 1, wherein each of R⁸, R⁹, R¹⁵ and R¹⁶ are independently selected from hydrogen, a halide, a methyl group, a perfluorinated methyl group, or a partially fluorinated methyl group.

### Example 3

The compound according to Example 1 or 2, wherein each of R⁵, R⁶, R⁷, R¹⁷ and R¹⁸ is independently selected from hydrogen, a halide, a methyl group, a perfluorinated methyl group, or a partially fluorinated methyl group.

### Example 4

The compound according to any of Examples 1-3, wherein Y may be hydrogen, a halide, or methoxy.

### Example 5

The compound according to any of Examples 1-4, wherein each of R², R³, R⁴, and R¹⁰ is independently selected from hydrogen, a halide, a methyl group, a perfluorinated methyl group, or a partially fluorinated methyl group.

### Example 6

The compound according to any of Examples 1-5, wherein each of R¹, R¹⁹, and R²⁰ is independently selected from hydrogen, a halide, a methyl group, a perfluorinated methyl group, or a partially fluorinated methyl group.

### Example 7

The compound according to any of Examples 1-6, wherein ring D is selected from:

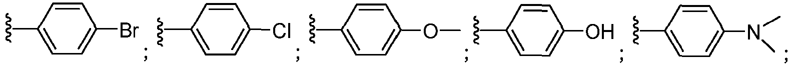

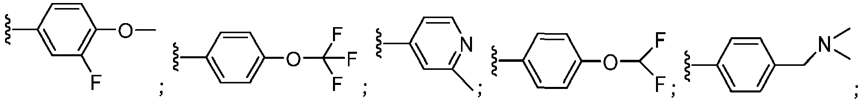

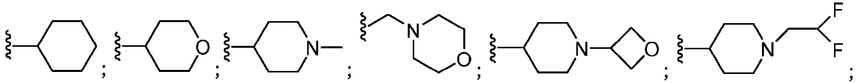

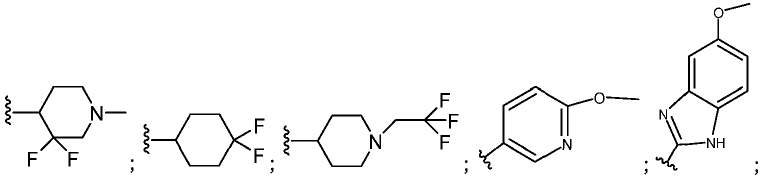

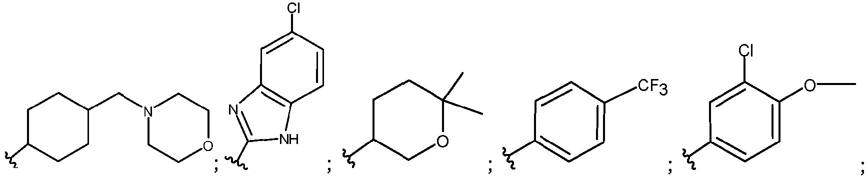

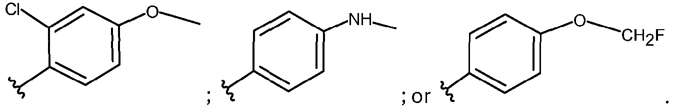

### Example 8

The compound according to any of Examples 1-7, wherein the compound is selected from Compounds 1-50:

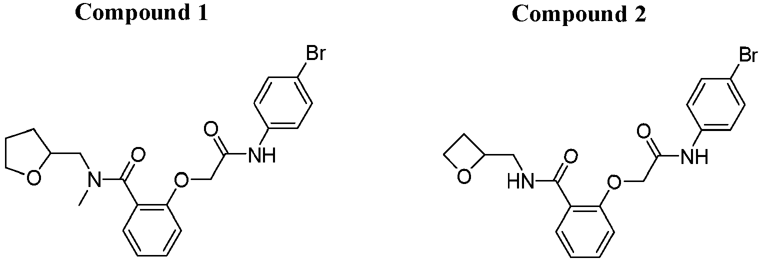

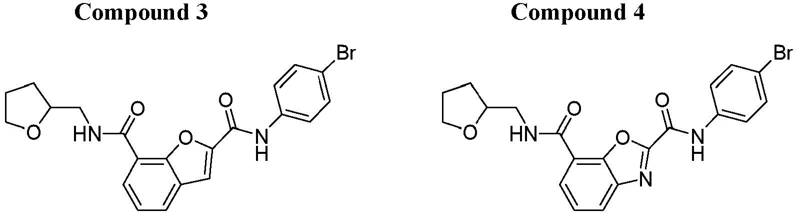

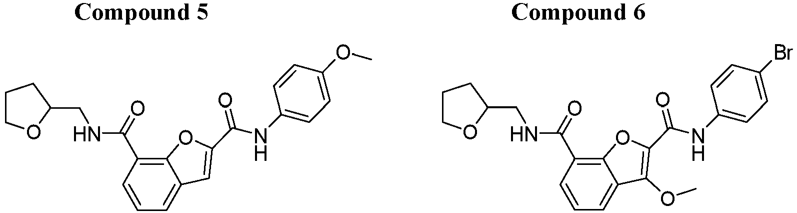

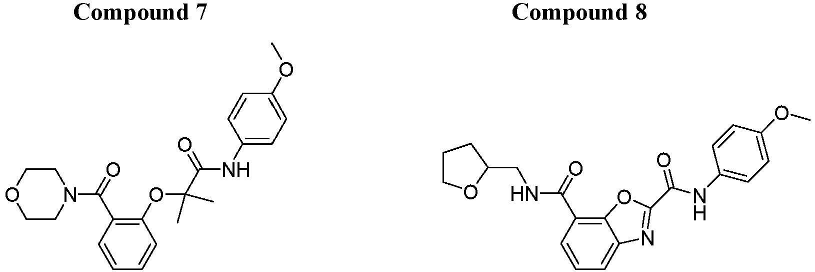

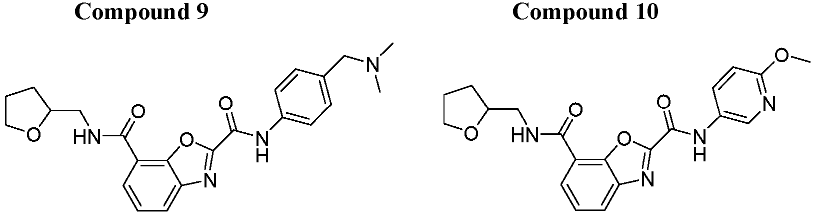

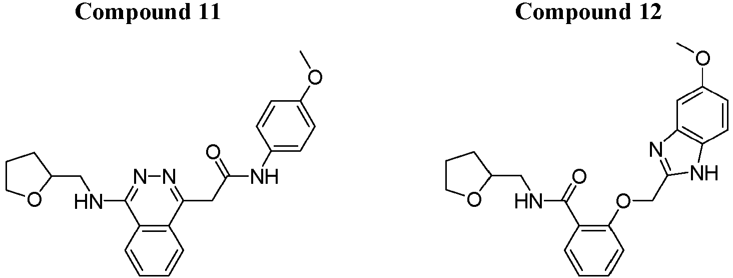

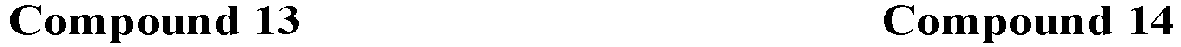

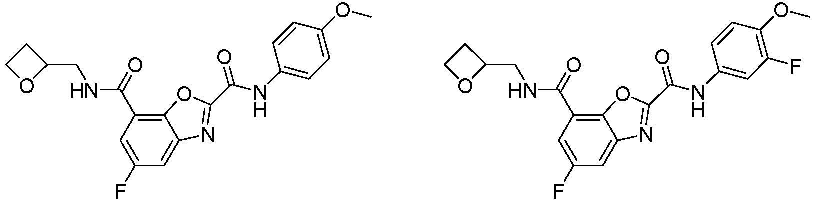

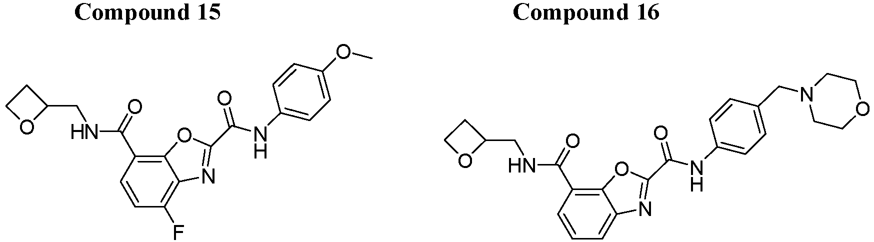

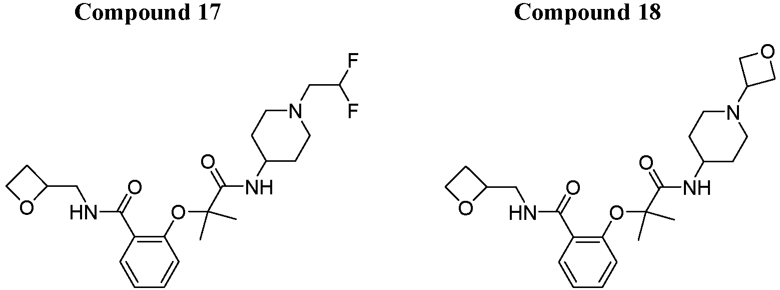

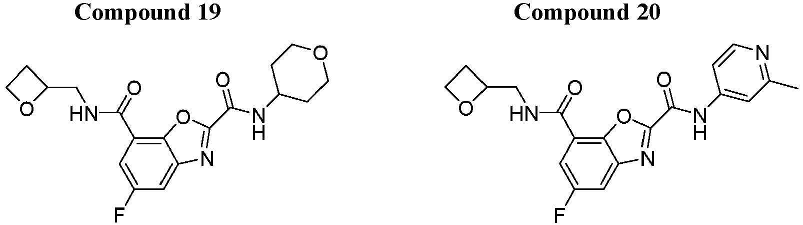

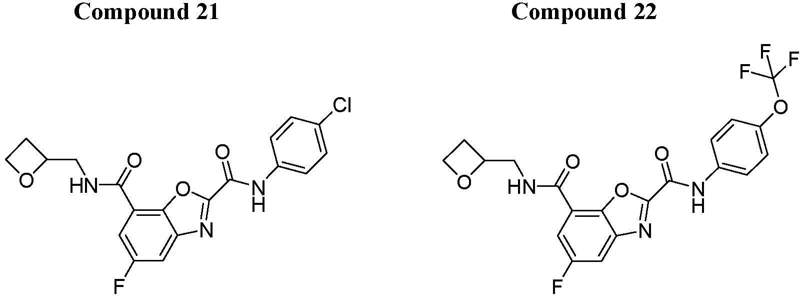

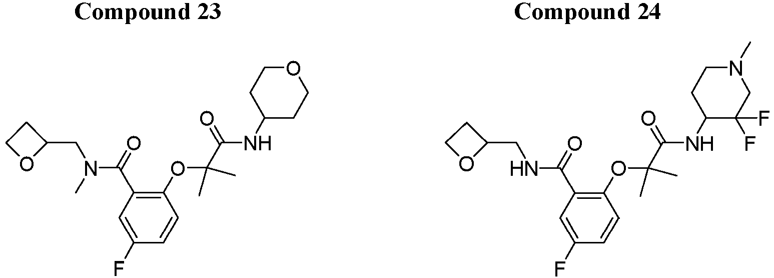

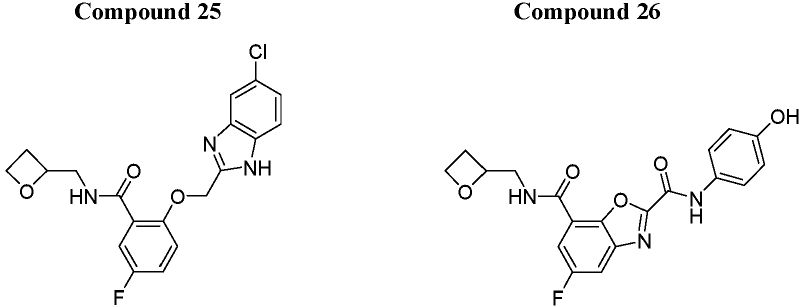

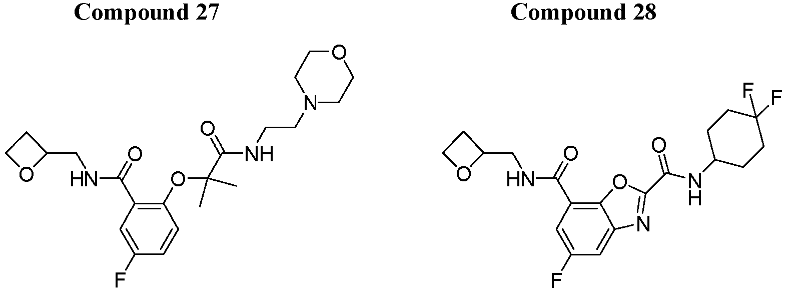

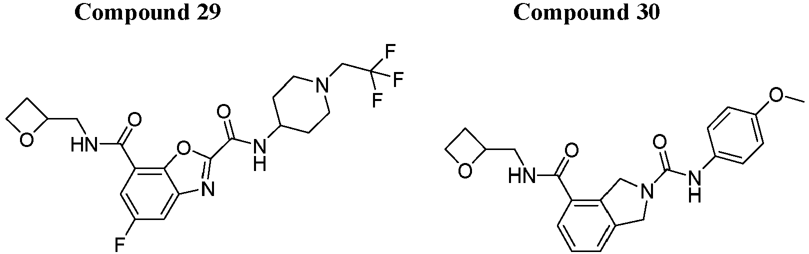

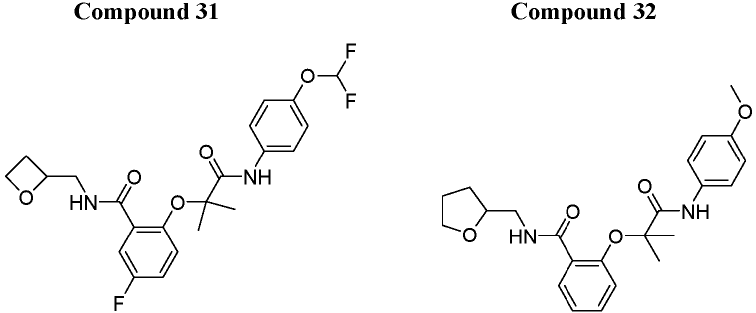

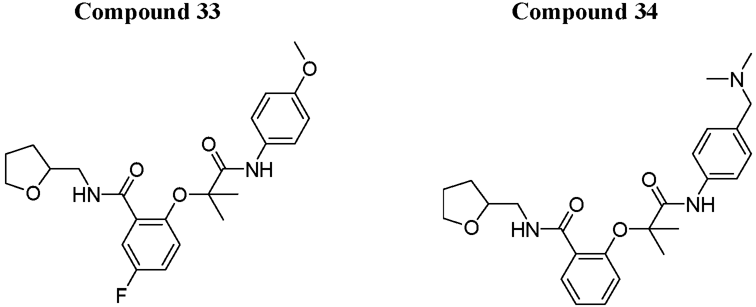

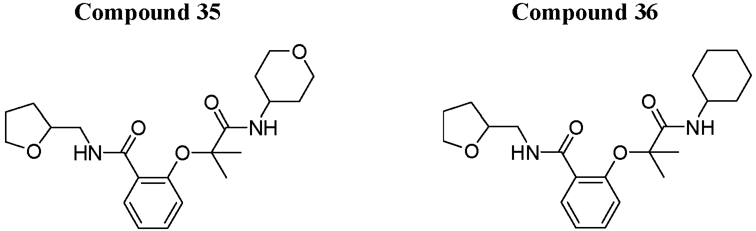

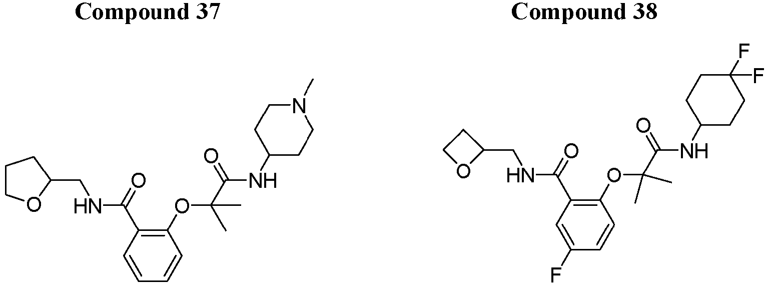

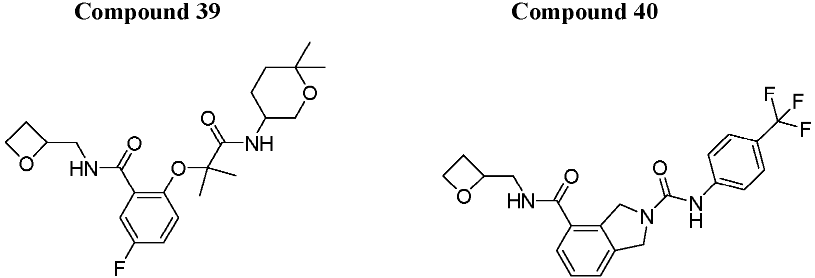

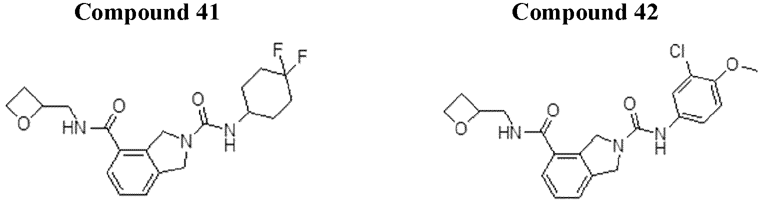

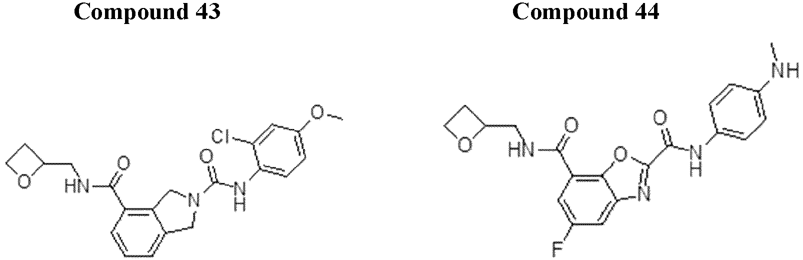

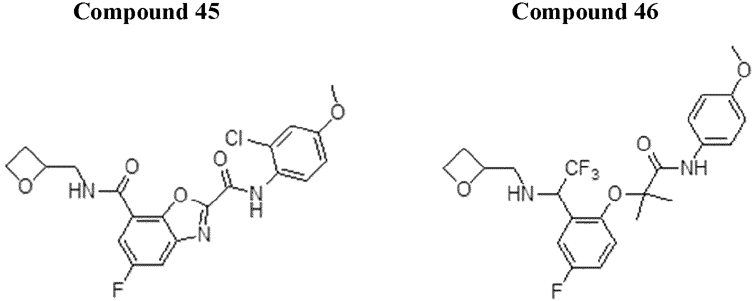

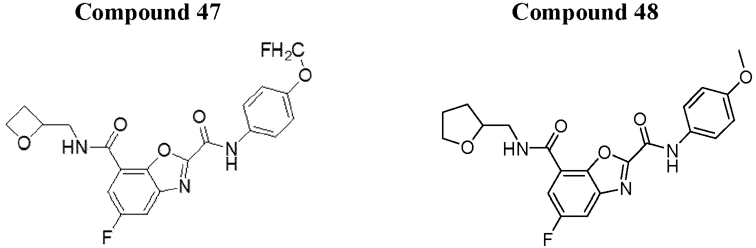

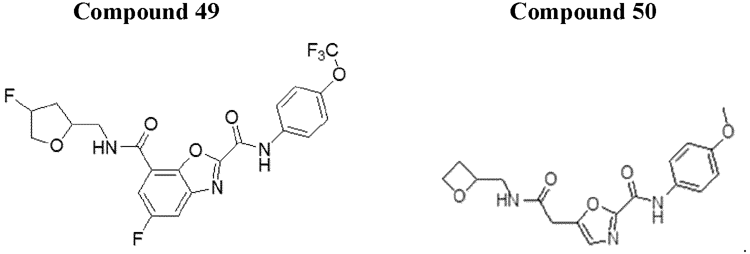

### Example 9

The compound according to any of Examples 1-7, wherein the compound is represented by one of Formula 6-9: 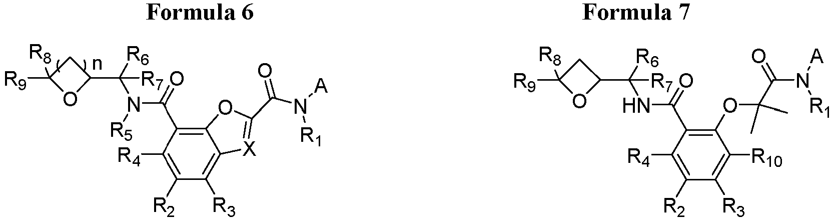 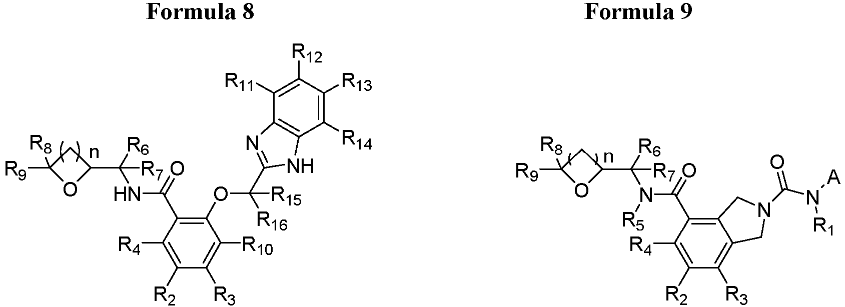 in Formulae 6 through 9:
n, X, and R¹ through R¹⁶ are as defined in Example 1, and
moiety A in Formulae 6, 7 and 9 is a substituted or unsubstituted aryl, heteroaryl, cyclic, or heterocylic ring or fused ring system bonded directly to Formulae 6, 7 or 9, or bonded to Formula 6, 7, or 9 via a linking -CH₂- group.

### Example 10

The compound according to Example 9, wherein moiety A is selected from: 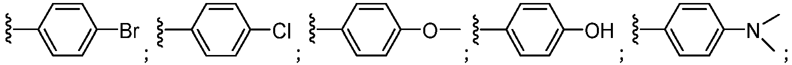 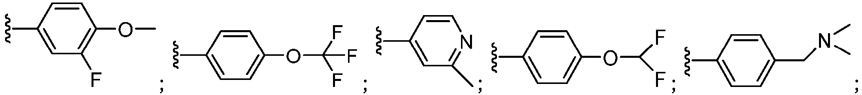 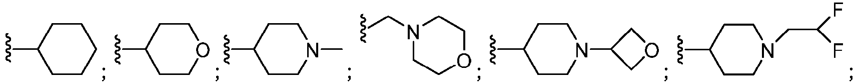 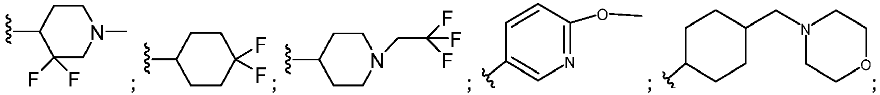 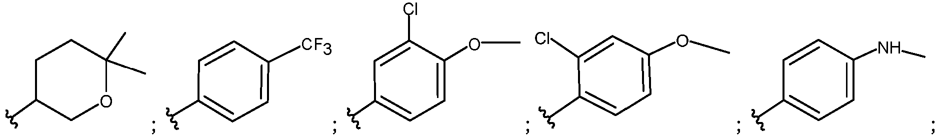 or

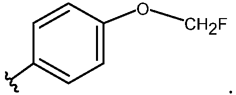

### Example 11

The compound according to any of Examples 9-10, wherein the compound represented by Formula 6 is selected from Compounds 3-6, 8-10, 13-16, 19-22, 26, 28, 29, 44, 45, and 47-49:

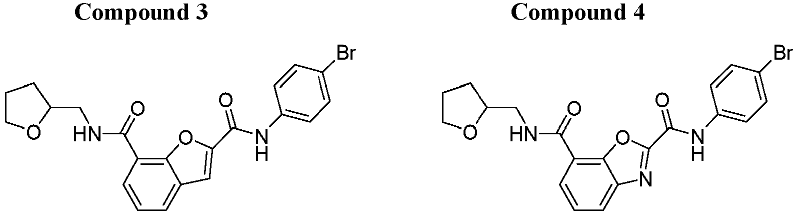

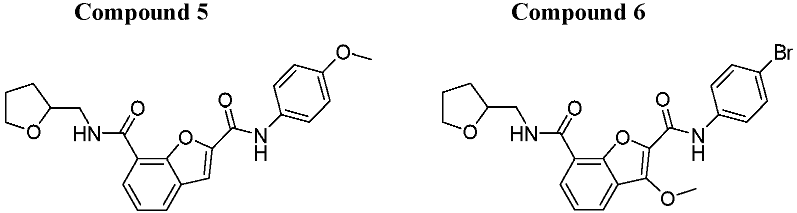

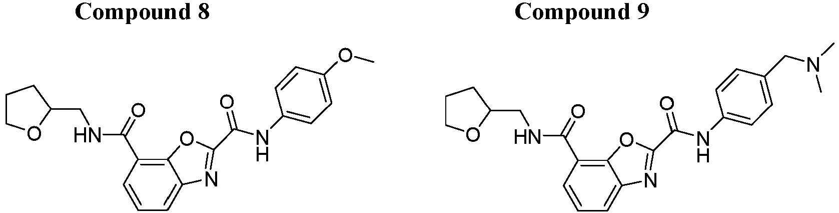

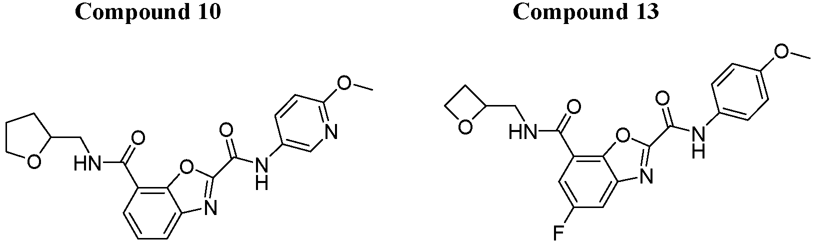

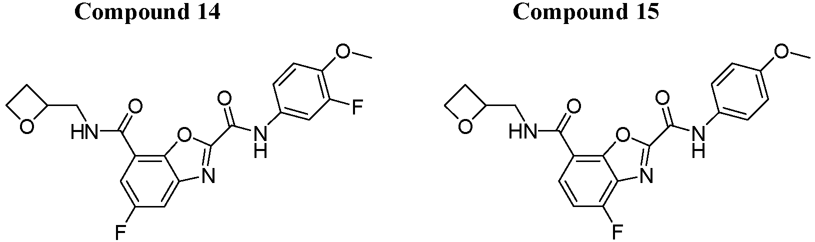

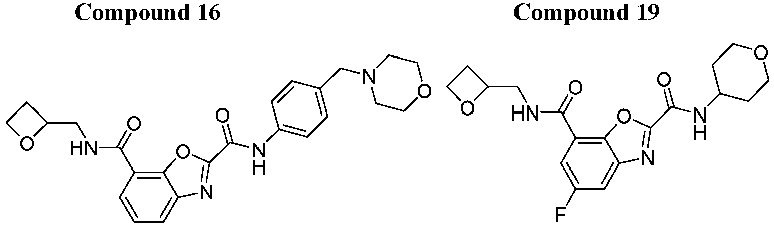

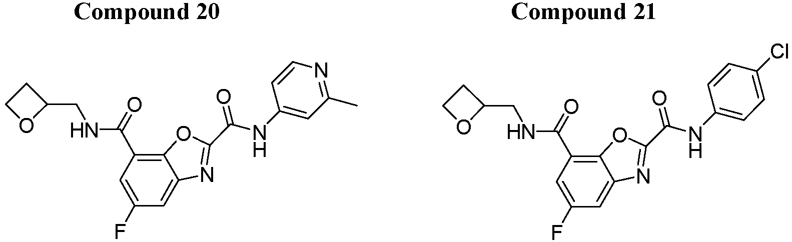

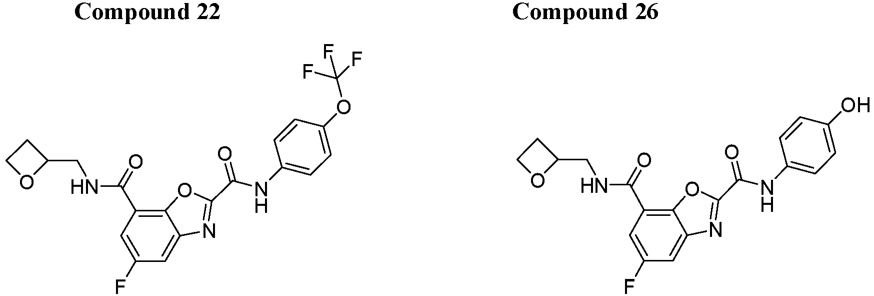

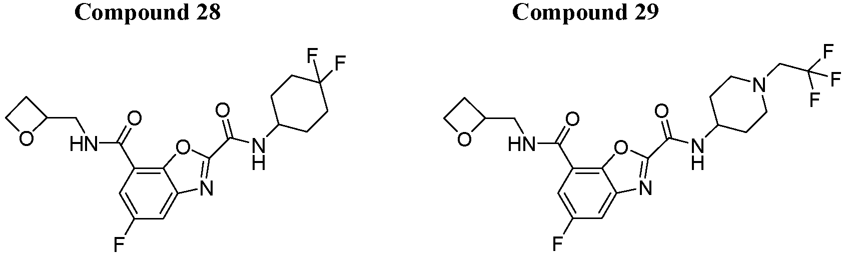

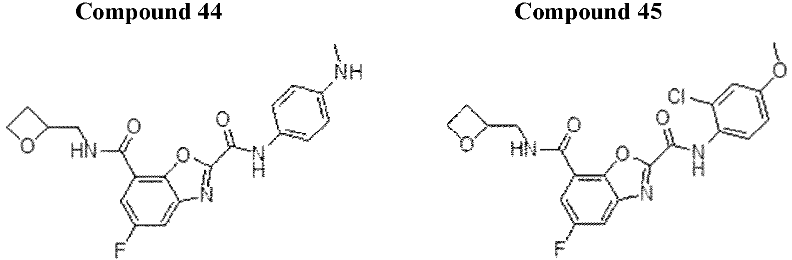

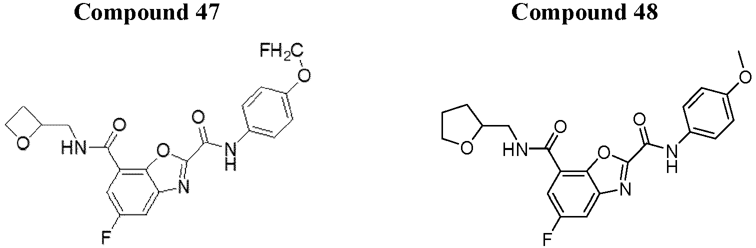

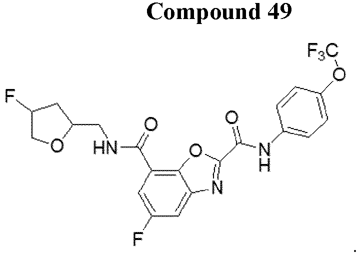

### Example 12

The compound according to any of Examples 9-11, wherein the compound represented by Formula 7 is selected from Compounds 1, 2, 7, 12, 17, 18, 23, 24, 27, 31-39, and 46:

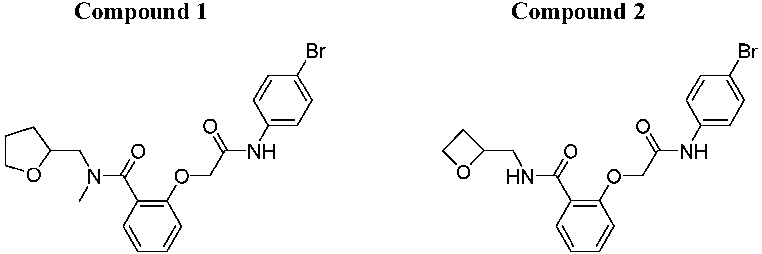

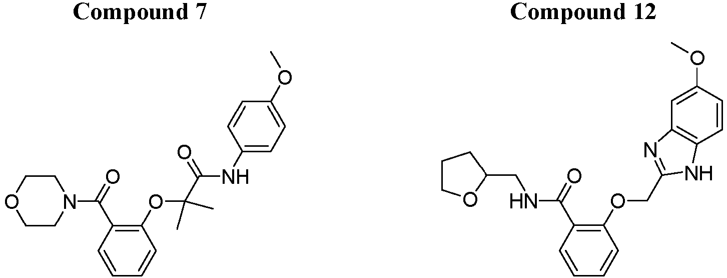

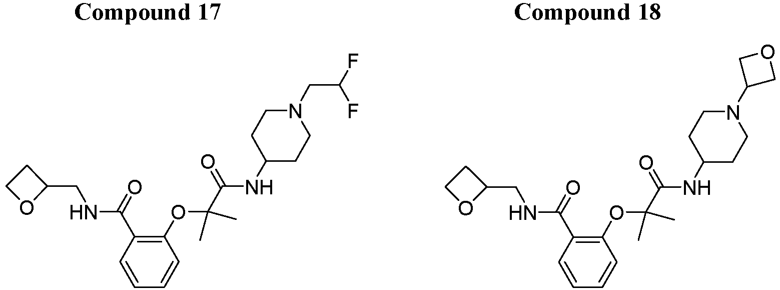

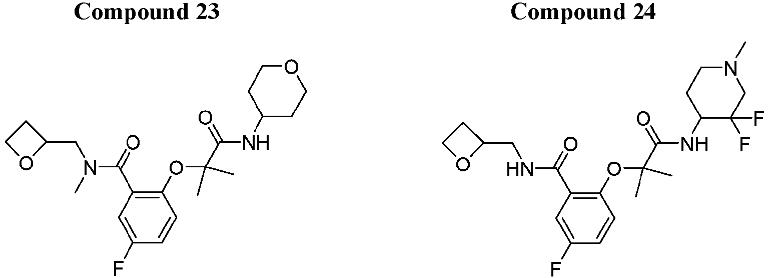

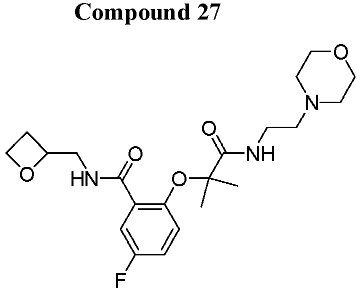

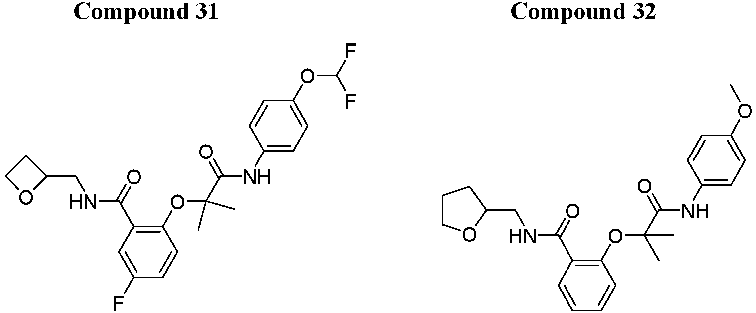

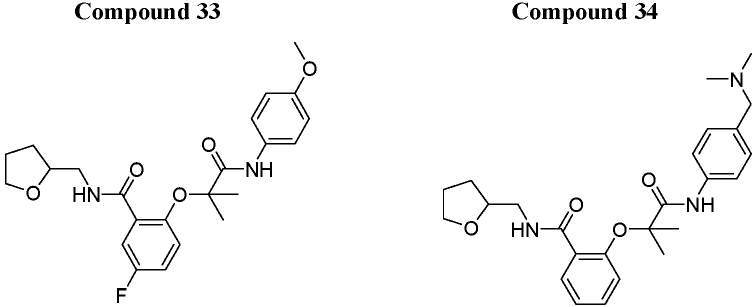

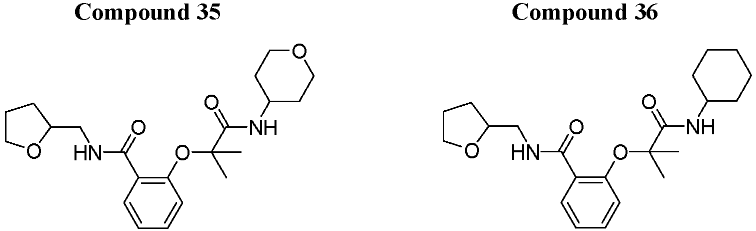

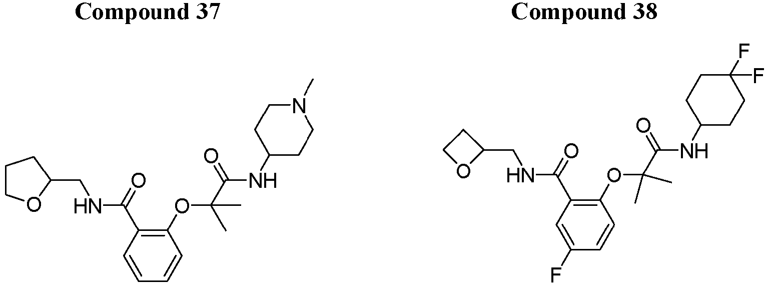

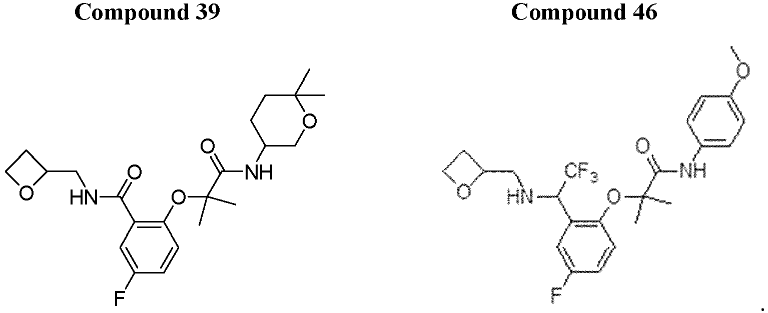

### Example 13

The compound according to any of Examples 9-12, wherein the compound represented by Formula 8 is Compound 25:

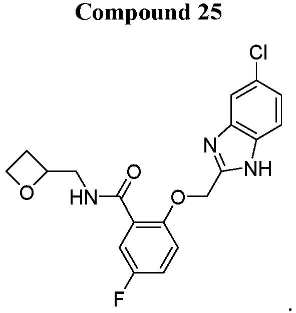

### Example 14

The compound according to any of Examples 9-13, wherein the compound represented by Formula 9 is selected from Compounds 30, and 40-43:

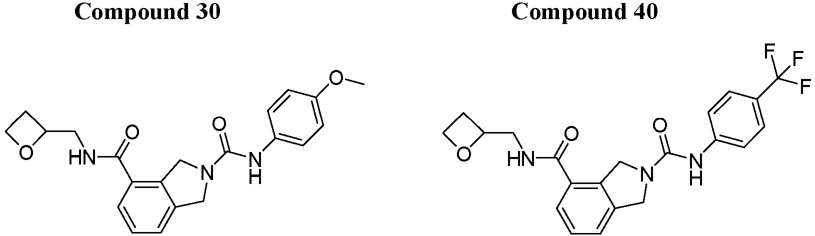

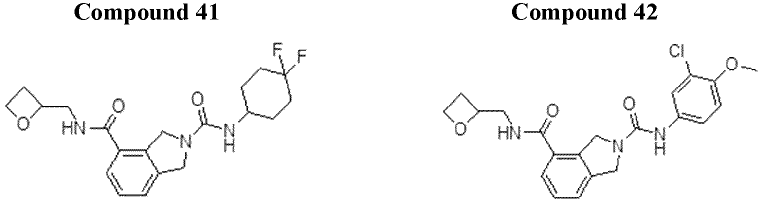

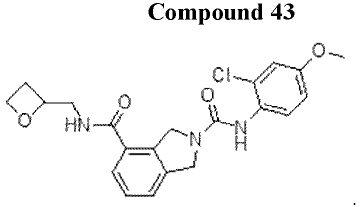

### Example 15

Use of the compound according to any of Examples 1-14 for the manufacture of a medicament for the treatment of Gaucher disease or Parkinson's disease.

### Example 16

A pharmaceutical composition, comprising: one or more of the compounds according to any of Examples 1-14 or pharmaceutically acceptable salts or prodrugs thereof; and a pharmaceutically acceptable carrier and/or excipient.

### Example 17

The pharmaceutical composition according to Example 16, further comprising one or more additional therapeutic agents for the treatment of Gaucher disease or Parkinson's disease.

### Example 18

The pharmaceutical composition according to any of Examples 16-17, formulated for oral, transdermal, inhalation, infusion, or parenteral administration.

### Example 19

A method of treating Gaucher disease or Parkinson's disease, comprising administering to a subject in need thereof, a therapeutically effective amount of one or more of the compounds according to any of Examples 1-14 or pharmaceutically acceptable salts or prodrugs thereof.

### Example 20

The method according to Example 19, wherein the one or more compounds or pharmaceutically acceptable salts or prodrugs thereof are administered orally in the form of a tablet, a capsule, or a liquid formulation.

### Example 21

The method according to any of Examples 19-20, wherein the one or more compounds or pharmaceutically acceptable salts or prodrugs thereof are administered parenterally via intravenous, intramuscular, or subcutaneous injection.

### Example 22

The method according to any of Examples 19-20, wherein the one or more compounds or pharmaceutically acceptable salts or prodrugs thereof are administered topically as a transdermal patch or cream.

### Example 23

The method according to any of Examples 19-20, wherein the one or more compounds or pharmaceutically acceptable salts or prodrugs thereof are administered via inhalation as an aerosolized formulation.

### Example 24

The method according to any of Examples 19-23, wherein the one or more compounds or pharmaceutically acceptable salts or prodrugs thereof are administered in combination with one or more additional therapeutic agents for the treatment of Gaucher disease or Parkinson's disease.

### Example 25

A method of treating Gaucher disease or Parkinson's disease, comprising administering to a subject in need thereof, the pharmaceutical composition according to any of Examples 16-18.

### Example 26

The method according to Example 25, wherein the pharmaceutical composition is administered orally in the form of a tablet, a capsule, or a liquid formulation.

### Example 27

The method according to any of Examples 25-26, wherein the pharmaceutical composition is administered parenterally via intravenous, intramuscular, or subcutaneous injection.

### Example 28

The method according to any of Examples 25-26, wherein the pharmaceutical composition is administered topically as a transdermal patch or cream.

### Example 29

The method according to any of Examples 25-26, wherein the pharmaceutical composition is administered via inhalation as an aerosolized formulation.

### Example 30

The method according to any of Examples 25-29, wherein the pharmaceutical composition is administered in combination with one or more additional therapeutic agents for the treatment of Gaucher disease or Parkinson's disease.

## Claims

1. A compound represented by Formula 1: 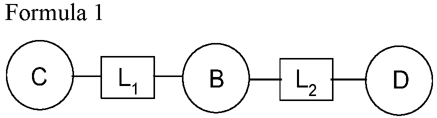
in Formula 1, ring C is selected from moieties represented by Formulae 2A and 2B: 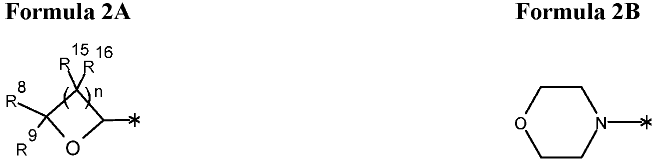 in Formulae 2A and 2B:
n is 1 or 2,
each of R⁸, R⁹, R¹⁵ and R¹⁶ is independently selected from hydrogen, a halide, a C1-C4 alkyl group, or a C1-C4 haloalkyl group, and
when n is 2, the multiple R¹⁵s or R¹⁶s may be the same or different from each other;
in Formula 1, Li is selected from moieties represented by Formulae 3A, 3B, and 3C: 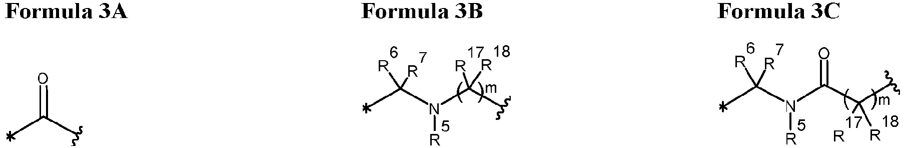 in Formulae 3A, 3B, and 3C:
m is 0 or 1,
each of R⁵, R⁶, R⁷, R¹⁷ and R¹⁸ is independently selected from hydrogen, a halide, a C1-C4 alkyl group, or a C1-C4 haloalkyl group;
in Formula 1, ring B is selected from moieties represented by Formulae 4A, 4B, 4C, 4D, and 4E: 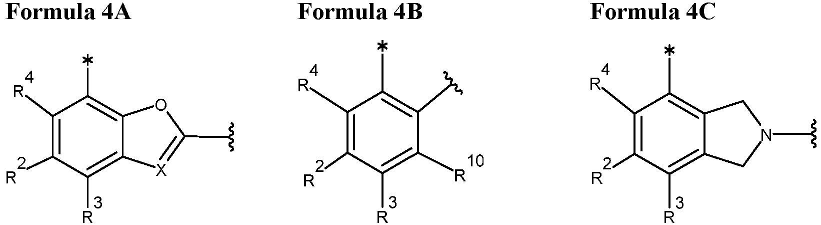 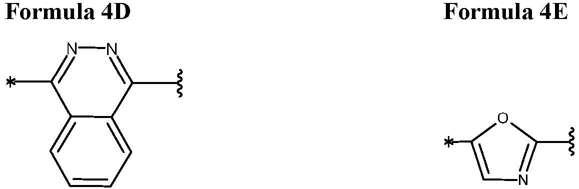
in Formulae 4A, 4B, 4C, 4D, and 4E:
X is C, N or C-Y, where Y is hydrogen, a halide, or a C1-C4 alkoxy group;
each of R², R³, R⁴, and R¹⁰ is independently selected from hydrogen, a halide, a C1-C4 alkyl group, or a C1-C4 haloalkyl group;
in Formula 1, L₂ is selected from moieties represented by Formulae 5A, 5B, and 5C: 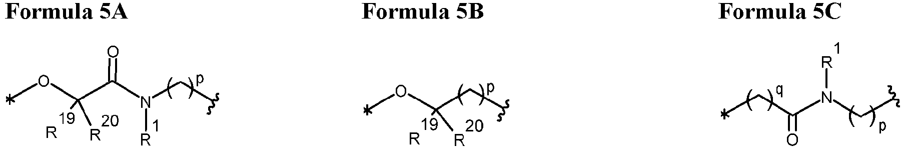
in Formulae 5A, 5B, and 5C:
both p and q are independently 0 or 1; and
each of R¹, R¹⁹, and R²⁰ is independently selected from hydrogen, a halide, a C1-C4 alkyl group, or a C1-C4 haloalkyl group;
in Formula 1, ring D is a substituted or unsubstituted aryl, heteroaryl, cyclic, or heterocylic ring or fused ring system.

2. The compound according to claim 1, wherein:
each of R⁸, R⁹, R¹⁵ and R¹⁶ are independently selected from hydrogen, a halide, a methyl group, a perfluorinated methyl group, or a partially fluorinated methyl group; and/or
each of R⁵, R⁶, R⁷, R¹⁷ and R¹⁸ is independently selected from hydrogen, a halide, a methyl group, a perfluorinated methyl group, or a partially fluorinated methyl group; and/or
Y may be hydrogen, a halide, or methoxy; and/or
each of R², R³, R⁴, and R¹⁰ is independently selected from hydrogen, a halide, a methyl group, a perfluorinated methyl group, or a partially fluorinated methyl group: and/or
each of R¹, R¹⁹, and R²⁰ is independently selected from hydrogen, a halide, a methyl group, a perfluorinated methyl group, or a partially fluorinated methyl group.

3. The compound according to claim 1 or 2, wherein ring D is selected from:

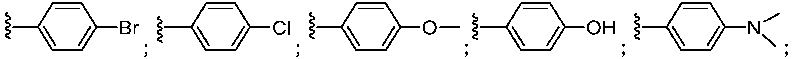

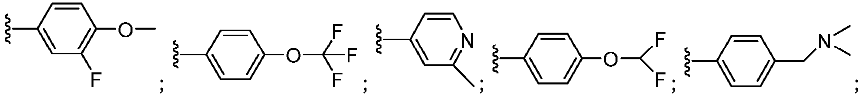

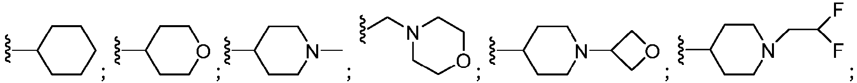

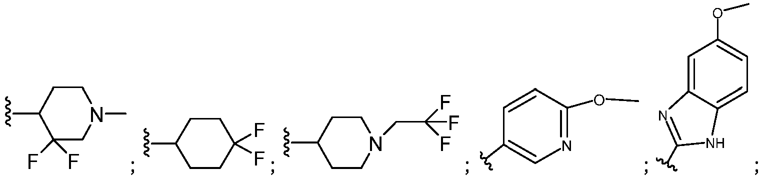

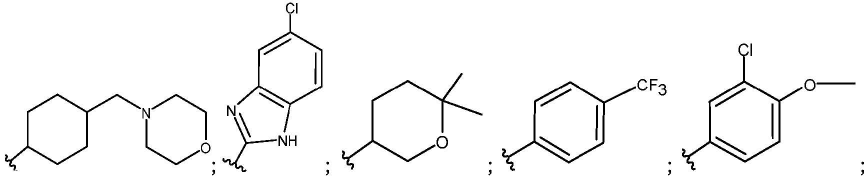

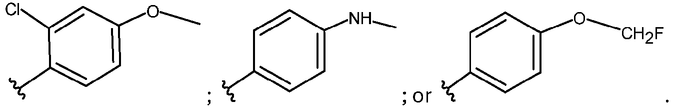

4. The compound according to any of claims 1-3, wherein the compound is selected from Compounds 1-50:

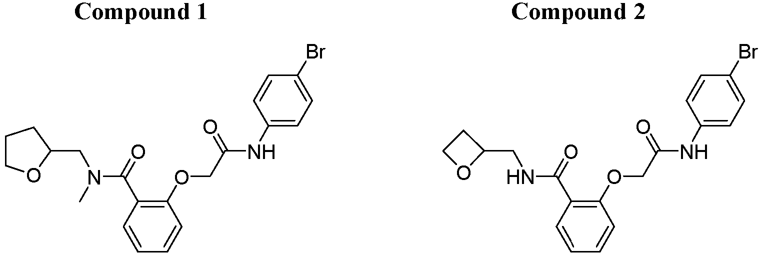

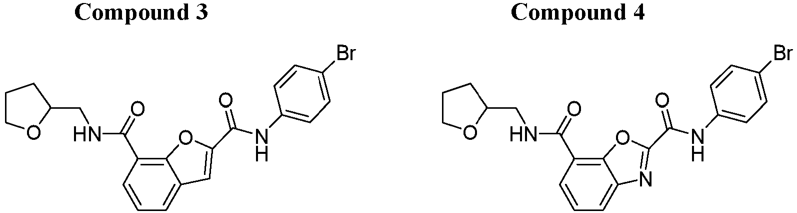

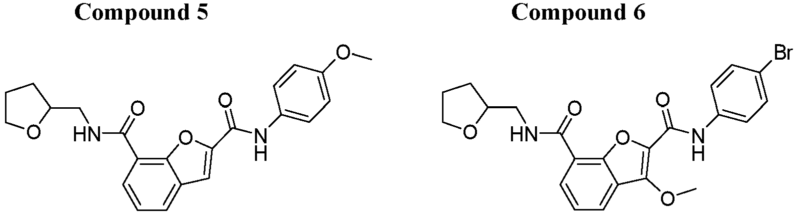

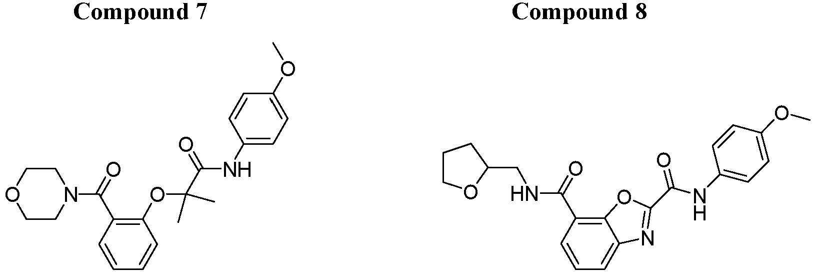

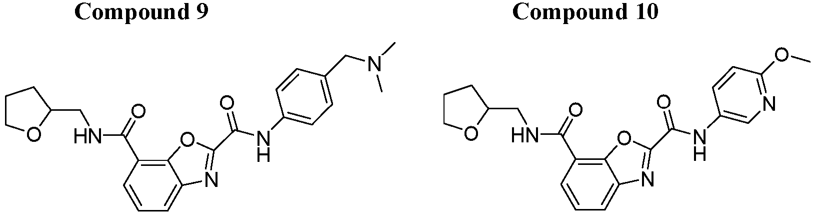

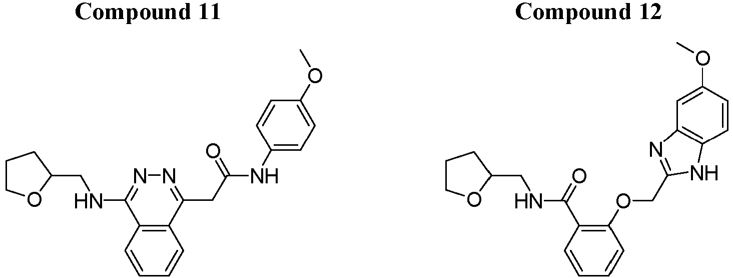

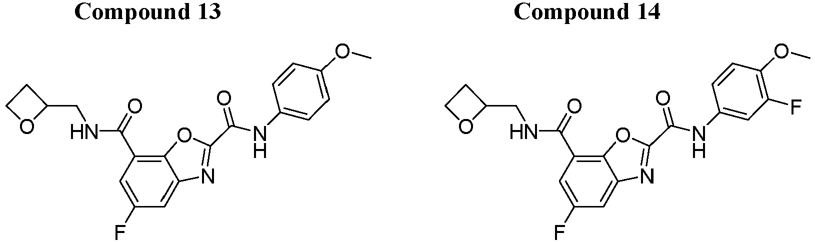

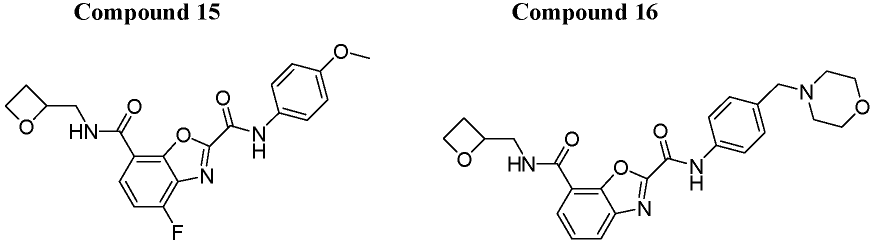

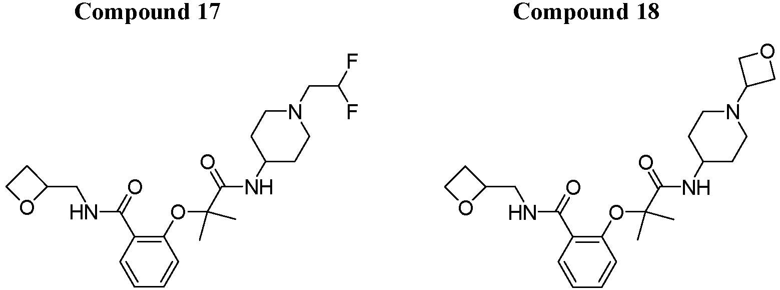

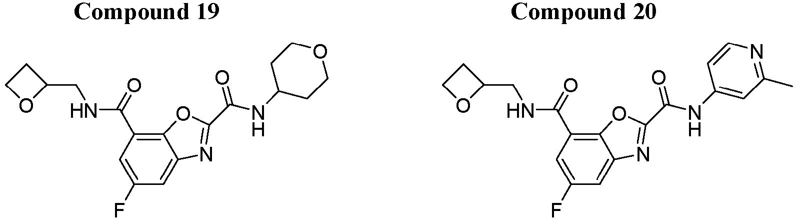

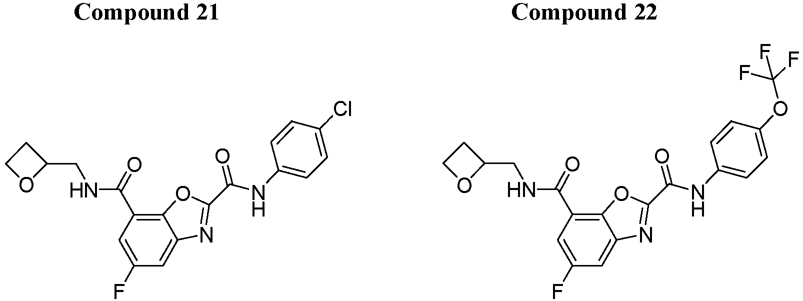

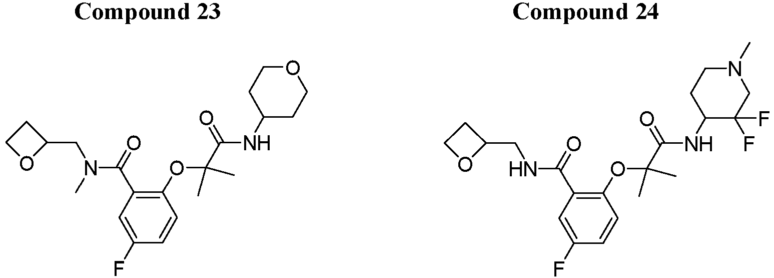

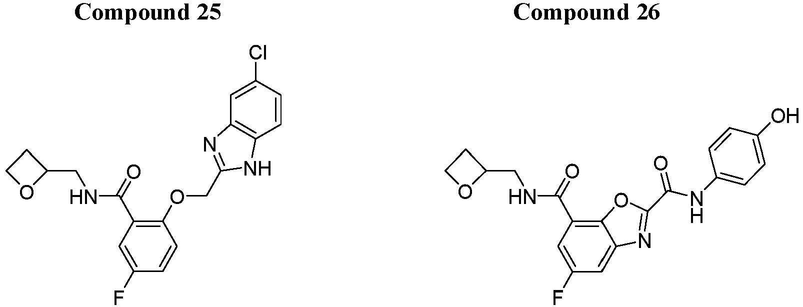

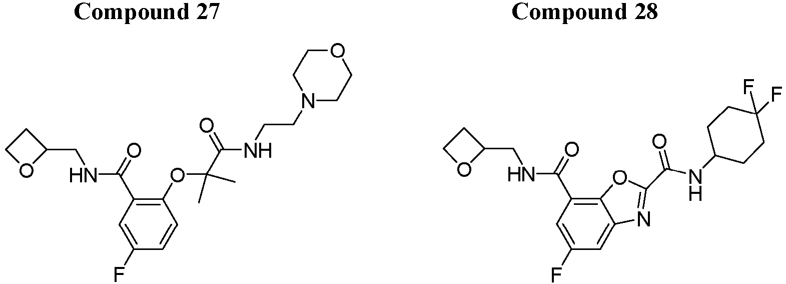

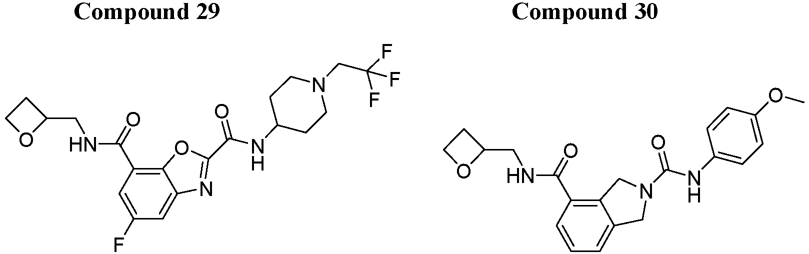

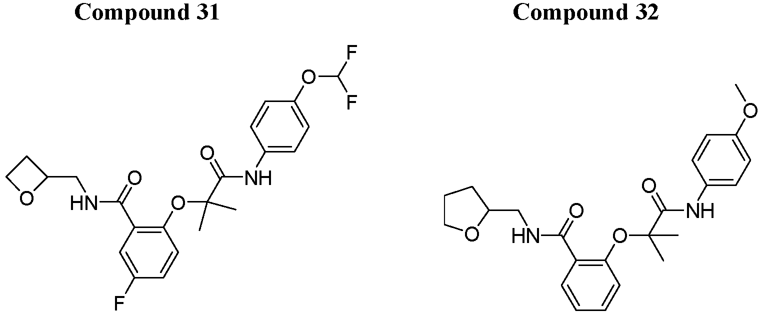

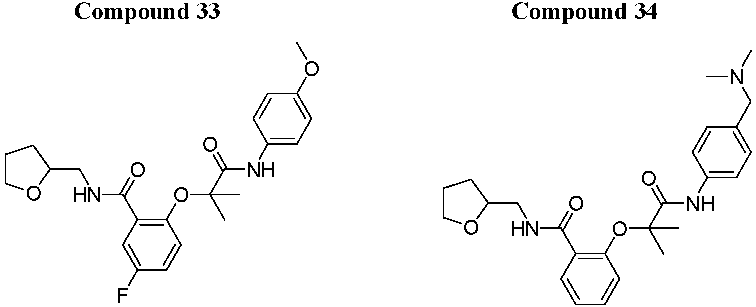

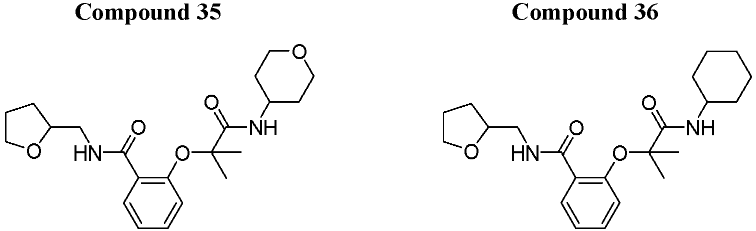

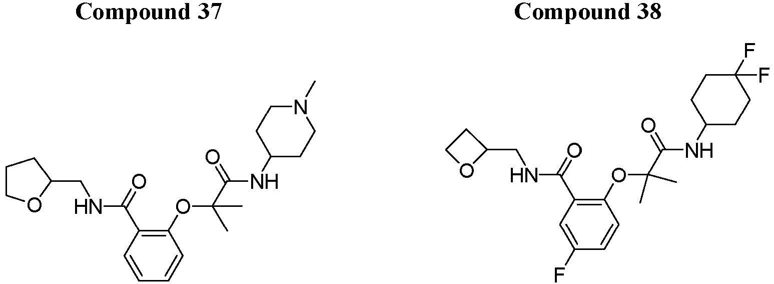

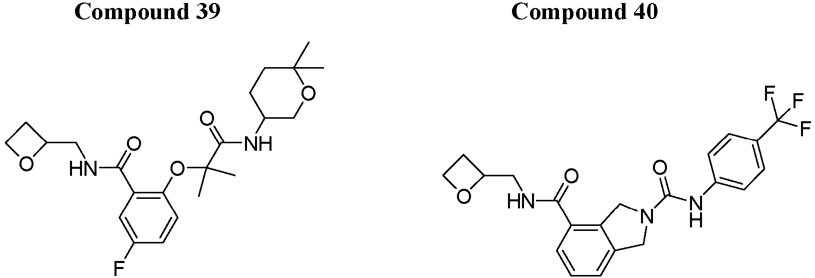

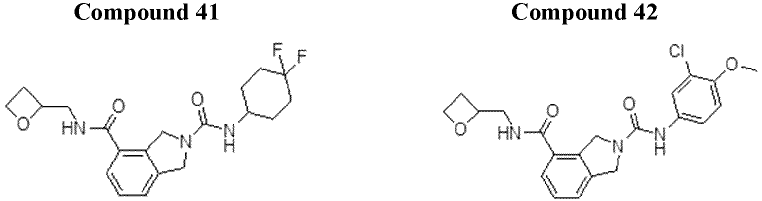

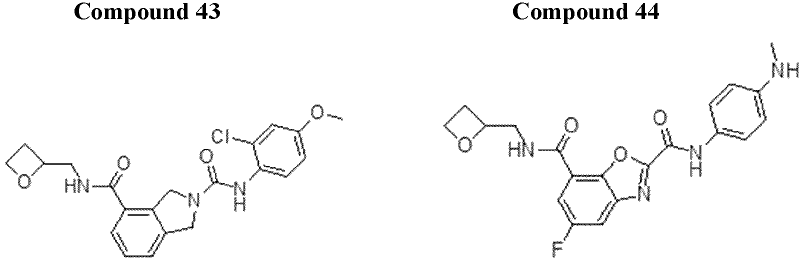

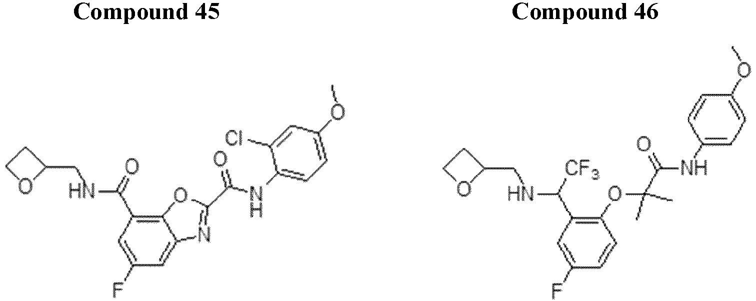

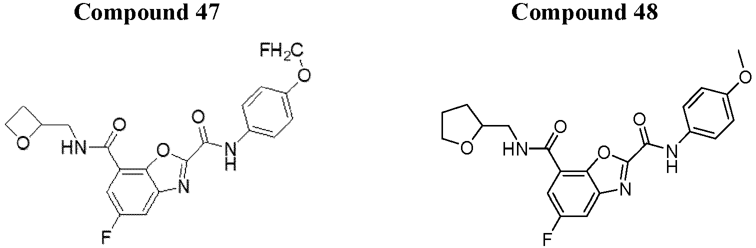

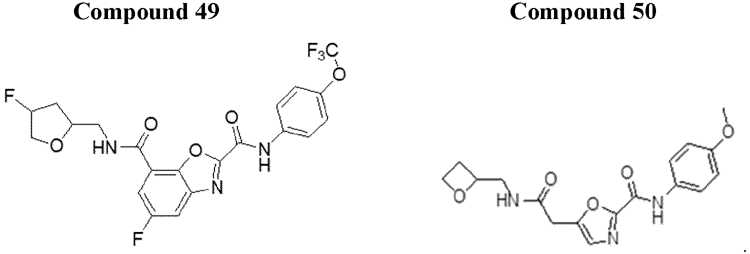

5. The compound according to any of claims 1-3, wherein the compound is represented by one of Formula 6-9: 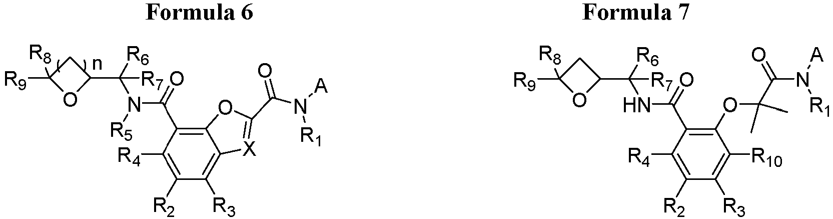 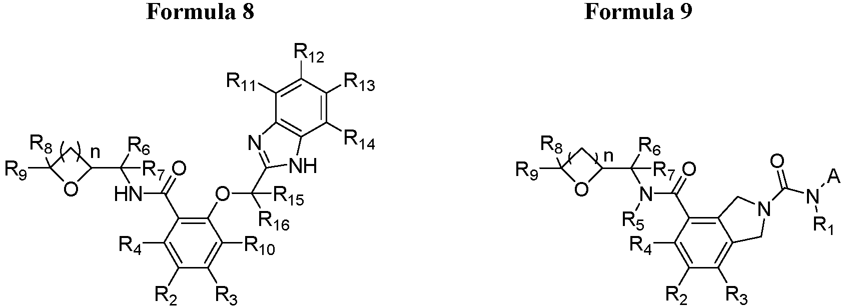 in Formulae 6 through 9:
n, X, and R¹ through R¹⁶ are as defined in claim 1, and
moiety A in Formulae 6, 7 and 9 is a substituted or unsubstituted aryl, heteroaryl, cyclic, or heterocylic ring or fused ring system bonded directly to Formulae 6, 7 or 9, or bonded to Formula 6, 7, or 9 via a linking -CH₂- group.

6. The compound according to claim 5, wherein moiety A is selected from: 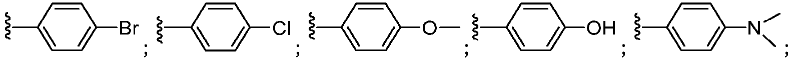 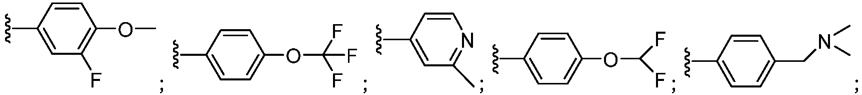 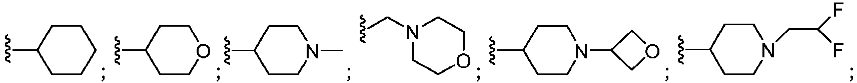 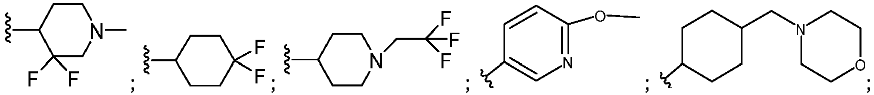 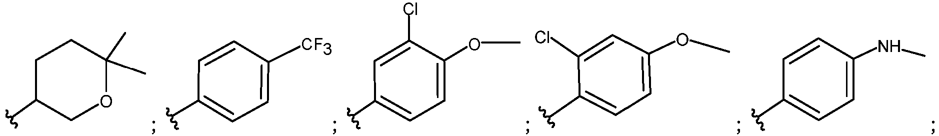 or

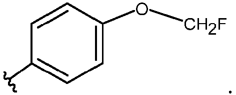

7. The compound according to any of claims 5-6, wherein:
the compound represented by Formula 6 is selected from Compounds 3-6, 8-10, 13-16, 19-22, 26, 28, 29, 44, 45, and 47-49; and/or
the compound represented by Formula 7 is selected from Compounds 1, 2, 7, 12, 17, 18, 23, 24, 27, 31-39, and 46; and/or
the compound represented by Formula 8 is Compound 25; and/or
the compound represented by Formula 9 is selected from Compounds 30, and 40-43:

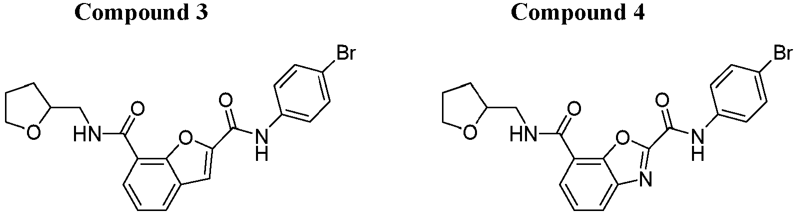

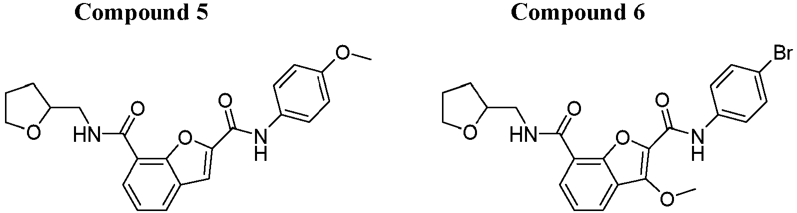

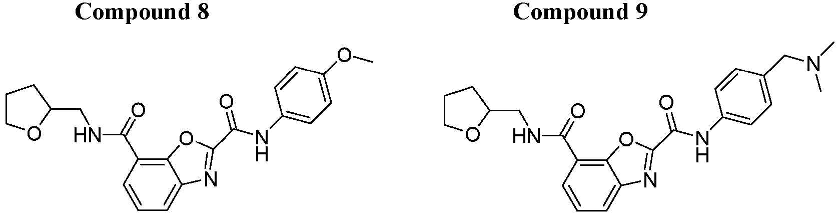

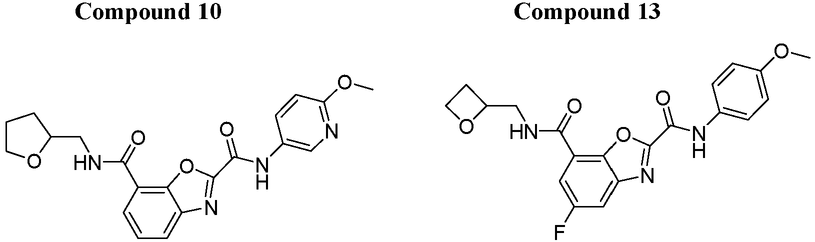

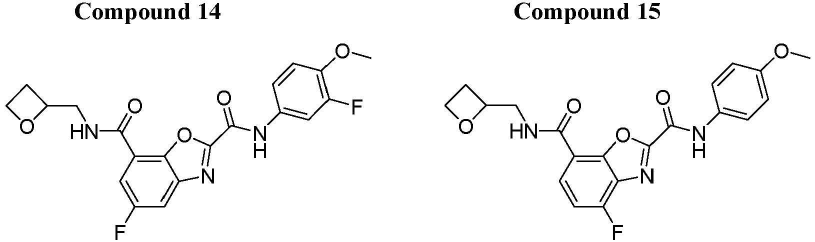

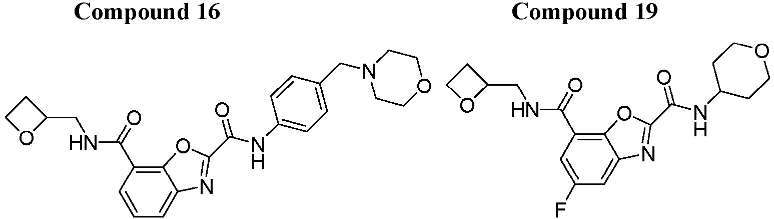

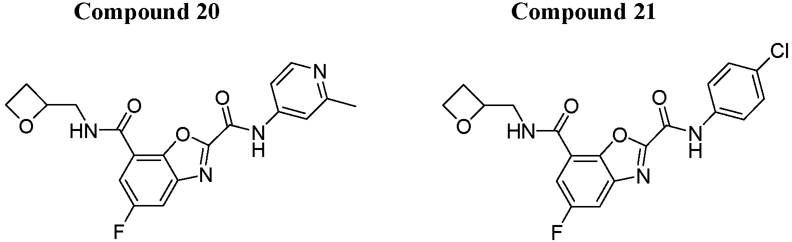

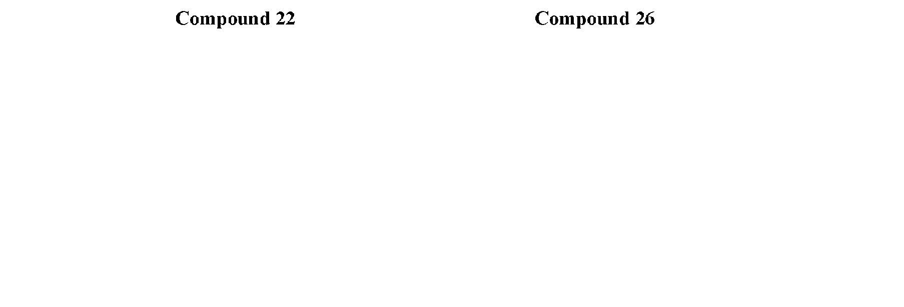

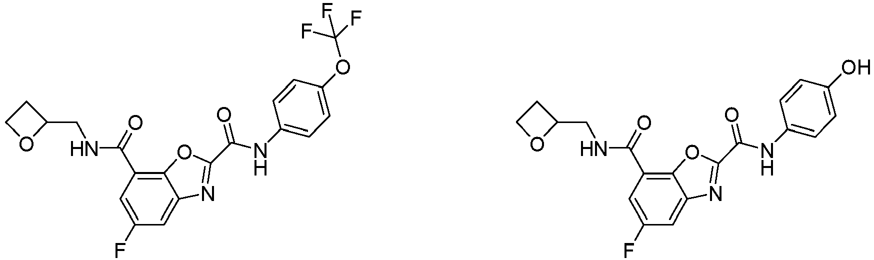

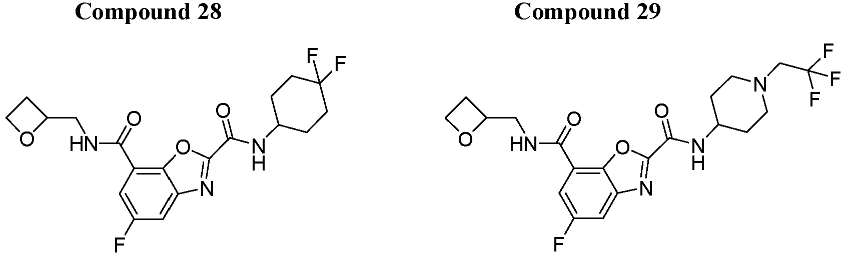

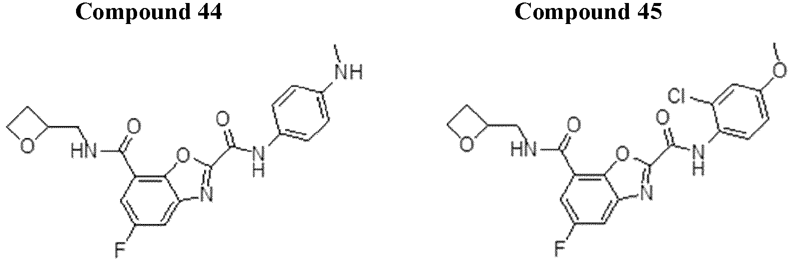

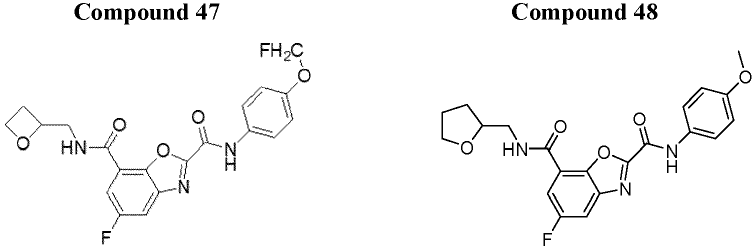

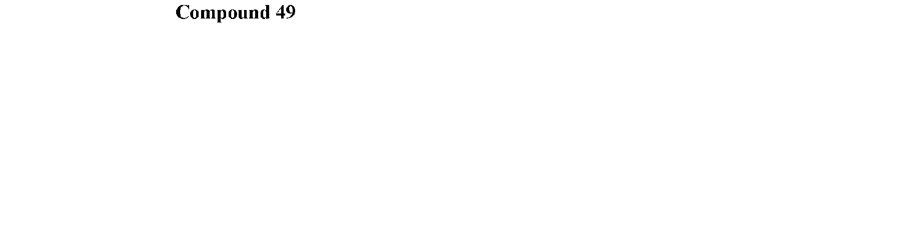

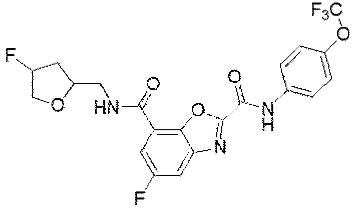

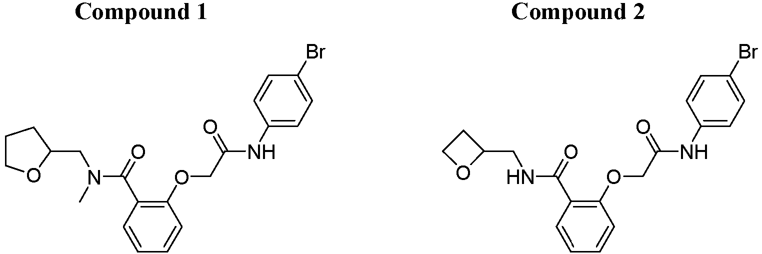

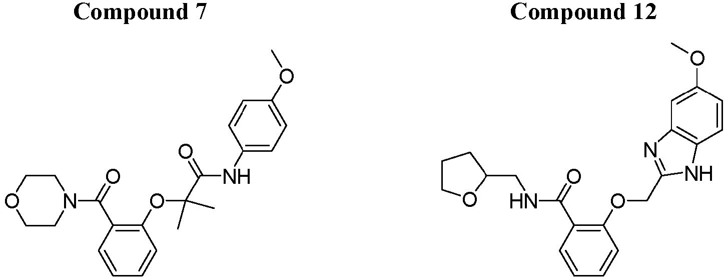

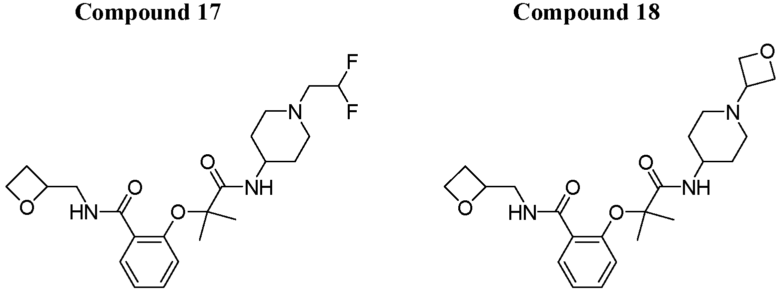

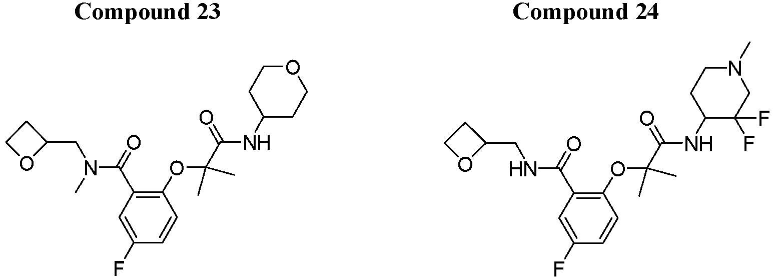

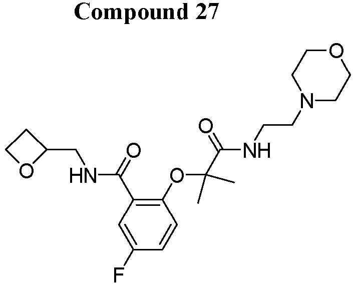

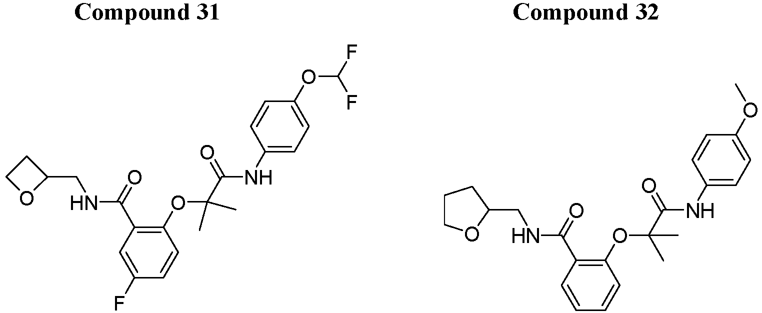

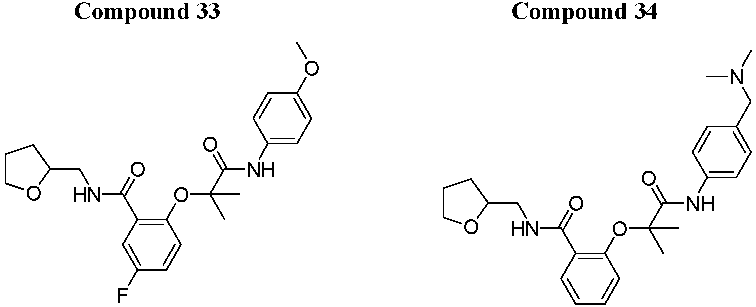

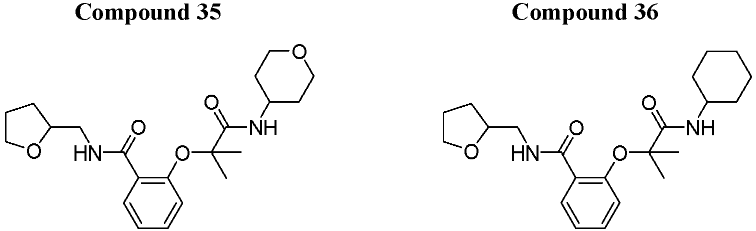

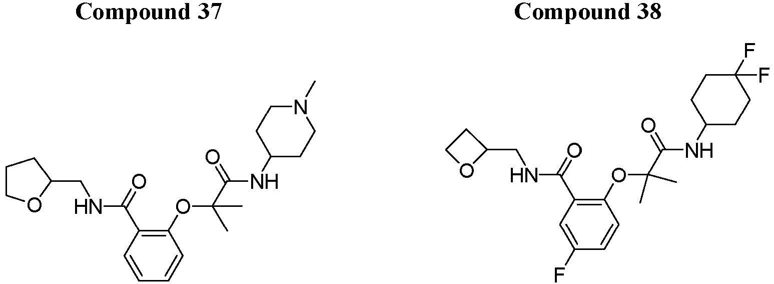

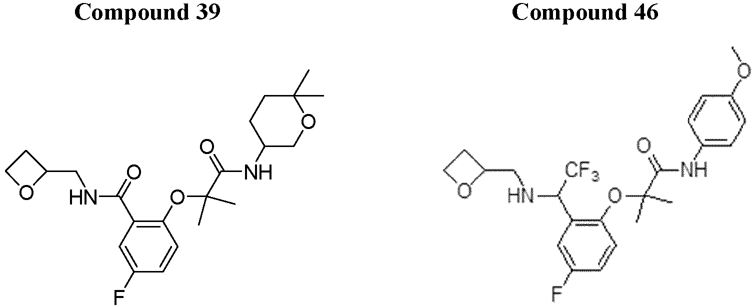

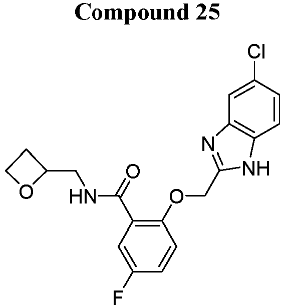

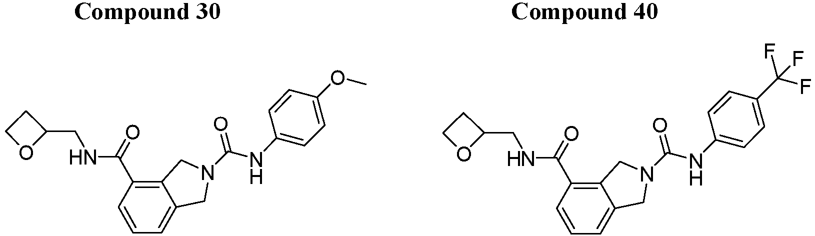

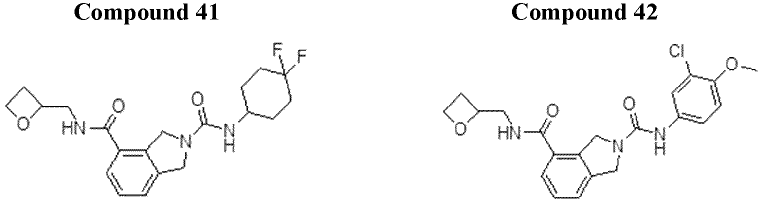

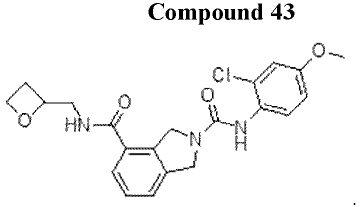

8. A compound according to any of claims 1-7 for use in the treatment of Gaucher disease or Parkinson's disease.

9. A pharmaceutical composition, comprising:
one or more of the compounds according to any of claims 1-7 or pharmaceutically acceptable salts or prodrugs thereof; and
a pharmaceutically acceptable carrier and/or excipient.

10. The pharmaceutical composition according to claim 9, further comprising one or more additional therapeutic agents for the treatment of Gaucher disease or Parkinson's disease.

11. The pharmaceutical composition according to any of claims 9-10, formulated for oral, transdermal, inhalation, infusion, or parenteral administration.

12. The pharmaceutical composition according to any of claims 9-11, formulated for intravenous, intramuscular, or subcutaneous injection.

13. The pharmaceutical composition according to any of claims 9-11, formulated for topical administration as a transdermal patch or cream.

14. The pharmaceutical composition according to any of claims 9-11, formulated for administration via inhalation as an aerosolized formulation.

15. The pharmaceutical composition according to any of claims 9-11, formulated for oral administration in the form of a tablet, a capsule, or a liquid formulation.
